# EUROPEAN PATENT SPECIFICATION

(11) **EP 2 073 899 B1**
(45) Date of publication and mention of the grant of the patent: **02.03.2016**
(21) Application number: 07815348.3
(22) Date of filing: 11.10.2007
(51) Int. Cl.: A61P 35/00, A61K 31/404, A61K 31/7068, A61K 31/282, A61K 31/553, A61K 31/337, A61K 31/704

(54) **THE USE OF A DNA DAMAGING AGENT AND A LIGAND FOR THE TREATMENT OF CANCER**
VERWENDUNG EINES DNA-SCHÄDIGENDEN MITTELS UND EINES LIGANDEN ZUR BEHANDLUNG VON KREBS
UTILISATION D'UN AGENT ENDOMMAGEANT L'ADN ET D'UN LIGAND POUR LE TRAITEMENT DE CANCER

(30) Priority: 11.10.2006 US 851213 P
(43) Date of publication of application: 01.07.2009
(73) Proprietor: MEDVET SCIENCE PTY. LTD., Stepney, South Australia 5069 (AU)
(72) Inventor: BROWN, Michael Paul, St. Georges, South Australia 5064 (AU); AL-EJEH, Fares, Adelaide, South Australia 5000 (AU); DARBY, Jocelyn Margaret, Blackwood, South Australia 5051 (AU)
(74) Representative: Dzieglewska, Hanna Eva
(86) International application number: PCT/AU2007/001543
(87) International publication number: WO 2008/043148

(56) References cited:
- EP-A1- 1 354 952
- EP-A1- 1 354 953
- EP-A2- 0 282 057
- WO-A1-2005/051424
- WO-A2-2005/039648
- CHEN SHAOLIANG ET AL: "Pivotal study of iodine-131-labeled chimeric tumor necrosis treatment radioimmunotherapy in patients with advanced lung cancer." JOURNAL OF CLINICAL ONCOLOGY : OFFICIAL JOURNAL OF THE AMERICAN SOCIETY OF CLINICAL ONCOLOGY 1 MAR 2005 LNKD- PUBMED:15735129, vol. 23, no. 7, 1 March 2005 (2005-03-01), pages 1538-1547, XP002600649 ISSN: 0732-183X
- MASTERS G R ET AL: "Synergistic effects of combined therapy using paclitaxel and [<90>Y-DOTA]776.1 on growth of OVCAR-3 ovarian carcinoma xenografts" 1 September 2006 (2006-09-01), GYNECOLOGIC ONCOLOGY, ACADEMIC PRESS, LONDON, GB LNKD- DOI:10.1016/J.YGYNO.2005.12.004, PAGE(S) 462 - 467 , XP024921970 ISSN: 0090-8258 [retrieved on 2006-09-01] * page 463, right-hand column, paragraphs 3,4 * * page 464, left-hand column, paragraph 2 * * page 466, right-hand column, paragraph 3 *
- BEHR THOMAS M ET AL: "Improved treatment of medullary thyroid cancer in a nude mouse model by combined radioimmunochemotherapy: Doxorubicin potentiates the therapeutic efficacy of radiolabeled antibodies in a radioresistant tumor type" CANCER RESEARCH, vol. 57, no. 23, 1 December 1997 (1997-12-01), pages 5309-5319, XP002600650 ISSN: 0008-5472
- GOLD D V ET AL: "COMBINED 90YTTRIUM-DOTA-LABELED PAM4 ANTIBODY RADIOIMMUNOTHERAPY AND GEMCITABINE RADIOSENSITIZATION FOR THE TREATMENT OF A HUMAN PANCREATIC CANCER XENOGRAFT" INTERNATIONAL JOURNAL OF CANCER, JOHN WILEY & SONS, INC, UNITED STATES, SWITZERLAND, GERMANY LNKD- DOI:10.1002/IJC.20004, vol. 109, 1 January 2004 (2004-01-01), pages 618-626, XP008055317 ISSN: 0020-7136
- ANDERSON ET AL.: "A Phase I safety and imaging study using radiofrequency ablation (RFA) followed by 131I-chTNT-1/B radioimmunotherapy adjuvant treatment of hepatic metastases" CANCER THERAPY, vol. 1, 1 December 2003 (2003-12-01), pages 283-291, XP002600651
- AL-EJEH FARES ET AL: "Chemotherapy Synergizes with Radioimmunotherapy Targeting La Autoantigen in Tumors" PLOS ONE, vol. 4, no. 2, February 2009 (2009-02), XP002600652 ISSN: 1932-6203
- SHTEMENKO N. ET AL.: 'Dichlorotetra-mu-Isobutyratodirhenium (III): Enhancement of Cisplatin Action and RBC-Stabilizing Properties' ANTICANCER RESEARCH vol. 27, July 2007 - August 2007, pages 2487 - 2492, XP008104949
- FUEGER B.J. ET AL.: 'Effects of Chemotherapeutic Agents on Expression of Somatostatin Receptors in Pancreatic Tumor Cells' THE JOURNAL OF NUCLEAR MEDICINE vol. 42, no. 12, 2001, pages 1856 - 1862, XP008104974
- DAUER M. ET AL.: 'Chemosensitization of Pancreatic Carcinoma Cells to Enhance T Cell-Mediated Cytotoxicity Induced by Tumor Lysate-Pulsed Dendritic Cells' JOURNAL OF IMMUNOTHERAPY vol. 28, no. 4, 2005, pages 332 - 342, XP008104975

## Description

### FIELD OF THE INVENTION

The present disclosure relates generally to a method of treating a neoplastic condition and to agents useful for same. More particularly, it is directed to a method of facilitating the treatment of a metastatic neoplastic tumour in a localised manner by effecting the exposure of neoplastic cell intra-cellular molecules, preferably intra-nuclear molecules, suitable for use as a therapeutic target. The co-localisation of tumour cells and metastases to discrete tissue locations thereby renders the method useful in terms of the delivery of bystander-based therapy.

### BACKGROUND OF THE INVENTION

Bibliographic details of the publications referred to by author in this specification are collected alphabetically at the end of the description.

The reference in this specification to any prior publication (or information derived from it), or to any matter which is known, is not, and should not be taken as an acknowledgment or admission or any form of suggestion that that prior publication (or information derived from it) or known matter forms part of the common general knowledge in the field of endeavour to which this specification relates.

Malignant tumours, or cancers, grow in an uncontrolled manner, invade normal tissues, and often metastasize and grow at sites distant from the tissue of origin. In general, cancers are derived from one or only a few normal cells that have undergone a poorly understood process called malignant transformation. Cancers can arise from almost any tissue in the body. Those derived from epithelial cells, called carcinomas, are the most common kinds of cancers. Sarcomas are malignant tumours of mesenchymal tissues, arising from cells such as fibroblasts, muscle cells, and fat cells. Solid malignant tumours of lymphoid tissues are called lymphomas, and marrow and blood-borne malignant tumours of lymphocytes and other hematopoietic cells are called leukemias.

Cancer is one of the three leading causes of death in industrialised nations. As treatments for infectious diseases and the prevention of cardiovascular disease continues to improve, and the average life expectancy increases, cancer is likely to become the most common fatal disease in these countries. Therefore, successfully treating cancer requires that all the malignant cells be removed or destroyed without killing the patient. An ideal way to achieve this would be to induce an immune response against the tumour that would discriminate between the cells of the tumour and their normal cellular counterparts. However, immunological approaches to the treatment of cancer have been attempted for over a century with unsustainable results.

Accordingly, current methods of treating cancer continue to follow the long used protocol of surgical excision (if possible) followed by radiotherapy and/or chemotherapy, if necessary. The success rate of this rather crude form of treatment is extremely variable but generally decreases significantly as the tumour becomes more advanced and metastasises. Further, these treatments are associated with severe side effects including disfigurement and scarring from surgery (e.g. mastectomy or limb amputation), severe nausea and vomiting from chemotherapy, and most significantly, the damage to normal tissues such as the hair follicles, gut and bone marrow which is induced as a result of the relatively non-specific targeting mechanism of the toxic drugs which form part of most cancer treatments.

Solid tumours cause the greatest number of deaths from cancer and mainly comprise tumours of the linings of the bronchial tree and the alimentary tract that are known as carcinomas. In the year 2000 in Australia, cancer accounted for 30% of male deaths and 25% of female deaths (Cancer in Australia 2000, 2003) and it accounted for 24% of male and 22% of female deaths in the US in year 2001 (Arias et al. 2003, National Vital Statistics Reports 52: 111-115). Solid tumours are not usually curable once they have spread or 'metastasised' throughout the body. The prognosis of metastatic solid tumours has improved only marginally in the last 50 years. The best chance for the cure of a solid tumour remains in the use of local treatments such as surgery and/or radiotherapy when the solid tumour is localised to its originating lining and has not spread either to the lymph nodes that drain the tumour or elsewhere. Nonetheless, even at this early stage, and particularly if the tumour has spread to the draining lymph nodes, microscopic deposits of cancer known as micrometastases may have already spread throughout the body and will subsequently lead to the death of the patient. In this sense, cancer is a systemic disease that requires systemically administered treatments. Of the patients who receive surgery and/or radiotherapy as definitive local treatment for their primary tumour and who have micrometastases, a minor proportion may be cured or at least achieve a durable remission from cancer by the addition of adjuvant systemic treatments such as cytotoxic chemotherapy or hormones.

Conventionally, solid cancer has been treated locally with surgery and/or radiotherapy, and during its metastatic stage with systemically administered cytotoxic drugs, which often interfere with the cell cycle of both normal and malignant cells. The relative selectivity of this approach for the treatment of malignant tissues is based to some extent on the more rapid recovery of normal tissues from cytotoxic drug damage. More recently, the targeted therapy of cancer has aimed to improve the therapeutic ratio of cancer treatment by enhancing its specificity and/or precision of delivery to malignant tissues while minimising adverse consequences to normal non-malignant tissues. Two of the major classes of targeted therapy are (i) the small molecule inhibitors such as the tyrosine kinase inhibitors imatinib mesylate (Glivec^{®}), gefitinib (Iressa^{®}) and erlotinib (Tarceva^{®}), and (ii) the monoclonal antibodies (mAb) such as rituximab (Mabthera^{®}) and trastuzumab (Herceptin^{®}).

In parallel to the development of targeted therapies, combining at least two conventional anticancer treatments such as chemotherapy and radiotherapy in novel ways has been another approach to the development of cancer therapeutics. By exploiting synergistic interactions between the different modalities of treatment, combined modality treatment seeks to improve treatment efficacy so that the therapeutic ratio for the combined treatment is superior to that for each of the individual treatments.

Combined modality treatment using external beam radiation and radiosensitising chemotherapeutic drugs such as 5-fluorouracil and cisplatin (chemoradiotherapy) has improved survival in a number of solid tumours such as those of head and neck, lung, oesophagus, stomach, pancreas and rectum because of both improved local tumour control and reduced rates of distant failure (TS Lawrence. Oncology (Huntington) 17, 23-28, 2003). Although radiosensitising drugs increase tumour response, they also increase toxicity to adjacent normal tissues, which is especially true of the potent new generation radiosensitisers, gemcitabine and docetaxel. However, decreasing the radiation volume allows cytotoxic doses of gemcitabine to be better tolerated clinically (Lawrence TS. Oncology (Huntington) 17, 23-28, 2003). Chemoradiotherapy may overcome mutually reinforcing resistance mechanisms, which may only manifest in vivo.

Radioimmunotherapy (RIT) is a systemic treatment that takes advantage of the specificity and avidity of the antigen-antibody interaction to deliver lethal doses of radiation to cells that bear the target antigen. Radio-isotopes that emit β-particies (e_{.}g_{.} ¹³¹Iodine, ⁹⁰Yttrium, 188 Rhenium, and ⁶⁷Copper) are usually used to label monoclonal antibodies (mAb) for therapeutic applications. The energy from β-radiation is released at relatively low intensity over distances measured in millimeters (Waldmann, Science 252: 1657-1662,1991; Bender et al., Cancer Research 52: 121-126,1992; O'Donoghue et al. Journal of Nuclear Medicine 36: 1902-1909, 1995; Griffiths et al. International Journal of Cancer 81: 985-992, 1999). Thus, high-energy β-emitters such as ⁹⁰Yttrium are useful for the treatment of larger and heterogeneous solid tumours (Liu et al. Bioconjugate Chemistry 12:7-34, 2001). Research interest in radioimmunotherapy has been reawakened because in spite of the low radiation doses delivered, significant and unexpected biological effects of RIT upon surrounding host cells have been observed (Xue et al. Proceedings of the National Academy of Sciences of the United States of America 99: 13765-13770,2002). Furthermore, the lower but biologically effective dose of radiation delivered by RIT had greater cytocidal effects than a larger dose of radiation conveyed as external beam radiotherapy *(*Dadachova et al. Proceedings of the National Academy of Sciences of the United States of America 101: 14865-14870,2004). Nonetheless, the efficiency of RIT as a treatment for solid tumours may be hampered by the low penetration of antibody through the tissue barriers that surround the target antigen in the tumour, which will consequently extend circulatory half life of the antibody *(*Britz-Cunningham et al. Journal of Nuclear Medicine 44: 1945-1961, 2003). Furthermore, RIT is often impeded by the heterogeneity of the target antigen's expression within the tumour. Thus, although RIT affords molecular targeting of tumour cells, the major limitation of RIT remains the toxicity that may result from large doses of radiation that are delivered systemically in order to achieve sufficient targeting (Britz-Cunningham *et al.* 2003, *supra*; Christiansen et al. Molecular Cancer Therapy 3: 1493-1501, 2004). Altogether, a useful therapeutic index using RIT has proven difficult to achieve clinically (Sellers et al. Journal of Clinical Investigation 104: 1655-1661, 1999).

Tumour associated antigens, which would allow differential targeting of tumours while sparing normal cells, have also been the focus of cancer research. Although abundant ubiquitous antigens may provide a more concentrated and accessible target for RIT, studies adopting this have been extremely limited.

Accordingly, there is an urgent and ongoing need to develop improved systemic therapies for solid cancers, in particular metastatic cancers.

In work leading up to the present invention it has been determined that the induction of neoplastic cell death using a DNA damaging agent, such as a cytotoxic agent, results in the exposure of intracellular, in particular intranuclear, molecules which were not previously exposed to the extracellular environment. Still further, the levels of some of these molecules are actively increased as a result of one or both of the initial neoplastic transformation of the cell and delivery of the cytotoxic cell death signal. When considered in light of the fact that the induction of neoplastic cell death by this method generally takes longer than the induction of normal cell death, the exposure of these nuclear molecules by this method provides a means for generating a target to which a further more concentrated cell death effector mechanism can be more selectively delivered. Since the neoplastic cells which have initially undergone cell death to expose the target nuclear molecules are located proximally to neoplastic cells which remain viable, the application of a targeted second step cell death effector mechanism will induce the death of proximally located viable neoplastic cells. The two step method of the invention therefore enables the initial delivery of a lower dose systemic cytotoxin since the objective is not to attempt to kill all neoplastic cells, but merely to kill a subpopulation of these cells and thereby provide a target to which a more concentrated cell death effector mechanism can be subsequently directed. By reducing the concentration and overall volume of cytotoxin,, which usually non-specifically targets a broad range of normal and neoplastic cells, the method of the present invention provides a means of delivering a highly effective second step treatment regime in a more localised manner, thereby reducing unwanted side effects. By preferably targetting nuclear molecules which have been actively increased subsequently to the exposure of a neoplastic cell to a DNA damaging agent provides a means of still further minimising the incidence of the induction of normal tissue cell death due to the localisation of significant concentrations of the second step effector mechanism to normal cells which have undergone cell death. Accordingly, the method of the present invention provides a means for improving the localised delivery of concentrated doses of treatment to a tumour and its metastases in a manner which is characterised by significantly improved outcomes and/or reduced side effects relative to those which would normally be expected in the context of the conventional treatment of an equivalent type of tumour. This is an extremely significant development since current protocols directed to treating metastatic disease are based on the non-targeted systemic delivery of chemotherapeutic agents.

### SUMMARY OF THE INVENTION

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

As used herein, the term "derived from" shall be taken to indicate that a particular integer or group of integers has originated from the species specified, but has not necessarily been obtained directly from the specified source. Further, as used herein the singular forms of "a", "and" and "the" include plural referents unless the context clearly dictates otherwise.

Unless otherwise defined, all technical and scientific terms used herein have the same meaning as commonly understood by one of ordinary skill in the art to which this invention belongs.

One aspect of the present disclosure provides a method for the localisation treatment of a neoplastic condition in a subject, said method comprising:
(i) administering to said subject a DNA damaging agent for a time and under conditions sufficient to induce the damage of at least a subpopulation of neoplastic cells, wherein said cell damage is characterised by the exposure of intra-cellular molecules; and
(ii) administering to said subject a ligand directed to one or more of said intra-cellular molecules, which ligand is associated with an effector mechanism, for a time and under conditions sufficient to downregulate the growth of viable bystander neoplastic cells.

In another aspect the present disclosure provides a method for the localised treatment of a malignant neoplastic condition in a subject, said method comprising:
(i) administering to said subject a DNA damaging agent for a time and under conditions sufficient to induce the damage of at least a subpopulation of neoplastic cells, wherein said cell damage is characterised by the exposure of intra-cellular molecules; and
(ii) administering to said subject a ligand directed to one or more of said intra-cellular molecules, which ligand is associated with an effector mechanism, for a time and under conditions sufficient to downregulate the growth of viable bystander neoplastic cells.

In yet another aspect there is provided a method for the localised treatment of a metastatic neoplastic condition in a subject, said method comprising:
(i) administering to said subject a DNA damaging agent for a time and under conditions sufficient to induce the death of at least a subpopulation of neoplastic cells, wherein said cell death is characterised by the exposure of intra-cellular molecules and upregulation of the level of said intra-cellular molecules; and
(ii) administering to said subject a ligand directed to one or more of said intra-cellular molecules, which ligand is associated with an effector mechanism, for a time and under conditions sufficient to downregulate the growth of viable bystander neoplastic cells.

In still another aspect there is provided a method for the localised treatment of a metastatic neoplastic condition in a subject, said method comprising:
(i) administering to said subject a cytotoxic agent for a time and under conditions sufficient to induce the death of at least a subpopulation of neoplastic cells, wherein said cell death is characterised by the exposure of La; and
(ii) administering to said subject a ligand directed to La, which ligand is associated with an effector mechanism, for a time and under conditions sufficient to downregulate the growth of viable bystander neoplastic cells.

In yet still another aspect there is provided a method for the localised treatment of a metastatic neoplastic condition in a subject, said method comprising:
(i) administering to said subject a radiosensitising agent for a time and under conditions sufficient to induce the death of at least a subpopulation of said neoplastic cells, wherein said cell death is characterised by the exposure of nuclear molecules and the upregulation of the level of said nuclear molecules; and
(ii) administering to said subject a ligand directed to one or more of said nuclear molecules, which ligand is associated with an effector mechanism, for a time and under conditions sufficient to downregulate the growth of viable bystander neoplastic cells.

In still yet another aspect there is provided a method for the localised treatment of a metastatic neoplastic condition in a subject, said method comprising:
(i) administering to said subject gemcitibine and/or cisplatin for a time and under conditions sufficient to induce the death of at least a subpopulation of said neoplastic cells, wherein said cell death is characterised by the exposure of La and the upregulation of the level of La; and
(ii) administering to said subject a ligand directed to La, which ligand is associated with an effector mechanism, for a time and under conditions sufficient to downregulate the growth of viable bystander neoplastic cells.

In a further aspect there is provided a method for the localised treatment of a metastatic neoplastic condition in a subject, said method comprising:
(i) administering to said subject a cytotoxic agent for a time and under conditions sufficient to induce the death of at least a subpopulation of said neoplastic cells, wherein said cell death is characterised by the exposure of La and upregulation of the level of said La; and
(ii) administering to said subject an antibody directed to La, which antibody is associated with an effector mechanism, for a time and under conditions sufficient to downregulate the growth of viable bystander neoplastic cells.

In another further aspect there is provided a method for the localised treatment of a metastatic neoplastic tumour in a subject, said method comprising:
(i) administering to said subject a radiosensitising agent for a time and under conditions sufficient to induce the death of at least a subpopulation of said neoplastic cells, wherein said cell death is characterised by the exposure and upregulation of the level of La; and
(ii) administering to said subject an immunointeractive molecule directed to La or antigenic portions thereof, which immunointeractive molecule is associated with a radioisotope, for a time and under conditions sufficient to downregulate the growth of viable bystander neoplastic cells.

Another aspect is directed to the use of:
(i) a DNA damaging agent; and
(ii) a nuclear molecule ligand associated with an effector mechanism

in the manufacture of a medicament for the treatment of a neoplastic condition in a subject wherein
(i) administering to said subject said DNA damaging agent induces the death of at lest a subpopulation of neoplastic cells, wherein said cell death is characterised by the exposure of intra-cellular molecules; and
(ii) administering to said subject said ligand induces the downregulation of growth of viable bystander neoplastic cells.

In yet another further aspect, the present disclosure contemplates a pharmaceutical composition comprising the agents as hereinbefore defined together with one or more pharmaceutically acceptable carriers and/or diluents.

### BRIEF DESCRIPTION OF THE DRAWINGS

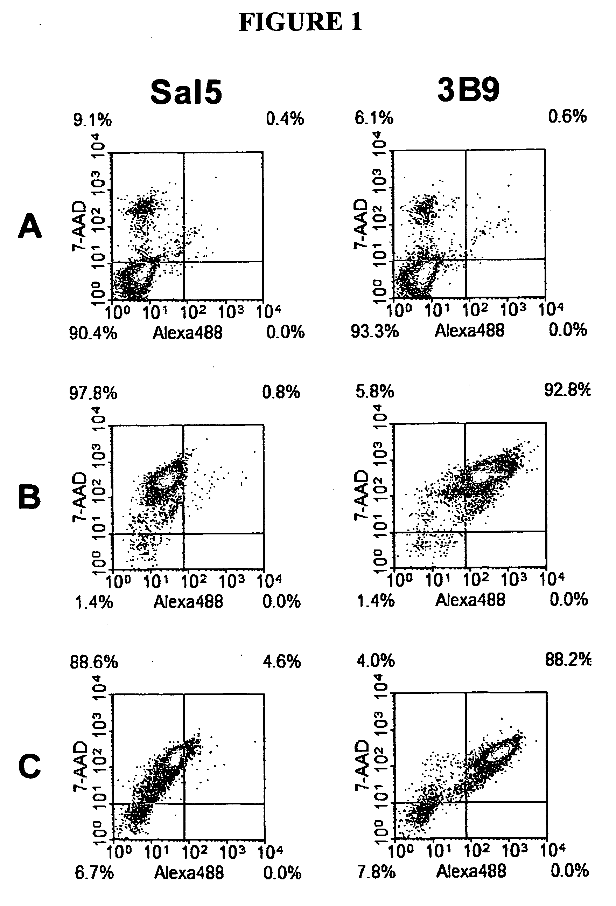

**Figure 1** depicts antigen-specific *in vitro* binding of anti-La mAb to EL4 thymic lymphoma cells after treatment with cytotoxic drugs. EL4 cells were cultured for 48 hours in the absence (**A** and **B**) or presence (**C**) of 20 µg/mL etoposide and 20 µg/mL cyclophosphamide. Untreated cells were incubated for 10 min. at room temperature (RT) in PBS (**A**) or 2 % paraformaldehyde (**B**) then diluted 1:10 in ice-cold PBS or ice-cold 100% methanol, respectively. Control cells (**A**), fixed and permeabilised cells (**B**) and cytotoxic drug-treated cells (**C**) were stained first with 5 µg/mL 3B9 or 5 µg/mL Sal5 (as the isotype control mAb) then with 2 µg/mL anti-mouse IgG Alexa₄₈₈-conjugated secondary antibody. 7-AAD 2 µg/mL was added to evaluate viability before cytofluographic analysis. Density plots show fluorescence from Alexa₄₈₈ (X-axis) and 7-AAD (Y-axis). 7AAD⁺ cells were defined as dead and were located in the upper outer quadrant if they specifically accumulated anti-La mAb (3B9).

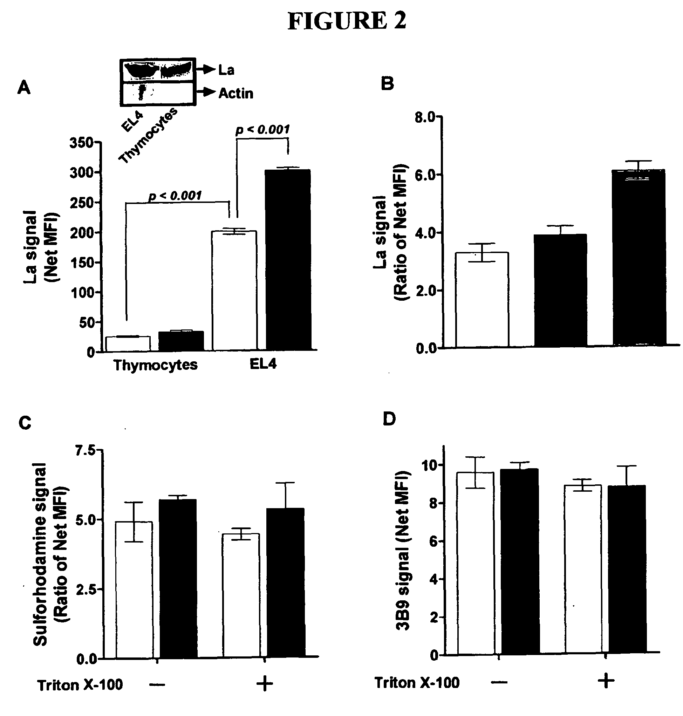

**Figure 2** depicts that La is overexpressed in malignant EL4 cells, and that La expression is further upregulated by *in vitro* treatment with DNA damaging drugs. Its expression in apoptotic EL4 cells is detergent resistant and also mediates selective detergent-resistant binding of La-specific mAb to apoptotic EL4 cells. (A) Cultured EL4 cells and normal mouse thymocytes were fixed using 2% paraformaldehyde at RT for 10 min. then resuspended 1:10 in ice-cold methanol (clear columns). Cells were also cultured for 48 hr in the presence of 20 µg/mL etoposide and 20 µg/mL cyclophosphamide PI⁺ and 3B9⁺ events (filled columns). Cells were stained with mAb then with anti-mouse IgG Alexa₄₈₈-conjugated secondary antibody and 7-AAD and analysed by flow cytometry using a gate restricted to 7-AAD⁺ events. Data are expressed as net mean fluorescence intensity (MFI) ± standard error of the mean (SEM) (*n*=3) of 7-AAD⁺ events. Net MFI for La-specific staining was obtained after subtraction of individual MFI values for Sal5 staining. **Inset:** Lysates of cultured cells were analysed by Western blotting for La protein expression using 3B9 together with an actin expression control for equivalence of loading. (**B**) EL4 cells and thymocytes were cultured for 48 hours in media containing 1 µM of the pan-kinase inhibitor staurosporine (STS) (clear columns), 20 µg/mL etoposide (filled columns), or 20 µg/mL cyclophosphamide and 20 µg/mL etoposide (grey columns). After incubation with 1% Triton X-100 for 5-10 min. at RT, cells were analysed by flow cytometry as described in A. Data are expressed as the ratio of the net MFI ± SEM (*n*=3) for La staining (after subtraction of individual MFI values for Sal5 staining) in EL4 cells to that in thymocytes. (**C**) EL4 cells and thymocytes were cultured in media containing 20 µg/mL etoposide (clear columns) or 10 µg/mL cisplatin (filled columns) for 96 hours. Cells were washed, stained with 1 µg/mL sulforhodamine B, washed again and incubated in the presence or absence of 1% Triton X-100 for 5 min. at RT and analysed by flow cytometry. Data are expressed as the ratio of the MFI ± SEM (*n*=4) for sulforhodamine staining in EL4 cells to that in thymocytes. (**D**) During 96 hours treatment with 20 µg/mL etoposide (clear columns) or 10 µg/mL cisplatin (filled columns), EL4 cells and thymocytes were incubated with either 50 µg/mL 3B9 or Sal5. Cells were washed and incubated with 1% Triton X-100 for 5 min at RT then washed again before staining with anti-mouse IgG Alexa₄₈₈-conjugated secondary antibody and 2 µg/mL 7-AAD. Data are expressed as the ratio of the net MFI ± SEM (*n*=3) for detergent-resistant 3B9 binding (after subtraction of individual MFI values for Sal5 binding) obtained in EL4 cells to that obtained in thymocytes.

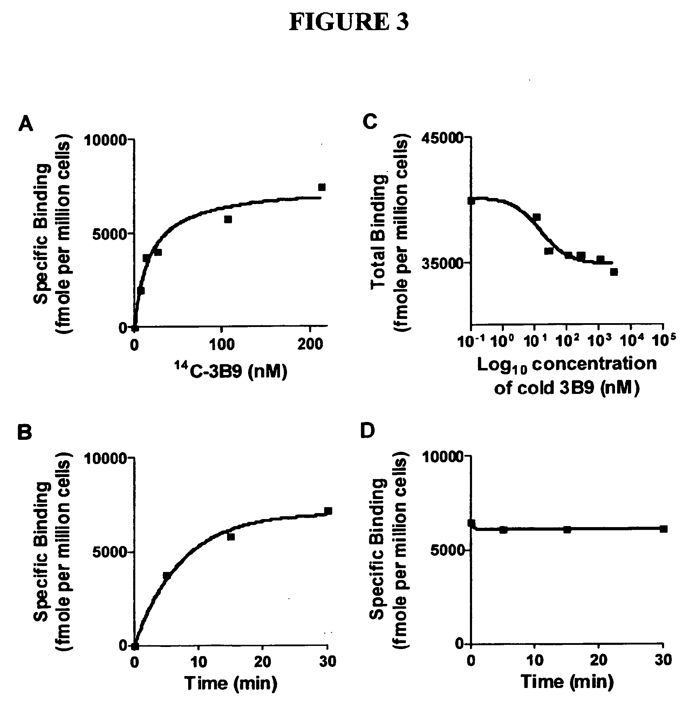

**Figure 3** depicts that La-specific mAb binds with high affinity to apoptotic EL4 cells in a specific, rapid, saturable and irreversible manner. Apoptosis was induced in EL4 cells after 48 hours treatment with 20 µg/mL etoposide and 20 µg/mL cyclophosphamide. (**A**) Treated cells were incubated at RT with increasing concentrations of ¹⁴C-3B9 in the presence or absence of 50-fold molar excess of unlabelled 3B9. Cells were washed and the radioactivity measured. Specific binding was calculated in units of femtomole bound per million cells and plotted as a function of ¹⁴C-3B9 concentration. The concentration required to reach half-maximal saturation of 10⁶ apoptotic cells was ~18nM (*n*=3). (**B**) Cells were incubated with ¹⁴C-3B9 and samples were removed at specified time points. Specific binding in units of femtomole bound per million cells was plotted as a function of time. The time required to reach half-maximal saturation of 10⁶ apoptotic cells was 5 min. (*n*=3). (**C**) Treated cells were incubated with a set concentration (100 nM) of ¹⁴C-3B9 in the presence of increasing concentrations of unlabelled 3B9. Radioactivity was measured and converted to units of femtomole bound per million cells and plotted as a function of the log-concentration of 3B9. The concentration of unlabelled 3B9 required to inhibit half-maximal binding of ¹⁴C-3B9 was ~28nM (*n*=5). (**D**) Cells were incubated with ¹⁴C-3B9 for 30 min. Cells were washed then incubated in binding buffer alone and samples were removed at specified time points. Samples were washed and specific binding in units of femtomole bound per million cells was plotted as a function of time. Bound antibody did not dissociate after cells were incubated in binding buffer for 30 min. at 37°C (*n*=3).

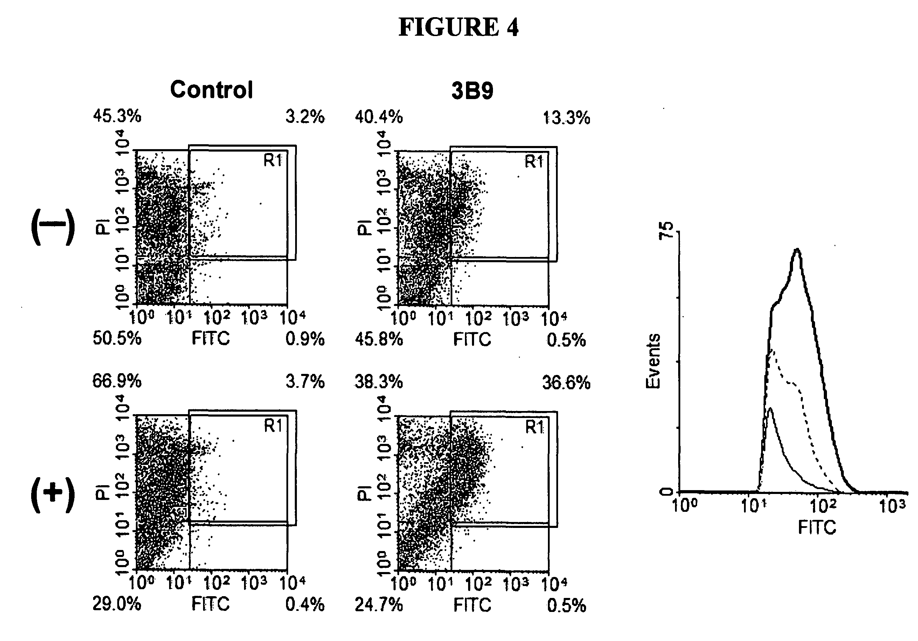

**Figure 4** depicts that anti-La mAb binds specifically to dead cells isolated from EL4 tumour explants and both proportionate and per cell binding increases after cytotoxic drug treatment. EL4 tumour-bearing mice (*n*=5) were treated or not with etoposide 67 mg/kg and cyclophosphamide 100 mg/kg by intraperitoneal injection on two occasions 24 hours apart. At 48 hours after the commencement of treatment, tumours were excised and single cell suspensions prepared. Cells were washed with PBS and incubated with 5 µg/mL FITC-conjugates of 3B9 or Sal5 isotype control. PI 0.5 µg/mL was added to evaluate cell viability and analysis performed using flow cytometry. Data shown are representative density plots and histogram overlays of FITC and PI emissions from stained cells that were isolated from EL4 tumour explants of mice treated (+; upper row of panels) or not (-; lower row of panels) with cytotoxic chemotherapy. Data depicted in the histogram overlays originated from region 1 (R1), which represents the FITC⁺ subset of PI⁺ events, and shows SaI5-FITC staining of tumour cells from chemotherapy-treated mice (grey fill), 3B9-FITC staining of tumour cells from untreated mice (dashed line) and chemotherapy-treated mice (thick line). After chemotherapy, the fraction of PI⁺ cells in tumor explants increased significantly from a mean (± SE) of 50 ± 2% to 70 ± 1% (*P* < 0.001). Similarly, the 3B9⁺ subset of PI⁺ cells increased significantly from 15 ± 1% to 38 ± 2% (*P* < 0.01) after chemotherapy, whereas isotype control staining was unaltered. Only the PI⁺ tumour subpopulation bound 3B9, which indicated that La was recognized specifically in dead tumour cells. Histogram analysis indicated that specific per cell binding of 3B9-FITC to PI⁺ cells was significantly augmented in tumors exposed *in vivo* to cytotoxic chemotherapy (net median fluorescence intensity ± SE of 18 ± 3 with chemotherapy and 1 ± 3 without chemotherapy, *P* < 0.05).

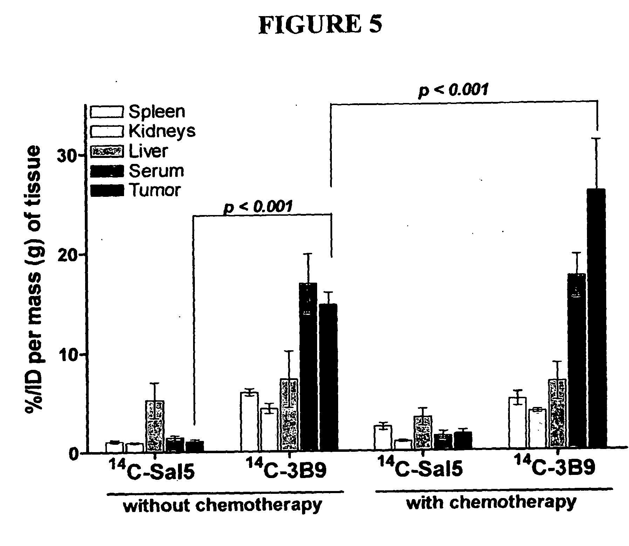

**Figure 5** depicts that biosynthetically labelled La-specific ¹⁴C-3B9 mAb is taken up preferentially by tumours, particularly after cytotoxic chemotherapy. Intrinsic labelling of the La-specific mAb indicates that tumour targetting results from antigen-binding activity of the antibody rather than from non-specific localisation of the radiolabel. EL4 tumour-bearing mice (*n*=4) were given intravenous injections of 100 µg of either ¹⁴C-3B9 or ¹⁴C-Sa15 at a dose of approximately 5 mg/kg. Then, mice were treated or not with etoposide 67 mg/kg and cyclophosphamide 100 mg/kg, which were given as two intraperitoneal injections 24 hours apart. Forty-eight hours after the commencement of treatment, the mice were killed and their organs collected for radioactivity measurement. Radioactivity was normalised to the mass of tissue counted (dpm/g of tissue) and the percentage accumulation over the injected dose (%/ID per mass of tissue) was calculated based on the specific radioactivity of the injected agents. Data are presented as %/ID ± SEM. Statistical comparisons were made using two-way analysis of variance, which was performed as a Bonferroni post-test comparison between all groups (*p* < 0.001; *n*=4).

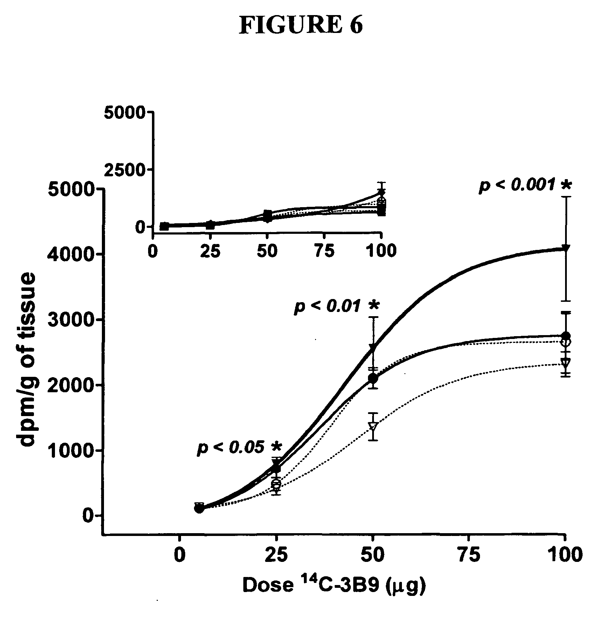

**Figure 6** depicts that the intra-tumoral accumulation of biosynthetically labelled La-specific ¹⁴C-3B9 mAb is dose-dependent and is augmented by cytotoxic chemotherapy. The biodistribution of different doses of ¹⁴C-labelled 3B9 was studied in EL4 tumour bearing mice with or without chemotherapy. ¹⁴C-3B9 accumulated significantly more only in the tumour with chemotherapy when its uptake approximately doubled at ¹⁴C-3B9 doses of 25 µg, 50 µg and 100 µg. Mice were given intravenous injections of 5, 25, 50 or 100 µg of ¹⁴C-3B9 or ¹⁴C-Sal5 and then treated or not with etoposide 67 mg/kg and cyclophosphamide 100 mg/kg, which were given as two intraperitoneal injections 24 hours apart. Forty-eight hours after commencement of treatment, the mice were killed and organs collected for radioactivity measurement. Radioactivity was normalised to the mass of tissue counted (dpm/g of tissue) and tissues included (◆) liver, (■) spleen, (▲) kidneys, (●) serum and (▼) tumour. Data are presented as dpm/g ± SEM. Closed symbols, with chemotherapy; open symbols, without chemotherapy. Statistical comparisons were made using two-way analysis of variance, which was performed as a Bonferroni post-test comparison between all groups (*n*>4).

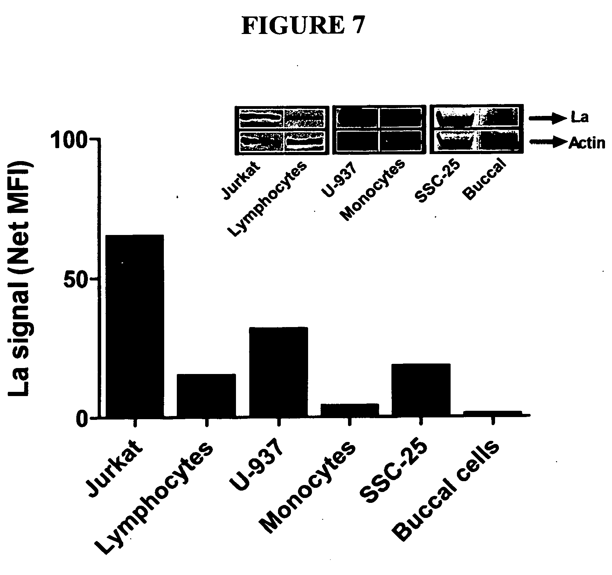

**Figure 7** depicts that La is overexpressed in human cancer cell lines. Cultured cells from cancer cell lines and cells of their normal counterpart cell type were fixed and permeabilised using paraformaldehyde and methanol. Cells were stained with 3B9 or Sal5 then with anti-mouse IgG Alexa₄₈₈ secondary antibody conjugate and 7-AAD before cytofluographic analysis was performed. Data are presented as the net MFI for 3B9 staining after subtraction of individual MFI values for Sal5 staining after first gating on 7-AAD⁺ events, which comprised >98 % of cells. Inset: Lysates of cultured cells were analysed by Western blotting for La protein expression using 3B9 together with an actin expression control for equivalence of loading.

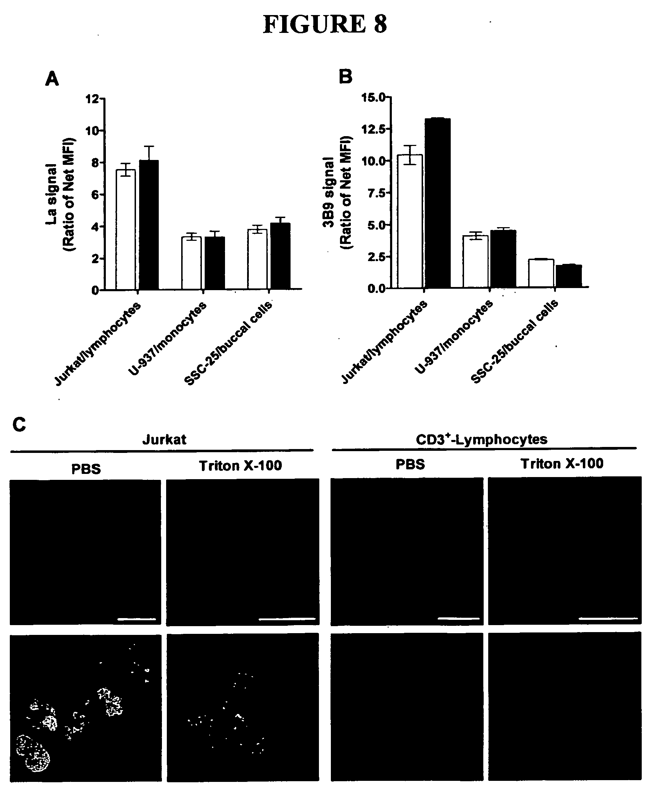

**Figure 8** depicts that both La and its binding by 3B9 in apoptotic human malignant cells is resistant to detergent treatment. (**A**) Cells were treated first with STS (clear columns) or etoposide (filled columns) for 48 h and then with Triton X-100. Cells were stained with 3B9 or Sal5 then with anti-mouse IgG Alexa₄₈₈ secondary antibody conjugate and 7-AAD before cytofluographic analysis was performed. Data are expressed as the ratio of the net MFI ± SEM (*n*≥4) for La-specific staining in the malignant cells to that in the corresponding normal cells. (**B**) During 96 hours treatment with 10 µg/mL cisplatin (clear columns) or 20 µg/mL etoposide (filled columns), cells were incubated with either 50 µg/mL 3B9 or Sal5. Cells were washed and incubated with 1% Triton X-100 for 5 min. at RT. Then cells were washed again before staining with anti-mouse IgG Alexa₄₈₈-conjugated secondary antibody and 2 µg/mL 7-AAD and analysed by flow cytometry. Data are expressed as the ratio of the net MFI ± SEM (*n*≥4) for detergent-resistant 3B9 binding (after subtraction of individual MFI values for Sal5 binding) obtained in the malignant cells to that obtained in the corresponding normal cells. (**C**) Cells were prepared as described in B, treated or not with Triton X-100, then stained with anti-mouse IgG Alexa₄₈₈ secondary antibody conjugate or sulforhodamine B before laser scanning confocal microscopy analysis. 3B9, green; sulforhodamine B, red; co-localised images, orange/yellow. Scale bar represents 20 µm.

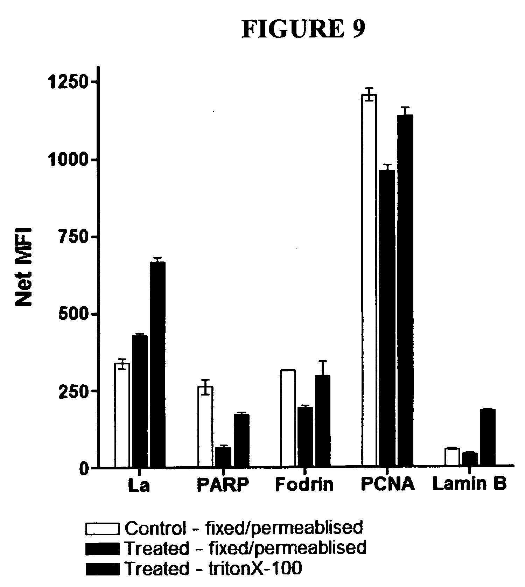

**Figure 9** depicts that in comparison with other nuclear antigens, La is preferentially retained in cisplatin-treated apoptotic Jurkat cells. Jurkat cells were cultured in the absence (control) or presence (treated) of 20 µg/mL cisplatin for 48 h. Then the cells were incubated as follows: (i) 15 min in 500 µL PBS (control - fixed and permeabilised), (ii) 10 min in 50 µL of 2 % paraformaldehyde then in 450 µL of ice-cold absolute methanol (treated -fixed and permeabilised), (iii) 10 min in 500 µL Triton X-100 then vortexing cells for 30 secs (treated - Triton X-100). Cells were washed with PBS and incubated for 30 min at room temperature (RT) in 5 µg/mL Sal5 isotype control mAb or 5 µg/mL of monoclonal antibodies specific for the antigens shown. Cells were washed and incubated (30 min, RT) with 2 µg/mL of anti-mouse IgG-Alexa₄₈₈. Finally, cells were washed, incubated with 2 mg/mL 7-AAD and analysed by flow cytometry. Data were obtained after gating on 7-AAD⁺ events and are presented as Net Mean Fluorescence Intensity (MFI) ± SEM (*n*=2) after subtraction of the MFI for isotype control staining. The upper and lower panels depict results from different experiments. PARP, Poly(ADP-ribose) polymerase; PCNA, proliferating cell nuclear antigen.

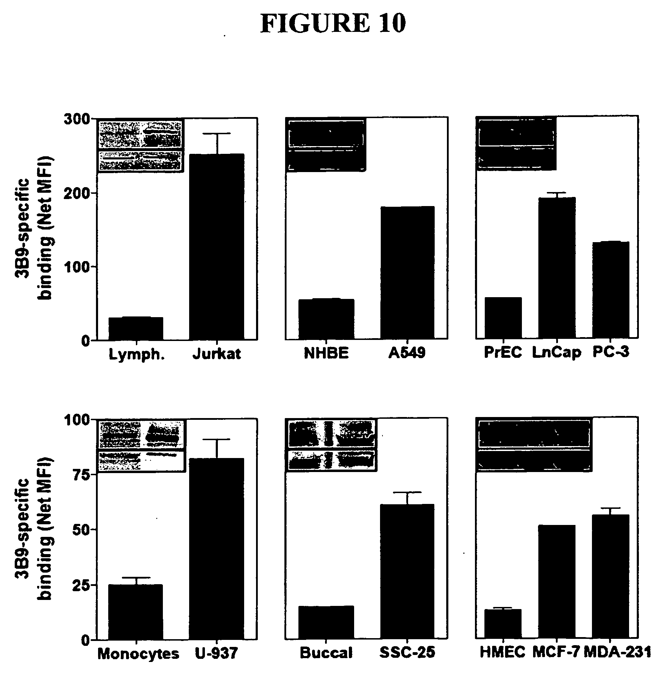

**Figure 10** depicts that La is overexpressed in human malignant cell lines. Panels show expression of La in malignant cell lines compared with its expression in the corresponding primary cell types using indirect immunofluorescence staining of fixed and permeabilized cells. 3B9-specific binding is shown as Net MFI ± SEM (*n*=3). **Insets:** Immunoblots of cell lysates from cells probed for expression of La with 3B9 (upper row of bands) or actin as a loading control (lower row of bands). Three independent experiments were performed and representative data are shown. **Key:** CD3-enriched peripheral blood lymphocytes (Lymph.) *vs.* Jurkat adult T-leukemia cells; Normal Human Bronchial Epithelium (NHBE) *vs.* A549 bronchogenic carcinoma cells; Prostate Epithelial Cells (PrEC) vs. PC-3 and LNCaP prostate cancer cells (PrEC and PC-3 lysates are shown in the inset); CD14-enriched monocytes *vs.* U-937 myeloid leukaemia cells; Buccal cavity epithelial cells (Buccal) *vs.* SSC-25 oropharyngeal squamous cell carcinoma cells; Human Mammary Epithelial Cells (HMEC) vs. MCF-7 and MDA-MB-231, breast cancer cell lines.

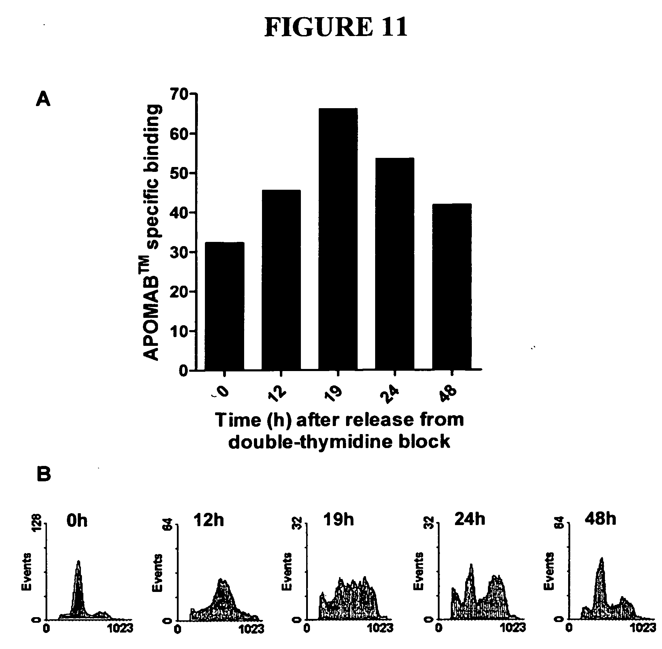

**Figure 11** is a graphical representation demonstrating that La expression is cell cycle dependent. Jurkat cells are incubated with 100µM thymidine for 18h then washed and incubated without thymidine for 16h. Cells are collected and re-incubated with 100µM thymidine for 18h for a double-thymidine block to synchronise cells at G1/S phase. At the time points indicated, cells are washed and (**A**) subjected to indirect immunofluorescence staining with Apomab or Sal5 isotype control mAb or (**B**) resuspended in 70% ethanol for cell cycle analysis by PI staining (50ng/mL). Apomab-specific binding is calculated from the net mean fluorescence intensity (MFI) value after subtraction of individual MFI values for staining with the Sal5 isotype control.

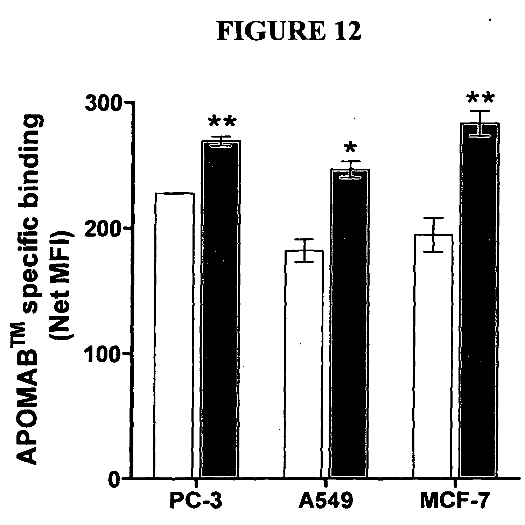

**Figure 12** is a graphical representation demonstrating that La expression increases in malignant cells after mitogenic stimulation. Cells are incubated with (filled bars) or without (clear bars) 200ng/mL phorbol 12-myristate 13-acetate (PMA) for 48h. Cells are fixed and permeabilised by incubation in 2% paraformaldehyde for 10min. followed by 1:10 dilution in -20°C methanol for 2-3min. Cells are washed extensively with PBS then subjected to indirect immunofluorescence staining with Apomab or Sal5 isotype control mAb. Apomab-specific binding is calculated from the net mean fluorescence intensity (MFI) value after subtraction of individual MFI values for staining with the Sal5 isotype control. Statistical analysis of Apomab-specific binding is done using two-way ANOVA and a Bonferroni post-test comparison (*, *P*<0.05; **, *P*<0.01).

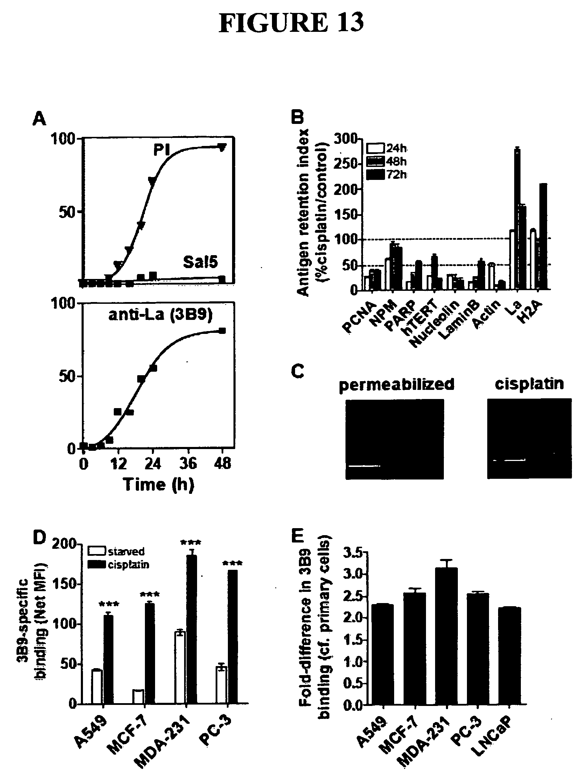

**Figure 13** depicts that La-specific 3B9 mAb preferentially binds apoptotic malignant cells *in vitro.* (A) Etoposide-treated (20µg/mL) Jurkat cells were stained with PI (▼), 3B9 or its control Sal5 mAb (■), and percentages of positively stained cells determined by cytofluography (Y-axis) are plotted as a function of time in hours (X-axis). (**B**) Jurkat cells were incubated in the presence or absence of 20µg/mL cisplatin. At specified times, cells were fixed, permeabilized and stained. Specific staining of different antigens was calculated as net MFI ± SEM and data are presented as an antigen retention index, which was calculated using the following formula: % retention = MFI from cisplatin-treated cells/MFI from control cells X 100%). The antigen retention index was calculated in two independent experiments each comprising triplicate samples. Data shown are representative of one of these experiments.(**C**) Permeabilized Jurkat cells and cisplatin-treated Jurkat cells were stained with 7-AAD (red) and 3B9 (green) and viewed by confocal microscopy. Scale bars represent 5µm of actual length. (**D**) Cell death was induced by 72h treatment with 20µg/mL cisplatin or by serum deprivation. Cells were stained with 3B9 and 7-AAD and data are shown as Net MFI ± SEM (*n*=2) for the 7-AAD⁺ population. Data shown are representative of identical assays performed on three separate occasions. (**E**) Cell death was induced in cultured cells after 48h treatment with 20µg/mL cisplatin. For each cell type, 3B9-specific binding was calculated as net MFI and the fold-difference in 3B9 binding was calculated as the ratio of specific binding in the malignant cells to that in the corresponding primary cell type: MCF-7 and MDA-MB-231 cells *vs.* HMEC; A549 cells *vs.* NHBE cells and LNCaP and PC-3 cells *vs* PrEC. Data are presented as the fold difference ± SEM (*n*=2).

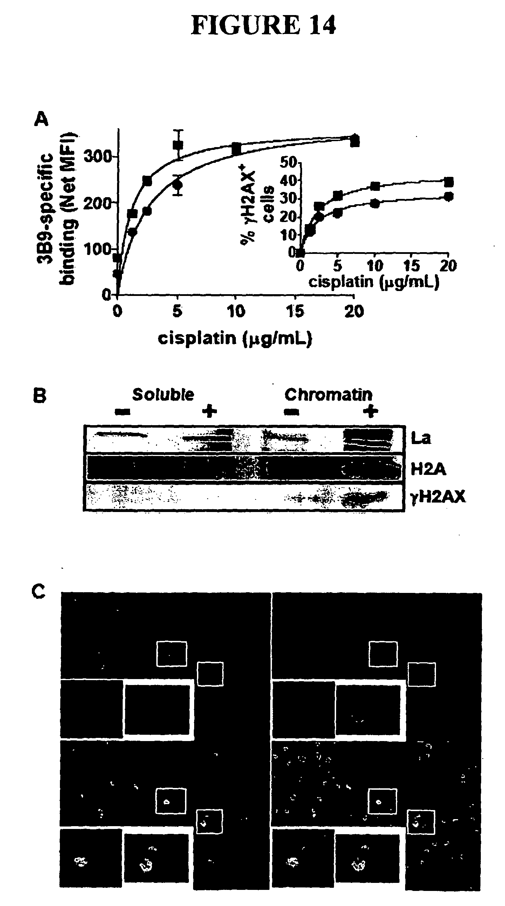

**Figure 14** depicts that 3B9 binding is augmented by DNA damaging agents. (**A**) Jurkat cells were incubated with increasing concentrations of cisplatin in the absence (●) or presence (■) of 100 ng/mL trichostatin A (TSA). At 48h, cells were analyzed for 3B9 binding. Inset: Samples were removed at 3h from incubation with cisplatin in the absence (●) or presence (■) of 100ng/mL TSA, fixed and permeabilized and stained for γH2AX. (**B**) Immunoblots of soluble and chromatin fractions from a chromatin-binding assay after Jurkat cells were treated with 20 µg/mL cisplatin for 3 h (+) or not (-). We inferred that La redistributed to DNA double-stranded breaks after observing increased chromatin-associated La and γH2AX. (**C**) Adherent cultures of PANC-1 cells were incubated in slide chambers for 3h with 20µg/mL cisplatin. Cells were fixed, permeabilized and stained with 3B9 and anti-γH2AX. Images were acquired for 3B9 binding (green) and γH2AX staining (red) and overlayed to investigate co-localization (orange-yellow). 3B9 and γH2AX overlays were superimposed on transmission images of cells to ensure nuclear localization of detected antigens. Shown are representative data of four independent experiments, which produced similar results.

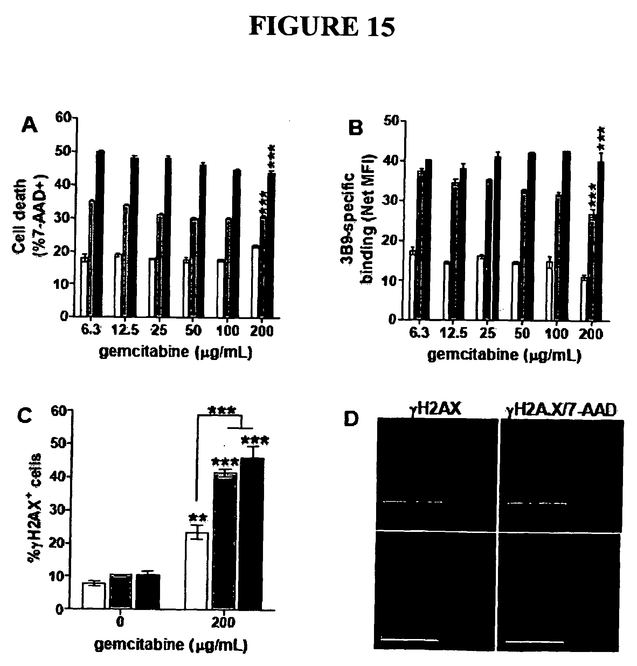

**Figure 15** depicts that 3B9 binding to dead PANC-1 cells is augmented after combination treatment with gemcitabine and TSA produced additive cytotoxicity and DNA-damaging effects. PANC-1 cells were incubated with increasing concentrations of gemcitabine in the absence (clear columns) or presence of 100ng/mL (hatched columns) or 200ng/mL (filled columns) of TSA. Analysis was done for viability using 7-AAD (**A**), 3B9-specific binding (**B**) and DNA damage using γH2AX staining (**C**). Data are presented as mean ± SEM from three independent experiments. Photomicrographs show PANC-1 cells treated with gemcitabine and TSA (+) or not (-), which were stained with γH2AX-specific mAb and 7-AAD (**D**).

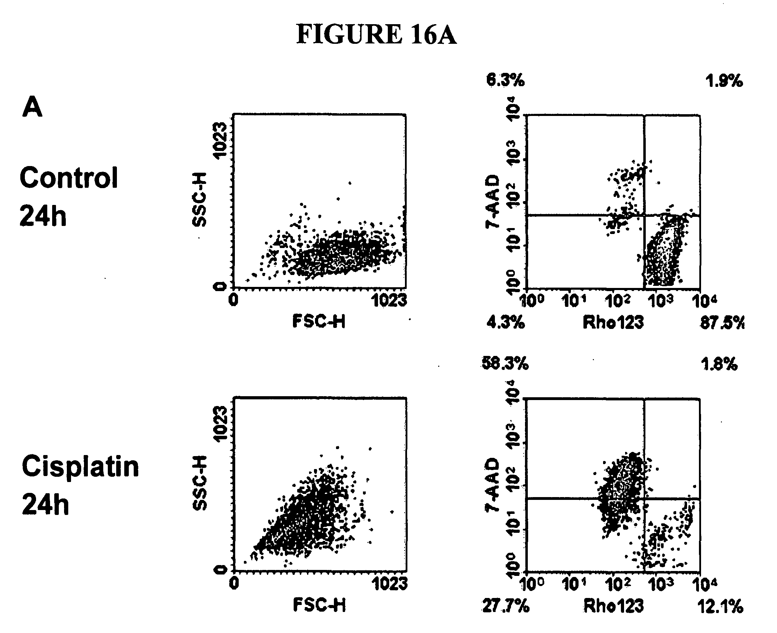

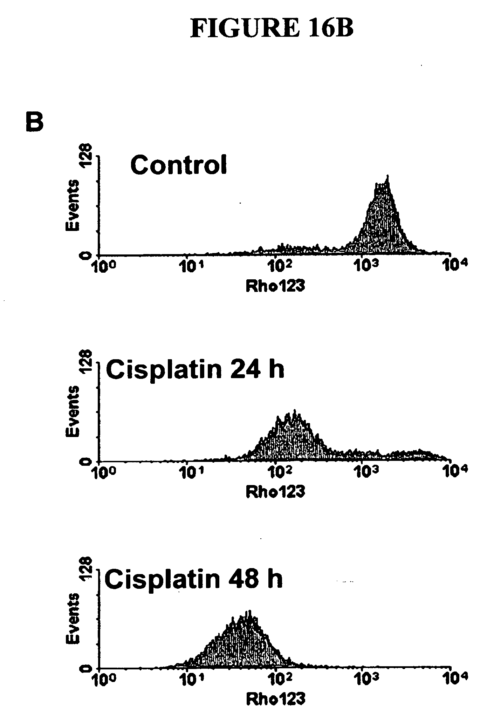

**Figure 16** is a graphical representation depicting that cisplatin-induced cell death is demonstrated to be apoptotic. Jurkat cells were incubated in the presence or absence of 20µg/mL cisplatin. Cell death was assessed using the DNA-binding dye, 7-AAD, and the mitochondrial membrane potential dye, Rhodamine 123. **A**. Dotplots are shown of untreated control cells and cells treated with cisplatin for 24h; left-hand panels, forward scatter (size) vs. side scatter (internal complexity); right hand panels, 7-AAD vs. rhodamine-123. **B**. Histograms are shown of rhodamine-123 staining for untreated control cells and cells treated with cisplatin for 24h and 48h.

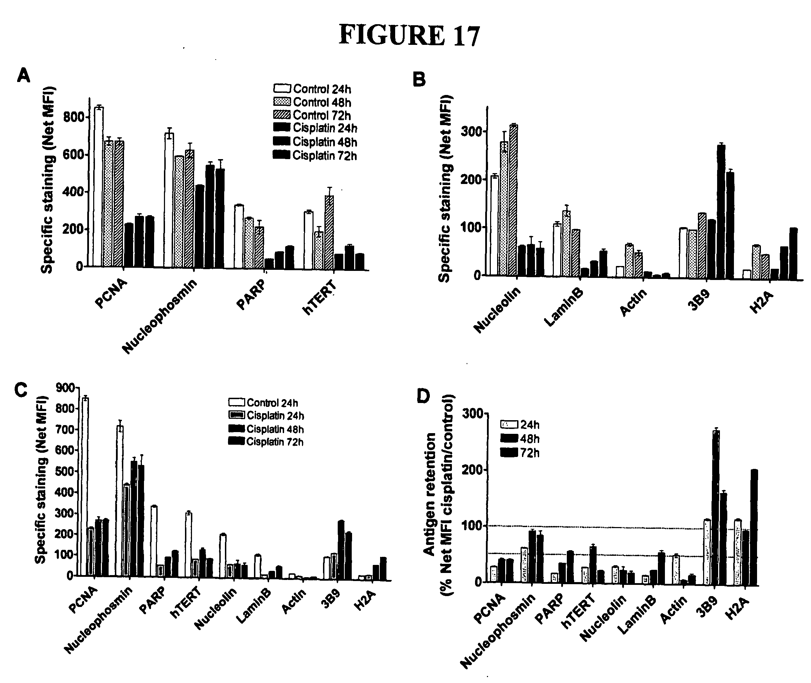

**Figure 17** is a graphical representation depicting that the La antigen is preferentiallly retained in dead Jurkat cells 48h after cisplatin-induced apoptosis. Jurkat cells were incubated in the presence or absence of 20µg/mL cisplatin. At the specified time points cells were removed and cell pellets were resuspended in 2% paraformaldehyde and incubated for 10 min. at RT then diluted 1:10 in ice-cold 100% methanol for another 2min. Pellets were collected and washed in PBS then incubated (30min. at RT) with 10µg/mL of antigen-specific murine mAb (PCNA, Nucleolin, Nucleophosmin, PARP, 3B9 (Apomab™), Sal5 and Lamin B), 125µL of rat anti-hTERT or 20ug/mL rat IgG₂ₐₖ matched isotype mAb or with rabbit anti-actin, rabbit anti-H2A or rabbit IgG control Ab at 10 µg/mL. Cells were collected and washed with PBS then incubated for 30 min. at RT with 2µg/mL of relevant secondary antibodies conjugated to Alexa₄₈₈. Cells were washed and samples were analysed by flow cytometry. (**A** and **B**) specific staining for the different antigens is presented as the net MFI (after substraction of the corresponding isotoype Ab) ± SEM. (**C**) Simplified form of panels A and B with control cells from 24h only shown. (**D**) antigen retention index was calculated from A and B using the following formula: % retention = specific signal from cisplatin treated cells/specific signal from control cells X 100%.

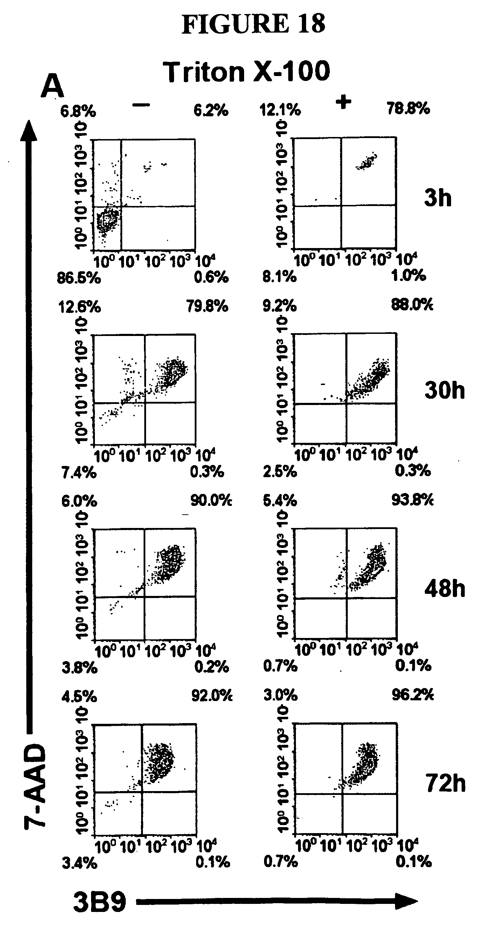

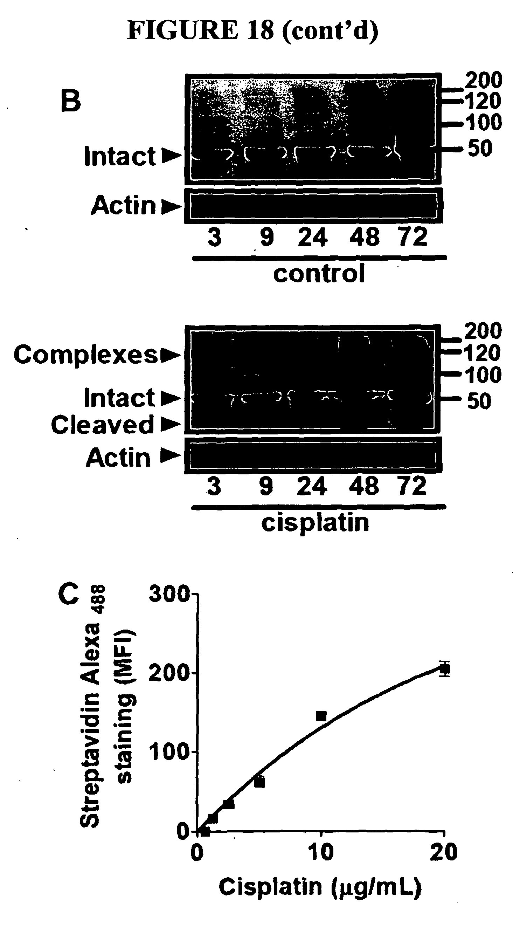

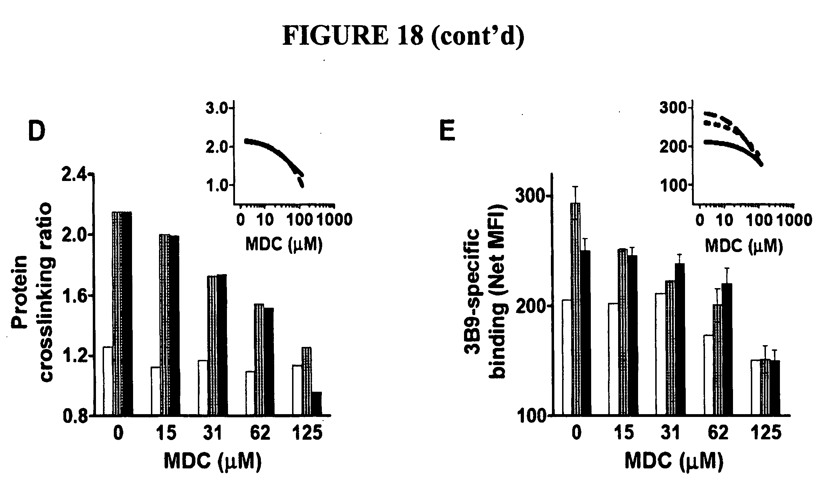

**Figure 18** depicts that La antigen is crosslinked during apoptosis. (**A**) Jurkat cells incubated in 20µg/mL cisplatin were removed at specified time points and incubated for 10min. in 1% Triton X-100 solution before 3B9 binding analysis. Data are presented as density plots of staining with 3B9 *vs.* 7-AAD. (**B**) Immunoblots of cell lysates of control and cisplatin-treated Jurkat cells were probed with 3B9 for expression of La (top panels) or actin as a loading control (bottom panels) at specified times. (**C**) Jurkat cells incubated in the absence or presence of increasing concentrations of cisplatin were labeled with cadaverine-biotin added at 100µM for 48h. Cells were stained with strepatavidin-Alexa₄₈₈ and analyzed by flow cytomtery. Data shown are MFI ± SEM (n=3). (**D**) Jurkat cells incubated in the absence or presence of 20µg/mL cisplatin were incubated with increasing concentrations of MDC. Cells were analyzed at specified times (24 □, 48 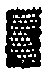 and 72h ■) for sulforhodamine 101 staining. **Inset** summarizes the dependence of the protein cross-linking ratio on MDC concentration at 48h (-) and 72h (■■■) after cisplatin treatment of Jurkat cells. (**E**) Jurkat cells incubated with cisplatin in the absence or presence of increasing concentrations of MDC were stained with 3B9. 3B9-specific binding is presented as Net MFI ± SEM (*n*=3). Inset summarizes the dependence of 3B9-specific binding on MDC concentration at 24h (-), 48h (--) and 72h (■■■) after cisplatin treatment of Jurkat cells. Data are representative of three identical and independent assays, which produced similar results.

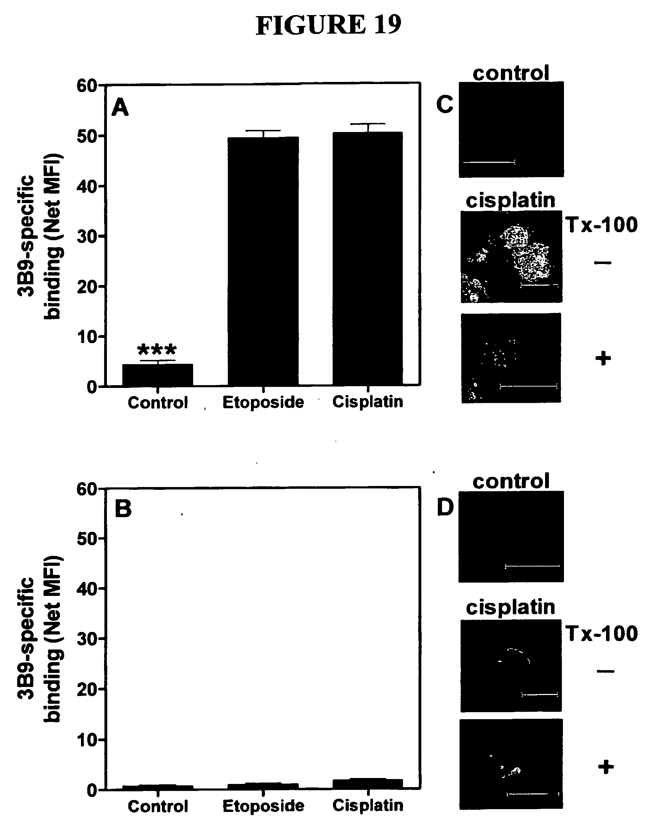

**Figure 19** depicts that the preferential binding of 3B9 to dead malignant cells after cytotoxic drug treatment is detergent-resistant and co-localizes with other intracellular proteins. Cultures of Jurkat cells (**A**) and their counterpart primary CD3-enriched peripheral blood lymphocytes (**B**) were either left untreated (control) or treated with etoposide or cisplatin for 48h in the presence of 50µg/mL of 3B9 or its Sal5 control mAb. After 48h, cells were treated or not with 1% Triton X-100 before fluorocytometric analysis. Data shown are the net MFI ± SEM of 7-AAD⁺ events from four independent assays. Statistical comparisons were made between control and treated cells. Photomicrographs show representative Jurkat cells (**C**) and CD3-enriched peripheral blood lymphocytes (**D**), which had been treated with 20µg/mL cisplatin in the presence of 50µg/mL 3B9, treated or not with 1% Triton X-100 and stained with sulforhodamine (red) and Alexa₄₈₈-conjugated anti-mouse IgG (green). Control: non-permeabilized and untreated Jurkat cells. Scale bars represent either 40µm (PBS) or 20µm (Triton X-100) of actual length.

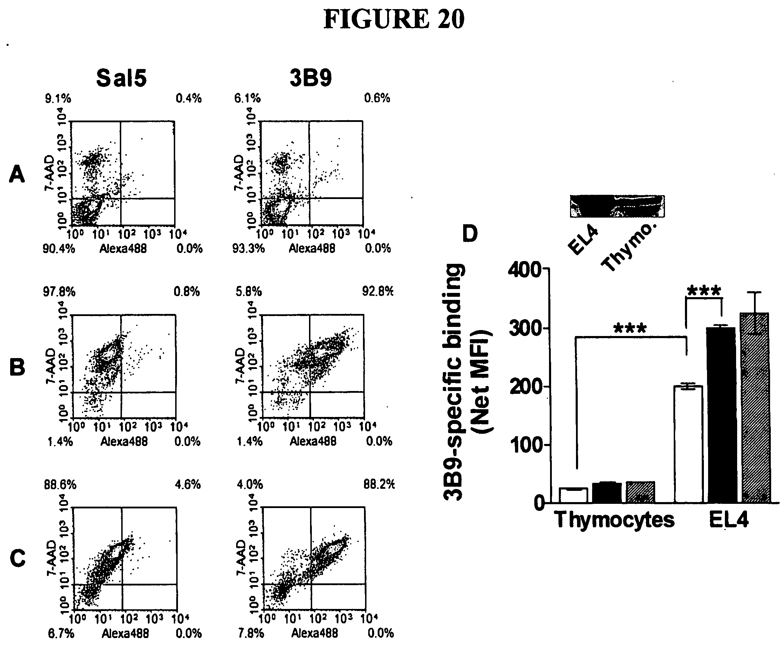

**Figure 20****: La-specific mAb binds specifically to malignant EL4 cells after *in vitro* treatment with cytotoxic drugs.** EL4 cells were cultured for 48h in the absence (**A** and **B**) or presence (**C**) of cyclophosphamide and etoposide. Untreated control cells (**A**), fixed and permeabilized cells (**B**) and cytotoxic drug-treated cells (**C**) were stained with 7-AAD and Sal5 isotype control or La-specific 3B9 mAb. Density plots show fluorescence from Alexa₄₈₈ (X-axis) and 7-AAD (Y-axis). (**D**) EL4 cells and syngeneic murine thymocytes were fixed and permeabilized (clear columns) or cultured for 48h with cyclophosphamide and etoposide and subsequently treated (striped columns) or not (filled columns) with Triton X-100. 3B9-specific binding is presented as net MFI ± SEM (*n*=3) of 7-AAD⁺ events, ***, *P*<0.001. **Inset:** Immunoblots of cell lysates were probed for expression of La using 3B9.

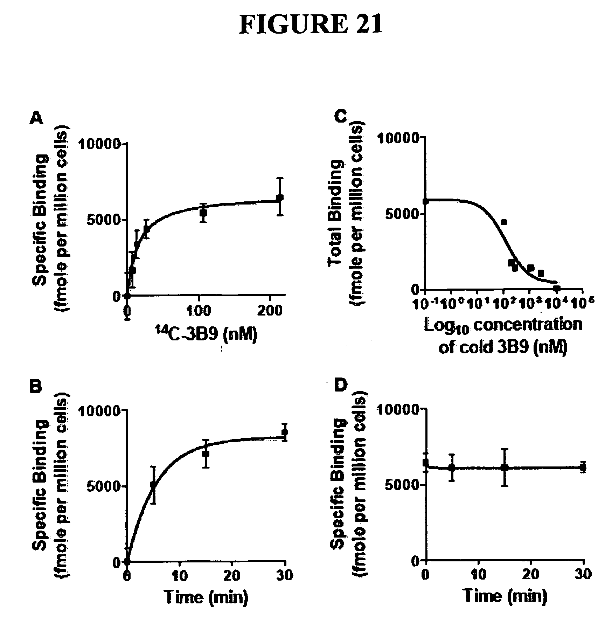

**Figure 21****:** depicts that biosynthetically labelled La-specific ¹⁴C-3B9 mAb is taken up preferentially by tumours, particularly after cytotoxic chemotherapy. Intrinsic labelling of the La-specific mAb indicates that tumour targetting results from antigen-binding activity of the antibody rather than from non-specific localisation of the radiolabel. EL4 tumour-bearing mice (*n*=4) were given intravenous injections of 100 µg of either ¹⁴C-3B9 or ¹⁴C-Sal5 at a dose of approximately 5 mg/kg. Then, mice were treated or not with etoposide 67 mg/kg and cyclophosphamide 100 mg/kg, which were given as two intraperitoneal injections 24 hours apart. Forty-eight hours after the commencement of treatment, the mice were killed and their organs collected for radioactivity measurement. Radioactivity was normalised to the mass of tissue counted (dpm/g of tissue) and the percentage accumulation over the injected dose (%/ID per mass of tissue) was calculated based on the specific radioactivity of the injected agents. Data are presented as %/ID ± SEM. Statistical comparisons were made using two-way analysis of variance, which was performed as a Bonferroni post-test comparison between all groups (*p* < 0.001; *n*=4).

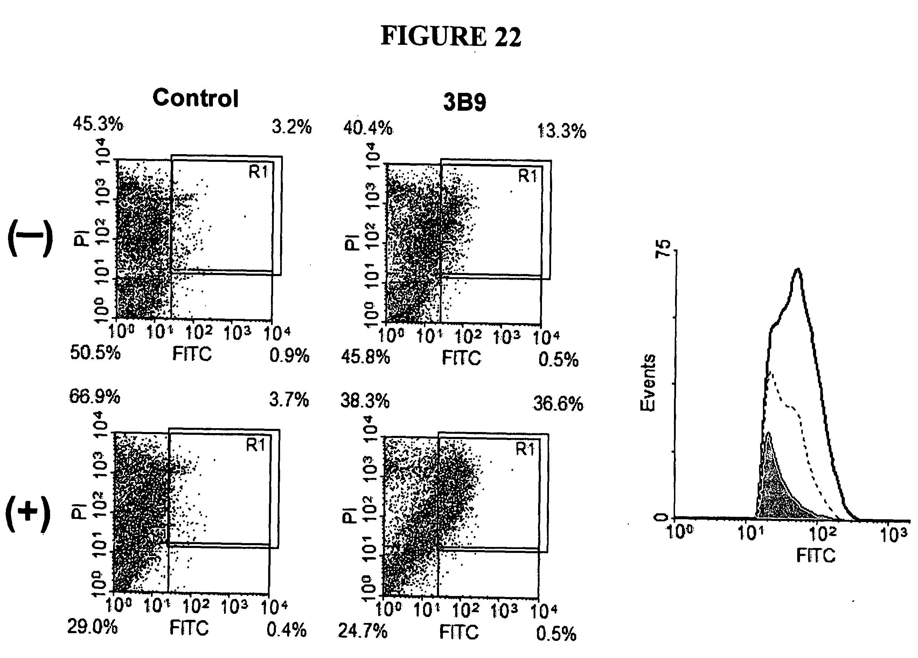

**Figure 22****:** depicts that anti-La mAb binds specifically to dead cells isolated from EL4 tumour explants and both proportionate and per cell binding increases after cytotoxic drug treatment. EL4 tumour-bearing mice (*n*=5) were treated or not with etoposide 67 mg/kg and cyclophosphamide 100 mg/kg by intraperitoneal injection on two occasions 24 hours apart. At 48 hours after the commencement of treatment, tumours were excised and single cell suspensions prepared. Cells were washed with PBS and incubated with 5 µg/mL FITC-conjugates of 3B9 or Sal5 isotype control. PI 0.5 µg/mL was added to evaluate cell viability and analysis performed using flow cytometry. Data shown are representative density plots and histogram overlays of FITC and PI emissions from stained cells that were isolated from EL4 tumour explants of mice treated (+; upper row of panels) or not (-; lower row of panels) with cytotoxic chemotherapy. Data depicted in the histogram overlays originated from region 1 (R1), which represents the FITC⁺ subset of PI⁺ events, and shows Sal5-FITC staining of tumour cells from chemotherapy-treated mice (grey fill), 3B9-FITC staining of tumour cells from untreated mice (dashed line) and chemotherapy-treated mice (thick line). After chemotherapy, the fraction of PI⁺ cells in tumor explants increased significantly from a mean (± SE) of 50 ± 2% to 70 ± 1% (*P* < 0.001). Similarly, the 3B9⁺ subset of PI⁺ cells increased significantly from 15 ± 1% to 38 ± 2% (*P* < 0.01) after chemotherapy, whereas isotype control staining was unaltered. Only the PI⁺ tumour subpopulation bound 3B9, which indicated that La was recognized specifically in dead tumour cells. Histogram analysis indicated that specific per cell binding of 3B9-FITC to PI⁺ cells was significantly augmented in tumors exposed *in vivo* to cytotoxic chemotherapy (net median fluorescence intensity ± SE of 18 ± 3 with chemotherapy and 1 ± 3 without chemotherapy, *P* < 0.05).

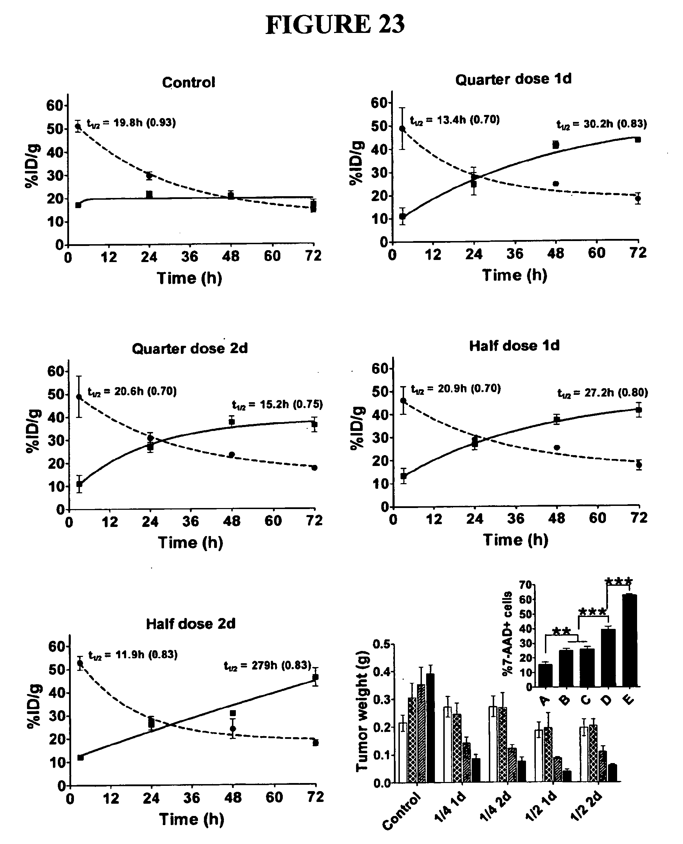

**Figure 23** depicts that time-activity curves of ¹¹¹In-DOTA-3B9 in EL-4 tumour-bearing mice treated or not with cytotoxic chemotherapy. EL4 tumour-bearing mice (*n*=*5*) were left untreated (control) or treated with cyclophosphamide and etoposide in four different regimens of escalating dose intensity. For each analysis time point after ¹¹¹In-DOTA-3B9 administration, data are expressed as percentage of organ radioactivity normalized to organ weight and divided by radioactivity of injected dose (%ID/g). Data are presented as mean ± SEM and time activity curves were fitted for blood (- -) and tumour (-). Half-life (t_{1/2}) values for blood clearance and tumour accumulation are displayed next to each fitted curve and regression values are shown in brackets. Lower right-hand panel: column graph showing tumour weights (g) from control and treated mice, which are expressed as mean ± SEM measured at 3h (clear), 24h (light hatched), 48h (dark hatched) and 72h (filled) post-injection. Inset: In separate experiments, single cell suspensions prepared from EL4 tumours (*n*=4) were analyzed by flow cytometry for 7-AAD binding at 48h post-injection for control mice (A) or mice treated with the following chemotherapy regimens: quarter dose 2d (B), half dose 1d (C), half dose 2d (D), and half dose 2d analyzed at 72h post-injection (E). Data are shown as mean ± SEM of percentage 7-AAD⁺ tumour cells. Statistical comparisons were made with control tumours.

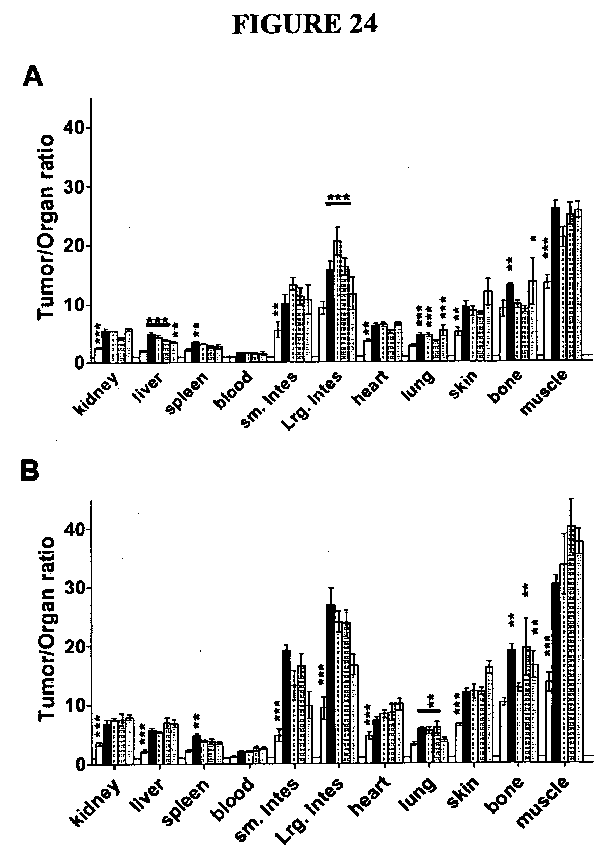

**Figure 24** depicts that tumour targeting efficiency of ¹¹¹In-DOTA-3B9 is enhanced by cytotoxic chemotherapy. The accumulation of ¹¹¹In-DOTA-3B9 in the tumour (%ID/g) measured at (A) 48h and (B) 72h was divided on that of other organs at these time points. The ratio is presented as the mean ± SEM from control mice (clear bars), mice treated with quarter dose 1d (black bars), quarter dose 2d (streaked bars), half dose 1d (dark filled bars) or half dose 2d (light filled bars). Asterisks (* *P<0.05*, ** *P<0.01* or *** *P<0.001*) denote significant differences in comparison to control mice. Solid line represent ratio of 1. Only tumour/muscle ratio was significantly different in chemotherapy treated mice at 24h after administration (*P<0.001*) (data not shown).

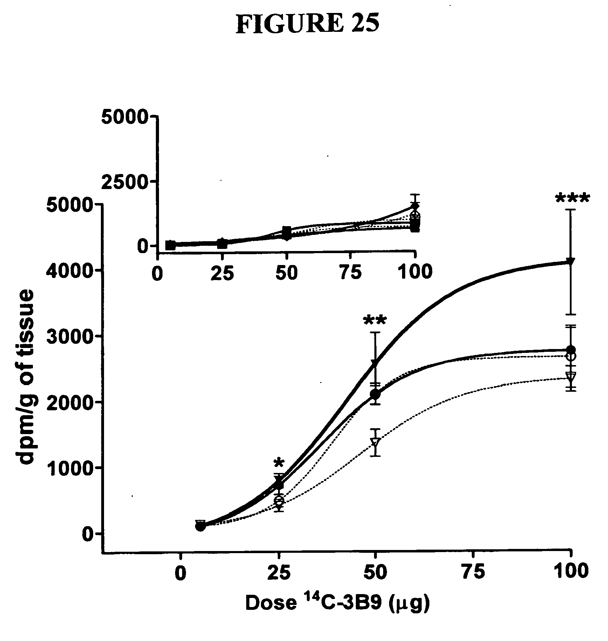

**Figure 25** depicts that the intra-tumoral accumulation of biosynthetically labelled La-specific ¹⁴C-3B9 mAb is dose-dependent and is augmented by cytotoxic chemotherapy. The biodistribution of different doses of ¹⁴C-labelled 3B9 was studied in EL4 tumour bearing mice with or without chemotherapy. ¹⁴C-3B9 accumulated significantly more only in the tumour with chemotherapy when its uptake approximately doubled at ¹⁴C-3B9 doses of 25 µg, 50 µg and 100 µg. Mice were given intravenous injections of 5,25, 50 or 100 µg of ¹⁴C-3B9 or ¹⁴C-Sal5 and then treated or not with etoposide 67 mg/kg and cyclophosphamide 100 mg/kg, which were given as two intraperitoneal injections 24 hours apart. Forty-eight hours after commencement of treatment, the mice were killed and organs collected for radioactivity measurement. Radioactivity was normalised to the mass of tissue counted (dpm/g of tissue) and tissues included (◆) liver, (■) spleen, (▲) kidneys, (●) serum and (▼) tumour. Data are presented as dpm/g ± SEM. Closed symbols, with chemotherapy; open symbols, without chemotherapy. Statistical comparisons were made using two-way analysis of variance, which was performed as a Bonferroni post-test comparison between all groups (*n*>4).

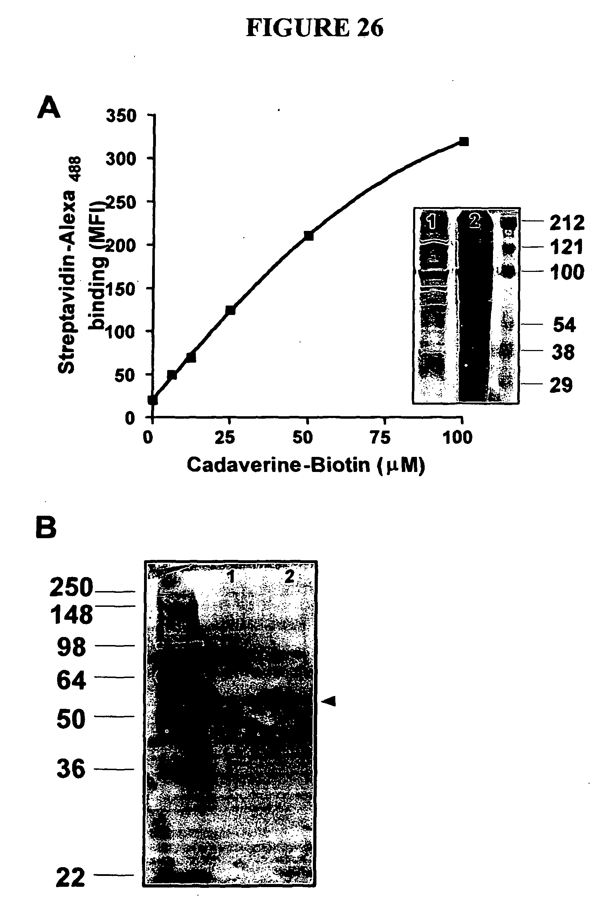

**Figure 26** depicts that incorporation of cadaverine-biotin into the target for Apomab™ during apoptosis. (**A**) Jurkat cells are induced to undergo apoptosis using 20µg/mL cisplatin for 48h with or without increasing concentrations of cadaverine-biotin. Cells are incubated with 2µg/mL of streptavidin-Alexa₄₈₈ for 20 min., washed, stained with 2µg/mL 7-AAD to assess viability and then analysed by flow cytometry. Data are presented as the MFI ± SEM from two independent assays. > 90% Jurkat cells are 7-AAD⁺ and Alexa₄₈₈ fluorescence emitted from 7-AAD⁺ cells is plotted as a function of cadaverine-biotin concentration. **Inset:** Iysates (12 µg) of cells incubated in the absence (lane 1) or the presence (lane 2) of 10µM cadaverine-biotin are fractionated on 12% SDS-PAGE. The gel is transferred to PVDF membrane and probed using streptavidin-AP. (**B**) Lysates of cells incubated in the absence (lane 1) or presence of 100µM cadaverine-biotin (lane 2) are first immunoprecipitated using Apomab™ before probing the immunoblots with streptavidin-AP. Arrow head, position of putative La band in lane 2.

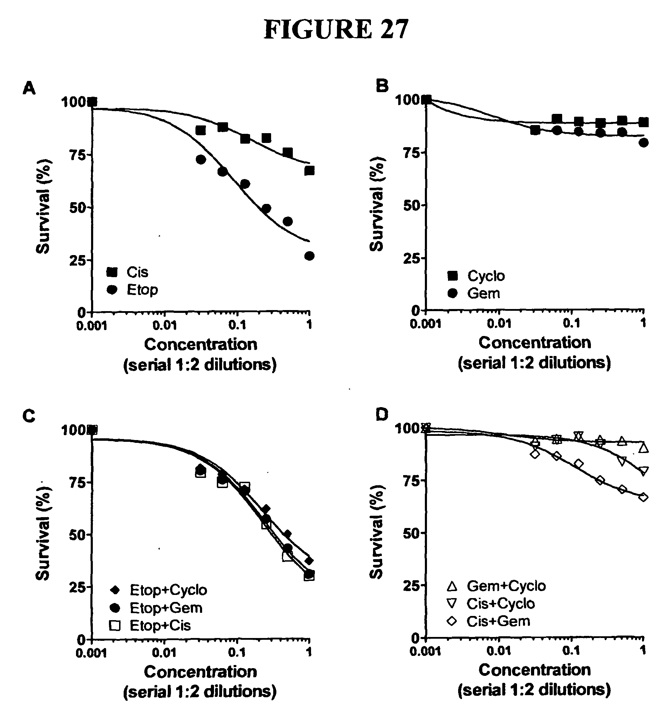

**Figure 27** depicts that different cytotoxic drugs differentially impair survival of primary ALL blasts. ALL blasts were cultured in the presence or absence of serial 1:2 dilutions of **A.** 40 µg/mL etoposide, or 20 µg/mL cisplatin, or **B.** 200 µg/mL gemcitabine, or 20 µg/mL cyclophosphamide, or **C.** and **D.** combinations of these drugs at the same concentrations also in serial dilution. The percentage of viable ALL blasts (7-AAD-negative) in medium without incubation of drug was 66.3%, which was normalised to 100% survival for comparing the effects of drug treatment on survival of ALL blasts. Sigmoidal dose-response curves were fitted to the data using GraphPad Prism v.4.0 software.

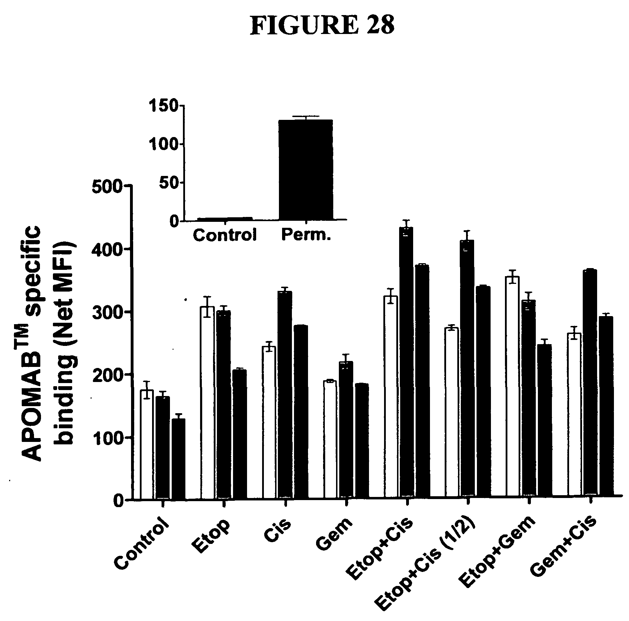

**Figure 28** depicts that Apomab binds specifically to 7-AAD⁺ primary ALL blasts after treatment with cytotoxic drugs *in vitro.* Cells were incubated in the absence (control) or presence of 40 µg/mL etoposide (Etop), 20 µg/mL cisplatin (Cis), 200 µg/mL gemcitabine (Gem), 40 µg/mL etoposide and 20 µg/mL cisplatin (Etop+Cis), 20 µg/mL etoposide and 10 µg/mL cisplatin (Etop+Cis [1/2]), 40 µg/mL etoposide and 200 µg/mL gemcitabine (Etop+Gem), and 200 µg/mL gemcitabine and 20 µg/mL cisplatin (Gem+Cis). 7-AAD⁺ events were gated and specific Apomab binding was determined as the net MFI after subtraction of the net MFI value for the Sal5 isotype control mAb. Columns, net MFI; bars, SE; (*n*=2). Apomab-specific binding was analysed at 24h (clear columns), 48h (grey columns) and 72h (black columns) after cells were incubated with cytotoxic drugs. Inset, Apomab-specific binding to viable ALL blasts, which were left untreated (control) or permeabilised.

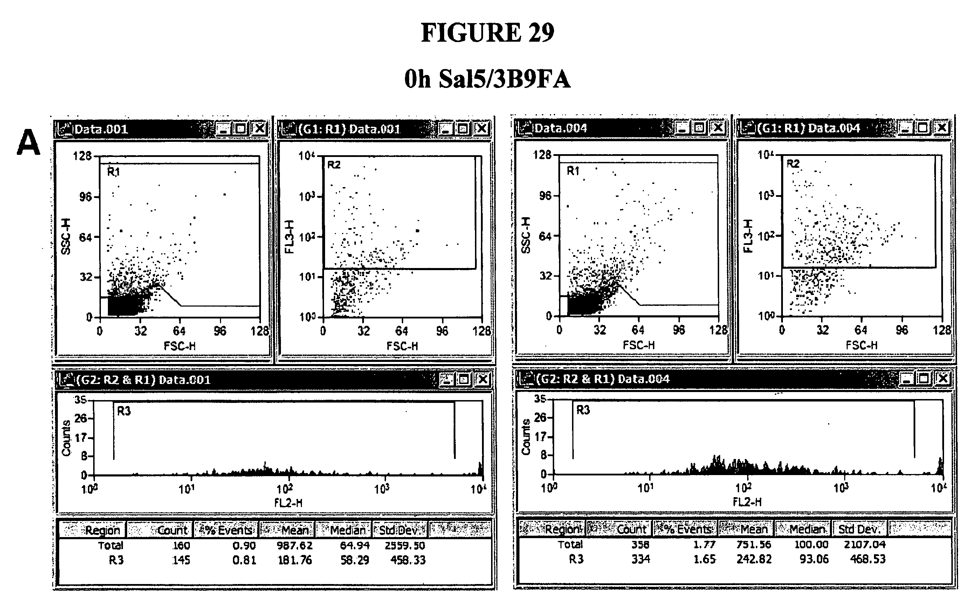

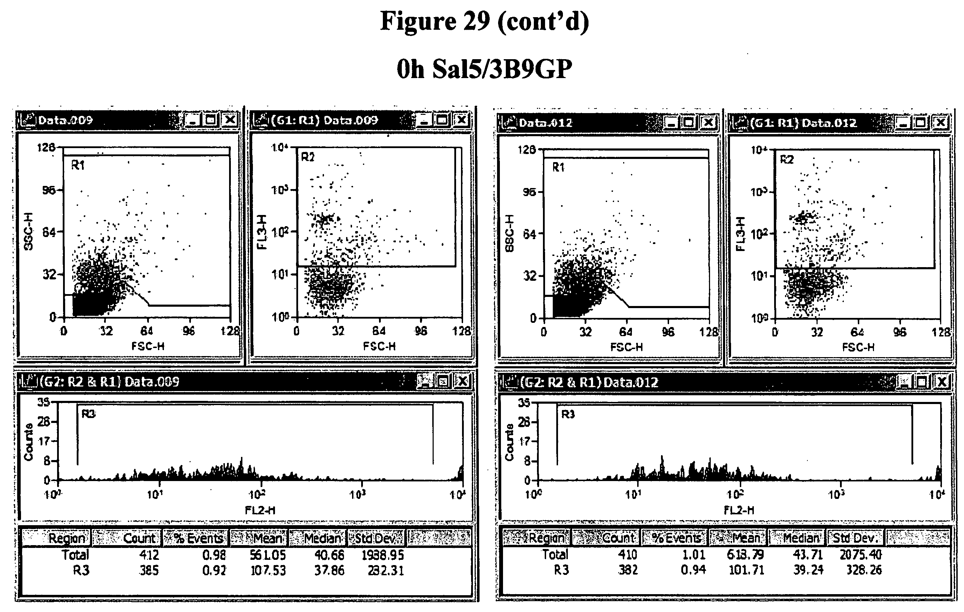

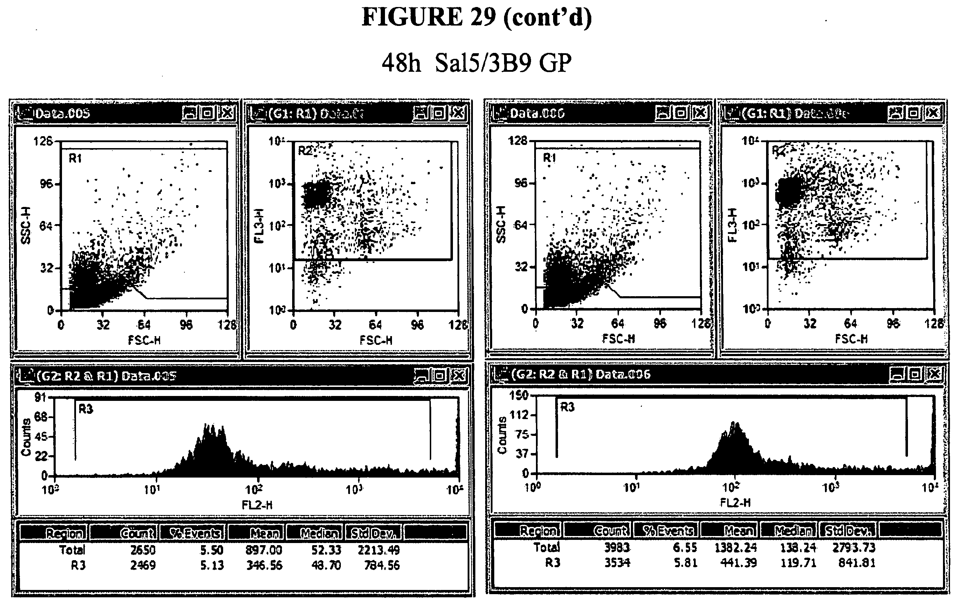

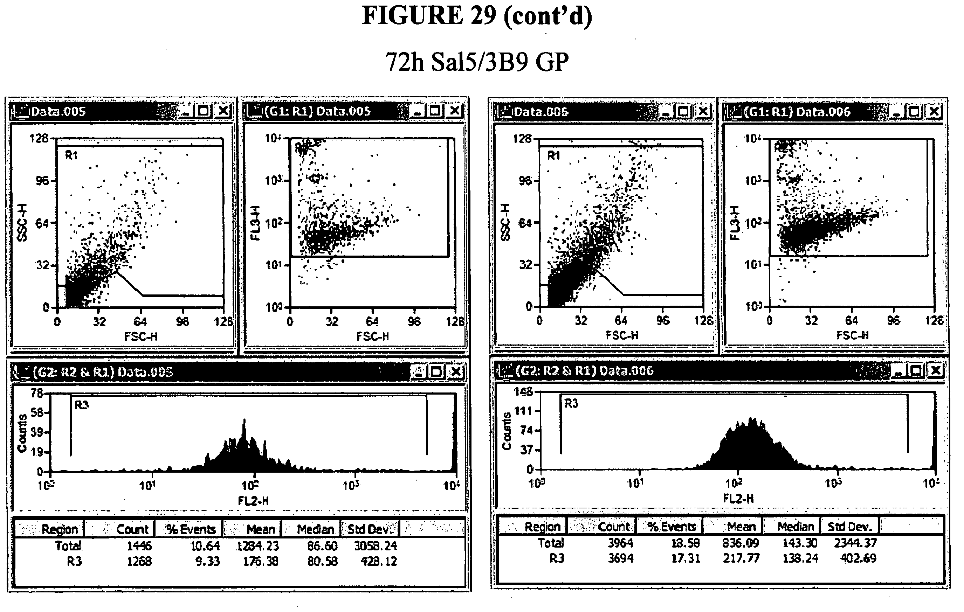

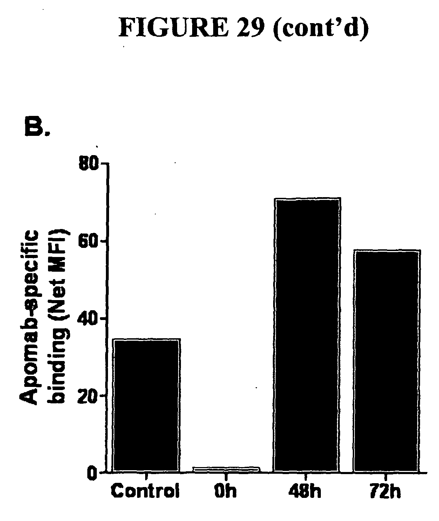

**Figure 29** depicts that Apomab binds to BerEP4-enriched cells from an SCLC patient particularly after cytotoxic chemotherapy was administered to the patient. Blood samples (2.5 mL) collected in heparinised tubes were enriched for circulating epithelial cells using the BerEP4 epithelial marker using the CELLection™ Epithelial Enrich kit as per manufacturer instructions (Dynal^{®} Biotech, Invitrogen, USA). Briefly, 250 µL of BerEP4-beads was mixed with the blood sample for 30 min at RT. Beads were bound to Dynal^{®} magnet and washed 5 times with PBS. Bead-bound cells were released using PBS containing 0.1 % BSA and DNase I as described by the manufacturer. Beads were bound to Dynal^{®} magnet and released cells were removed to a fresh tube, centrifuged and washed with PBS. Samples were incubated (30 min at RT) with anti-mouse IgG (2 µg/mL) to block the mouse BerEP4 antibody remaining on the isolated cells. Samples were washed with PBS and aliquots were stained with Apomab or matching irrelevant isotype (Sal5). Finally, cells were washed with PBS and analysed by flow cytometry after incubation (15 min) with 2 µg/mL 7-AAD at RT. **A.** FSC vs.SSC density plots were constructed for all samples. A region (R1) was drawn based on control blood to exclude events related to normal blood cells. Density plots of FSC *vs.* FL-3 (7-AAD) were constructed from gate Rl. Viable cells are shown as red and dead cells as green. A second region (R2) was constructed to select dead (7-AAD⁺) cells. FL-2 histograms of Sal5 (left) and 3B9 (right) staining are shown in green and are gated on 7-AAD⁺ cells. **B.** A summary column graph shows Apomab-specific binding as net median fluorescence intensity (MFI) (after subtraction of MFI values for Sal5 staining). *Notes:* (i) A 24h time point is not shown because bead separation failed. (ii) In the case of 48 and 72 h samples, a population with very high fluorescence was observed and was not different between control and positive staining. Therefore, an R3 region was drawn to exclude these populations from the histograms.

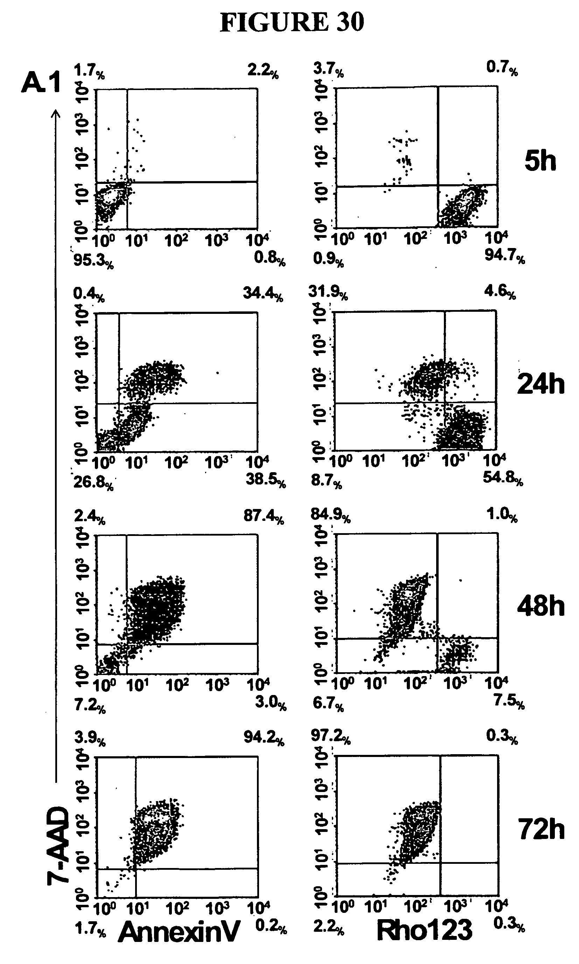

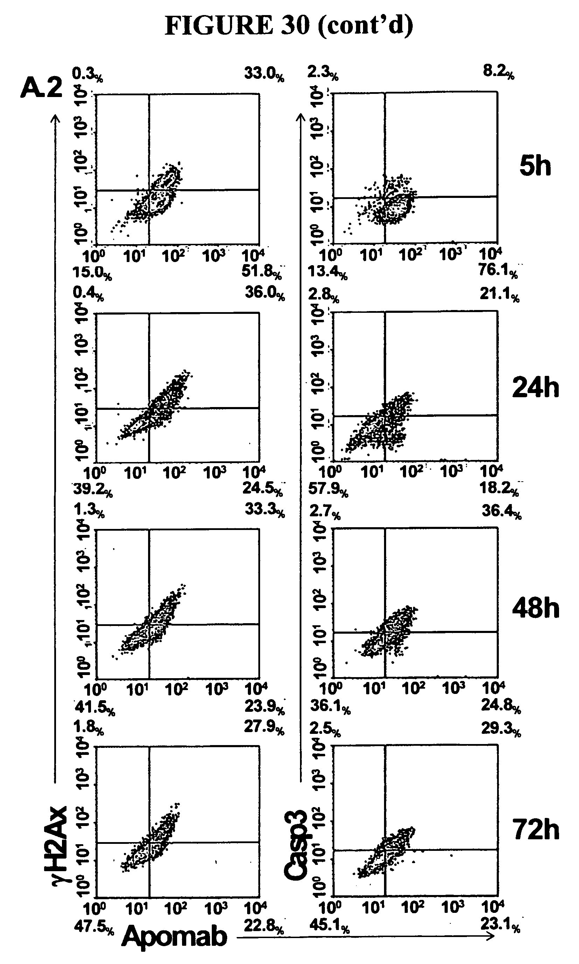

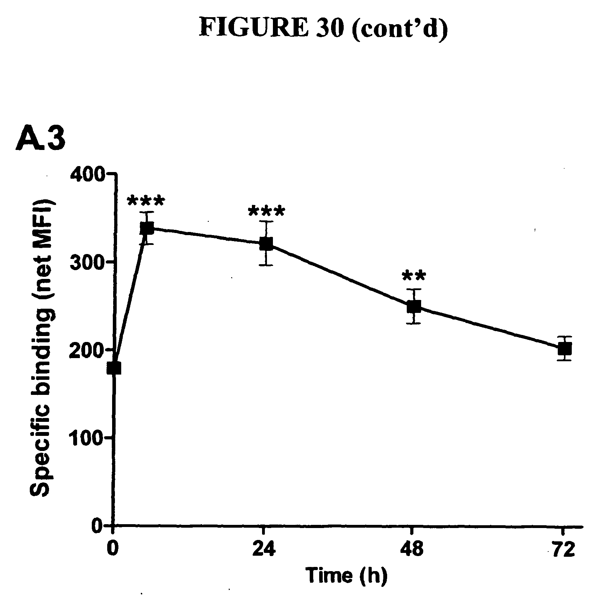

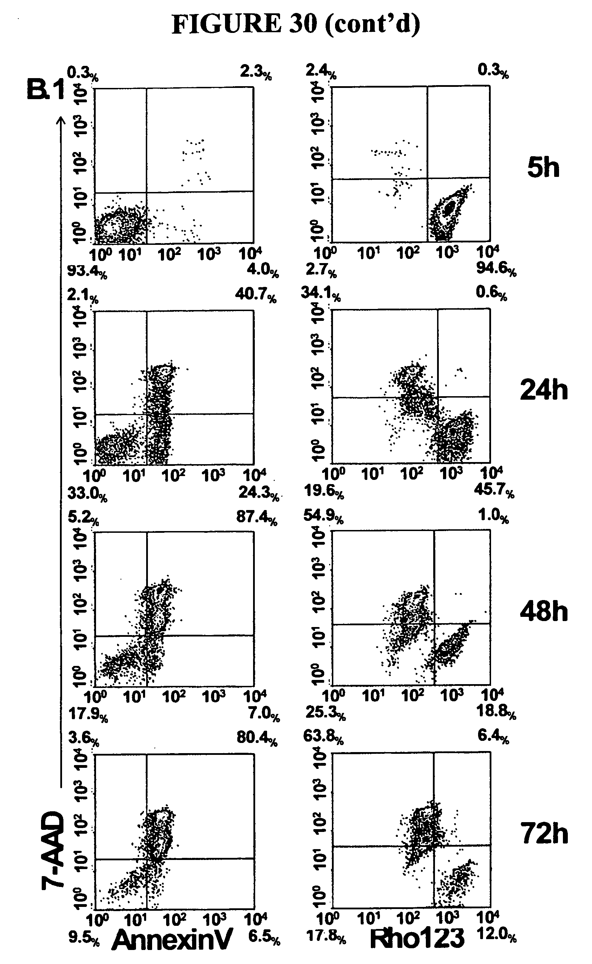

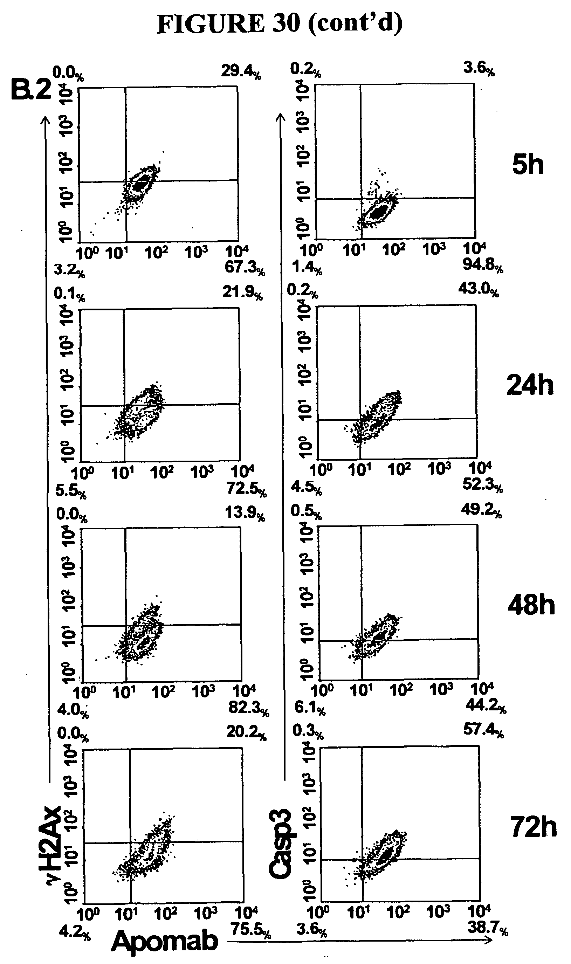

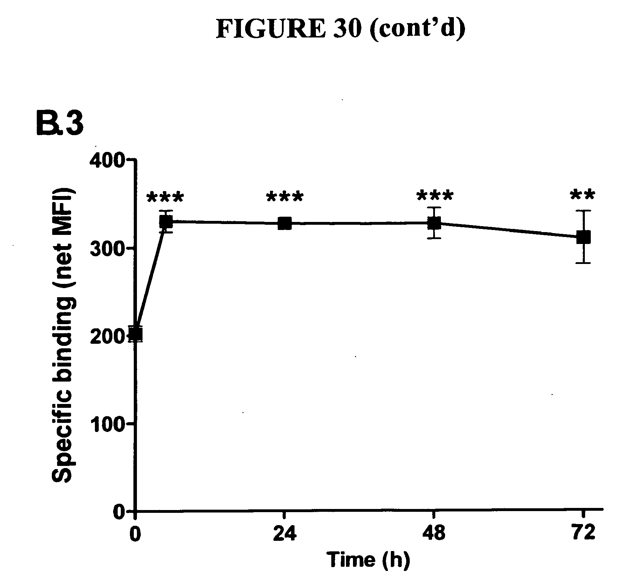

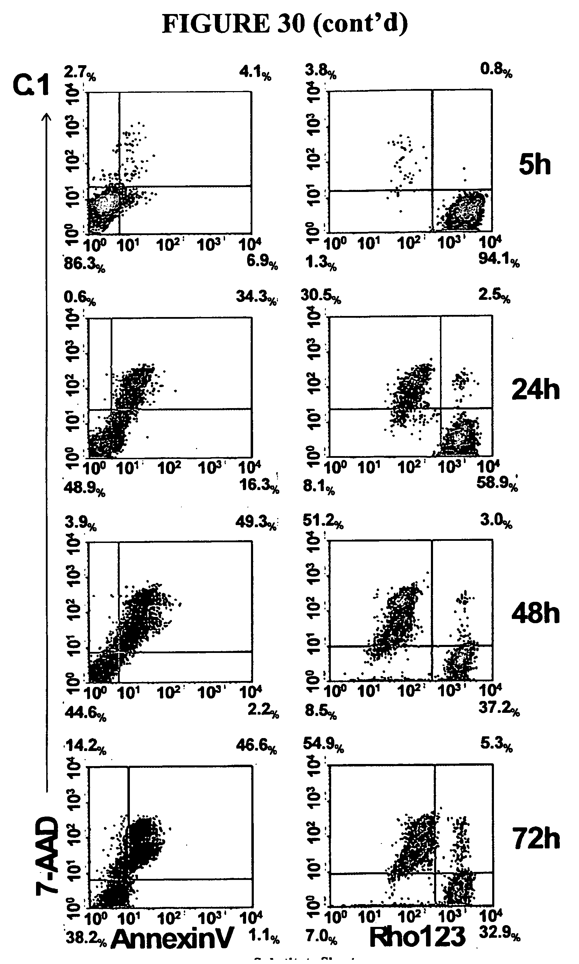

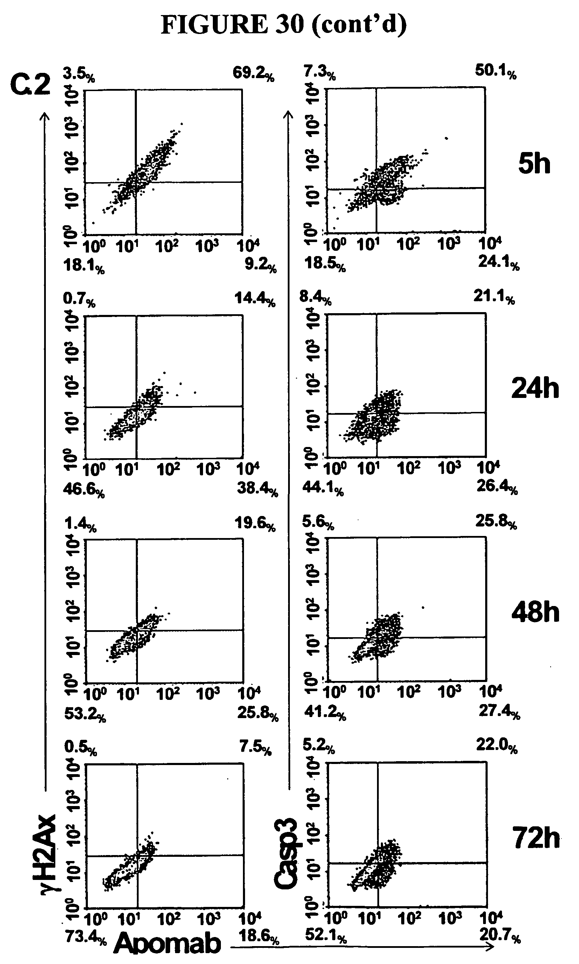

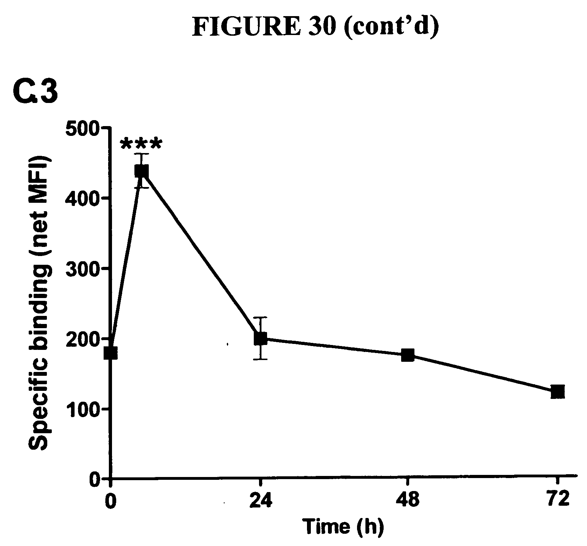

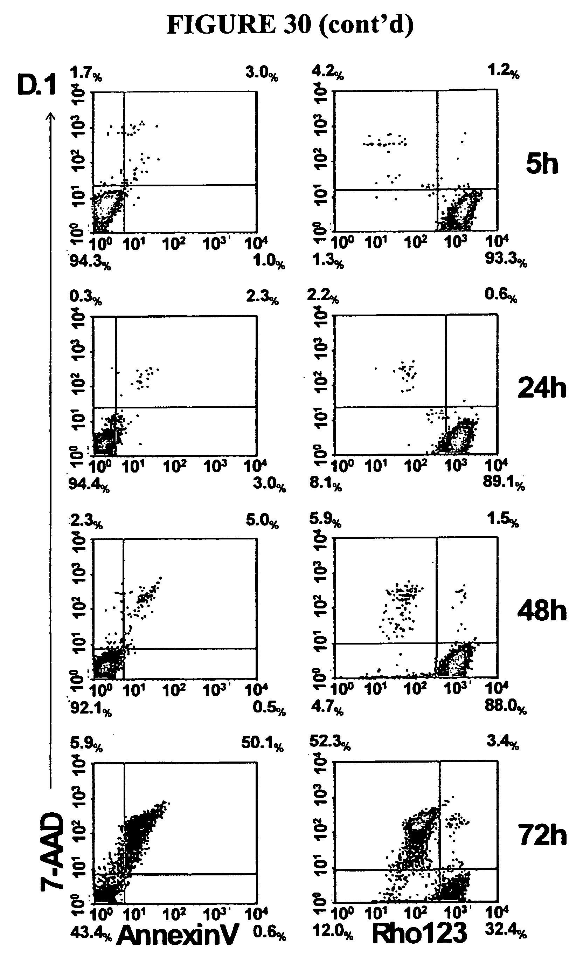

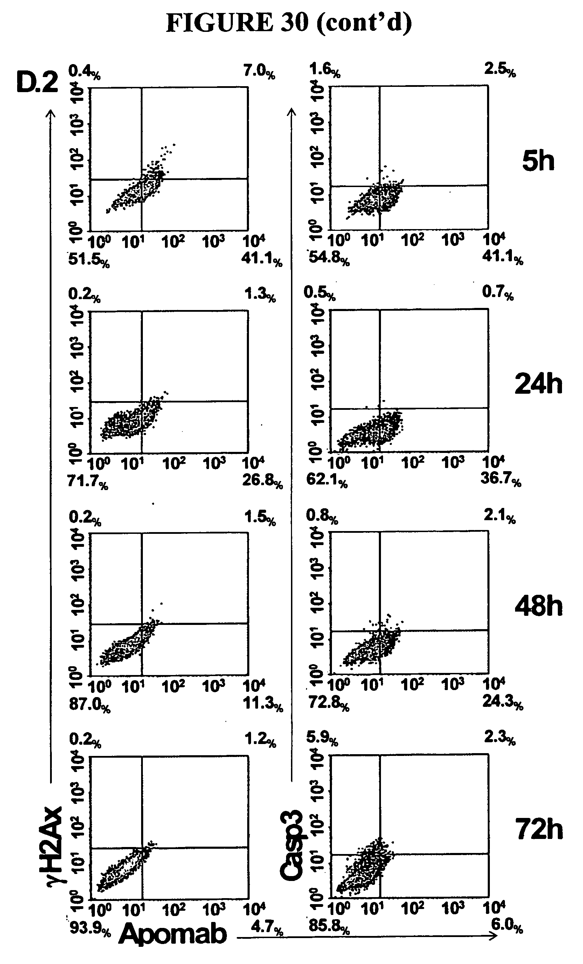

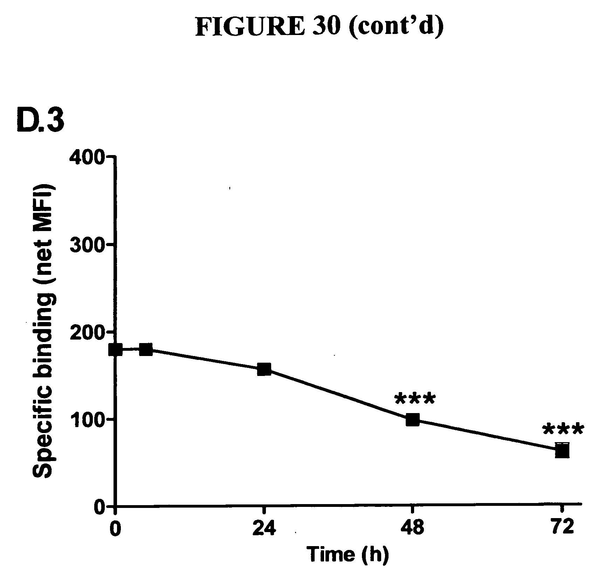

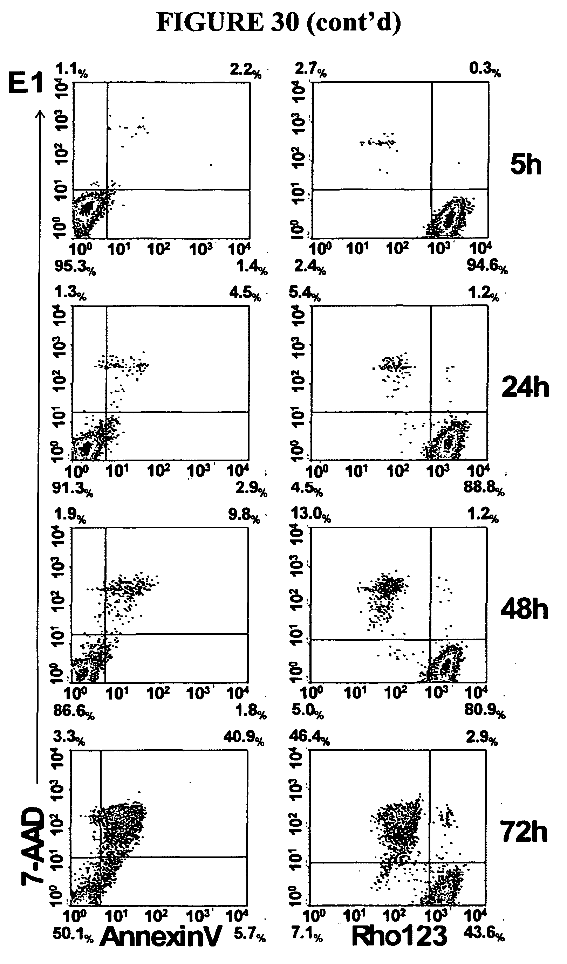

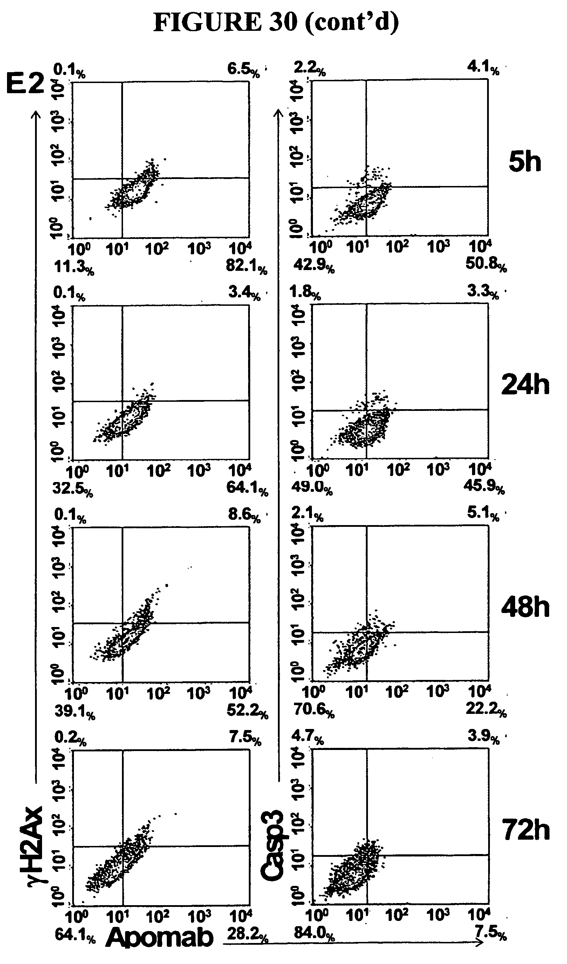

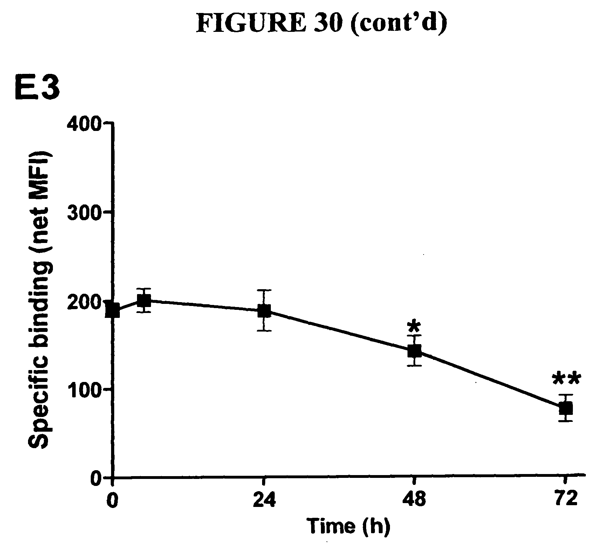

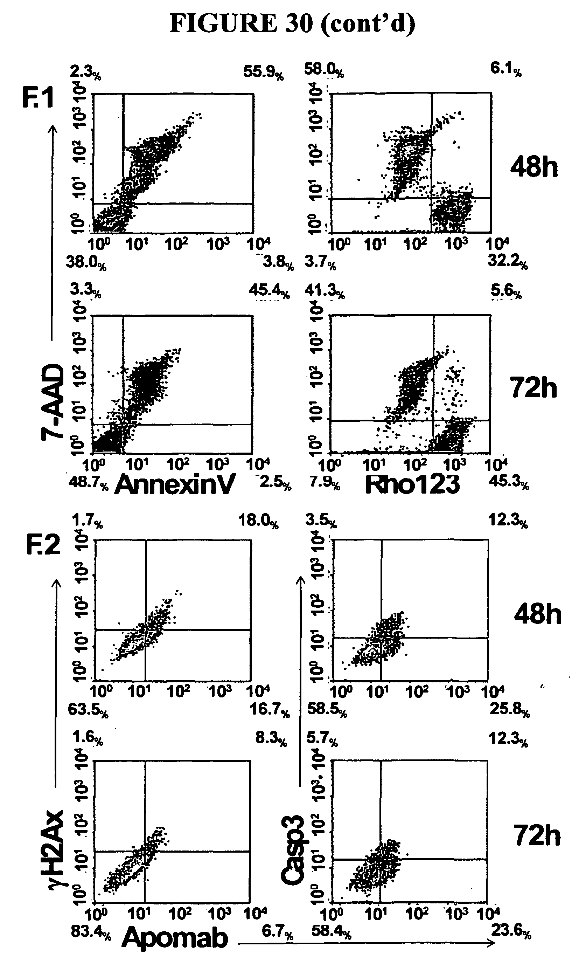

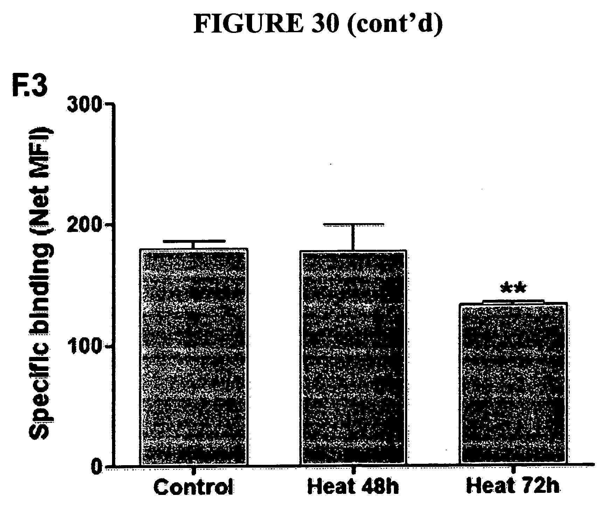

**Figure 30** depicts that activators of the intrinsic apoptotic pathway extends the kinetics of Apomab binding compared with extrinsic apoptotic pathway activators. Jurkat cells were treated with stimuli that induced apoptosis *via* mainly either the intrinsic pathway using **A.** cisplatin 20 µg/mL or **B.** γ -radiation 15 Gy, or the extrinsic pathway using **C.** anti-Fas mAb 250 ng/mL. In addition, Jurkat cells were stressed by either **D.** allowing overgrowth in the culture medium or **E.** serum withdrawal. **F.** Necrosis was induced by heat treatment. Aliquots of the Jurkat cell cultures were removed at the indicated time points and stained with annexin **V**, rhodamine 123 or 7-AAD without fixation or permeabilisation, or were fixed and permeabilised and then stained with either Sal5 or 3B9 and γH2AX, or Sal5 or 3B9 and anti-activated caspase-3 mAb.**, *P*<0.01; ***, *P*<0.001;

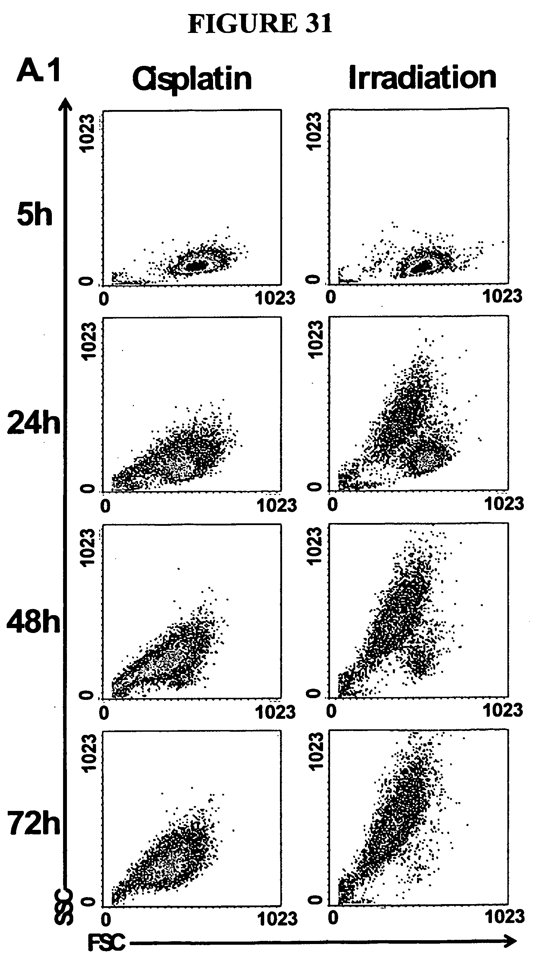

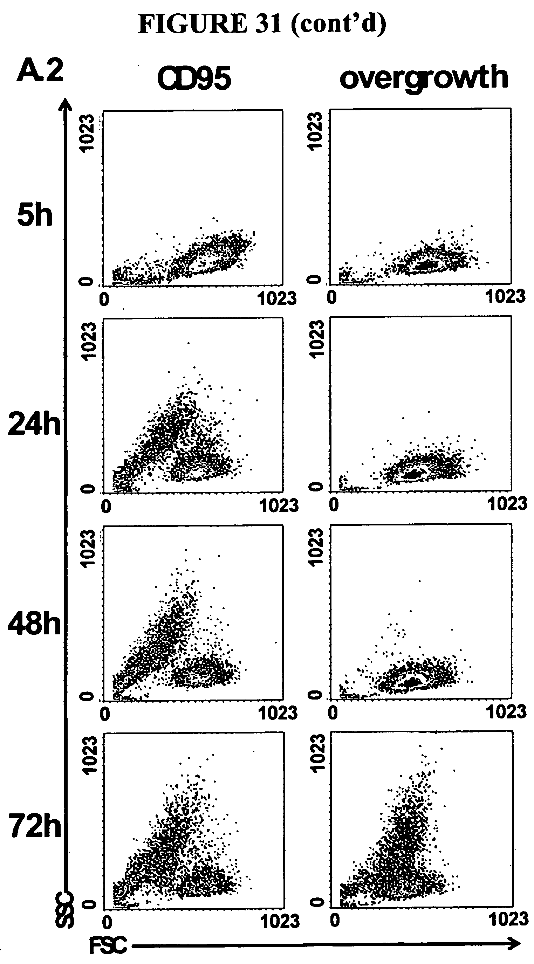

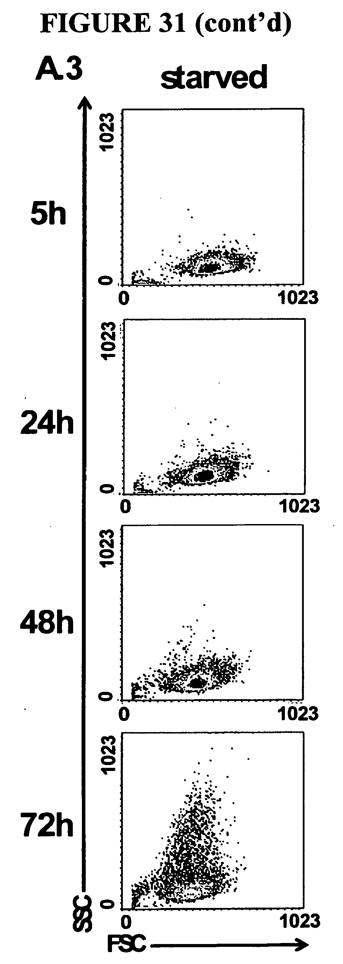

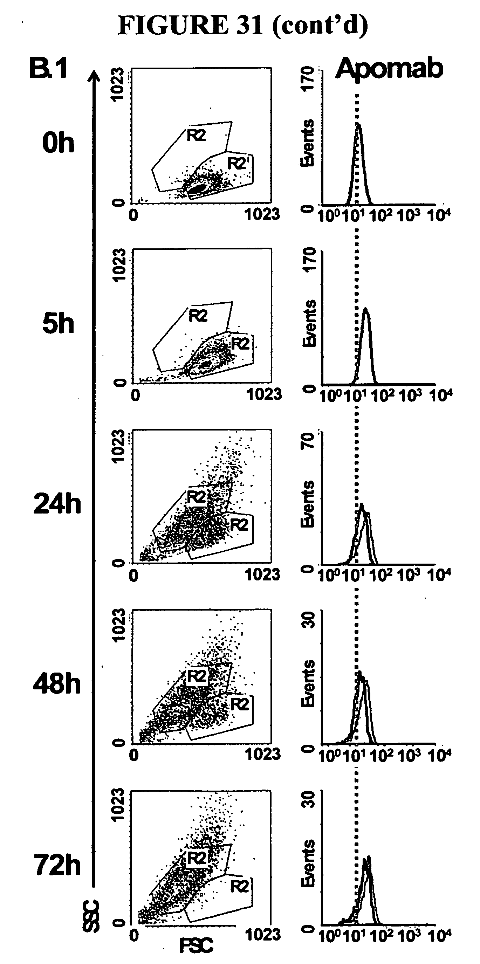

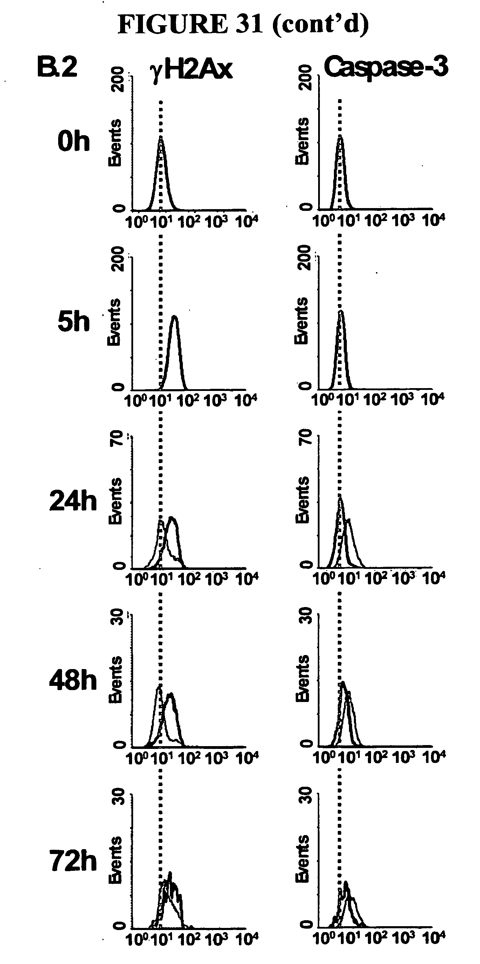

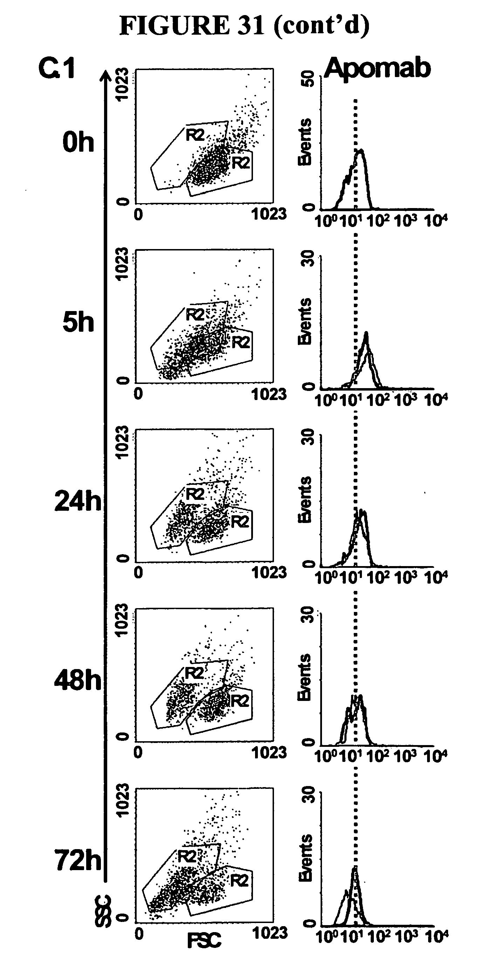

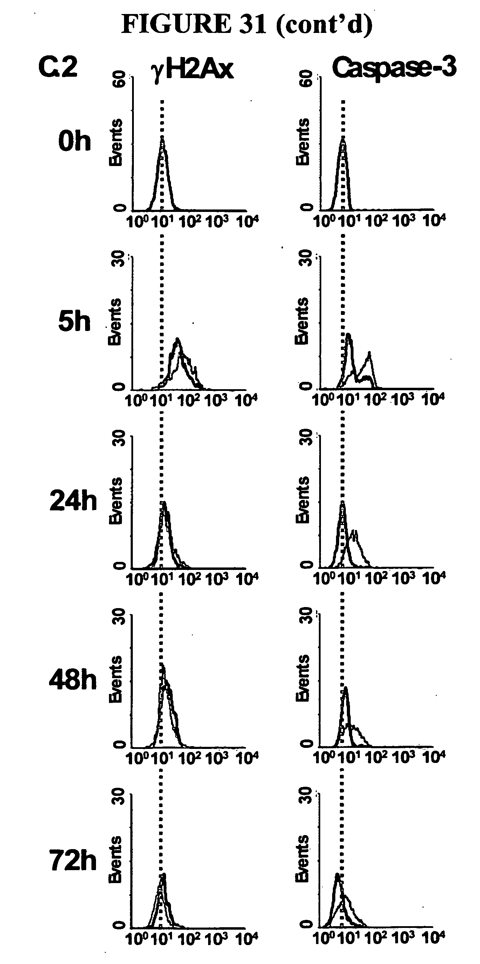

**Figure 31** depicts that γ-radiation and Fas ligation induce apobody formation with different kinetics and different patterns of γH2AX and La induction. **A.** Scatter plots are shown for each of the experimental conditions indicated in Fig. 4. **B.** Jurkat cells were γ-irradiated to a dose of 15 Gy, or treated with 250 ng/mL anti-Fas (CD95) mAb. At the specified time points, cells were fixed and permeabilised and stained with either Apomab and anti-γH2AX or Apomab and anti-activated caspase-3. Gates R1 and R2 were drawn from FSC vs. SSC plots and Apomab, γH2AX or activated caspase-3⁺ staining are shown for the different gates (R1, black; R2, red)

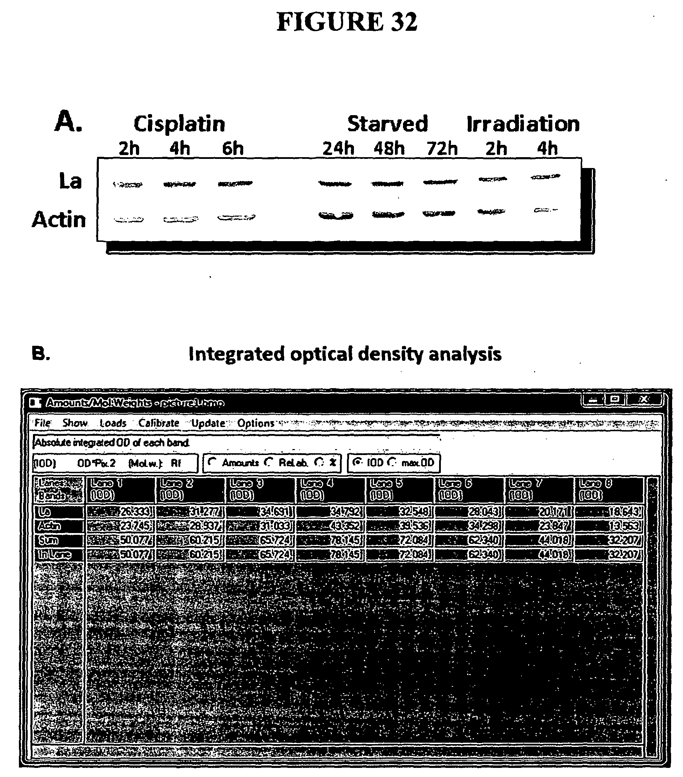

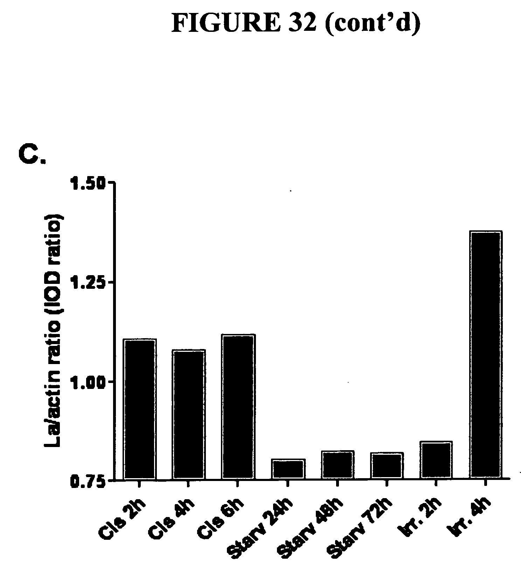

**Figure 32** depicts that DNA-damaging treatment induces early expression of La protein in Jurkat cells *in vitro.* **A.** Lysates of cultured Jurkat cells were prepared at the indicated time points after treatment or not and electrophoresed by SDS-PAGE. After transfer, the blots were probed with Apomab or anti-actin antibodies, and **B.** integrated optical density analysis performed. **C.** The results of this analysis are shown in a column graph.

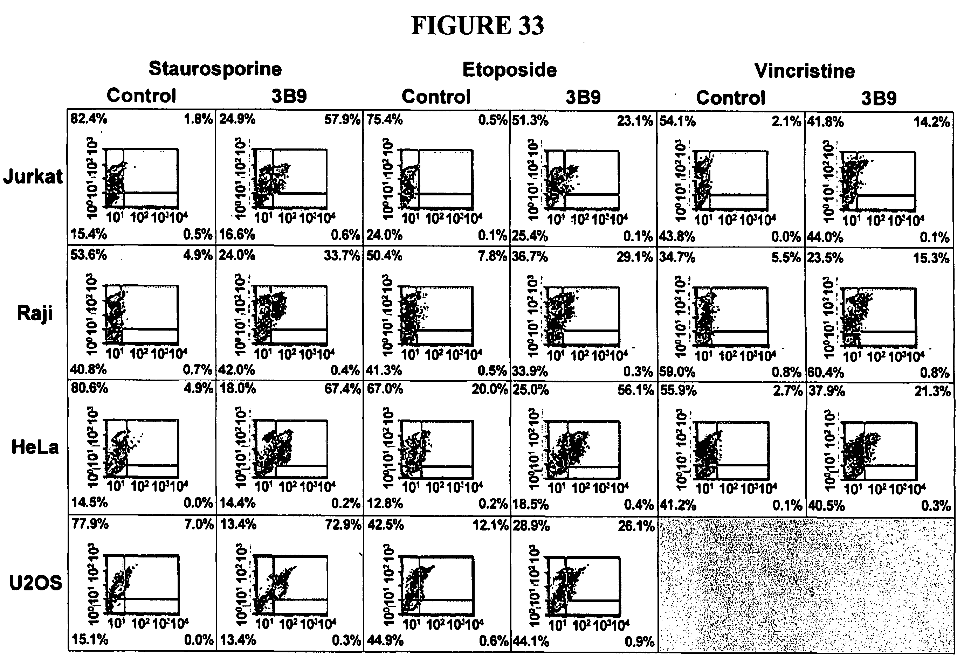

**Figure 33** depicts that cytotoxic drugs induce binding of 3B9 (Apomab) to malignant human cell lines after cell death *in vitro.* Staining with propidium iodide (PI) is indicated on the γ-axis. Jurkat, T cell leukemia; Raji, B cell leukemia; HeLa, cervical carcinoma; U2OS, osteosarcoma.

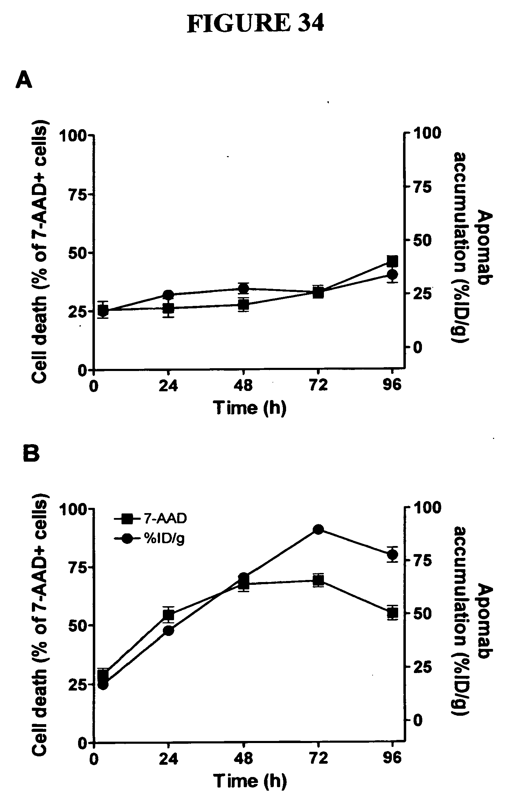

**Figure 34** depicts that after CE chemotherapy, tumour Apomab accumulation *in vivo* is disproportionately higher than the tumour cell death rate, which suggests that the La target antigen is induced by DNA-damaging chemotherapy. EL4 tumour cell death and Apomab accumulation were analysed at the indicated time points in A. untreated control and **B.** treated mice (*n*=3). Apomab accumulation was calculated from ¹¹¹In-Apomab accumulation after accounting for radioactive decay of ¹¹¹Indium. Data points, mean %7-AAD⁺ cells (left Y-axis, squares) and mean %ID/g Apomab (right Y-axis, circles); bars, SE.

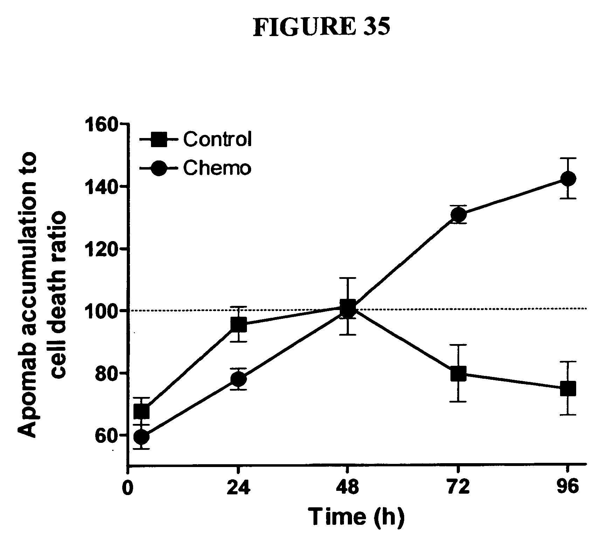

**Figure 35** depicts that after CE chemotherapy, the ratio of tumour Apomab accumulation to tumour cell death declines in control tumours and is enhanced in treated tumours, which suggests that the La target antigen is induced by DNA-damaging chemotherapy. Tumour Apomab accumulation (%ID/g) was directly related to tumour cell death (%7-AAD⁺ cells) at each of the indicated time points for EL4 tumours derived from control or treated mice (n=3). Data points, mean ratio; bars, SE; control (squares); chemotherapy (circles); dotted line, 100%.

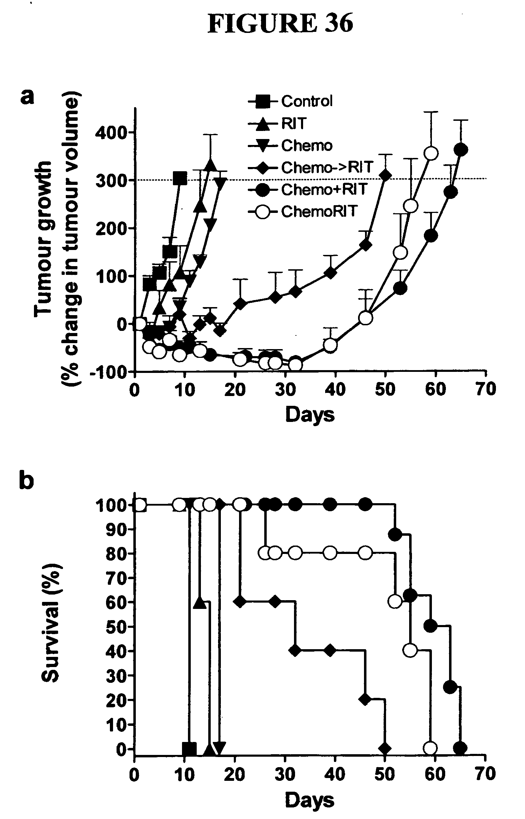

**Figure 36** Depisites that chemotherapy and RIT combination is synergetic against human prostate carcinoma models. Balb/c nude mice were injected subcutaneously in the right flanks with 5x10⁶ LnCap cells prepared in matrigel: PBS (50:50) solution. Mice bearing LnCap tumors (~100mm³ in volume) were left untreated (control), treated with chemotherapy alone (50mg/kg gemcitabine on day 1, 4, 7 and 10 and 0.5mg/kg cisplatin on day 1 and 7) or treated with 2.50MBq of ⁹⁰Y-DOTA-3B9 on day 1 alone (RIT). The following combination treatments were used; (1) Chemo→RIT: 50mg/kg gemcitabine on day 1, 4, 7 and 10 and 0.5mg/kg cisplatin on day I and 7 with 2.50MBq ⁹⁰Y-DOTA-3B9 on day8, (2) Chemo+RIT: 50mg/kg gemcitabine on day 1, 4 and 7 and 0.5mg/kg cisplatin on day 1 and 7 with 2.50MBq ⁹⁰Y-DOTA-3B9 on day2 or (3) ChemoRIT: 50mg/kg gemcitabine on day 1, 4 and 7 and 0.5mg/kg cisplatin on day 1 and 7 with 2.50MBq ⁹⁰Y-DOTA-3B9 on day 1 and 4. Tumor volume was calculated from tumor dimensions and the average change in tumor volume±SEM (n>5 mice per group) is shown in panel (a). Survival curves in panels (b) was based on tumor reaching maximum size except for one mouse which showed irreversible weight loss which belonged to LnCap tumor bearing mice treated with chemoRIT combination.

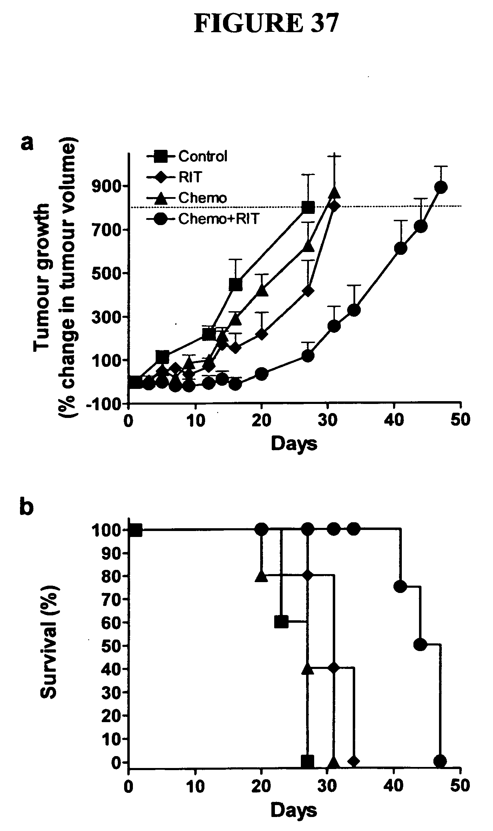

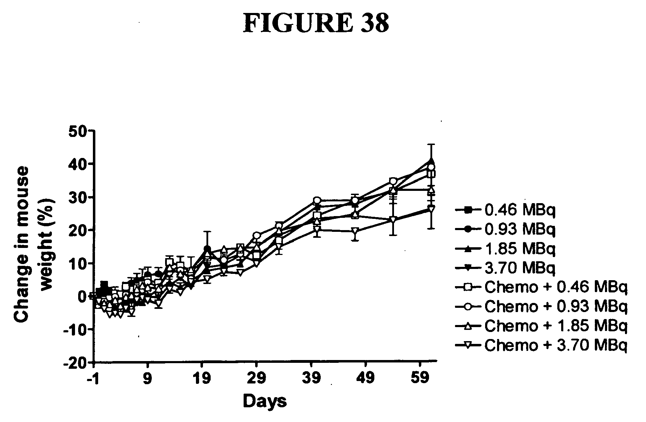

**Figure 37** Depisites that chemotherapy and RIT combination is synergetic against human pancreatic carcinoma models. Balb/c nude mice were injected subcutaneously in the right flanks with 5x10⁶ Panel cells prepared in matrigel: PBS (50:50) solution. Mice bearing Mice bearing Panc-1 tumors (~55mm³ in volume) were left untreated (control), treated with chemotherapy alone (50mg/kg gemcitabine on day 1, 4, and 7 and 0.5mg/kg cisplatin on day 1 and 7), treated with 2.50MBq of ⁹⁰Y-DOTA-3B9 on day 1 alone (RIT) or combination of chemotherapy as above with RIT with 2.5MBq of ⁹⁰Y-DOTA-3B9 on day2. Tumor volume was calculated from tumor dimensions and the average change in tumor volume±SEM (n>5 mice per group) is shown in panel (a). Survival curves in panels (b) was based on tumor reaching maximum size. **Figure 38** depicts that change of mouse weight in mono-RIT and combination of chemotherapy and RIT treatment of EL4-tumour bearing mice. Groups of 5 mice each treated with 19mg/kg etoposide and 25mg/kg cyclophosphamide 24h before injection of escalating doses of 90Y-Apomab. Groups of 5 mice each were not treated with chemotherapy received matched escalating doses of 90Y-Apomab. Mice were weighed daily and percentage change was calculated at each time point as the difference in weight compared to initial weight and divided on the initial weight. Data shown is the mean + SEM (n=5).

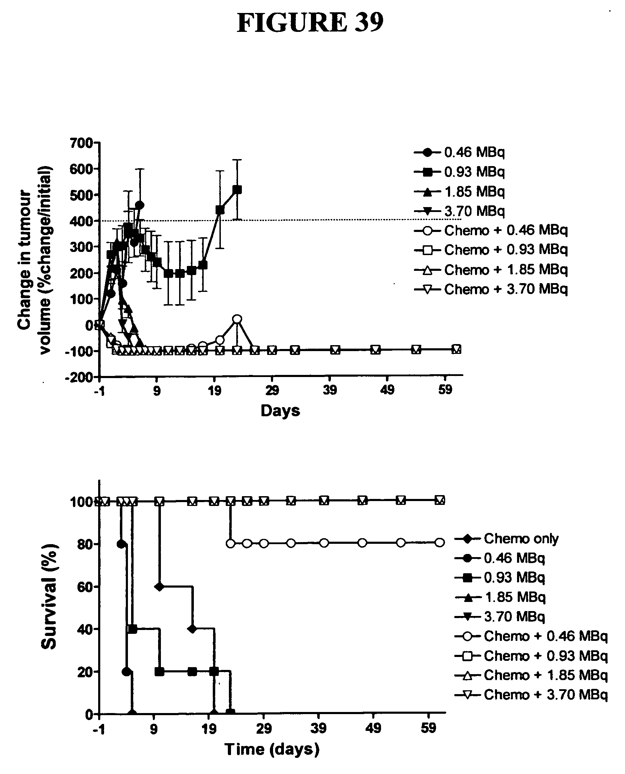

**Figure 39** depicts that combination of chemotherapy and RIT cured EL4-tumour bearing mice. Groups of 5 mice each were treated as described above and group of 5 mice treated with chemotherapy alone. (Top) Tumour volume was measured at the specified time points and percentage change was calculated at each time point as the difference in tumour volume compared to initial volume and divided on the initial volume. Data shown is the mean + SEM (n=5). (Bottom) Survival curves were constructed based on based on tumor reaching maximum size.

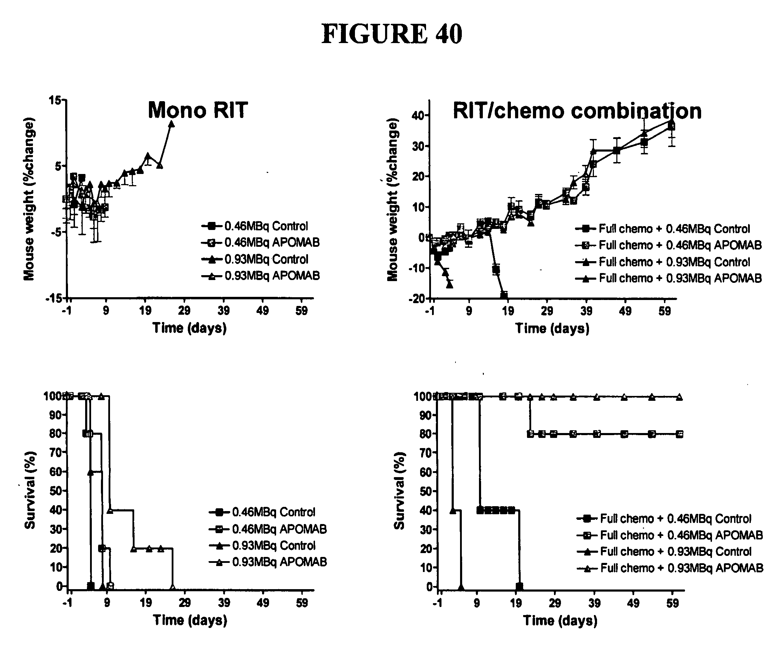

**Figure 40** depicits that therapeutic effect of 90Y-Apomab alone or in combination with chemotherapy is specific. Groups of 5 mice each that bear EL4 tumours were treated with 90Y-Apomab alone or with matched doses of 90Y-labelled control antibody (MonoRIT). Doses of 90Y-Apomab or 90Y-Sal5 were also combined with etoposide and cyclophosphamide chemotherapy (RIT/chemotherapy combination). Top panels show percentage change in mouse weight and bottom panels show survival curves constructed based on tumors reaching maximum size except in the case of 90Y-control combination with chemotherapy where mice were killed due to irreversible toxicity (>15% weight loss).

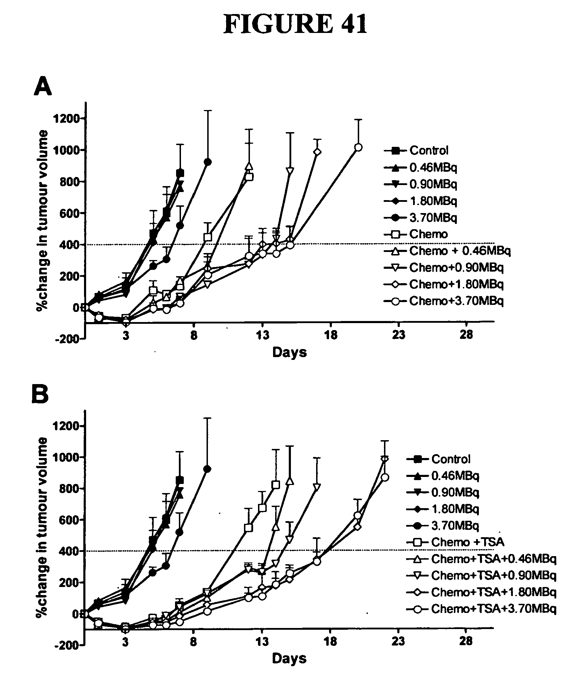

**Figure 41** depicts that combination of chemotherapy and radioimmunotherapy are synergetic against Lewis Lung carcinoma. B6 mice bearing 51±6mm3 subcutaneous Lewis Lung carcinoma in groups of 5 mice each were left untreated (control) or treated with Gem/Cis 2d chemotherapy (Chemo) alone, Gem/Cis/TSA 2d (Chemo+TSA) alone, escalating doses of ⁹⁰Y-Apomab alone or combinations of Chemo or Chemo+TSA and escalating doses of ⁹⁰Y-Apomab. Chemotherapy was administered by i.v. injections on day 0 and 1 while ⁹⁰Y-Apomab was administered by i.v. injection on day 2. Tumours were monitored for volume change measured as the percentage of the difference in tumour volume compared to that on day 0 and divided on the tumour volume on day 0 (initial volume). Data shown are the mean±SEM for %change in tumour volume. Mice were killed when tumours reached 500mm3 volume (corresponding to ~800% change in tumour volume compared to initial volume). (A) combinations with Gem/Cis 2d (chemo); (B) combinations with Gem/Cis 2d (chemo) and TSA. Dotted line represents 50% of the 800% end point (i.e. 400%).

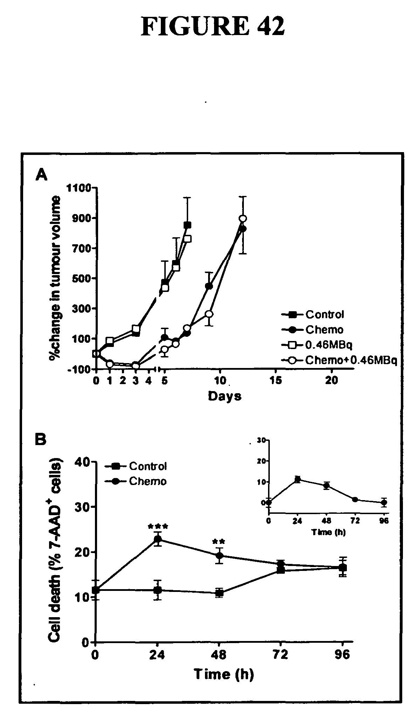

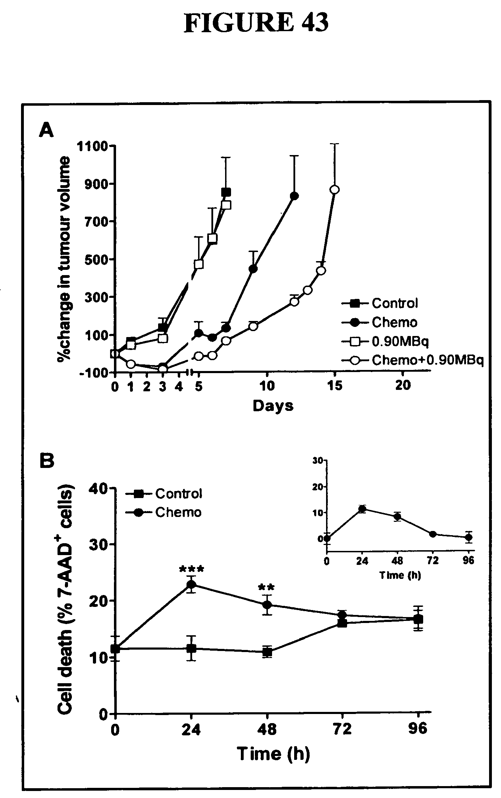

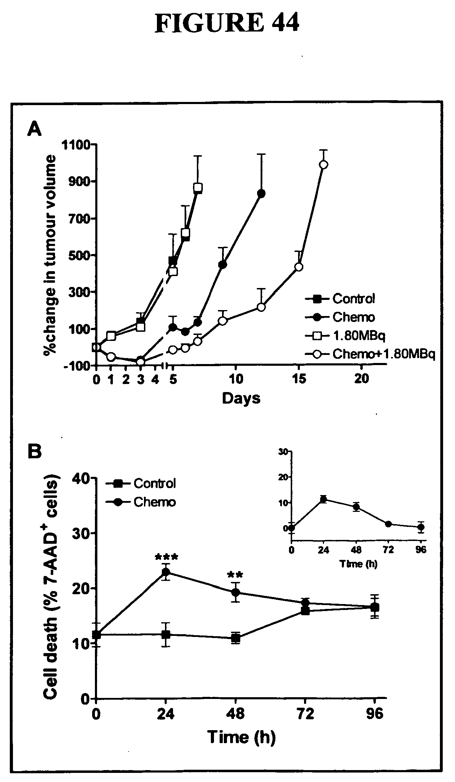

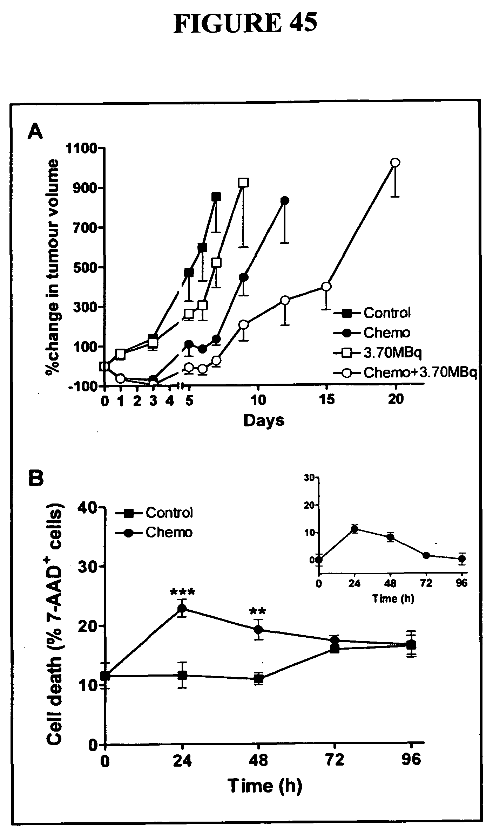

**Figures 42-45** depicts that radiosensitising chemotherapy augments carcinoma-inhibitory effects of La-directed RIT. The upper panel (**A**) in each figure shows growth curves for tumours of control and treated mice. Treatments included chemotherapy alone, radioimmunotherapy (RIT) alone, or in combined with chemotherapy at RIT doses of 1. 0.46 Gy, **2.** 0.90 Gy, **3.** 1.80 Gy, or **4.** 3.70 Gy per mouse. The lower panel (**B**) in each figure shows kinetic curves of the dead cell content (7-AAD⁺) of tumours from mice left untreated (control, filled squares) or treated (chemo, filled circles). **Inset,** tumour cell death due to chemotherapy (after subtraction of control values). **, *P*<0.01; ***, *P*<0.001.

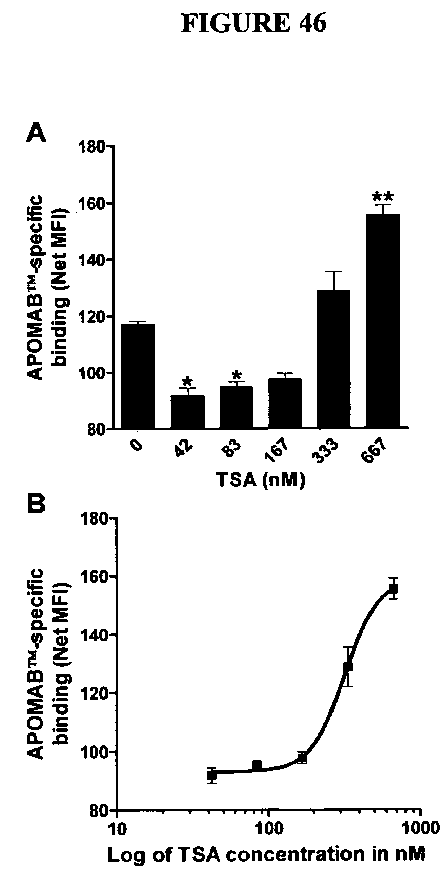

**Figure 46** depicts that the histone deacetylase inhibitor (HDACi) trichostatin A (TSA) exerts dose-dependent synergy with cisplatin to augment Apomab™-specific binding to dead Jurkat cells. Jurkat cells were incubated for 48h with 10µg/mL cisplatin together with increasing concentrations of TSA. Cells were subjected to indirect immunofluorescence staining using Apomab™ or its Sal5 isotype control mAb and viability was assessed using 7-AAD. Apomab™-specific binding was calculated as the Net MFI after gating on 7-AAD⁺ events. Data are presented as the mean ± SEM from two separate experiments. (**A**) Apomab™-specific binding to cisplatin-treated Jurkat cells is plotted as a function of TSA concentration. Binding in the presence of TAS was compared with binding in the absence of TSA using two-way ANOVA and a Bonferroni post-test comparison (*, *P*<0.05; **, *P*<0.01). (**B**) TSA concentration data were log-transformed to suggest a dose-response effect of TSA dose on Apomab™-specific binding. GraphPad Prism software was used to find a sigmoidal-dose response with variable slope as the best-fit model for the data (*r*² = 0.98).

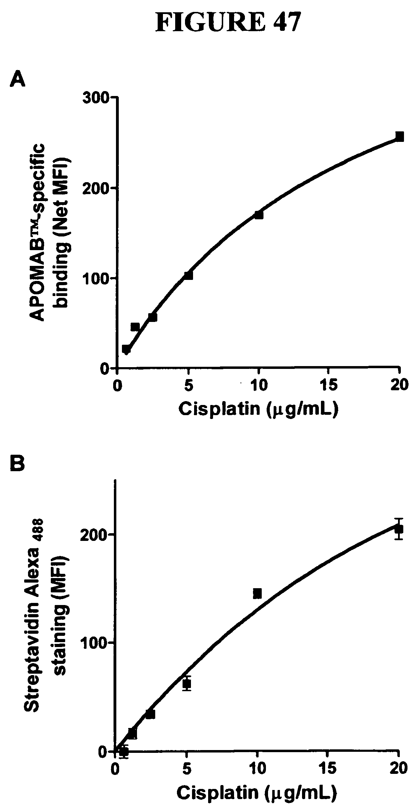

**Figure 47** depicts that the extent of Apomab™ binding to apoptotic Jurkat cells *in vitro* correlates directly with both cisplatin dose and TG2 protein cross linking activity. (**A**) Jurkat cells were incubated for 48h in increasing concentrations of cisplatin and then subjected to indirect immunofluorescence staining using 5µg/mL Apomab™ followed by 2µg/mL anti-mouse IgG Alexa₄₈₈ antibody. (**B**) Jurkat cells were incubated with 100µM cadaverine-biotin and increasing concentrations of cisplatin. After 48h, cells were incubated at RT for 15min. with 2µg/mL streptavidin-Alexa₄₈₈ then washed and analysed by flow cytometry. Data (*n*=3) shown were gated on 7-AAD⁺ events and are presented as (A) net MFI (±SEM) for Apomab™-specific binding or as (B) MFI (±SEM) for cadaverine-biotin incorporation.

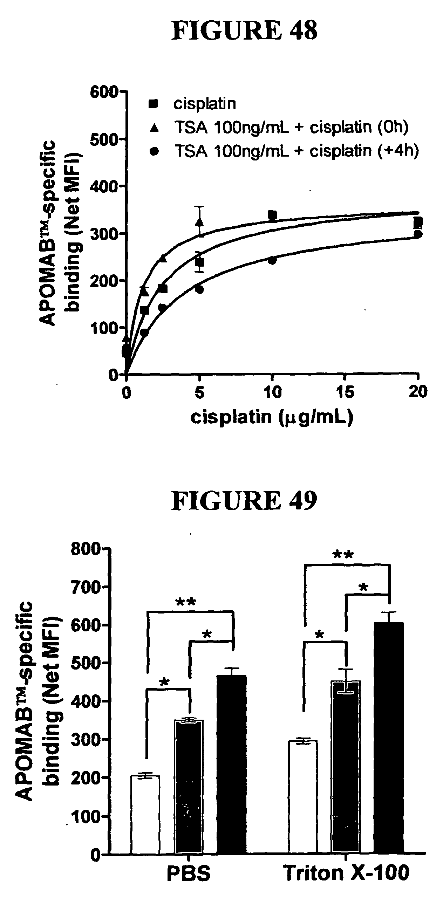

**Figure 48** depicts that the synergistic effect of TSA on Apomab™-specific binding to cisplatin-treated Jurkat cells is schedule-dependent. Jurkat cells were treated with increasing concentrations of cisplatin alone (■), increasing concentrations of cisplatin in the presence of 100ng/mL TSA (▲), or increasing concentrations of cisplatin added 4h from the time of addition of 100ng/mL TSA (•). After 48h, cells were subjected to indirect immunofluorescence staining using Apomab™ or its Sal5 isotype control mAb and viability was assessed using 7-AAD. Apomab™-specific binding was calculated as the Net MFI after gating on 7-AAD⁺ events. Data are shown as the Net MFI ± SEM (*n*=3).
**Figure 49** depicts that the histone deacetylase inhibitor (HDACi) trichostatin A (TSA) synergises with cisplatin to augment detergent-resistant Apomab™-specific binding to dead Jurkat cells. Jurkat cells were incubated in 200ng/mL TSA (clear bars) or 20µg/mL cisplatin (grey bars) singly or in combination (black bars). After 48h, cells were subjected to indirect immunofluorescence staining using Apomab™ or its Sal5 isotype control mAb and viability was assessed using 7-AAD. Apomab™-specific binding was calculated as the Net MFI after gating on 7-AAD⁺ events. Data are shown as the Net MFI ± SEM (*n*=3). Statistical analysis was performed using two-way ANOVA and a Bonferroni post-test comparison (*, *P*<0.01; **, *P<*0.001).

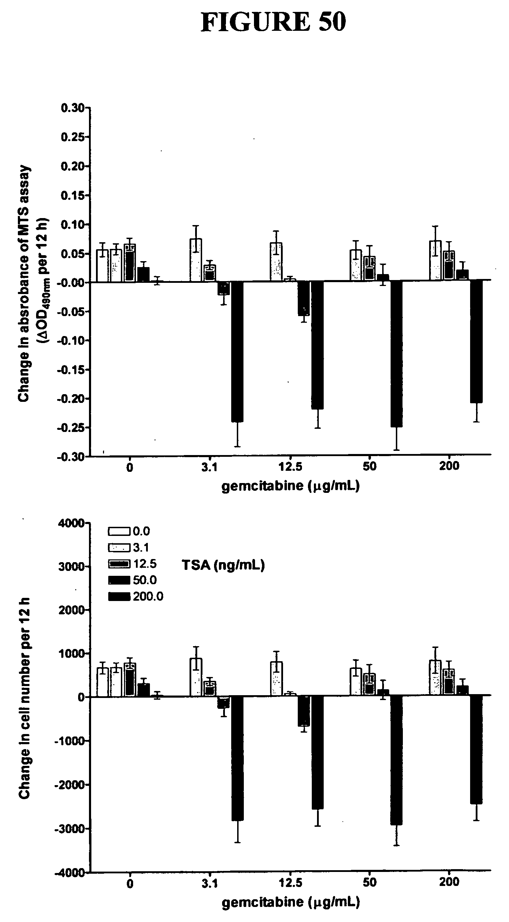

**Figure 50** depicts that gemcitabine and TSA synergise to reduce proliferation rates among PANC-1 cells *in vitro.* PANC-1 cells (2 x10⁴ cells) were seeded in flat bottom 96-well plates overnight using phenol-free RPMI-1640 medium containing 10 % FCS (medium). A serial dilution of 2 x 10⁴ cells was used to construct a standard curve for the MTS assay. Triplicate wells were incubated with increasing concentrations of TSA (nM) with or without the addition of increasing concentrations of gemcitabine. Three identical plates were incubated for 24, 48 or 72h then medium was decanted and fresh medium (100µL) containing 20µL MTS/PMS substrate was added to all wells. Assays were performed according to the manufacturer's instructions. The change in the absorbance reading over time was calculated for each condition in units of change per 12 h (upper panel), which was converted to units of cell number change per 12h (lower panel) using a standard curve of the absorbance reading as a function of time (r² = 0.99).

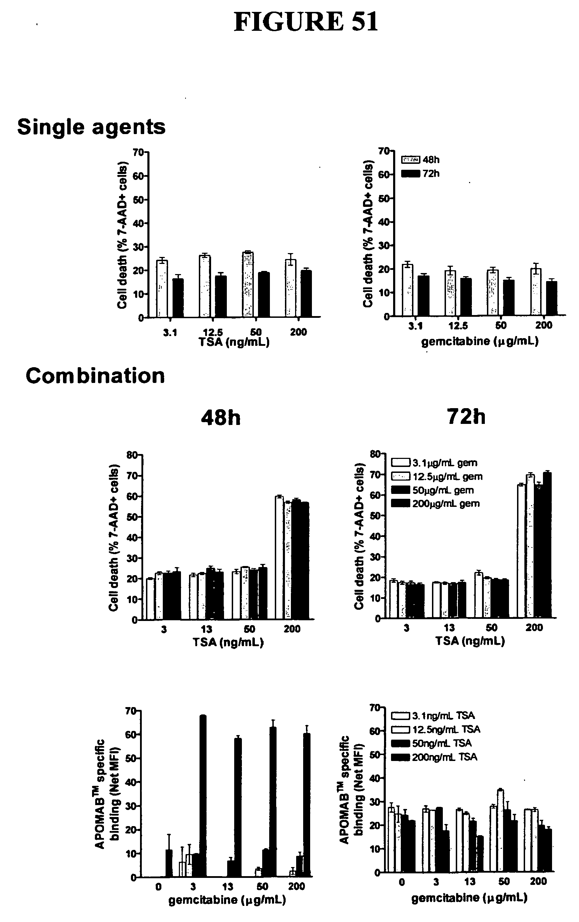

**Figure 51** depicts that gemcitabine and TSA synergise to increase cell death among PANC-1 cells, which subsequently demonstrate peak Apomab™-specific binding 48h after induction of cell death. PANC-1 cells (2 x 10⁴ cells) were seeded in flat bottom 96-well plates overnight using phenol-free RPMI-1640 medium containing 10 % FCS (medium). Cells were incubated with the specified concentrations of gemcitabine or TSA singly or in combination. Cells were collected (from media and EDTA detached adherent cells) and subjected to indirect immunofluorescence staining with Apomab™ or its Sal5 isotype control mAb and assessed for viability with 7-AAD staining. Data are shown as the percentage of cells staining with 7-AAD (upper four panels) or as net MFI ± SEM (*n*=3) for Apomab™-specific binding to 7-AAD⁺ cells (lower two panels).

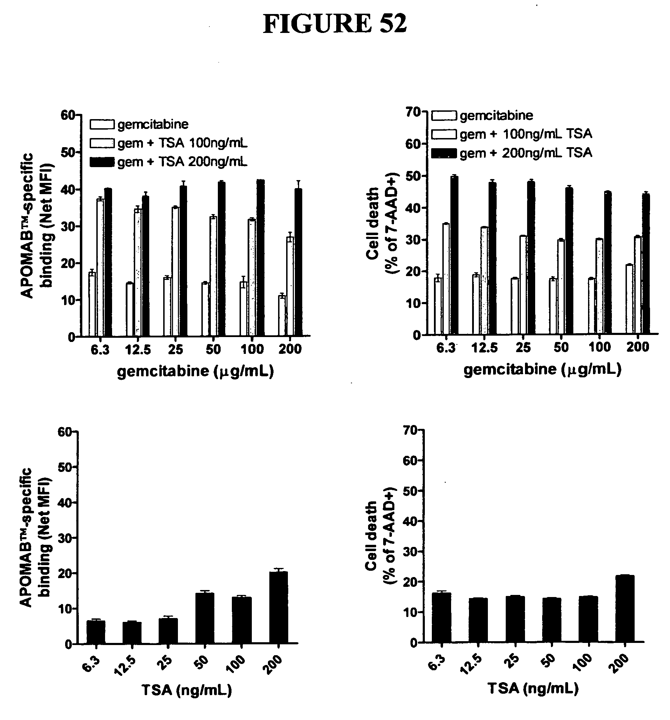

**Figure 52** depicts that gemcitabine and TSA synergise to increase cell death among PANC-1 cells, which subsequently demonstrate augmented Apomab™-specific binding. PANC-1 cells were incubated with increasing concentrations of gemcitabine alone (clear bars), increasing concentrations of TSA alone (black bars), increasing concentrations of gemcitabine and 100ng/mL TSA (light grey bars) or increasing concentrations of gemcitabine and 200ng/mL TSA (dark grey bars). After 48h, cells were collected (from media and EDTA detached adherent cells) and subjected to indirect immunofluorescence staining with Apomab™ or its Sal5 isotype control mAb and assessed for viability with 7-AAD staining. Data are shown as net MFI ± SEM (*n*=3) for Apomab™-specific binding (left-hand two panels) or as the percentage of cells staining with 7-AAD (right-hand two panels).

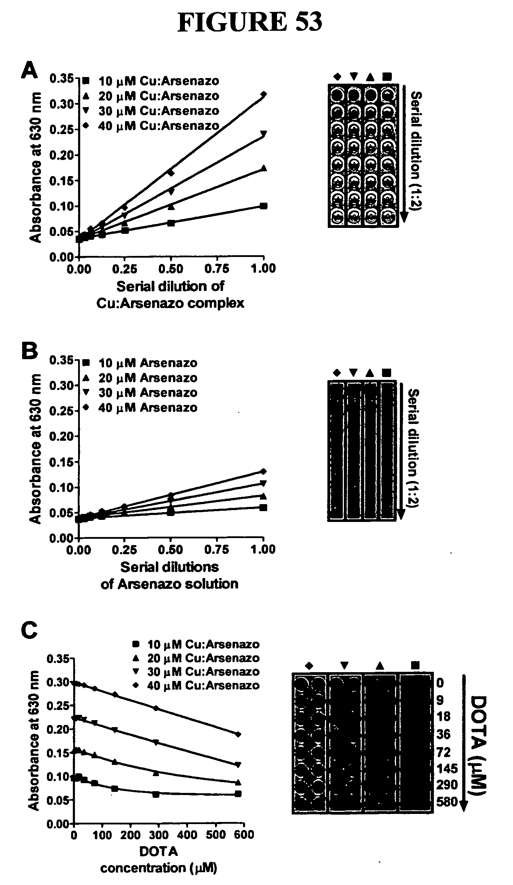

**Figure 53** depicts that modification of Cu:Arsenazo(III) assay. (A) Cu:Arsenazo(III) reagent prepared at different concentrations from stock solution was serially diluted 1:2 using milliQ water in 96-wells titre plate. Absorbance was measured at 630nm and was plotted as a function of the serial dilution performed. (**B**) Arsenazo(III) solutions prepared identically as described in A however without the addition of Cu were serially diluted 1:2 using milliQ water. Absorbance was measured at 630nm and plotted as a function of the dilutions performed. (**C**) Aliquots (10µL) of DOTA standard solutions were added in duplicates to 96-wells plate. Cu:Arsenazo(III) reagent prepared at four different concentration was added to these wells (190µL). Plate was incubated at 37°C for 20 min, absorbance was measured at 630nm and plotted as a function of DOTA concentration. Subsequent Cu:Arsenazo(III) assays were performed as described in this panel.

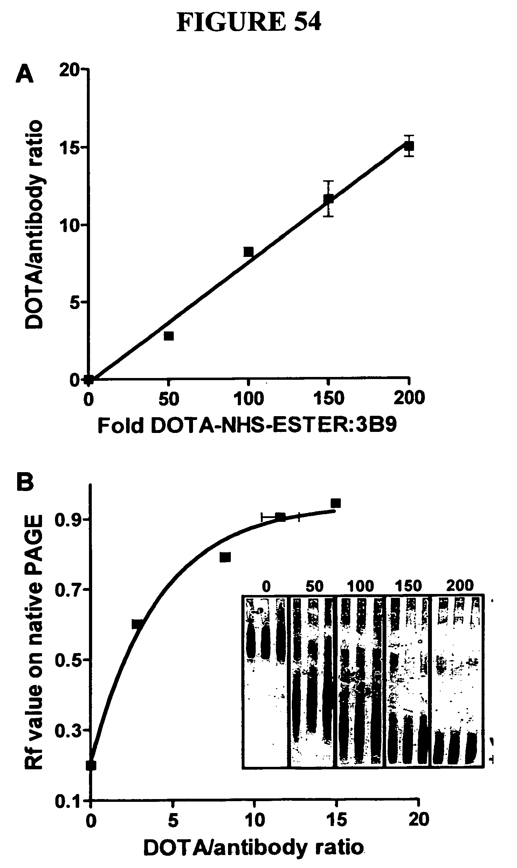

**Figure 54** depicts that conjugation condition affects DOTA/antibody ratio and net charge of the immunoconjugates. 3B9 was conjugated, at three separate occasions, with increasing concentrations of DOTA-NHS-ESTER calculated to be at 0-, 50-, 100-, 150- and 200-fold molar excess to the concentration of antibody. Conjugates were purified as described in Methods section. (**A**) DOTA concentration in the purified conjugates was measured using 10mL aliquots in modified Cu:Arsenzo(III) assay as described in Figure 1C. The protein concentration was measured using the BCA protein assay kit as described in methods. The DOTA/antibody ratio was calculated as the concentration of DOTA (µM) to that of antibody (µM) and the mean ratio ± standard error of the mean (SEM) (*n*=3) was plotted as a function of the concentration of DOTA-NHS-ESTER added during conjugation. (**B**) Samples of 3B9 incubated in the absence or presence of increasing concentrations of DOTA-NHS-ESTER (0-200 fold molar excess) as described above (triplicate reactions) were fractionated in native PAGE. Gel was stained with BBR250 and documented (inset). The (-) and (+) symbols denote the cathode and anode with the migration direction during electrophoresis is indicated by the arrow. The Rf value for the detected bands was calculated using GelPro Analyzer and was plotted as a function of the DOTA/antibody ratio calculated from panel A.

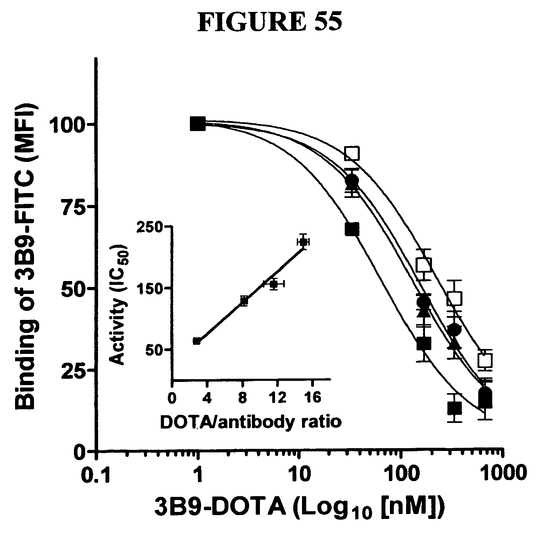

**Figure 55** depicts that increased DOTA/antibody ratio affects antibody avidity. Fixed and permeabilized Jurkat cells were incubated with 33.3nM of 3B9-FITC in the absence or presence of increasing 3B9-DOTA concentrations which were prepared at (□) 50-fold, (▲) 100-fold, (●) 150-fold and (■) 200-fold molar excess of DOTA-NHS-ESTER to antibody. Cells were washed and analyzed by flow cytometry and the mean fluorescent intensity (MFI±SEM, *n*=3) for 3B9-FITC binding was plotted as a function of the log10 concentration of 3B9-DOTA (nM) added to incubation. Competition binding curve was fitted to the data using GraphPad which found that data did not significantly deviate from the fitted model and reproduced the concentration of 3B9-DOTA required to inhibit half of the binding of 3B9-FITC (IC₅₀, in nM). **Inset:** The IC₅₀ (nM±SEM) of the different conjugates which is indicative of avidity was plotted as a function of the DOTA/antibody ratio. Linear correlation (*r²*=0.99) was fitted by GraphPad and described the relationship between avidity and the extent of conjugation taking place on the antibody.

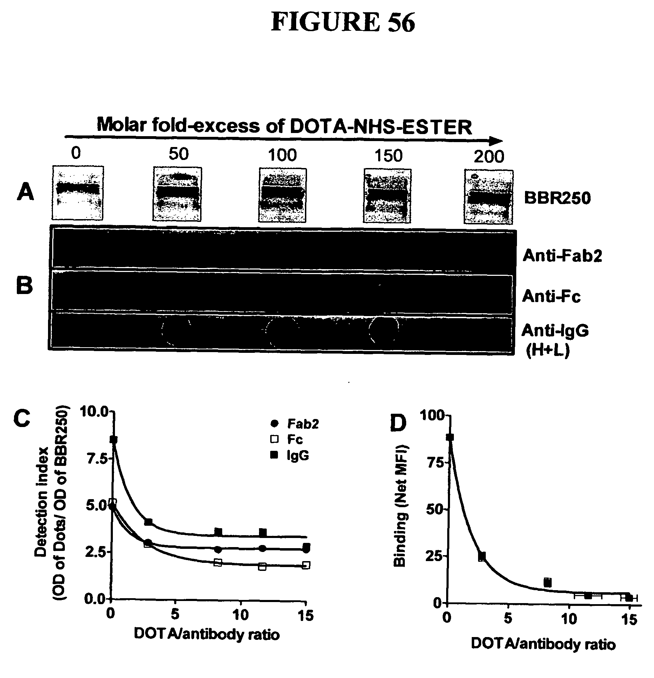

**Figure 56** depicts that conjugation modifies Fc and Fab regions of monoclonal antibody. Samples of 3B9 incubated in the absence or presence of increasing concentration of DOTA-NHS-ESTER (0-200 fold molar excess) were used for (**A**) non-reducing SDS-PAGE and BBR250 staining or (**B**) dot blot for staining with anti-mouse (Fab)₂, anti-mouse Fc or anti-mouse whole IgG. (**C**) The optical density of dots detected using the anti-mouse antibodies was normalized to the optical density of BBR250 reflecting the amount of IgG present in sample. This ratio was plotted as a function of the DOTA/antibody ratio achieved by the corresponding conjugation condition. A one-phase exponential decay curve was fitted to the data using GraphPad. This assay was performed at three separate occasions and similar data was obtained. (**D**) Fixed and permeabilized Jurkat cells were subjected to indirect immunofluorescent staining using 3B9 or 3B9-DOTA followed by anti-mouse IgG Alexa₄₈₈ conjugated antibody as described in Methods. Cells were analyzed by flow cytometry and data shown is the Net MFI (signal from 3B9 or 3B9-DOTA after subtraction of the signal from Sal5 or corresponding Sal5-DOTA). Error bars are the SEM from triplicate incubations and the drawn curve is a one-phase exponential decay model fitted using GraphPad.

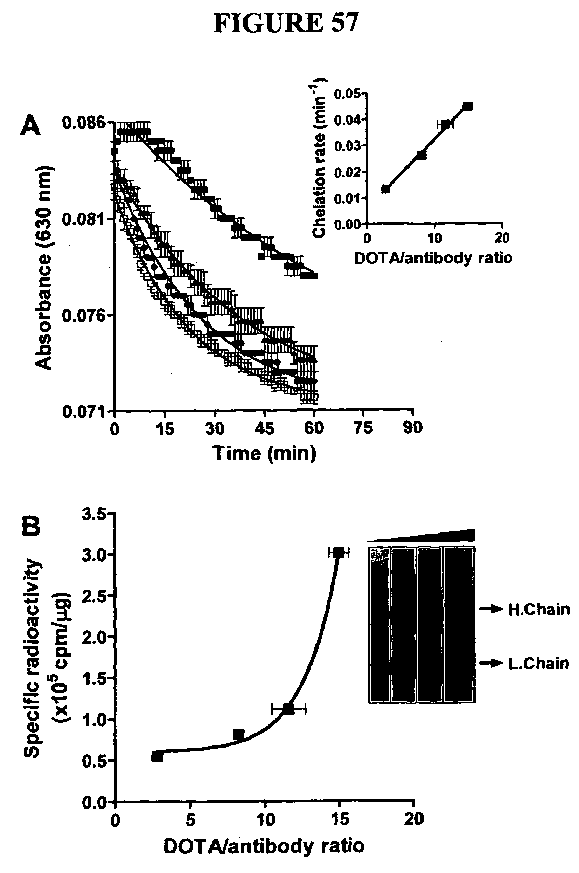

**Figure 57** depicts that DOTA/antibody ratio affects metal chelation rate and radionuclide loading capacity. (**A**) Identical amounts of 3B9-DOTA prepared at increasing concentrations of DOTA-NHS-ESTER were incubated with Terbium:Arsenazo(IIII) reagent which was assayed kinetically for 1h at 37°C measuring absorbance at 630nm. Absorbance was plotted as a function of time for 3B9-DOTA prepared at (■) 50-fold, (▲) 100-fold, (●) 150-fold or (□) 200-fold molar excess of DOTA-NHS-ESTER. Data presented is the mean ± SEM and was fitted using GraphPad to a one-phase exponential decay model which provided a measurement of chelation rate (min⁻¹) SEM. **Inset:** chelation rate was plotted as a function of DOTA/antibody ratio which showed a direct correlation. (**B**) Identical amounts of 3B9-DOTA prepared at increasing concentrations of DOTA-NHS-ESTER were labelled with ¹¹¹In. After purification, the concentrations of antibody and radioactivity in the samples were measured as described in Methods and specific radioactivity expressed as cpm/µg was plotted as a function of DOTA/antibody ratio. **Inset:** Identical amounts of reduced samples of ¹¹¹In-labelled DOTA-3B9 prepared at increasing DOTA-NHS-ESTER concentrations were fractionated by SDS-PAGE. Gel was exposed to x-ray film and documented. The heavy and light chains of the IgG are marked by arrows. Note the increase in the intensity of bands with increasing DOTA concentrations (◢) and the labelling of both heavy and light chains.

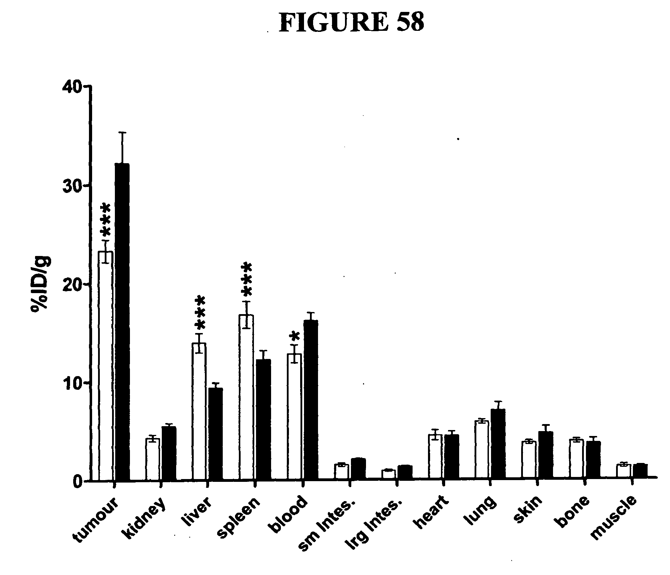

**Figure 58** depicts that modification of monoclonal antibody by conjugation is reflected *in vivo.* C57BL/6 mice bearing EL4 tumors and treated with cytotoxic chemotherapy as described in Methods received an intravenous injection at time 0 containing 100µg of ¹¹¹In-DOTA-3B9 prepared at 200-fold molar excess of DOTA-NHS-ESTER (clear bars) or 100µg of ¹¹¹In-DOTA-3B9 prepared at 50-fold molar excess of DOTA-NHS-ESTER (filled bars). Mice were euthanized at 48h and blood, tumors and other organs were collected, weighed and counted using gamma counter. Data presented is the accumulation in the organs calculated as the percentage of radioactivity in the organs per mass or organ to the radioactivity of the injected dose at time (%ID/g). Error bars are the SEM from 5 mice at each treatment and asterisks denote statistically different data (* *P<0.05*, ** *P<0.001*).

### DETAILED DESCRIPTION OF THE INVENTION

The present invention is predicated, in part, on the determination that intra-cellular molecules, in particular intra-nuclear molecules, which are exposed to the extracellular environment as a result of DNA damaging treatment regimes provide a suitable target to enable a more localised second step neoplastic treatment regime. Still further, the use of molecules which are overexpressed in malignant cells and/or, as determined in the context of the development of the present invention, the level of which are increased due to the first step exposure to a DNA damaging agent provides a highly valuable and effective means of effecting the preferential delivery of a second step treatment to populations of neoplastic cells, thereby minimising the inadvertent exposure of normal cells. This is a significant difference to the notion of merely binding pre-existing dead neoplastic cells in which the levels of the subject nuclear molecule are unlikely to have been increased unless said death was induced via a cell damage mechanism and/or dissipation or clearance of the dead cell components has not occurred. The selectivity of this method can be rendered still more pronounced by designing the timing of the two-step treatment regimen to take advantage of the fact that the induction of normal cell death, and thereby the clearance of these cells, occurs more quickly than the induction of neoplastic cell death. Accordingly, the timing of the delivery of the second step treatment can be designed to occur at a point at which the proportion of any collaterally induced normal dead cells is at their lowest point. Since the objective of the first step treatment is merely to kill or to sensitise for killing (i.e. lower the threshold for killing) a subpopulation of neoplastic cells sufficient to provide a suitable target to enable the targeted delivery of a more toxic second step treatment, the method of the invention conveniently enables the delivery of lower concentrations of first step systemic treatments, thereby minimising unwanted side effects, prior to the delivery of a localised higher dose second step treatment. Again, the reduction in the concentration of the non-targeted first step treatment also contributes to reducing the side effects which would be apparent in the context of conventional systemic cytotoxic treatment regimes where this treatment step would be delivered at a much higher dose and, further, often in the context of multiple repeated rounds over a period of months. This development now provides a realistic means of moving away from the treatment of metastatic disease via the non-targeted, systemic delivery of chemotherapy.

Accordingly, one aspect provides a method for the treatment of a neoplastic condition in a subject, said method comprising:
(i) administering to said subject a DNA damaging agent for a time and under conditions sufficient to induce the damage of at least a subpopulation of neoplastic cells, wherein said cell damage is characterised by the exposure of intra-cellular molecules; and
(ii) administering to said subject a ligand directed to one or more of said intra-cellular molecules, which ligand is associated with an effector mechanism, for a time and under conditions sufficient to downregulate the growth of viable bystander neoplastic cells.

Reference to "localised" treatment hereafter should be understood as a reference to treatment which is focussed on the sites of neoplastic cells, as opposed to acting on non-neoplastic tissue. However, it should be appreciated that the method of the present invention is designed to effect an increased incidence of localised treatment but may not necessarily entirely eliminate the incidence of neoplastic tissue effects although this is usefully reduced.

Reference to a "neoplastic condition "should be understood as a reference to a condition characterised by the presence of development of encapsulated or unencapsulated growths or aggregates of neoplastic cells. Reference to a "neoplastic cell" should be understood as a reference to a cell exhibiting abnormal growth. The term "growth" should be understood in its broadest sense and includes reference to enlargement of neoplastic cell size as well as proliferation.

The phrase "abnormal growth" in this context is intended as a reference to cell growth which, relative to normal cell growth, exhibits one or more of an increase in individual cell size and nuclear/cytoplasmic ratio, an increase in the rate of cell division, an increase in the number of cell divisions, a decrease in the length of the period of cell division, an increase in the frequency of periods of cell division or uncontrolled proliferation and evasion of apoptosis. Without limiting the present invention in any way, the common medical meaning of the term "neoplasia" refers to "new cell growth" that results as a loss of responsiveness to normal growth controls, eg. to neoplastic cell growth. Neoplasias include "tumours" which may be benign, pre-malignant or malignant. The term "neoplasm" should be understood as a reference to a lesion, tumour or other encapsulated or unencapsulated mass or other form of growth or cellular aggregate which comprises neoplastic cells.

The term "neoplasm", in the context of the present invention should be understood to include reference to all types of cancerous growths or oncogenic processes, metastatic tissues or malignantly transformed cells, tissues or organs irrespective of histopathologic type or state of invasiveness.

The term "carcinoma" is recognised by those skilled in the art and refers to malignancies of epithelial or endocrine tissues including respiratory system carcinomas, gastrointestinal system carcinomas, genitourinary system carcinomas, testicular carcinomas, breast carcinomas, prostate carcinomas, endocrine system carcinomas and melanomas. Exemplary carcinomas include those forming from tissue of the breast. The term also includes carcinosarcomas, e.g. which include malignant tumours composed of carcinomatous and sarcomatous tissues. An "adenocarcinoma" refers to a carcinoma derived from glandular tissue or in which the tumour cells form recognisable glandular structures.

The neoplastic cells comprising the neoplasm may be any cell type, derived from any tissue, such as an epithelial or non-epithelial cell. Reference to the terms "malignant neoplasm" and "cancer" and "carcinoma" herein should be understood as interchangeable.

The term "neoplasm" should be understood as a reference to a lesion, tumour or other encapsulated or unencapsulated mass or other form of growth or cellular aggregate which comprises neoplastic cells. The neoplastic cells comprising the neoplasm may be any cell type, derived from any tissue, such as an epithelial or non-epithelial cell. Examples of neoplasms and neoplastic cells encompassed by the present invention include, but are not limited to central nervous system tumours, retinoblastoma, neuroblastoma, paediatric tumours, head and neck cancers (e.g. squamous cell cancers), breast and prostate cancers, lung cancer (both small and non-small cell lung cancer), kidney cancers (e.g. renal cell adenocarcinoma), oesophagogastric cancers, hepatocellular carcinoma, pancreaticobiliary neoplasias (e.g. adenocarcinomas and islet cell tumours), colorectal cancer, cervical and anal cancers, uterine and other reproductive tract cancers, urinary tract cancers (e.g. of ureter and bladder), germ cell tumours (e.g. testicular germ cell tumours or ovarian germ cell tumours), ovarian cancer (e.g. ovarian epithelial cancers), carcinomas of unknown primary, human immunodeficiency associated malignancies (e.g. Kaposi's sarcoma), lymphomas, malignant melanomas, sarcomas, endocrine tumours (e.g. of thyroid gland), mesothelioma and other pleural or peritoneal tumours, neuroendocrine tumours and carcinoid tumours.

Preferably, the present disclosure is directed to the treatment of a malignant neoplastic condition and even more preferably a metastatic neoplastic condition. It would be appreciated that although the method of the invention can be applied to the treatment of any neoplasm (whether a solid tumour or not, it is particularly useful in terms of the treatment of metastasised neoplasms. Without limiting the present invention to any one theory or mode of action, non-metastasised primary tumours are treatable either by the method of the disclosure or by conventional treatment regimes such as surgical excision of the tumour or radiotherapy. However, tumours which have metastasised are not curable by either of these conventional treatment regimes due to the often extensive spread and growth of metastatic nodules. Accordingly, such conditions are currently only treatable by the administration of systemic chemotherapy, this treatment regime often causing severe side effects for limited curative potential, for example due to the emergence of chemoresistant neoplastic cells. Still further, even in the context of primary tumours which appear not to have metastasised, chemotherapy is still often recommended following surgery and radiation in case metastatic spread has occurred but is not yet detectable. This is a particularly common practice in the context of cancers which are traditionally regarded as aggressive, such as breast and colon cancers. The method now provides an alternative to the application of aggressive systemic chemotherapy treatment regimes. Since the systemic administration of the DNA damaging agent is only required to kill a proportion of the total neoplastic cell population, so as to provide a localised target for a more aggressive second step treatment regime (preferably a radioimmunotherapy regime) the occurrence of side effects can be minimised via the administration of lower doses of the DNA damaging agent, such as a cytotoxic agent, and the existence or occurrence of chemoresistant cells becomes less relevant, or at least be susceptible to elimination by means of a second-step effector activity such as a β particle, an α particle or a cytotoxin such as calicheamicin, which may exert beneficial therapeutic effects via non-crossreactive mechanisms of action.

The present disclosure therefore more particularly provides a method for the treatment of a malignant neoplastic condition in a subject, said method comprising:
(i) administering to said subject a DNA damaging agent for a time and under conditions sufficient to induce the damage of at least a subpopulation of neoplastic cells, wherein said cell damage is characterised by the exposure of intra-cellular molecules; and
(ii) administering to said subject a ligand directed to one or more of said intra-cellular molecules, which ligand is associated with an effector mechanism, for a time and under conditions sufficient to downregulate the growth of viable bystander neoplastic cells.

Preferably, said malignant condition is a metastatic malignant condition. Reference to "metastatic" should be understood as a reference to a condition with either has undergone metastatisation or may have undergone metastatisation.

As detailed hereinbefore, the method is based on a two step procedure wherein in the first step sufficient DNA damaging agent is administered to induce some neoplastic cell damage. By "damage" is meant that the integrity of the cellular and nuclear membranes is compromised such that the molecules comprising these compartment are exposed to the extracellular environment. Without limiting the present invention to any one theory or mode of action, this type of damage is usually inextricably linked to the cell death process since the permeabilisation of the cell membrane is often regarded as defining cell death. However, to the extent that this may not be the case, it should be understood that the induction of damage to the cell membrane such that the intracellular molecules are exposed to the extracellular environment falls within the scope of the present disclosure. Preferably, said damage is death.

According to this preferred embodiment there is provided a method for the treatment of a metastatic neoplastic condition in a subject, said method comprising:
(i) administering to said subject a DNA damaging agent for a time and under conditions sufficient to induce the death of at least a subpopulation of neoplastic cells, wherein said cell death is characterised by the exposure of intra-cellular molecules; and
(ii) administering to said subject a ligand directed to one or more of said intracellular molecules, which ligand is associated with an effector mechanism, for a time and under conditions sufficient to downregulate the growth of viable bystander neoplastic cells.

The cell death process, whether it is induced to occur via an apoptotic mechanism or some other mechanism such as paraptosis or autophagy, ultimately results in the breakdown of the cellular structures and the exposure of both intracytoplasmic and intra-nuclear molecules (herein collectively referred to as intra-cellular molecules). Without limiting the present invention to any one theory or mode of action, the nucleus is compartmentalized structurally and functionally although membranes do not divide these compartments. The two largest interacting parts of the nucleus are DNA-containing chromatin and ribonucleoprotein (RNP)-containing structures, which include RNA. Large morphological and molecular changes in the nucleus occur during irreversible commitment to apoptosis. The classical nuclear changes of apoptosis include condensation and margination of chromatin and nuclear fragmentation. During apoptosis, it is believed that both chromatin collapse and alterations of the nuclear envelope result from the cleavage of structural nuclear proteins such as lamins and RNPs such as La, which is mediated by caspases and other proteases. Other nuclear components such as the nucleolus undergo profound structural rearrangements during apoptosis. However, during apoptosis, chromatin and RNP-containing nuclear structures remain segregated.

Molecular modifications in the nucleolus, which is composed of approximately 350 proteins and in the nucleus include up- or downregulation of protein expression or even the expression of new proteins. In addition to alterations in protein expression, apoptosis-related modifications include degradation, post-translational modification and translocation. More than 120 proteins have been reported to undergo modification during apoptosis. Approximately 20 to 40% of these proteins contain the RNA-binding domain found in RNPs which constitute only 1.8% of the human proteome.

In eukaryotic cells, the perichromatin compartment contains the machinery for transcription, splicing and gene silencing. RNPs comprise integral components of the transcriptional and splicing machinery, which are identifiable ultrastructurally as perichromatin granules (PG), perichromatin fibrils (PF) and interchromatin granules (IG). These structures may be characterized by immuno-electron microscopy using antibodies directed against the component proteins. PF contain newly synthesized RNA and PG are involved in the storage and nucleo-cytoplasmic transport of mRNA. During apoptosis, ultrastructural rearrangement of PG, PF and IG is observed and results in the creation of new fibro-granular structures called Heterogenous Ectopic RNP-Derived Structures (HERDS). These aggregates are extruded into the cytoplasm and move toward the cell surface as part of apoptotic blebs, which are eventually released as apoptotic bodies. During even the latter stages of apoptosis, RNPs in the HERDS can be labelled by specific antibodies.

The nucleolus is the dominant nuclear substructure and among other functions, it acts as a ribosome factory. During apoptosis, the nucleolus is disassembled and eventually disappears and its components may aggregate with nucleoplasmic RNPs in the nucleus, cytoplasm and apoptotic blebs. RNA molecules are also extruded from the nucleus and are readily detectable in the cytoplasm of apoptotic cells including in HERDS. However, the identification of nucleolar proteins in HERDS marks irreversible progression of HERDS formation and commitment to apoptosis.

Preferably, said intracellular molecules are nuclear molecules.

Examples of intra-cellular molecules suitable for targetting in accordance with the method include, but are not limited to:
(i) Heterogeneous ectopic ribonucleoprotein-derived structures (HERDS);
(ii) Ribonucleoproteins (RNPs);
(iii) RNA species;
(iv) DNA damage response proteins;
(v) Apoptosis-related neo-epitopes, such as those generated by cleavage and those generated by protein modification;
(vi) Malignancy and cell death-related peptides;
(vii) Tissue transglutaminase.

Preferably, said intra-cellular molecule is a nuclear molecule. Reference to "nuclear molecule" should be understood as a reference to any proteinaceous or non-proteinaceous molecule which is permanently or transiently present in the nucleus. The molecule may be one which is transiently expressed, for example in response to a specific signal, or it may be one which is constitutively expressed.

More preferably, said nuclear molecule is RNP-1, hnRNP or La. Most preferably, said nuclear molecule is La.

Accordingly, there is preferably provided a method for the treatment of a malignant neoplastic condition in a subject, said method comprising:
(i) administering to said subject a DNA damaging agent for a time and under conditions sufficient to induce the damage of at least a subpopulation of said neoplastic cells, wherein said cell damage is characterised by the exposure of nuclear molecules; and
(ii) administering to said subject a ligand directed to one or more of said nuclear molecules, which ligand is associated with an effector mechanism, for a time and under conditions sufficient to downregulate the growth of viable bystander neoplastic cells.

Preferably said nuclear molecule is La.

Reference herein to "La" includes reference to all forms of La or their homologues, or orthologs or derivatives. Reference to "La" should be understood to include reference to any isoforms which arise from alternative splicing of La mRNA or mutants or polymorphic variants of La. It should also be understood that "La" is a molecule which is alternatively termed SS-B.

Reference to the "exposure" of a nuclear molecule should be understood as a reference to rendering the subject molecule accessible to the extracellular environment such that a ligand present in the extracellular environment can interact with said molecule. To this end, it should be understood that the exposure of these molecules may occur in the context of a dead cell or a dying cell.

The selectivity of the method is enabled by virtue of the fact that normal cells die and are cleared more quickly than neoplastic cells. Accordingly, the targeted second step treatment, if timed appropriately, can be designed to predominantly target the nuclear molecules of dead neoplastic cells by virtue of the fact that normal cells which may have been killed in the first step treatment have been, or are in the process of, clearance. However, in a related aspect it has also been determined that not only are the levels of a range of intra-cellular molecules, in particular nuclear molecules, increased upon transformation of a cell, they are still further increased upon the induction of cell death by a DNA damaging agent. This provides an additional and highly valuable mechanism by which differentiation between normal cells and neoplastic cells can occur, thereby minimising the incidence of bystander killing which targets non-malignant tissue. Without limiting the present invention to any one theory or mode of action, by minimising the availability of intra-cellular molecules, such as nuclear molecules, derived from normal cells, there occurs a reduced incidence of binding of the ligand of the second step treatment to regions of normal cellular tissue. Still further, to the extent that the targeted intra-cellular molecule is one in respect of which the levels are increased upon cellular transformation and/or cytotoxic cell death, the overall effect of the second step ligand binding is reduced in the context of normal cells due to the fact that the concentration of ligand binding to any given normal intracellular molecules is reduced due to the lower concentration of available molecule. This therefore correlates to a lower concentration of the cell death inducing effector mechanism to which the normal tissue proximally located to the normal intracellular target molecule is exposed and in fact enable the design of ligands which, per unit, exhibit a lower dose of the effector mechanism than would previously have been envisioned since increased concentration of these ligands are now enabled to be bound at any neoplastic growth of interest.

Accordingly, there is still more particularly provided a method for the treatment of a metastatic neoplastic condition in a subject, said method comprising:
(i) administering to said subject a DNA damaging agent for a time and under conditions sufficient to induce the death of at least a subpopulation of neoplastic cells, wherein said cell death is characterised by the exposure of intra-cellular molecules and upregulation of the level of said intra-cellular molecules; and
(ii) administering to said subject a ligand directed to one or more of said intra-cellular molecules, which ligand is associated with an effector mechanism, for a time and under conditions sufficient to downregulate the growth of viable bystander neoplastic cells.

As detailed hereinbefore, the DNA damaging agent which is administered in the first treatment round is one which preferably induces cell death, thereby leading to membrane permeability and exposure of the nuclear molecules of the targeted cell. Due to the fact that the objective of this treatment step is only to induce the death of a subpopulation of the subject neoplastic cells, the DNA damaging agent which is selected for use may be administered at lower doses or in some other manner which achieves this objective but which is at a lower dose than if the treatment was required to be administered in the context of being the sole systemic treatment regime. Accordingly, this minimises unwanted side effects.

Reference to a "DNA damaging agent" should be understood as a reference to any proteinaceous or non-proteinaceous agent which acts to damage cellular DNA. the agent may be a cytotoxic agent or a non-cytotoxic agent. Without limiting the present invention to any one theory or mode of action, many such agents function via the induction of apoptotic processes. However, this is not the only mechanism by which such agents function and it is conceivable that the subject DNA damage may be induced by some other mechanism. Examples of DNA damaging agents include, but are not limited to, the traditionally understood chemotherapy agents such as Actinomycin D, Arsenic Trioxide, Asparaginase, Bleomycin, Busulfan, Carboplatin, Carmustine, Chlorambucil, Cisplatin, Corticosteroids, Cyclophosphamide, Daunorubicin, Docetaxel, Doxorubicin, Epirubicin, Etoposide, Fludarabine, Fluorouracil, Gemcitabine, Hydroxyurea, Idarubicin, Ifosfamide, Irinotecan, Lomustine, Melphalan, Mercaptopurine, Methotrexate, Mitomycin, Mitoxantrone, Oxaliplatin, Paclitaxel, Procarbizine, Raltitrexed, Streptozocin, Thioguanine, Thiotepa, Topotecan, Treosulfan, Vinblastine, Vincristine, Vindesine, Vinorelbine. Other means of inducing DNA damage include ionising radiation as well as the use of molecules such as inhibitors of poly-(ADP ribosyl) transferase (PARP) or agents which induce DNA damage as part of a synergistic process with another agent, for example e.g. Gemcitabine or Irinotecan and CHK1/2 inhibitors such as CBP-501 or AZD7762. In addition, new classes of antineoplastic agents such as histone deacetylase inhibitors (HDACi) e.g. vorinostat, BH3 mimetics e.g. ABT737, and Tumor Necrosis Factor-Related Apoptotis-Inducing Ligand (TRAIL), are pro-apoptotic particularly when administered in conjunction with conventional cytotoxic agents. Hence, singly or in combination, these pro-apoptotic compounds will likely increase the amount of La-specific signal detectable in the malignant neoplasm.

Preferably, the subject agent is a radiosensitising agent. Reference to "radiosensitising agent" should be understood as a reference to an agent which potentiates the effectiveness of radiation therapy in destroying unwanted cells. For example, and without limiting the present invention to any one theory or mode of action, some radiosensitising agents function by arresting cell cycling at a stage which renders the cell particular sensitive to radiation. Examples of radiosensitising agents include, but are not limited to, platinum drugs, carmustine, topotecan, tumour necrosis factor, antimetabolites such as gemcitabine, 5-fluorouracil, capectibine, fludarabine, taxanes such as paclitaxel and docetaxel, Epidermal Growth Factor Receptor (EGFR) inhibitors such as cetuximab, erlotinib and gefitinib, histone deacetylase inhibitors such as vorinostat, DNA-dependent protein kinase inhibitors, CHK1/2 inhibitors, and hypoxic radiosensitizers such as nitroimidazole compounds.

According to this preferred embodiment there is provided a method for the treatment of a metastatic neoplastic condition in a subject, said method comprising:
(i) administering to said subject a radiosensitising agent for a time and under conditions sufficient to induce the death of at least a subpopulation of said neoplastic cells, wherein said cell death is characterised by the exposure of nuclear molecules and the upregulation of the level of said nuclear molecules; and
(ii) administering to said subject a ligand directed to one or more of said nuclear molecules, which ligand is associated with an effector mechanism, for a time and under conditions sufficient to downregulate the growth of viable bystander neoplastic cells.

Preferably, said radiosensitising agent is gemcitabine and/or cisplatin and said nuclear molecule is La.

It should be understood that the method is not limited to the use of only one DNA damaging agent but may extend to the sequential or simultaneous use of two or more agents. It would also be appreciated that the body of knowledge in relation to the characteristics and use of DNA damaging agents is extensive and the person of skill in the art could design an administration protocol to meet the parameters of the present invention as a matter of routine procedure.

According to this preferred embodiment there is provided a method for the treatment of a metastatic neoplastic condition in a subject, said method comprising:
(i) administering to said subject gemcitibine and/or cisplatin for a time and under conditions sufficient to induce the death of at least a subpopulation of said neoplastic cells, wherein said cell death is characterised by the exposure of La and the upregulation of the level of La; and
(ii) administering to said subject a ligand directed to La, which ligand is associated with an effector mechanism, for a time and under conditions sufficient to downregulate the growth of viable bystander neoplastic cells.

As detailed hereinbefore, the exposure of the nuclear molecules of a subpopulation of the neoplastic cells of interest provides a more selective target to which an effector mechanism can be targeted in order to achieve the bystander killing of proximally located viable neoplastic cells. In addition to providing a means of reducing the side effects associated with non-specific systemic treatments such as chemotherapy, this method also provides a means of reducing some of the problems associated with neoplastic cell non-responsiveness to conventional treatment regimes. Without limiting the present invention to any one theory or mode of action, systemic anti-cancer treatments usually kill by chemotherapy induced apoptosis. In many cases of advanced cancer, however, some cancer cells are resistant to the selected chemotherapy agent and conventional external beam radiotherapy may not be an available option due to the perceived or actual likelihood of the existence of metastases or micro-metastases. These resistant tumour cells are the source of clinical relapse of disease that ultimately kills most patients with advanced cancers and a significant proportion of patients with earlier stage cancers. In those patients with advanced cancers who could be shown to be responding to the initial modality of treatment because tumour cell kill could be documented *in vivo,* additional gains in survival and quality of life may be made if another non-cross resistant treatment modality were also to be employed either further to or, preferably, alternatively to the conventional treatment regime. Accordingly, the method provides a means of either avoiding or else overcoming this problem by targetting a suitable therapy, such as a radiation based therapy regimen or immunotoxin, to a neoplastic condition in a manner which can target both the primary tumour and metastases.

Although the general notion of targeted therapy is not new, to date the success of targeted therapy has been limited by virtue of meeting the criteria which have been required of a potential target antigen, these being:
(i) cell surface location
(ii) high cell surface antigen density
(iii) lack of internalisation of the antigen; and
(iv) lack of appreciable antigen shedding from the cell surface.

Significant limitations exist in terms of the identification of such antigens, in particular antigens which are also ideally tumour-specific. The determination that nuclear molecules can be usefully revealed and targeted not only to provide target specificity, thereby minimising unwanted side effects, but to enable the direct *in vivo* delivery of radiotherapy, the external beam-based delivery of which is not suitable for the treatment of metastases or micrometastases, is therefore particularly valuable. Still further, directed delivery of radiotherapy provides a means of potentially more effectively treating metastases which are, often, chemoresistant and therefore effectively impervious to further chemotherapy.

To this end, reference to a "ligand" should be understood as a reference to any molecule having specificity (not necessarily exclusive specificity, although this is preferable) and binding affinity for a nuclear molecule. Examples of ligands include immunointeractive molecules, affibodies, phylomers, aptamers, peptidomimetic agents, lanthamide metals (which interact with RNA species), enzymatic substrates (which interact with cell death-related enzymes) and putrescine (which interacts with tissue transglutaminase). To the extent that a preferred embodiment of the present invention is directed to targetting La, said ligand is preferably an immunointeractive molecule. Although a preferred immunointeractive molecule is an immunoglobulin molecule, the present invention extends to other immunointeractive molecules such as antibody fragments, single chain antibodies, deimmunized antibodies including humanized antibodies and T-cell associated antigen-binding molecules (TABMs). Most preferably, the immunointeractive molecule is an antibody such as a polyclonal or monoclonal antibody. It should be understood that the subject ligand may be linked, bound or otherwise associated to any other proteinaceous or non-proteinaceous molecule or cell.

The ligand is "directed to" the nuclear molecule, for example La, or, to the extent that the ligand is an immunointeractive molecule, to an antigenic determinant or epitope. It should be understood that the molecule may not necessarily exhibit complete exclusivity, although this is preferable. For example, antibodies are known to sometimes crossreact with other antigens. An antigenic determinant or epitope includes that part of the molecule to which an immune response can be directed. The antigenic determinant or epitope may be a Bell epitope or where appropriate a T-cell receptor binding molecule.

The second step of the present invention may also be designed such that a ligand is directed to one or more nuclear molecules. Accordingly, the present invention may be designed to administer two or more ligands directed to different targets, for example as a means of increasing the dose of effector mechanism which is delivered to a population of neoplastic cells. It also provides a convenient means of simultaneously delivering two different effector mechanisms.

Preferably, the subject nuclear molecule is La and the subject ligand is an antibody.

According to this preferred embodiment there is provided a method for the treatment of a neoplastic condition in a subject, said method comprising:
(i) administering to said subject a DNA damaging agent for a time and under conditions sufficient to induce the death of at least a subpopulation of said neoplastic cells, wherein said cell death is characterised by the exposure of La and upregulation of the level of said La; and
(ii) administering to said subject an antibody directed to La, which antibody is associated with an effector mechanism, for a time and under conditions sufficient to downregulate the growth of viable bystander neoplastic cells.

Preferably, said DNA damaging agent is a radiosensitising agent or a cytotoxic agent such as calicheamicin or a maytansinoid.

The use of antibodies, in particular monoclonal antibodies, to detect nuclear molecules such a La is a preferred method. Discussion hereinafter in relation to La should be understood as non-limiting and is intended to be exemplary of any cellular antigen. Antibodies may be prepared by any of a number of means. For the detection of human La, for example, human-human monoclonal antibody hybridomas may be derived from B cells, which have been obtained from patients who make anti-La autoantibodies because they have systemic autoimmune diseases such as systemic lupus erythematosis (SLE) or Sjorgren's syndrome (Ravirajan et al. Lupus 1(3):157-165,1992). Antibodies are generally but not necessarily derived from non-human animals such as primates, livestock animals (e.g. sheep, cows, pigs, goats, horses), laboratory test animals (e.g. mice, rats, guinea pigs, rabbits) and companion animals (e.g. dogs, cats). Generally, antibody based assays are conducted *in vitro* on cell or tissue biopsies. However, if an antibody is suitably deimmunized or, in the case of human use, humanized, then the antibody can be labelled with, for example, a nuclear tag, administered to a patient and the site of nuclear label accumulation determined by radiological techniques. The La antibody is regarded, therefore, as a cellular apoptosis targeting agent. Accordingly, the present invention extends to deimmunized forms of the antibodies for use in cellular apoptosis imaging in human and non-human patients. This is described further below.

Currently available antibodies include SW3 and 3B9.

For the generation of antibodies to La, this molecule is required to be extracted from a biological sample whether this be from animal including human tissue or from cell culture if produced by recombinant means. The La can be separated from the biological sample by any suitable means. For example, the separation may take advantage of any one or more of La's surface charge properties, size, density, biological activity and its affinity for another entity (e.g. another protein or chemical compound to which it binds or otherwise associates). Thus, for example, separation of La from the biological fluid may be achieved by any one or more of ultra-centrifugation, ion-exchange chromatography (e.g. anion exchange chromatography, cation exchange chromatography), electrophoresis (e.g. polyacrylamide gel electrophoresis, isoelectric focussing), size separation (e.g., gel filtration, ultra-filtration) and affinity-mediated separation (e.g. immunoaffinity separation including, but not limited to, magnetic bead separation such as Dynabead™ separation, immunochromatography, immuno-precipitation). Choice of the separation technique(s) employed may depend on the biological activity or physical properties of the La sought or from which tissues it is obtained.

Preferably, the separation of La from the biological fluid preserves conformational epitopes present on the protein and, thus, suitably avoids techniques that cause denaturation of the enzyme. Persons of skill in the art will recognize the importance of maintaining or mimicking as close as possible physiological conditions peculiar to La (e.g. the biological fluid from which it is obtained) to ensure that the antigenic determinants or active sites on La, which are exposed to the animal, are structurally identical to that of the native protein. This ensures the raising of appropriate antibodies in the immunised animal that would recognize the native protein. In a preferred embodiment, La is separated from the biological fluid using any one or more of affinity separation, gel filtration and ultra-filtration.

Immunization and subsequent production of monoclonal antibodies can be carried out using standard protocols as for example described by Kohler and Milstein, Nature 256: 495-499, 1975; Kohler and Milstein, Eur. J. Immunol. 6(7): 511-519, 1976; Coligan et al., Current Protocols in Immunology, John Wiley & Sons, Inc., 1991-1997, or Toyama et al., "Monoclonal Antibody, Experiment Manual", published by Kodansha Scientific, 1987. Essentially, an animal is immunized with a La-containing biological fluid or fraction thereof by standard methods to produce antibody-producing cells, particularly antibody-producing somatic cells (e.g. B lymphocytes). These cells can then be removed from the immunized animal for immortalization.

Where a fragment of La is used to generate antibodies, it may need to first be associated with a carrier. By "carrier" is meant any substance of typically high molecular weight to which a non- or poorly immunogenic substance (e.g. a hapten) is naturally or artificially linked to enhance its immunogenicity.

Immortalization of antibody-producing cells may be carried out using methods which are well-known in the art. For example, the immortalization may be achieved by the transformation method using Epstein-Barr virus (EBV) (Kozbor et al., Methods in Enzymology 121: 140, 1986). In a preferred embodiment, antibody-producing cells are immortalized using the cell fusion method (described in Coligan *et al.,* 1991-1997, *supra*), which is widely employed for the production of monoclonal antibodies. In this method, somatic antibody-producing cells with the potential to produce antibodies, particularly B cells, are fused with a myeloma cell line. These somatic cells may be derived from the lymph nodes, spleens and peripheral blood of humans with circulating La-reactive antibodies, and primed animals, preferably rodent animals such as mice and rats. Mice spleen cells are particularly useful. It would be possible, however, to use rat, rabbit, sheep or goat cells, or cells from other animal species instead.

Specialized myeloma cell lines have been developed from lymphocytic tumours for use in hybridoma-producing fusion procedures (Kohler and Milstein, 1976, *supra*; Shulman et al., Nature 276: 269-270, 1978; Volk et al., J. Virol. 42(1): 220-227, 1982). These cell lines have been developed for at least three reasons. The first is to facilitate the selection of fused hybridomas from unfused and similarly indefinitely self-propagating myeloma cells. Usually, this is accomplished by using myelomas with enzyme deficiencies that render them incapable of growing in certain selective media that support the growth of hybridomas. The second reason arises from the inherent ability of lymphocytic tumour cells to produce their own antibodies. To eliminate the production of tumour cell antibodies by the hybridomas, myeloma cell lines incapable of producing endogenous light or heavy immunoglobulin chains are used. A third reason for selection of these cell lines is for their suitability and efficiency for fusion.

Many myeloma cell lines may be used for the production of fused cell hybrids, including, e.g. P3X63-Ag8, P3X63-AG8.653, P3/NS1-Ag4-1 (NS-1), Sp2/0-Ag14 and S194/5.XXO.Bu.1. The P3X63-Ag8 and NS-1 cell lines have been described by Köhler and Milstein (1976, *supra*). Shulman *et al.* (1978, *supra*) developed the Sp2/0-Ag14 myeloma line. The S194/5.XXO.Bu.1 line was reported by Trowbridge, J. Exp. Med. 148(1): 313-323,1978.

Methods for generating hybrids of antibody-producing spleen or lymph node cells and myeloma cells usually involve mixing somatic cells with myeloma cells in a 10:1 proportion (although the proportion may vary from about 20:1 to about 1:1), respectively, in the presence of an agent or agents (chemical, viral or electrical) that promotes the fusion of cell membranes. Fusion methods have been described (Kohler and Milstein, 1975, *supra*; 1976, *supra*; Gefter et al., Somatic Cell Genet. 3: 231-236, 1977; Volk *et al.,* 1982, *supra*). The fusion-promoting agents used by those investigators were Sendai virus and polyethylene glycol (PEG).

Because fusion procedures produce viable hybrids at very low frequency (e.g. when spleens are used as a source of somatic cells, only one hybrid is obtained for roughly every 1x10⁵ spleen cells), it is preferable to have a means of selecting the fused cell hybrids from the remaining unfused cells, particularly the unfused myeloma cells. A means of detecting the desired antibody-producing hybridomas among other resulting fused cell hybrids is also necessary. Generally, the selection of fused cell hybrids is accomplished by culturing the cells in media that support the growth of hybridomas but prevent the growth of the unfused myeloma cells, which normally would go on dividing indefinitely. The somatic cells used in the fusion do not maintain long-term viability in *in vitro* culture and hence do not pose a problem. In the example of the present invention, myeloma cells lacking hypoxanthine phosphoribosyl transferase (HPRT-negative) were used. Selection against these cells is made in hypoxanthine/aminopterin/thymidine (HAT) medium, a medium in which the fused cell hybrids survive due to the HPRT-positive genotype of the spleen cells. The use of myeloma cells with different genetic deficiencies (drug sensitivities, etc.) that can be selected against in media supporting the growth of genotypically competent hybrids is also possible.

Several weeks are required to selectively culture the fused cell hybrids. Early in this time period, it is necessary to identify those hybrids which produce the desired antibody, so that they may subsequently be cloned and propagated. Generally, around 10% of the hybrids obtained produce the desired antibody, although a range of from about 1 to about 30% is not uncommon. The detection of antibody-producing hybrids can be achieved by any one of several standard assay methods, including enzyme-linked immunoassay and radioimmunoassay techniques as, for example, described in Kennet et al. (eds) Monoclonal Antibodies and Hybridomas: A New Dimension in Biological Analyses, pp. 376-384, Plenum Press, New York, 1980 and by FACS analysis.

Once the desired fused cell hybrids have been selected and cloned into individual antibody-producing cell lines, each cell line may be propagated in either of two standard ways. A suspension of the hybridoma cells can be injected into a histocompatible animal. The injected animal will then develop tumours that secrete the specific monoclonal antibody produced by the fused cell hybrid. The body fluids of the animal, such as serum or ascites fluid, can be tapped to provide monoclonal antibodies in high concentration. Alternatively, the individual cell lines may be propagated *in vitro* in laboratory culture vessels. The culture medium containing high concentrations of a single specific monoclonal antibody can be harvested by decantation, filtration or centrifugation, and subsequently purified.

The cell lines are tested for their specificity to detect the La by any suitable immunodetection means. For example, cell lines can be aliquoted into a number of wells and incubated and the supernatant from each well is analyzed by enzyme-linked immunosorbent assay (ELISA), indirect fluorescent antibody technique, or the like. The cell line(s) producing a monoclonal antibody capable of recognizing the target La but which does not recognize non-target epitopes are identified and then directly cultured *in vitro* or injected into a histocompatible animal to form tumours and to produce, collect and purify the required antibodies.

These antibodies are La specific. This means that the antibodies are capable of distinguishing La from other molecules. More broad spectrum antibodies may be used provided that they do not cross react with molecules in a normal cell.

In one embodiment, the subject antibody is anti-human La monoclonal antibodies, 8G3 and 9A5 (Bachmann et al. Proc Natl Acad Sci USA 83 (20):7770-7774, 1986), anti-human La monoclonal antibody (mAb), La1B5 (Mamula et al. J Immunol 143(9):2923-2928, 1989), anti-human La monoclonal antibodies (Carmo-Fonseca et al. ExpCell Res 185(1):73-85, 1989), anti-human and anti-bovine La monoclonal antibodies, SW1, SW3 and SW5 (Pruijn et al. EurJBiochem 232(2):611-619, 1995), anti-human and anti-rodent La mAb, La4B6 (Troster et al. J Autoimmunity 8(6):825-842, 1995) or anti-human and anti-murine La mAb, 3B9 (Tran et al. Arthritis Rheum 46(1):202-208, 2002) or derivative, homologue, analogue, chemical equivalent, mutant or mimetic thereof.

Since the monoclonal antibody is destined for *in vivo* use, it may be desirable to deimmunise the antibody. The deimmunization process may take any of a number of forms including the preparation of chimeric antibodies which have the same or similar specificity as the monoclonal antibodies prepared. Chimeric antibodies are antibodies whose light and heavy chain genes have been constructed, typically by genetic engineering, from immunoglobulin variable and constant region genes belonging to different species. Thus, once a hybridoma producing the desired monoclonal antibody is obtained, techniques are used to produce interspecific monoclonal antibodies wherein the binding region of one species is combined with a non-binding region of the antibody of another species (Liu et al., Proc. Natl. Acad. Sci. USA 84: 3439-3443, 1987). For example, complementary determining regions (CDRs) from a non-human (e.g. murine) monoclonal antibody can be grafted onto a human antibody, thereby "humanizing" the murine antibody (European Patent Publication No. 0 239 400; Jones et al., Nature 321: 522-525, 1986; Verhoeyen et al., Science 239: 1534-1536, 1988; Richmann et al., Nature 332: 323-327, 1988). In this case, the deimmunizing process is specific for humans. More particularly, the CDRs can be grafted onto a human antibody variable region with or without human constant regions. The non-human antibody providing the CDRs is typically referred to as the "donor" and the human antibody providing the framework is typically referred to as the "acceptor". Constant regions need not be present, but if they are, they must be substantially identical to human immunoglobulin constant regions, i.e. at least about 85-90%, preferably about 95% or more identical. Hence, all parts of a humanized antibody, except possibly the CDRs, are substantially identical to corresponding parts of natural human immunoglobulin sequences. Thus, a "humanized antibody" is an antibody comprising a humanized light chain and a humanized heavy chain immunoglobulin. A donor antibody is said to be "humanized", by the process of "humanization", because the resultant humanized antibody is expected to bind to the same antigen as the donor antibody that provides the CDRs. Reference herein to "humanized" includes reference to an antibody deimmunized to a particular host, in this case, a human host.

It will be understood that the deimmunized antibodies may have additional conservative amino acid substitutions which have substantially no effect on antigen binding or other immunoglobulin functions. Exemplary conservative substitutions may be made according to Table 1.

**TABLE 1**

| **ORIGINAL RESIDUE** | **EXEMPLARY SUBSTITUTIONS** |
|---|---|
| Ala | Ser |
| Arg | Lys |
| Asn | Gln, His |
| Asp | Glu |
| Cys | Ser |
| Gln | Asn |
| Glu | Asp |
| Gly | Pro |
| His | Asn, Gln |
| Ile | Leu, Val |
| Leu | Ile, Val |
| Lys | Arg, Gln, Glu |
| Met | Leu, Ile |
| Phe | Met, Leu, Tyr |
| Ser | Thr |
| Thr | Ser |
| Trp | Tyr |
| Tyr | Trp, Phe |
| Val | He, Leu |

Exemplary methods which may be employed to produce deimmunized antibodies according to the present invention are described, for example, in references Richmann *et al.,* 1988, *supra*; European Patent Publication No. 0 239-400; Chou et al. (U.S. Patent No. 6,056,957); Queen et al. (U.S. Patent No. 6,180,370); Morgan et al. (U.S. Patent No. 6,180,377).

Thus, in one embodiment, the present disclosure contemplates the use of a deimmunized antibody molecule having specificity for an epitope recognized by a monoclonal antibody to La wherein at least one of the CDRs of the variable domain of said deimmunized antibody is derived from the said monoclonal antibody to La and the remaining immunoglobulin-derived parts of the deimmunized antibody molecule are derived from an immunoglobulin or an analogue thereof from the host for which the antibody is to be deimmunized.

This aspect involves manipulation of the framework region of a non-human antibody.

The present disclosure extends to the use of mutants, analogues and derivatives of the subject antibodies but which still retain specificity for La.

The terms "mutant" or "derivatives" includes one or more amino acid substitutions, additions and/or deletions.

As used herein, the term "CDR" includes CDR structural loops which covers the three light chain and the three heavy chain regions in the variable portion of an antibody framework region which bridge β strands on the binding portion of the molecule. These loops have characteristic canonical structures (Chothia et al., J. Mol. Biol. 196: 901, 1987; Chothia et al., J. Mol. Biol. 227: 799, 1992).

By "framework region" is meant region of an immunoglobulin light or heavy chain variable region, which is interrupted by three hypervariable regions, also called CDRs. The extent of the framework region and CDRs have been precisely defined (see, for example, Kabat et al., "Sequences of Proteins of Immunological Interest", U.S. Department of Health and Human Services, 1983). The sequences of the framework regions of different light or heavy chains are relatively conserved within a species. As used herein, a "human framework region" is a framework region that is substantially identical (about 85% or more, usually 90-95% or more) to the framework region of a naturally occurring human immunoglobulin. The framework region of an antibody, that is the combined framework regions of the constituent light and heavy chains, serves to position and align the CDRs. The CDRs are primarily responsible for binding to an epitope of La.

As used herein, the term "heavy chain variable region" means a polypeptide which is from about 110 to 125 amino acid residues in length, the amino acid sequence of which corresponds to that of a heavy chain of a monoclonal antibody, starting from the amino-terminal (N-terminal) amino acid residue of the heavy chain. Likewise, the term "light chain variable region" means a polypeptide which is from about 95 to 130 amino acid residues in length, the amino acid sequence of which corresponds to that of a light chain of a monoclonal antibody, starting from the N-terminal amino acid residue of the light chain. Full-length immunoglobulin "light chains" (about 25 Kd or 214 amino acids) are encoded by a variable region gene at the NH₂-terminus (about 110 amino acids) and a κ or λ constant region gene at the COOH-terminus. Full-length immunoglobulin "heavy chains" (about 50 Kd or 446 amino acids), are similarly encoded by a variable region gene (about 116 amino acids) and one of the other aforementioned constant region genes, e.g. γ (encoding about 330 amino acids).

The term "immunoglobulin" or "antibody" is used herein to refer to a protein consisting of one or more polypeptides substantially encoded by immunoglobulin genes. The recognized immunoglobulin genes include the κ, λ, α, γ (IgG₁, IgG₂, IgG₃, IgG₄), 8, ε and µ constant region genes, as well as the myriad immunoglobulin variable region genes. One form of immunoglobulin constitutes the basic structural unit of an antibody. This form is a tetramer and consists of two identical pairs of immunoglobulin chains, each pair having one light and one heavy chain. In each pair, the light and heavy chain variable regions are together responsible for binding to an antigen, and the constant regions are responsible for the antibody effector functions. In addition to antibodies, immunoglobulins may exist in a variety of other forms including, for example, Fv, Fab, Fab' and (Fab')₂.

The disclosure also contemplates the use and generation of fragments of monoclonal antibodies produced by the method including, for example, Fv, Fab, Fab' and F(ab')₂ fragments. Such fragments may be prepared by standard methods as for example described by *Coligan et al.* (1991-1997, *supra*).

The present disclosure also contemplates synthetic or recombinant antigen-binding molecules with the same or similar specificity as the monoclonal antibodies of the invention. Antigen-binding molecules of this type may comprise a synthetic stabilised Fv fragment. Exemplary fragments of this type include single chain Fv fragments (sFv, frequently termed scFv) in which a peptide linker is used to bridge the N terminus or C terminus of a V*_{H}* domain with the C terminus or N-terminus, respectively, of a V*_{L}* domain. ScFv lack all constant parts of whole antibodies and are not able to activate complement. Suitable peptide linkers for joining the V*_{H}* and V*_{L}* domains are those which allow the V*_{H}* and V*_{L}* domains to fold into a single polypeptide chain having an antigen binding site with a three dimensional structure similar to that of the antigen binding site of a whole antibody from which the Fv fragment is derived. Linkers having the desired properties may be obtained by the method disclosed in U.S. Patent No 4,946,778. However, in some cases a linker is absent. ScFvs may be prepared, for example, in accordance with methods outlined in Krebber *et al. (*Krebber et al., J. Immunol. Methods 201(1): 35-55, 1997). Alternatively, they may be prepared by methods described in U.S. Patent No 5,091,513, European Patent No 239,400 or the articles by Winter and Milstein (Winter and Milstein, Nature 349: 293, 1991) and Plückthun *et al.* (Plückthun et al., In Antibody engineering: A practical approach 203-252, 1996).

Alternatively, the synthetic stabilized Fv fragment comprises a disulphide stabilized Fv (dsFv) in which cysteine residues are introduced into the V*_{H}* and V*_{L}* domains such that in the fully folded Fv molecule the two residues will form a disulphide bond therebetween. Suitable methods of producing dsFv are described, for example, in (Glockshuber et al., Biochem. 29: 1363-1367, 1990; Reiter et al., Biochem. 33: 5451-5459, 1994; Reiter et al., Cancer Res. 54: 2714-2718, 1994; Reiter et al., J. Biol. Chem. 269: 18327-18331, 1994; Webber et al., Mol. Immunol. 32: 249-258,1995).

Also contemplated as synthetic or recombinant antigen-binding molecules are single variable region domains (termed dAbs) as, for example, disclosed in *(*Ward et al., Nature 341: 544-546, 1989; Hamers-Casterman et al., Nature 363: 446-448, 1993; Davies & Riechmann, FEBS Lett. 339: 285-290,1994).

Alternatively, the synthetic or recombinant antigen-binding molecule may comprise a "minibody". In this regard, minibodies are small versions of whole antibodies, which encode in a single chain the essential elements of a whole antibody. Suitably, the minibody is comprised of the V*_{H}* and V*_{L}* domains of a native antibody fused to the hinge region and CH3 domain of the immunoglobulin molecule as, for example, disclosed in U.S. Patent No 5,837,821.

In an alternate embodiment, the synthetic or recombinant antigen binding molecule may comprise non-immunoglobulin derived, protein frameworks. For example, reference may be made to (Ku & Schutz, Proc. Natl. Acad. Sci. USA 92: 6552-6556,1995) which discloses a four-helix bundle protein cytochrome b562 having two loops randomized to create CDRs, which have been selected for antigen binding.

The synthetic or recombinant antigen-binding molecule may be multivalent (i.e. having more than one antigen binding site). Such multivalent molecules may be specific for one or more antigens. Multivalent molecules of this type may be prepared by dimerization of two antibody fragments through a cysteinyl-containing peptide as, for example disclosed by (Adams et al., Cancer Res. 53: 4026-4034, 1993; Cumber et al., J. Immunol. 149: 120-126, 1992). Alternatively, dimerization may be facilitated by fusion of the antibody fragments to amphiphilic helices that naturally dimerize (Plünckthun, Biochem. 31: 1579-1584, 1992) or by use of domains (such as leucine zippers *jun* and *fos*) that preferentially heterodimerize (Kostelny et al., J. Immunol. 148: 1547-1553, 1992).

The present disclosure further encompasses chemical analogues of amino acids in the subject antibodies. The use of chemical analogues of amino acids is useful *inter alia* to stabilize the molecules such as if required to be administered to a subject. The analogues of the amino acids contemplated herein include, but are not limited to, modifications of side chains, incorporation of unnatural amino acids and/or their derivatives during peptide, polypeptide or protein synthesis and the use of crosslinkers and other methods which impose conformational constraints on the proteinaceous molecule or their analogues.

Examples of side chain modifications include modifications of amino groups such as by reductive alkylation by reaction with an aldehyde followed by reduction with NaBH₄; amidination with methylacetimidate; acylation with acetic anhydride; carbamoylation of amino groups with cyanate; trinitrobenzylation of amino groups with 2, 4, 6-trinitrobenzene sulphonic acid (TNBS); acylation of amino groups with succinic anhydride and tetrahydrophthalic anhydride; and pyridoxylation of lysine with pyridoxal-5-phosphate followed by reduction with NaBH₄.

The guanidine group of arginine residues may be modified by the formation of heterocyclic condensation products with reagents such as 2,3-butanedione, phenylglyoxal and glyoxal.

The carboxyl group may be modified by carbodiimide activation *via* O-acylisourea formation followed by subsequent derivatisation, for example, to a corresponding amide. Sulphydryl groups may be modified by methods such as carboxymethylation with iodoacetic acid or iodoacetamide; performic acid oxidation to cysteic acid; formation of a mixed disulphides with other thiol compounds; reaction with maleimide, maleic anhydride or other substituted maleimide; formation of mercurial derivatives using 4-chloromercuribenzoate, 4-chloromercuriphenylsulphonic acid, phenylmercury chloride, 2-chloromercuri-4-nitrophenol and other mercurials; carbamoylation with cyanate at alkaline pH.

Tryptophan residues may be modified by, for example, oxidation with N-bromosuccinimide or alkylation of the indole ring with 2-hydroxy-5-nitrobenzyl bromide or sulphenyl halides. Tyrosine residues on the other hand, may be altered by nitration with tetranitromethane to form a 3-nitrotyrosine derivative.

Modification of the imidazole ring of a histidine residue may be accomplished by alkylation with iodoacetic acid derivatives or N-carbethoxylation with diethylpyrocarbonate.

Examples of incorporating unnatural amino acids and derivatives during peptide synthesis include, but are not limited to, use of norleucine, 4-amino butyric acid, 4-amino-3-hydroxy-5-phenylpentanoic acid, 6-aminohexanoic acid, t-butylglycine, norvaline, phenylglycine, ornithine, sarcosine, 4-amino-3-hydroxy-6-methylheptanoic acid, 2-thienyl alanine and/or D-isomers of amino acids. A list of unnatural amino acid, contemplated herein is shown in Table 2.

**TABLE 2**

| Non-conventional amino acid | Code | Non-conventional amino acid | Code |
|---|---|---|---|
| α-aminobutyric acid | Abu | L-N-methylalanine | Nmala |
| α-amino-α-methylbutyrate | Mgabu | L-N-methylarginine | Nmarg |
| aminocyclopropane-carboxylate | Cpro | L-N-methylasparagine | Nmasn |
| | | L-N-methylaspartic acid | Nmasp |
| aminoisobutyric acid | Aib | L-N-methylcysteine | Nmcys |
| aminonorbornyl- | Norb | L-N-methylglutamine | Nmgln |
| carboxylate | | L-N-methylglutamic acid | Nmglu |
| cyclohexylalanine | Chexa | L-Nmethylhistidine | Nmhis |
| cyclopentylalanine | Cpen | L-N-methylisolleucine | Nmile |
| D-alanine | Dal | L-N-methylleucine | Nmleu |
| D-arginine | Darg | L-N-methyllysine | Nmlys |
| D-aspartic acid | Dasp | L-N-methylmethionine | Nmmet |
| D-cysteine | Dcys | L-N-methylnorleucine | Nmnle |
| D-glutamine | Dgln | L-N-methylnorvaline | Nmnva |
| D-glutamic acid | Dglu | L-N-methylornithine | Nmorn |
| D-histidine | Dhis | L-N-methylphenylalanine | Nmphe |
| D-isoleucine | Dile | L-N-methylproline | Nmpro |
| D-leucine | Dleu | L-N-methylserine | Nmser |
| D-lysine | Dlys | L-N-methylthreonine | Nmthr |
| D-methionine | Dmet | L-N-methyltryptophan | Nmtrp |
| D-ornithine | Dorn | L-N-methyltyrosine | Nmtyr |
| D-phenylalanine | Dphe | L-N-methylvaline | Nmval |
| D-proline | Dpro | L-N-methylethylglycine | Nmetg |
| D-serine | Dser | L-N-methyl-t-butylglycine | Nmtbug |
| D-threonine | Dthr | L-norleucine | Nle |
| D-tryptophan | Dtrp | L-norvaline | Nva |
| D-tyrosine | Dtyr | α-methyl-aminoisobutyrate | Maib |
| D-valine | Dval | α-methyl-γ-aminobutyrate | Mgabu |
| D-α-methylalanine | Dmala | α-methylcyclohexylalanine | Mchexa |
| D-α-methylarginine | Dmarg | α-methylcylcopentylalanine | Mcpen |
| D-α-methylasparagine | Dmasn | α-methyl-α-napthylalanine | Manap |
| D-α-methylaspartate | Dmasp | α-methylpenicillamine | Mpen |
| D-α-methylcysteine | Dmcys | N-(4-aminobutyl)glycine | Nglu |
| D-α-methylglutamine | Dmgln | N-(2-aminoethyl)glycine | Naeg |
| D-α-methylhistidine | Dmhis | N-(3-aminopropyl)glycine | Norn |
| D-α-methylisoleucine | Dmile | N-amino-α-methylbutyrate | Nmaabu |
| D-α-methylleucine | Dmleu | α-napthylalanine | Anap |
| D-α-methyllysine | Dmlys | N-benzylglycine | Nphe |
| D-α-methylmethionine | Dmmet | N-(2-carbamylethyl)glycine | Ngln |
| D-α-methylornithine | Dmorn | N-(carbamylmethyl)glycine | Nasn |
| D-α-methylphenylalanine | Dmphe | N-(2-carboxyethyl)glycine | Nglu |
| D-α-methylproline | Dmpro | N-(carboxymethyl)glycine | Nasp |
| D-α-methylserine | Dmser | N-cyclobutylglycine | Ncbut |
| D-α-methylthreonine | Dmthr | N-cycloheptylglycine | Nchep |
| D-α-methyltryptophan | Dmtrp | N-cyclohexylglycine | Nchex |
| D-α-methyltyrosine | Dmty | N-cyclodecylglycine | Ncdec |
| D-α-methylvaline | Dmval | N-cylcododecylglycine | Ncdod |
| D-N-methylalanine | Dnmala | N-cyclooctylglycine | Ncoct |
| D-N-methylarginine | Dnmarg | N-cyclopropylglycine | Ncpro |
| D-N-methylasparagine | Dnmasn | N-cycloundecylglycine | Ncund |
| D-N-methylaspartate | Dnmasp | N-(2,2-diphenylethyl)glycine | Nbhm |
| D-N-methylcysteine | Dnmcys | N-(3,3-diphenylpropyl)glycine | Nbhe |
| D-N-methylglutamine | Dnmgln | N-(3-guanidinopropyl)glycine | Narg |
| D-N-methylglutamate | Dnmglu | N-(1-hydroxyethyl)glycine | Nthr |
| D-N-methylhistidine | Dnmhis | N-(hydroxyethyl))glycine | Nser |
| D-N-methylisoleucine | Dnmile | N-(imidazolylethyl))glycine | Nhis |
| D-N-methylleucine | Dnmleu | N-(3-indolylyethyl)glycine | Nhtrp |
| D-N-methyllysine | Dnmlys | N-methyl-γ-aminobutyrate | Nmgabu |
| N-methylcyclohexylalanine | Nmchexa | D-N-methylmethionine | Dnmmet |
| D-N-methylornithine | Dnmorn | N-methylcyclopentylalanine | Nmcpen |
| N-methylglycine | Nala | D-N-methylphenylalanine | Dnmphe |
| N-methylaminoisobutyrate | Nmaib | D-N-methylproline | Dnmpro |
| N-(1-methylpropyl)glycine | Nile | D-N-methylserine | Dnmser |
| N-(2-methylpropyl)glycine | Nleu | D-N-methylthreonine | Dnmthr |
| D-N-methyltryptophan | Dnmtrp | N-(1-methylethyl)glycine | Nval |
| D-N-methyltyrosine | Dnmtyr | N-methyla-napthylalanine | Nmanap |
| D-N-methylvaline | Dnmval | N-methylpenicillamine | Nmpen |
| γ-aminobutyric acid | Gabu | N-(*p*-hydroxyphenyl)glycine | Nhtyr |
| L-*t*-butylglycine | Tbug | N-(thiomethyl)glycine | Ncys |
| L-ethylglycine | Etg | penicillamine | Pen |
| L-homophenylalanine | Hphe | L-α-methylalanine | Mala |
| L-α-methylarginine | Marg | L-α-methylasparagine | Masn |
| L-α-methylaspartate | Masp | L-α-methyl-*t*-butylglycine | Mtbug |
| L-α-methylcysteine | Mcys | L-methylethylglycine | Metg |
| L-α-methylglutamine | Mgln | L-α-methylglutamate | Mglu |
| L-α-methylhistidine | Mhis | L-α-methylhomophenylalanine | Mhphe |
| L-α-methylisoleucine | Mile | N-(2-methylthioethyl)glycine | Nmet |
| L-α-methylleucine | Mleu | L-α-methyllysine | Mlys |
| L-α-methylmethionine | Mmet | L-α-methylnorleucine | Mnle |
| L-α-methylnorvaline | Mnva | L-α-methylornithine | Morn |
| L-α-methylphenylaianine | Mphe | L-α-methylproline | Mpro |
| L-α-methylserine | Mser | L-α-methylthreonine | Mthr |
| L-α-methyltryptophan | Mtrp | L-α-methyltyrosine | Mtyr |
| L-α-methylvaline | Mval | L-N-methylhomophenylalanine | Nmhphe |
| N-(N-(2,2-diphenylethyl) carbamylmethyl)glycine | Nnbhm | N-(N-(3,3-diphenylpropyl) carbamylmethyl)glycine | Nnbhe |
| 1-carboxy-1-(2,2-diphenyl-ethylamino)cyclopropane | Nmbc | | |

Crosslinkers can be used, for example, to stabilize 3D conformations, using homo-bifunctional crosslinkers such as the bifunctional imido esters having (CH₂)ₙ spacer groups with n=1 to n=6, glutaraldehyde, N-hydroxysuccinimide esters and hetero-bifunctional reagents which usually contain an amino-reactive moiety such as N-hydroxysuccinimide and another group specific-reactive moiety such as maleimido or dithio moiety (SH) or carbodiimide (COOH).

Without limiting the present invention to any one theory or mode of action, it has been observed that immunointractive molecules directed to La may become fixed, thereby providing a robust bystander effector mechanism. Still further, La has also been observed to become localised to the cellular cytoplasm, subsequently to the induction of cell damage, or may become associated with apoptotic bodies.

The ligand hereinbefore described is designed to deliver to the tumour site an anti-neoplastic treatment regime designed to downregulate the growth of the tumour cells or, most preferably, induce the death of these cells. To this end, reference to an "effector mechanism" should be understood as a reference to any suitable mechanism which, when localised to the site of neoplastic cells (by virtue of the ligand based targetting), either directly or indirectly downregulates the growth of, and preferably kills, proximally located viable neoplastic cells. The effector mechanism may be any proteinaceous or non-proteinaceous molecule or group of molecules which achieve this outcome. Examples of effector mechanisms suitable for use in the method include, but are not limited to:
(i) Use of a ligand which has been linked to a cytokine, chemokine or other factor, such as macrophage, dendritic cell and/or T cell activators which act to induce or enhance one or more aspects of an immune response, thereby augmenting bystander killing. For example, the chemotactic peptide, N-formyl-methionyl-leucyl-phenylalanine (FMLP) *(*Morikawa et al. Cancer Immunol Immunother 27(1):1-6, 1988) and the novel bacterial lipopeptide, JBT 2002 (Shinohara et al. J Immunother 23(3):321-331, 2000) are both activators of tumour associated macrophages.
(ii) Use of a ligand which has been conjugated to a toxin.
   Reference to "toxin" should be understood as a reference to any suitable proteinaceous or non-proteinaceous molecule which achieves the object of providing a signal which reduces, prevents or otherwise inhibits the proliferation, differentiation or maintenance of the subject cell (herein referred to as "down-regulating the growth" of said cell). The subject toxin may act by a variety of means including providing its signal via direct contact with a subject cell or emitting a molecule or particle, such as radiation in the case of a radioactive isotope toxin, which provides the signal to the subject cell. Preferably the toxin is a radioisotope and even more preferably a radioisotope which is highly toxic over a short range and exhibits a short half life thereby minimizing the occurrence of inadvertent toxicity on proximally located non-target cells. Nevertheless, without limiting the present invention in any way, radionuclides have varying energies and half-lives and it is increasingly possible to match these characteristics to the different tasks of treating small and large metastases. The radio-isotopes, which are most commonly used to label ligands for therapeutic applications, emit β-particles such as ¹³¹Iodine, ⁹⁰Yttrium, ¹⁸⁸Rhenium, ¹⁷⁷Lutetium or ⁶⁷Copper. The energy from β-radiation is released at relatively low intensity over distances measured in millimeters. Thus, high-energy β-emitters such ash ⁹⁰Yttrium are useful for the treatment of larger and heterogeneous solid tumours (Liu et al., Bioconjugate Chem 12:7-23,2001).
   Kassis and Adelstein defined optimal conditions for delivering an effective therapeutic dose of targeted radiation to a tumour using a β-emitting radionuclide in an effort to overcome the limiting effects imposed by heterogeneity of the target antigen distribution within the tumour. The distances between these hot foci must be equal to or less than twice the maximum range of the emitted energetic β-particles. The concentration of radiolabelled reagent in each hot focus must be sufficiently high to produce a cumulative crossfire dose of ≥ 10Gy to surrounding tumour cells (Kassis et al., Nucleotide Med 46:4S-12S, 2005).
   Radiation is targeted to dead tumour cells so as to deliver radiation-induced killing to nearby viable tumour cells in a form of bystander killing. In one preferred embodiment the ligand is associated with a β-emitting radionuclide such as ⁹⁰Y to treat tumours up to several centimetres in size where heterogeneous uptake of the ligand in the tumour and crossfire would result in effective absorption of the radiation dose across the whole tumour. In contrast, micrometastases can be targeted with an α-emitting radionuclide such as ²¹³Bi, which deposits high levels of radiation energy no more than several cell diameters (Sharkey and Goldenberg, J Nucleotide Med 46:115S-127S, 2005). Very few cells will survive impact by an alpha particle.
   It should nevertheless be understood that the radioisotopes used may include alpha-, beta- and gamma-emitting radioisotopes, depending on the clinical context. Examples of alpha-emitting radioisotopes suitable for use in the method include, but are not limited to, Tb-149 or Bi-213. It should be understood that the toxin which is utilised in the method may be in a purified, partially purified or unpurified form. It may also form a component of a larger molecule. The toxin may be naturally occurring or it may be synthetically or recombinantly produced.
   Other examples of molecules which should be understood to fall within the scope of "toxin" include ricin, colicheamicin, maytansinoid prodrugs (as antibody-directed prodrug converting enzyme therapy [ADEPT]) and novel biotherapeutic agents, such as catalytic antibodies.

Reference to an effector mechanism being "associated" with a ligand, for example, an antibody or other immunointeractive molecule should be understood as a reference to any covalent or noncovalent interactive mechanism which achieves linking of the two molecules. This includes, but is not limited to the use of peptide bonds, ionic bonds, hydrogen bonds, van Der Waals forces or any other interactive bonding mechanism.

It would be appreciated that the two steps of the present invention are preferably performed sequentially. However, it should also be understood that this method may be modified to incorporate other steps. For example, one may seek to perform a diagnostic/screening step after administration of the DNA damaging agent in order to assess the effectiveness of the first step. Such screening step may also be applied later in the treatment regime to monitor the effectiveness of the treatment. It would also be appreciated that it is well within the skill of the person in the art, and in light of the teaching provided herein, to select and design an administration protocol for the elements herein described including, for example, the DNA damaging agent, target nuclear molecule, ligand and the effector mechanism.

Reference to "growth" of a cell or neoplasm should be understood as a reference to the proliferation, differentiation and/or maintenance of viability of the subject cell, while "down-regulating the growth" of a cell or neoplasm is a reference to the process of cellular senescence or to reducing, preventing or inhibiting the proliferation, differentiation and/or maintenance of viability of the subject cell. In a preferred embodiment the subject growth is proliferation and the subject down-regulation is killing. In this regard, killing may be achieved either by delivering a fatal hit to the cell or by delivering to the cell a signal which induces the cell to apoptose.

In a particularly preferred embodiment there is provided a method for the treatment of a neoplastic tumour in a subject, said method comprising:
(i) administering to said subject a radiosensitising agent for a time and under conditions sufficient to induce the death of at least a subpopulation of said neoplastic cells, wherein said cell death is characterised by the exposure and upregulation of the level of La; and
(ii) administering to said subject an immunointeractive molecule directed to La or antigenic portions thereof, which immunointeractive molecule is associated with a radioisotope, for a time and under conditions sufficient to downregulate the growth of viable bystander neoplastic cells.

Preferably, said radiosensitising agent is one or more of Camptosar, Cisplatin, Docetaxel, Gemcitabina, Gemcitabine, Gemzar, Irinotecan, Platinex, Platinol, Trihydrate, Vinorelbina, Vinorelbine and said radioisotope is ⁹⁰Y or ²¹³Bi.

More preferably, said immunointeractive molecule is a monoclonal antibody.

Still more preferably, said neoplastic tumour is metastatic.

Reference herein to a "subject" should be understood to encompass humans, primates, livestock animals (eg. sheep, pigs, cattle, horses, donkeys), laboratory test animals (eg. mice, rabbits, rats, guinea pigs), companion animals (eg. dogs, cats) and captive wild animals (eg. foxes, kangaroos, deer). Preferably, the mammal is a human.

It should be understood that the term "treatment" does not necessarily imply that a subject is treated until total recovery. Accordingly, treatment includes reducing the severity of an existing condition, amelioration of the symptoms of a particular condition or preventing or otherwise reducing the risk of developing a particular condition.

Administration of the DNA damaging agent and the ligand, in the form of pharmaceutical compositions, may be performed by any convenient means. The pharmaceutical composition is contemplated to exhibit therapeutic activity when administered in an amount which depends on the particular case. The variation depends, for example, on the human or animal and the particular agent, ligand and effector mechanism selected for use. A broad range of doses may be applicable. Dosage regimes may be adjusted to provide the optimum therapeutic response.

Routes of administration include, but are not limited to, respiratorally, intratracheally, nasopharyngeally, intravenously, intraperitoneally, subcutaneously, intracranially, intradermally, intramuscularly, intraoccularly, intrathecally, intracereberally, intranasally, infusion, orally, rectally, *via* IV drip patch and implant.

In accordance with these methods, the agent defined in accordance with the present invention may be coadministered with one or more other compounds or molecules. By "coadministered" is meant simultaneous administration in the same formulation or in two different formulations via the same or different routes or sequential administration by the same or different routes. For example, the subject agent may be administered together with an agonistic agent in order to enhance its effects. By "sequential" administration is meant a time difference of from seconds, minutes, hours or days between the administration of the two types of molecules. These molecules may be administered in any order.

Another aspect is directed to the use of:
(i) a DNA damaging agent; and
(ii) an intra-cellular molecule ligand associated with an effector mechanism
in the manufacture of a medicament for the treatment of a neoplastic condition in a subject wherein
(i) administering to said subject said DNA damaging agent induces the cellular damage of at least a subpopulation of neoplastic cells, wherein said cell death is characterised by the exposure of intra-cellular molecules; and
(ii) administering to said subject said ligand induces the downregulation of growth of viable bystander neoplastic cells.

Examples of neoplasms and neoplastic cells include, but are not limited central nervous system tumours, retinoblastoma, neuroblastoma, paediatric tumours, head and neck cancers (eg. squamous cell cancers), breast and prostate cancers, lung cancer (both small and non-small cell lung cancer), kidney cancers (eg. renal cell adenocarcinoma), oesophagogastric cancers, hepatocellular carcinoma, pancreaticobiliary neoplasias (eg. adenocarcinomas and islet cell tumours), colorectal cancer, cervical and anal cancers, uterine and other reproductive tract cancers, urinary tract cancers (eg. of ureter and bladder), germ cell tumours (eg. testicular germ cell tumours or ovarian germ cell tumours), ovarian cancer (eg. ovarian epithelial cancers), carcinomas of unknown primary, human immunodeficiency associated malignancies (eg. Kaposi's sarcoma), lymphomas, malignant melanomas, sarcomas, endocrine tumours (eg. of thyroid gland), mesothelioma and other pleural tumours, neuroendocrine tumours and carcinoid tumours.

More particular aspects in relation to the DNA damaging agent, intracellular molecule, ligand, effector mechanism and cell damage are hereinbefore described in detail.

In yet another further aspect, the present disclosure contemplates a pharmaceutical composition comprising the agents as hereinbefore defined together with one or more pharmaceutically acceptable carriers and/or diluents. Said agents are referred to as the active ingredients.

The pharmaceutical forms are preferably suitable for injectable use and include sterile aqueous solutions (where water soluble) or dispersions and sterile powders for the extemporaneous preparation of sterile injectable solutions or dispersion. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol and liquid polyethylene glycol, and the like), suitable mixtures thereof, and vegetable oils. The proper fluidity can be maintained, for example, by the use of a coating such as lecithin, by the maintenance of the required particle size in the case of dispersion and by the use of superfactants. The preventions of the action of microorganisms can be brought about by various antibacterial and antifungal agents, for example, parabens, chlorobutanol, phenol, sorbic acid, thimerosal and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various of the other ingredients enumerated above, as required, followed by filtered sterilisation. Generally, dispersions are prepared by incorporating the various sterilised active ingredient into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

When the active ingredients are suitably protected they may be orally administered, for example, with an inert diluent or with an assimilable edible carrier, or it may be enclosed in hard or soft shell gelatin capsule, or it may be compressed into tablets, or it may be incorporated directly with the food of the diet. It would be appreciated, for example, that some chemotherapy agents can be delivered orally. For oral therapeutic administration, the active compound may be incorporated with excipients and used in the form of ingestible tablets, buccal tablets, troches, capsules, elixirs, suspensions, syrups, wafers, and the like. Such compositions and preparations should contain at least 1% by weight of active compound. The percentage of the compositions and preparations may, of course, be varied and may conveniently be between about 5 to about 80% of the weight of the unit. The amount of active compound in such therapeutically useful compositions in such that a suitable dosage will be obtained. Preferred compositions or preparations are prepared so that an oral dosage unit form contains between about 0.1 µg and 2000 mg of active compound.

The tablets, troches, pills, capsules and the like may also contain the components as listed hereafter: a binder such as gum, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavouring agent such as peppermint, oil of wintergreen, or cherry flavouring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavouring such as cherry or orange flavour. Of course, any material used in preparing any dosage unit form should be pharmaceutically pure and substantially non-toxic in the amounts employed. In addition, the active compound(s) may be incorporated into sustained-release preparations and formulations.

Yet another aspect is directed to a kit comprising a DNA damaging agent and an intracellular molecule ligand associated with an effector mechanism.

More particular aspects in relation to the DNA damaging agent, the intracellular molecule, ligand and effector mechanism are hereinbefore defined in detail.

The present invention is further described by reference to the following non-limiting examples.

### EXAMPLE 1

### IN VIVO TARGETTING OF THE RIBONUCLEOPROTEIN La IN A MOUSE TUMOUR MODEL : RATIONALE FOR THE DEVELOPMENT OF COMBINED CHEMO- AND RADIOIMMUNOTHERAPY FOR HUMAN MALIGNANCIES

### MATERIALS AND METHODS

### Materials

Cell culture media, RPMI-1640, DMEM and Ham's F12, and fetal calf serum (FCS) were all purchased from JRH Biosciences Inc. (KS, USA). Trypsin-EDTA solution, trypan blue, propidium iodide (PI), bovine serum albumin (BSA), hydrocortisone and staurosporine (STS) were obtained from Sigma-Aldrich Co. (MO, USA). Hybond-P membrane (PVDF), ECF^{™} substrate, L-[U-¹⁴C]Leucine, D-[U-¹⁴C]Glucose and Protein G purification columns were purchased from Amersham Biosciences, (NJ, USA). The miniPERM bioreactor was obtained from Vivascience (Hannover, Germany) and the BCA Protein Reagent Assay from Pierce Biotechnology Inc. (IL, USA). Solvable^{™} and UltimaGold^{™} were purchased from PerkinElmer Inc. (MA, USA). H₂O₂. The anti-poly(ADP-ribose) polymerase (PARP) monoclonal antibody (IgG1 mAb) clone C-2-10 was obtained from Oncogene^{™} Research Products (EMD Biosciences Inc., CA, USA). The anti-actin (N-20) affinity purified goat polyclonal antibody was purchased from Santa Cruz Biotechnology Inc. (CA, USA). Goat anti-mouse IgG alkaline phosphatase (AP)-conjugated antibody and rabbit anti-goat IgG AP-conjugated antibody were purchased from Johnson Laboratories (USA). The anti-La/SS-B IgG mAb 3B9 cell line (Tran *et al.,* 2002), prepared by Dr M. Bachmann (Oklahoma Medical Research Foundation, OK, USA), was a generous gift from Dr T.P. Gordon (Department of Immunology, Allergy, and Arthritis, Flinders Medical Centre, SA, Australia). The irrelevant 1D4.5 mAb Sal5 cell line, prepared by Dr L.K. Ashman (Medical Science Building, University of Newcastle, NSW, Australia), was a kind gift from Dr S. McColl (School of Molecular Biosciences, University of Adelaide, SA, Australia). These mAbs were affinity-purified on Protein G purification columns. Purified 3B9 and Sal5 mAbs were conjugated to fluorescein isothiocyanate (FITC) as described by manufacturer's instructions Sigma-Aldrich Co. (MO, USA). Anti-mouse IgG antibody conjugated to Alexa₄₈₈ and 7-AAD were purchased from Molecular Probes^{®} (Invitrogen, USA). Lymphoprep^{™} was purchased from Axis-Shield PoC AS (Oslo, Norway). Etoposide and vincristine for injection (Pfizer Inc., NY, USA) and cyclophosphamide, cisplatin were purchased.

### Cell lines and culture

The tumour cell lines, Jurkat (ATCC# TIB-152, acute T cell leukemia), EL4 (ATCC# TIB-39, mouse T-lymphocyte lymphoma), U-937 (ATCC# CRL-1593.2, monocytic leukemia) and Raji (ATCC# CCL-86, Burkitt's lymphoma) were routinely grown as suspension cultures in RPMI-1640 containing 5 % FCS and passaged every 48-72 h at 1:4 dilution. The U2OS osteosarcoma cell line (ATCC# HTB-96), SAOS-2 osteosarcoma cell line (ATCC# HTB-85) and HeLa cervical adenocarcinoma (ATCC# CCL-2) were routinely cultured in DMEM containing 5 % FCS and passaged every 48 - 72 h after detachment using trypsin-EDTA solution. The squamous cell carcinoma cell line, SCC-25 (ATCC# CRL-1628), was cultured in a 1:1 mixture of DMEM and Ham's F12 medium containing supplemented with 400 ng/mL hydrocortisone and 10 % FCS and passaged after detachment using trypsin-EDTA solution. Fresh blood from normal volunteers was subjected to Lymphoprep^{®} separation to isolate peripheral blood monocytic cells (PBMC). PBMC were cultured overnight in RPMI-1640 and 5 % FCS to separate suspension and adherent cells. The cells in suspension were defined as lymphocytes because more than 70% were CD3⁺ whereas the adherent cells were defined as monocytes because more than 70% were CD14⁺. Mouse thymocytes were obtained from mice. Finally, buccal cells were isolated from the gum lining of healthy volunteers as described.

Apoptosis in all cultures was induced by incubation of these cells in culture media described above and in the presence of specified concentrations of cytotoxic chemotherapy drugs (see Figure legends).

### Flow cytometry

Direct immunofluorescence staining was performed using 1-2 x 10⁵ cells at 10⁶ cells/mL for 30 min at room temperature in PBS containing 0.1 % BSA and 5 µg/mL of FITC-conjugated mAb. Cells were thoroughly washed using PBS and centrifugation at 450xg. Cells were resuspended in PBS containing 0.5 µg/mL PI and acquired immediately by a Becton-Dickinson FACScan™ flow cytometry system (BD Biosciences, CA, USA). Positive staining using the mAb-FITC conjugates was determined in comparison to FITC-conjugated isotype control mAb detected using the FL-1 channel (530-nm filter). Indirect immunofluorescent staining was performed using purified mouse antibodies followed by anti-mouse IgG conjugated to Alexa₄₈₈ detected using the FL-1 channel (530-nm filter). Cell viability was assessed by the exclusion of PI detected using the FL-2 (585-nm filter) or the exclusion of 7-AAD detected using the FL-3 (> 650 nm filter). Flow cytometry data was analysed using WinMDI v 2.8 (Scripps Research Institute, CA, USA). Unless otherwise specified, no gating was performed in any of the analysis shown in this paper.

### SDS-polyacrylamide gel electrophoresis (SDS-PAGE) and Western blotting

SDS-PAGE was performed as per manufacturer instructions using the Hoefer^{®} Mighty Small II SE 250 electrophoresis system (Amersham Biosciences, NJ, USA) under reducing condition using 12 % resolving polyacrylamide gel as per Laemmli (Nature 227: 680-685, 1970). Transfer of polyacrylamide gel onto Hybond-P membrane was carried out as per manufacturer instruction using the TE 22 Mini Tank Transfer Unit (Amersham Biosciences, NJ, USA). Blotting was performed as per standard procedure using 3B9 or anti-PARP mAbs followed by AP-conjugated anti-mouse IgG mAb or anti-actin polyclonal antibody (pAb) followed by AP-conjugated anti-goat IgG mAb. All blots were developed using the ECF^{™} substrate and scanned using the Fluorlmager^{™} 595 (Molecular Dynamics, Amersham Biosciences, NJ, USA) with 488 nm excitation laser and emission collected using 570 nm filter.

### Radioligand binding study with anti-La mAb

Sal5 isotype and 3B9 mAb were labelled with ¹⁴C by incubating the hybridoma cells (35 million cells) in 35 ml RPMI-1640 containing 10 % FCS in the production module of miniPERM bioreactor and 400 ml of RPMI-1640 containing 10 % FCS and 250 µCi of D- [U-¹⁴C]glucose and 250 µCi of L-[U-¹⁴C]Leucine in the nutrient module. The bioreactor was incubated in 5 % CO₂ humidified air at 37°C on a bottle-rotating device for 5 days. The medium in the production chamber was collected for antibody purification using protein G purification columns as per manufacturer instructions. Radioactivity of purified antibodies (10 µl sample) was counted in UltimaGold^{™} scintillation liquid (1 ml) for 20 min. using Tri-Carb 3100 β-counter (Packard, regularly calibrated using supplied ¹⁴C standards). Protein concentration was determined using BCA Protein Reagent Assay as per manufacturer instructions. The specific radioactivity of ¹⁴C-Sal5 and ¹⁴C-3B9 was 120.3 and 130.8 dpm/µg, respectively.

Saturation binding study was performed by incubating apoptotic EL4 cells (5 x 10⁵ cells) at 24 h after treatment with etoposide and cyclophosphamide with increasing concentration of ¹⁴C-3B9 in the absence (total) or presence (non specific) of 50-fold molar excess of unlabelled 3B9. After 30 min, cells were washed thoroughly using PBS and radioactivity was measured using the β-counter as described above. Specific binding was calculated as the difference between total and non-specific binding and plotted as a function of concentration of ¹⁴C-3B9. Competition binding curve was constructed by incubating apoptotic EL4 cells with ¹⁴C-3B9 in the presence of increasing concentrations of unlabelled 3B9. Radioactivity was measured as described earlier and-plotted as a function of unlabelled 3B9 concentration. Association time course was performed by incubation of apoptotic EL4 cells with ¹⁴C-3B9 in the absence or presence of 50-fold molar excess of unlabelled 3B9 for the specified times. Samples were washed and radioactivity was measure and specific binding was plotted as function of time.

### EL4 tumour model in C57BL/6 mice

EL4 cells, established from a lymphoma induced in a C57BL/6 mouse (Gorer, British Journal of Cancer 4: 372-379, 1950), were used to establish subcutaneous tumour implants in 6 - 8 weeks old C57BL/6 mice. Mice were housed and treated as per protocols approved by the Animal Ethics Committee at The University of Adelaide. Briefly, 10⁵ EL4 cells were injected subcutaneously in the right flank of each mouse. Once the tumour reached 1 cm diameter, mice were randomly divided into two groups one of which received intraperitoneal injection of etoposide and cyclophosphamide to achieve a dose of 76 mg/kg and 100 mg/kg, respectively (time 0). These two groups (untreated or treated) were used for the studied described below.

Treated mice received a second injection of etoposide and cyclophosphamide at 24 h after the first injection while untreated mice were left untreated. After 24 h (i.e. time point 48 h), all mice were euthanised, whole blood was obtained by cardiac puncture and EL4 tumours were excised from these sacrificed mice. Excised tumours tissue was disrupted to produce a single cell suspension, washed with PBS and used for immunofluorescent staining with 3B9 and PI and flow cytometry analysis as described earlier.

In other studies, treated mice that received the first chemotherapy injection at time 0, received a second injection of etoposide and cyclophosphamide at 24 h and an intravenous injection of specified amount of ¹⁴C-3B9 or ¹⁴C-Sal5. Untreated mice only received the intravenous injection of ¹⁴C-3B9 or ¹⁴C-Sal5. All mice were euthanased, whole blood was obtained by cardiac puncture and EL4 tumours as well as other organs were collected for radioactivity measurement. Serum and organs were solubilised using 1 ml of Solvable^{™} for 2 h at 50°C, decolourised using 100 µl of H₂O₂ (30 %). UltimaGold^{™} scintillation liquid (1 ml) was added and samples were counted for 10 min. using the β-counter.

### Results

### Anti-La mAb binds to apoptotic malignant EL4 thymic lymphoma cells in vitro

Cytofluographic analysis of cultured EL4 thymic lymphoblastic lymphoma cells, which were stained with the DNA binding dye 7-AAD as a test of membrane integrity, indicated that fewer than 10 % of the cultured cells were spontaneously apoptotic and did not bind La-specific 3B9 mAb (Figure 1A). In contrast, after EL4 cells were fixed and permeabilised, significantly more 3B9 bound than the isotype-matched control mAb, Sal5 (Figure 1B). This result indicated that specific binding of 3B9 to intracellular La depended on the loss of cell membrane integrity. After treatment of the cultured EL4 cells with the cytotoxic and DNA-damaging drugs, cyclophosphamide and etoposide, the majority of EL4 cells bound 7-AAD and, in contrast to the Sal5 control, virtually all 7-AAD⁺ cells bound 3B9 (upper outer quadrant, Figure 1C).

After cell permeabilisation, it was found that significantly higher levels of 3B9 bound to the malignant EL4 lymphoma cell line than to its normal murine counterpart cell type of the thymocyte (Figure 2A), which suggested that La was overexpressed in the malignant cells. This observation was confirmed by Western blot analysis using the 3B9 mAb to probe lysates of EL4 cells and thymocytes (Figure 2A - inset). Similarly, after cytotoxic drug treatment, which induced apoptosis of both EL4 cells and thymocytes, significantly higher levels of 3B9 bound to EL4 cells than to thymocytes (Figure 2A). Moreover, significantly more 3B9 bound EL4 cells after cytotoxic drug treatment than after cell permeabilisation, which suggested that cytotoxic drug treatment either induced higher levels of La expression and/or increased the availability of the 3B9 epitope on the La antigen (Figure 2A). In addition, treatment of the apoptotic EL4 and thymocytes with the non-ionic detergent, Triton X-100, did not alter the fluorescence intensity of 3B9-mediated detection of the La antigen in EL4 cells (Figure 2B and data not shown). Detergent resistance of the La signal suggested that the La antigen may be covalently cross linked during apoptosis induction. Further support for this contention is given by the result shown in Figure 2C. Sulforhodamine B stains proteins indiscriminately and sulforhodamine staining of malignant EL4 cells, which were rendered apoptotic by cytotoxic drug treatment, was also resistant to detergent treatment (Figure 2C). Surprisingly, Figure 2D shows that protein-protein cross linking in apoptotic cells was not confined to the La antigen but also included the 3B9 mAb itself. EL4 cells and thymocytes that were induced to undergo apoptosis in the presence of 3B9 mAb showed significantly higher binding of the detection reagent, anti-mouse IgG Alexa₄₈₈-conjugated antibody, than those cells undergoing apoptosis in the presence of the Sal5 isotype mAb (data not shown). This result further supports the notion that 3B9 binds specifically to apoptotic cells and preferentially to malignant apoptotic cells. Treatment of the apoptotic cells with Triton X-100 after antibody incubation indicated that binding of the 3B9 mAb was detergent resistant and suggested that 3B9 was cross linked to other proteins within the apoptotic malignant cells (Figure 2D).

### Anti-La mAb binds specifically, efficiently and irreversibly to apoptotic EL4 cells in vitro

La-specific mAb, which was biosynthetically labelled with the radioisotope carbon-14 (¹⁴C-3B9), bound to apoptotic EL4 cells after they were treated with cyclophosphamide and etoposide in a specific and saturable manner. The concentration of agent required to reach half-maximal saturation of one million apoptotic cells was 18 nM and maximal binding was ∼7500 femtomole/million apoptotic cells (Figure 3A). Half-maximal saturation of ¹⁴C-3B9 binding was found to occur within 5 minutes at room temperature (Figure 3B). Specific binding was largely irreversible as bound antibody did not dissociate when cells were incubated in binding buffer for 30 minutes at 37°C (Figure 3C). The concentration of unlabelled 3B9 required to inhibit half-maximal binding of ¹⁴C-3B9 (IC₅₀) was estimated to be 28 nM (Figure 3D). Altogether, these data describe specific, rapid, irreversible and high affinity binding of ¹⁴C-3B9 to apoptotic tumour cells. Binding was not detected when EL4 cells were tested in the absence of cytotoxic drugs (data not shown), which indicates that binding depends on apoptosis induction. It should be noted that these binding parameters relate to the binding of ¹⁴C-3B9 to apoptotic cells rather than the binding of antibody to the La antigen itself.

### Cytotoxic chemotherapy increases the target for 3B9 in the tumour mass

Single cell suspensions were prepared from tumour explants of EL4 tumour-bearing mice, which had been treated or not with cytotoxic chemotherapy. As illustrated in Figure 4, only the PI⁺ subpopulation of tumour cells displayed binding with the 3B9-FITC conjugate, which indicated that the La antigen had been recognised specifically in dead tumour cells. After the use of cytotoxic chemotherapy, the fraction of PI⁺ cells in the tumour explants increased significantly from 50 ± 2% to 70 ± 1% (P<0.0001) and, similarly, the 3B9⁺ subset of PI⁺ cells increased significantly from 15 ± 1 % to 28 ± 2% (P<0.01). In order to describe the effect of chemotherapy on 3B9 targeting to La, we analysed the frequency distributions of PI⁺ cells, which bound FITC conjugates and which are defined in the upper right quadrant of each density plot. As illustrated in the representative histograms in Figure 4, the specific binding of 3B9-FITC to PI⁺ cells was significantly augmented in tumours exposed *in vivo* to cytotoxic chemotherapy. For all tumour explant samples, the net MFI for La-specific staining (after subtraction of individual MFI values for Sal5 staining) was 18 ± 3 with chemotherapy and 1 ± 3 without chemotherapy (P<0.05). These results *provide in vivo* evidence of increased La expression and/or increased availability of the 3B9 epitope in dead cells obtained from tumours that were exposed to cytotoxic chemotherapy.

### La-specific mAb targets EL4 tumours in vivo especially after cytotoxic chemotherapy

EL4 tumour-bearing mice were used to demonstrate that 3B9 La-specific mAb targeted a tumour mass *in vivo* and that the 3B9 targeting was enhanced 48 hours after the mice were treated with cytotoxic chemotherapy. As illustrated graphically in Figure 5, 100µg ¹⁴C-labelled isotype control mAb (Sal5) did not accumulate significantly in any organ or tissue including the tumour. In contrast and compared with all other organs, 100µg ¹⁴C-3B9 accumulated significantly in serum and the tumour both before and after chemotherapy (P<0.001). Moreover, after the mice were treated with cytotoxic chemotherapy, only tumours accumulated significantly more ¹⁴C-3B9 (P<0.001) (Figure 5).

The biodistribution studies of ¹⁴C-3B9 in EL4 tumour-bearing mice were extended to include lower doses of the radiolabelled agent. Again, as shown in Figure 6, there was no significant accumulation of ¹⁴C-3B9 in any tissue except the tumour and only after administration of cytotoxic chemotherapy. The intra-tumoural accumulation of ¹⁴C-3B9 was dose-dependent and the sigmoidal dose-response relationship suggested that binding of the target was specific and saturable. In comparison to untreated mice, tumour uptake of ¹⁴C-3B9 with chemotherapy was significantly higher and demonstrated fold increases of 1.9, 1.9 and 1.8 at ¹⁴C-3B9 doses of 25 µg, 50 µg and 100 µg, respectively. Irrespective of the use of chemotherapy, no significant difference in the tumour uptake of ¹⁴C-3B9 was observed at a 5 µg dose. Although a 25µg dose of ¹⁴C-3B9 produced significant intra-tumoural accumulation 48 hours after administration of cytotoxic chemotherapy at an average 20 ± 3% of injected dose, the same dose of ¹⁴C-3B9 did not accumulate significantly in bone marrow, which received less than 3 ± 2% of the injected dose. These data further support the concept that La-specific 3B9 mAb selectively targets malignant tissues rather than critical normal tissues such as bone marrow, which is particularly susceptible to apoptosis after cytotoxic drug administration. Together with the previous data showing enhanced expression of La in EL4 tumour cells after *in vivo* use of cytotoxic chemotherapy (Figure 4), these data strengthen the contention that enhanced *in vivo* tumour targeting of 3B9 results from increased levels of cell death caused by cytotoxic chemotherapy.

### Anti-La as a potential tumour targeting agent for human malignancies

Using a murine tumour model, La-specific *in vitro* labelling of dead malignant cells and La-specific *in vivo* targeting of malignant tumours, which were both significantly augmented by the use of cytotoxic chemotherapy, have been described. Hence, it was sought to determine if similar findings applied to human malignant cells *in vitro.* First, it was found that in comparison to the counterpart normal cell type, La was overexpressed in the Jurkat T cell leukemia line (*cf.* CD3⁺ lymphocytes), the U-937 monocytic leukemia line (*cf.* CD14⁺ monocytes) and the oropharyngeal squamous cell carcinoma line SSC-25 (*cf.* normal buccal mucosal cells) (Figure 7). La overexpression was also found in the osteosarcoma lines, U2OS and SAOS-2 (*cf.* normal bone marrow stromal cells) and in the A549 bronchogenic carcinoma line (*cf.* normal human bronchial epithelium) (data not shown). Second, as shown earlier for EL4 tumour cells, cytofluographic analysis of human cancer cell lines showed that 3B9-FITC binding was evident only after treatment of these cells with cytotoxic drugs (Table 3). Third, La overexpression in malignant cells, which were rendered apoptotic by cytotoxic drug treatment, was resistant to detergent treatment (Figure 8A). Finally, as shown previously for EL4 cells and in contrast to normal primary cell counterparts, the detergent-resistant binding of 3B9 mAb to apoptotic human malignant cell lines was significantly higher as demonstrated by flow cytometry (Figure 8B) and laser scanning confocal microscopy (Figure 8C). These data suggested that 3B9 may be suitable for the *in vivo* targeting of dead cells within human tumours after cytotoxic chemotherapy and/or radiotherapy. For diagnostic purposes, 3B9 could be conjugated to radio-imaging agents and conjugation of 3B9 to cytotoxic radionuclides may provide a therapeutically useful means for delivery of bystander killing to surrounding viable malignant and supporting tissues.

**Table 3: Binding of La-specific mAb to apoptotic human cancer cell lines. Cell lines were cultured in the presence of 0.5 µM staurosporine (STS) (2 µM for Raji cell line), 20 µg/mL etoposide or 0.1 µg/mL vincristine. Cells were stained with Sal5-FITC or 3B9-FITC and analysed by dual-colour flow cytometry using PI for viability determination. After apoptosis induction, data shown are the percentages of PI⁺ and 3B9⁺ events after subtraction of the corresponding percentage of control (PI⁺ and Sal5⁺) events. ND, not determined.**

| **Cell line** | **Staurosporine** | **Etoposide** | **Vincristine** |
|---|---|---|---|
| **Jurkat** | 56.1% | 22.6% | 12.1% |
| **Raji** | 28.8% | 21.3% | 9.8% |
| **HeLa** | 62.5% | 36.1% | 18.6% |
| **U2OS** | 65.9% | 28.0% | ND |

### EXAMPLE 2

### NUCLEAR ANTIGENS SUITABLE FOR AUTO-TARGETED RADIOIMMUNOTHERAPY

The nuclear structural and molecular changes that accompany malignancy together with additional apoptosis-related changes in the expression levels, processing and translocation of the constituent nuclear proteins suggest that other of these proteins may be suitable targets for this approach. Hence, the criteria that have been developed to characterize targeting of the La antigen for the purpose of radio-imaging and radio-immunotherapy can be applied in order to define other appropriate nuclear target antigens for the application of the present technology (Apomab criteria). In particular, radiosensitising chemotherapy creates new abundant targets in the tumour mass, which were not previously available, and these revealed antigens may be targeted using monoclonal antibodies, which are conjugated to therapeutic radionuclides in order to deliver cytotoxic radiation to the radiosensitised tumour and supporting tissue.

The Apomab criteria used to determine nuclear antigens (Ag) that are particularly suitable for targeting dead tumour cells for diagnostic and therapeutic purposes are:
1. Ag is overexpressed in malignancy.
2. Anti-cancer treatment induces or increases Ag expression in dead tumour cells.
3. Ag in dead tumour cells is accessible to Ag-specific monoclonal antibody.
4. Ag is retained preferentially in dead tumour cells by protein cross-linking.
5. Ag-specific monoclonal antibody is retained preferentially in dead tumour cells by protein cross-linking.

By applying these criteria in part or in full, antigens can be targeted that produce an enhanced signal to noise ratio for diagnostic imaging and an improved therapeutic ratio for targeted radiation. Although the La targeting data suggest that some of these antigens may be accessible at low levels in untreated tumours, it is found that cytotoxic chemotherapy exposes an abundance of antigen-specific binding sites. Therefore, the combination of cytotoxic radiosensitising chemotherapy or non-cytotoxic radiosensitising drug and the method of RIT will not only produce more effective and safer anti-cancer treatment than either modality (chemotherapy or RIT) alone but will also be considerably more advantageous than other methods of RIT that target pre-existing dead cells.

As an example of how the Apomab criteria may be applied experimentally, monoclonal antibodies specific for other nuclear antigens were tested in a fluorocytometric assay of apoptotic cisplatin-treated Jurkat cells (Figure 9). La consistently displayed increased expression in apoptotic cisplatin-treated Jurkat cells after treatment with the non-ionic detergent, Triton X-100. Detergent treatment dissociates viable cells whereas apoptotic cells and bodies are resistant to this treatment.

### Scaling of Auto-targeted radioimmunotherapy

Consistent with pre-clinical animal data, further *in vitro* data are presented to discuss how the method of RIT in combination with radiosensitising cytotoxic chemotherapy may be applied clinically. The data presented in Figure 10 show that in comparison both with control cells and the treatment of cells with gemcitabine, which is a radiosensitising anti-metabolite, the treatment of cells with the radiosensitising and highly DNA damaging agent, cisplatin, resulted in significant enhancement of the La signal. Interestingly, further significant enhancement of the La signal was obtained after combined treatment of cells with gemcitabine and cisplatin. *In vitro* studies using pancreatic cancer cell lines showed that although cisplatin produced synergistic cytotoxicity with gemcitabine, cisplatin did not enhance gemcitabine-mediated radiosensitisation (Symon Z et al. Int J Radiat Oncol Biol Physics 53, 140-145, 2002).

The scheduling and dose of the component modalities of combination therapy may need to be varied to achieve the optimal combination. In particular, use of full doses of potent radiosensitizing drugs such as gemcitabine to induce cytotoxicity may cause apoptotic cell death in susceptible normal tissues such as bone marrow and the intestinal mucosa. Using the mouse jejunum as a model tissue, Milas and colleagues studied the effects of gemcitabine and showed that the time course of apoptosis was short and that recovery was complete within a 24-36 hour period (Milas L et al. Cancer Res 59, 107-114, 1999). In contrast, changing views about the modes of cell death in carcinomas induced by ionizing radiation or cytotoxic drugs indicate that the time course of cell death in tumours may be extended over a period of days or longer (Brown JM, Attardi LD. Nature Rev Cancer 5, 231-237, 2005). Hence, a window of opportunity may open for the use of combined modality RIT and radiosensitising chemotherapy that minimises harm to normal apoptosis-sensitive tissues while maximising the potential for further radiation-induced cell death to the tumour. In which case, the critical factors that will determine the therapeutic ratio of the RIT will include the relative half-lives of the monoclonal antibody and its conjugated radionuclide.

Combination chemotherapy with the radiosensitising drugs, cisplatin and gemcitabine, produces higher response rates in pancreatic cancer patients than the use of single agent gemcitabine but it does not improve overall survival and is associated with greater toxicity compared with the use of single agent gemcitabine. Thus, despite the higher response rate, combination cisplatin/gemcitabine chemotherapy presumably fails to eradicate systemic metastases, which re-grow to kill the patient. Armed Apomab enables delivery of higher and potentially curative doses of radiation to tumour metastases by taking advantage of the more potent cytotoxic effects and hence anti-tumour properties of the cisplatin/gemcitabine combination. Consequently, the creation of tumour targets (Auto-targeting) may be scaled to the schedule and dose of cytotoxic chemotherapy. In particular, greater targeting of armed Apomab to tumours after combination cisplatin/gemcitabine chemotherapy, results from two major effects, which are shown schematically in Table 4:
(i) higher levels of pancreatic tumour cell apoptosis *in vitro and in vivo* than after either drug alone;
(ii) cisplatin is a more potent DNA damaging agent than gemcitabine and together the two agents produce a higher level of La expression specifically in dead tumour cells than after the use of single agent gemcitabine.

**Table 4. Hypothetical scheme, which shows how both the degree of tumour cytotoxicity induced by treatment of a tumour-bearing animal with cisplatin and/or gemcitabine together with the level of intra-tumoural La expression induced by cisplatin and/or gemcitabine may contribute to the overall level of binding within the tumour of a La-specific radio-immunoconjugate.**

| Experimental Condition | Level of tumour cell apoptosis | Level of La expression in dead tumour cells | Hypothesized % of injected dose of armed Apomab |
|---|---|---|---|
| control (spontaneous) | + | + | X |
| gemcitabine | ++ | + | 2X |
| cisplatin | +++ | ++ | 4X |
| cisplatin/gemcitabine | +++ | +++ | 5X |

The potential significance of the scaling concept is related to the dose-dependent effects that radiation has on both supporting tumour vasculature and the tumour cells themselves. If Apomab RIT were given in conjunction with combination cytotoxic chemotherapy such as cisplatin and gemcitabine then higher *in vivo* binding to apoptotic/necrotic tumours would occur than if it were given in conjunction with single agent chemotherapy. In which case, it would be expected that higher doses of radiation would be delivered to neighbouring viable tumour cells. For example, if a radiation dose of≥8-10Gy were delivered to a tumour by combination cisplatin/gemcitabine chemotherapy then apoptosis of tumour endothelial cells may result from release of ceramide (Z Fuks and R Kolesnick. Cancer Cell 8, 89-91, 2005). However, if Apomab RIT delivered a radiation dose below this threshold to the tumour then further radioenhancement may result if ceramide generation were increased, for example, by using an inhibitor of sphingosine kinase I (SK1) or other radiosensitising agents such as inhibitors of VEGF or bFGF. At lower doses of radiation (1.8-3Gy), different anti-tumour mechanisms come into play such as generation of reactive oxygen species, which induce production of the hypoxia-inducible factor-1α (HIF1α). HIF1α protects against tumour endothelial cell apoptosis thus inducing radioresistance but simultaneously sensitises tumour cells to radiation in a p53-dependent manner (Fuks and Kolesnick 2005, *supra*). Hence, HIF1α inhibitors or inhibitors of VEGF or bFGF may provide additional radiosensitisation when delivering lower doses of radiation (1.8-3Gy) to tumours using Apomab RIT.

### EXAMPLE 3

### ANALYSIS OF GENE EXPRESSION PROFILING DATA TO IDENTIFY SUITABLE TARGETS FOR TCS

### Introduction

Oncomine^{™} is a cancer-specific database containing microarray data from 962 studies of which 209 were analysed. The database contains 14,177 microarrays from 35 cancer types (information publicly available at the website www.oncomine.org). Several cancer signatures have been deduced from large scale analysis of data held in the database (Hampton, Jama 292(17): 2073, 2004; Rhodes et al., Proc Natl Acad Sci USA 101(25): 9309-14, 2004a; Rhodes et al., Neoplasia 6(1): 1-6, 2004b; Rhodes and Chinnaiyan, Nat Genet 37 Suppl: S31-7, 2005; Rhodes et al., Nat Genet 37(6): 579-83, 2005). 209 studies in the database as described below were analysed in order to investigate certain malignancy signatures, which may provide useful targets for the present invention.

### Method

In the catalogue of the database Oncomine^{™} on www.oncomine.org, the tissue of interest was selected and only analysed data are shown. Studies were viewed using the Advanced Analysis module only for overexpressed genes and enrichment for these genes was achieved using the following options: (1) InterPro for analysis of motifs, (2) Gene Ontogeny (GO) molecular function for the analysis of function and (3) GO cellular component for cellular compartmentation analysis. Two parameters were used to describe the gene sets deduced from the above analysis: (a) Odds Ratio and (b) P-value. Only the groups of interest and genes with a P-value lower than 1E-4 were considered significant for reporting herein.

### Results

As shown in Table 5 and Table 6, the ribonucleoprotein (RNP-1) motif was identified , which comprises the RNA recognition motif (RRM), to be at higher odds of being associated with a cancer signature compared to the nucleus as a cellular component. Similarly, the RNA binding gene set, which was enriched as a cellular function, was also at high odds of being associated with a cancer signature. Finally and more importantly, nucleolar and heteronuclear ribonucleoprotein (hnRNP) components appeared to have very high odds as gene sets in association with a cancer signature.

### Conclusion

Consequently, components with the RNP-1 motif generally and hnRNP complex specifically are suitable targets for the method of the present invention. This recommendation is justified by (1) association of hnRNP with cancer at the gene expression level (mRNA data reported herein), (2) the relative abundance of this protein and its correlation with cancer and cancer progression (see review Carpenter et al., Biochimica et Biophysica Acta 1765(2): 85-100, 2006), and (3) the reorganisation of the hnRNP network during apoptosis into the HERDS (Biggiogera et al., Biologie Cellulaire 96(8): 603-15. 2004) and the accessibility of the hnRNP network for detection with antibodies. Nucleolar proteins generally and specifically nucleophosmin represent suitable targets for this strategy. This is justified by the data shown in the analysis of the Oncomine^{™} data base as well as the literature regarding nucleolar function in cancer (Maggi and Weber, Cancer Investigation 23(7): 599-608, 2005) and, in particular, nucleophosmin as putative-proto-oncogene *(*Grisendi et al., Nature Reviews. Cancer 6(7): 493-505,2006).

**Table 5: mRNA expression in clinical tumors. La mRNA is upregulated in malignancy**

| **Tissue type** | **No. genes** | **Comparison** | **% of overexpressed genes** | **Enrichment type** | **Group** | **Odds Ratio** | **P-value** |
|---|---|---|---|---|---|---|---|
| Adrenal gland (Giordano *et al.,* 2003) | 12625 | Adrenocortical carcinoma *vs.* normal adrenal cortex, adrenocortical adenoma and macronodular hyperplasia | 2.3% | InterPro motif | **RNP-1 motif** | **2.98** | **7.8E-4** |
| | | | | | e.g. hnKNP A1/B2 | | 9E-4 |
| | | | | Cellular function | **RNA binding** | **2.06** | **4.4E-5** |
| | | | | Cellular component | **Nucleus** | **1.93** | 7.8E-23 |
| | | | | | **hnRNP complex** | **11.72** | 3.9E-4 |
| Brain (Phillips *et al.,* 2006) | 44792 | grade IV *vs.* grade III astrocytoma | 9.6% | InterPro motif | RNP-1 motif | **2.25** | **1.7E-5** |
| | | | | | e.g. La/SS-B | | 1.5E-10 |
| | | | | | hnRNP H1 | | 1.0E-8 |
| | | | | Cellular function | **RNA binding** | **1.78** | **9.6E-8** |
| | | | | Cellular component | **Nucleus** | **1.53** | **7.4E-17** |
| | | | | | **hnRNP complex** | **11.38** | 2.5E-5 |
| | | | | | **Nucleolus** | **4.38** | **6E-4** |
| | | | | | e.g. nucleolin | | 5.7E-5 |
| Brain (Phillips *et al., 2006,supra)* | 44792 | dead *vs*. alive in astrocytoma 5-year survival | 1.0% | InterPro motif | **RNP-1 motif** | **1.75** | **3.8E-4** |
| | | | | | e.g. La/SS-B | | 3.3E-5 |
| | | | | | hnRNP H1 | | 3.7E-4 |
| | | | | Cellular function | **RNA binding** | **1.81** | **5E-8** |
| | | | | Cellular component | **Nucleus** | **1.75** | **1E-27** |
| | | | | | **hnRNP complex** | **7.36** | **3.4E-4** |
| Brain (Khatua *et al.,* 2003) | 12625 | High grade *vs*. low grade astrocytoma | 1.3% | InterPro motif | **RNP-1 motif** | **2.59** | **1.6E-6** |
| | | | | | e.g. hnRNP A/B | | 2.3E-4 |
| | | | | Cellular function | **RNA binding** | **2.8** | 1.7E-14 |
| | | | | Cellular component | **Nucleus** | **1.88** | **2.2E-21** |
| | | | | | **hnRNP complex** | **17.47** | 2.4E-7 |
| | | | | | **Nucleolus** | **4.03** | 2.2E-4 |
| Brain (Shai *et al.,* 2003) | 12625 | Glioblastoma Multiforme *vs*. Normal white matter | 17% | InterPro motif | **RNP-1 motif** | **2.29** | **3.3E-5** |
| | | | | | e.g. hnRNP A0 | | 6.6E-10 |
| | | | | Cellular function | **RNA binding** | **2.43** | **7E-11** |
| | | | | Cellular component | **Nucleus** | **1.47** | **7.5E-9** |
| | | | | | e.g. PCNA | | 8.9E-7 |
| | | | | | **hnRNP complex** | **6.75** | **3.6E-4** |
| | | | | | **Nucleolus** | **4.38** | **6E-4** |
| | | | | | e.g. nucleolin | | 5.7E-5 |
| Brain (Liang *et al.,* 2005) | 23079 | Dead vs. alive glioblastoma multiforme 1 year survival | 0.2% | InterPro motif | **RNP-1 motif** | **2.70** | **1.2E-8** |
| | | | | | e.g. hnRNP A3 | | 2.5E-4 |
| | | | | Cellular function | **RNA binding** | **2.38** | **5.7E-12** |
| | | | | Cellular component | **Nucleus** | **1.75** | **4.8E-12** |
| | | | | | **Nucleolus** | **3.3** | **9.6E-4** |
| Brain (Rickman *et al.,* 2001) | 6668 | Glioma *vs*. normal neocortex of temporal lobe | 7% | InterPro motif | **RNP-1 motif** | **2.64** | **9.9E-5** |
| | | | | | e.g. hnRNP H1 | | 1.2E-4 |
| | | | | Cellular function | **RNA binding** | **3.13** | **1.1E-12** |
| | | | | Cellular component | **hnRNP complex** | **19.64** | **5.6E-6** |
| Breast (Wang *et al.,* 2005) | 22283 | Relapse vs. no disease in breast carcinoma - 5-year disease free survival | 1.1% | InterPro motif | **RNP-1 motif** | **2.08** | **2E-5** |
| | | | | | e.g. hnRNP A1 | | 3.7E-4 |
| | | | | Cellular function | **RNA binding** | **1.66** | **3E-5** |
| | | | | Cellular component | **Nucleus** | **1.44** | **1.1E-10** |
| Breast (Wang *et al.,* 2005, *supra*) | 22283 | Relapse *vs*. no disease in ER⁺ breast carcinoma; 5-year disease free survival | 0.8% | InterPro motif | **RNP-1 motif** | **2.51** | **4.7E-8** |
| | | | | Cellular function | **RNA binding** | **1.86** | **2.4E-7** |
| | | | | Cellular component | **Nucleus** | **1.5** | **6.4E-13** |
| Breast (Dairkee *et al.,* 2004) | 10761 | Breast cancer *vs*. Breast Cancer Culture and Immortalized Breast Cell-Line | 8.7% | InterPro motif | **RNP-1 motif** | **3.54** | **3.2E-8** |
| | | | | | e.g. hnRNP H1 | | 5.7E-6 |
| | | | | | hnRNP D | | 2.2E-5 |
| | | | | Cellular function | **RNA binding** | **2.46** | **2.4E-7** |
| | | | | Cellular component | **Nucleus** | **1.66** | **4.8E-11** |
| Mammary epithelial cells - oncogene transfected (Bild *et al.,* 2006) | 54675 | Activated H-Ras *vs.* GFP-transfected primary cells | 16.1% | InterPro motif | **RNP-1 motif** | **2.21** | **1.5E-7** |
| | | | | | e.g. hnRNP A/B | | 2.6E-11 |
| | | | | | Nucleolin | | 3.2E-9 |
| | | | | | La/SS-B | | 5.1E-5 |
| | | | | Cellular function | **RNA binding** | **2.08** | **3E-12** |
| | | | | Cellular component | **Nucleolus** | **4.2** | **3.4E-5** |
| | | | | | e.g. nucleophosmin | | 6.5E-7 |
| Mammary epithelial cells - oncogene transfected (Bild *et al.,* 2006, *supra*) | 54675 | c-Myc *vs*. GFP transfected primary cells | 3.7% | InterPro motif | **RNP-1 motif** | **2.5** | **7.6E-7** |
| | | | | | e.g. Nucleolin | | 2.8E-11 |
| | | | | | hnRNP A/B | | 9.8E-7 |
| | | | | | La/SS-B | | 8.5E-6 |
| | | | | Cellular function | **RNA binding** | **3.13** | **1.2E-20** |
| | | | | Cellular | **Nucleolus** | **12.57** | **9.3E-13** |
| | | | | component | e.g. nucleophosmin | | 7.2E-6 |
| Colon (Notterman *et al.,* 2001) | 6745 | Colon adenocarcinoma vs. normal colon | 7.2% | InterPro motif | **RNP-1 motif** | **4.11** | **1.2E-6** |
| | | | | | e.g. hnRNP F | | 2.2E-8 |
| | | | | | hnRNP D | | 5.7E-5 |
| | | | | | La/SS-B | | 6.7E-5 |
| | | | | Cellular function | **RNA binding** | **3.92** | **2E-16** |
| | | | | Cellular | Nucleus | **1.66** | **4.6E-8** |
| | | | | component | e.g. PCNA | | 1.1E-9 |
| | | | | | **hnRNP complex** | **20.15** | **4.8E-6** |
| | | | | | **Nucleolus** | **7.62** | **3.5E-5** |
| | | | | | **e.g. nucleophosmin** | | **9.2E-12** |
| Colon (Alon *et al.,* 1999) | 1988 | Colon adenocarcinoma *vs*. normal colon | 9.7% | InterPro motif | **RNP-1 motif** | **4.99** | **1.3E-5** |
| | | | | | e.g. hnRNP F | | 2.9E-4 |
| | | | | Cellular function | **RNA binding** | 5.79 | **2.6E-13** |
| | | | | Cellular component | **Nucleus** | 1.73 | **2.4E-4** |
| Liver (Chen *et al.,* 2002) | 22033 | Metastasis to liver *vs.* hepatocellular carcinoma | 6.2% | InterPro motif | **RNP-1 motif** | **2.33** | **1.2E-6** |
| | | | | | e.g. hnRNP A3 | | 1.2E-5 |
| | | | | Cellular function | **RNA binding** | **1.91** | 2.5E-7 |
| | | | | Cellular component | **Nucleus** | **1.38** | **1.7E-7** |
| Lung (Bhattacharjee *et al.,* 2001) | 11158 | Lung adenocarcinoma vs. normal lung | 31.1% | InterPro motif | **RNP-1 motif** | 2.72 | **6.3E-7** |
| | | | | | e.g. hnRNP C1/C2 | | 9.5E-7 |
| | | | | | hnRNP A3 | | 1.6E-6 |
| | | | | | La/SS-B | | 1.3E-5 |
| | | | | Cellular function | **RNA binding** | **3.1** | **7.4E-17** |
| | | | | Cellular component | **Nucleus** | **1.31** | **6.8E-5** |
| | | | | | **e.g. PARP-1** | | 3.5E-9 |
| Lung (Bhattacharjee *et al.,* 2001, *supra*) | 10881 | Small cell lung cancer *vs.* normal lung | 26.6% | InterPro motif | **RNP-1 motif** | **3.82** | **8.2E-12** |
| | | | | | e.g. hnRNP D | | 1E-4 |
| | | | | Cellular function | **RNA binding** | **3.04** | **8.3E-16** |
| | | | | Cellular | **Nucleus** | **2.19** | **1.3E-29** |
| | | | | component | e.g. PCNA | | 3.6E-6 |
| | | | | | PARP-1 | | 4.4E-6 |
| Lung (Bhattacharjee *et al.,* 2001, *supra*) | 10030 | Squamous cell lung carcinoma *vs.* normal lung | 7.1% | InterPro motif | **RNP-1 motif** | **2.39** | **2.7E-5** |
| | | | | | e.g. hnRNP A3 | | 8.5E-7 |
| | | | | Cellular function | **RNA binding** | 2.72 | 9.2E-13 |
| | | | | Cellular component | **Nucleus** | **1.48** | **3.4E-8** |
| | | | | | e.g. PARP-1 | | 5.7E-8 |
| | | | | | PCNA | | 7.7E-8 |
| | | | | | **hnRNP complex** | 6.27 | **8.1E-4** |
| Lymph (Hummel *et al.,* 2006) | 22215 | Ig-Myc fusion vs. translocation negative lymphoma | 27.9% | InterPro motif | **RNP-1 motif** | **3**.**94** | **2.2E-17** |
| | | | | | e.g. hnRNP A3 | | 4.4E-20 |
| | | | | | La/SS-B | | 5.6E-13 |
| | | | | | hnRNP A2/B1 | | 6.3E-11 |
| | | | | Cellular function | **RNA binding** | **4.11** | 5.9E-37 |
| | | | | Cellular component | **Nucleus** | **1.71** | 2.1E-21 |
| | | | | | **hnRNP complex** | **15.22** | 3.1E-6 |
| | | | | | **Nucleolus** | **8.68** | 4.9E-11 |
| | | | | | e.g. nucleophosmin | | 4.7E-28 |
| Lymph (Hummel *et al.,* 2006, *supra*) | 22215 | Definite Burkitt's lymphoma *vs*. not definite | 27.9% | InterPro motif | **RNP-1 motif** | **3.47** | **2.7E-14** |
| | | | | | e.g. La/SS-B | | 1.7E-9 |
| | | | | | hnRNP A3 | | 1.3E-6 |
| | | | | | Nucleolin | | 1.9E-4 |
| | | | | Cellular function | **RNA binding** | **4.31** | **5.6E-29** |
| | | | | Cellular component | **Nucleus** | **1.71** | **2.1E-21** |
| | | | | | **hnRNP complex** | **9.22** | **1.6E-4** |
| | | | | | **Nucleolus** | **3.43** | **2E-4** |
| | | | | | e.g. nucleophosmin | | 2.1E-5 |
| Mesothelioma (Gordon *et al.,* 2005) | 22215 | Malignant mesothelioma *vs*. pleura | 2.6% | InterPro motif | **RNP-1 motif** | **2.02** | **3.4E-5** |
| | | | | | e.g. hnRNP A/B | | 1E-5 |
| | | | | | hnRNP H1 | | 5.1E-4 |
| | | | | Cellular function | **RNA binding** | **1.81** | 5.3E-7 |
| | | | | Cellular component | **Nucleus** | **1.47** | 1.3E-7 |
| | | | | | **hnRNP complex** | **10.16** | 4.2E-5 |
| Ovary (Schaner *et al.,* 2003) | 21057 | Undifferentiated *vs*. clear cell, endometrioid and Serous Papillary histological subtypes of ovarian carcinoma | 1.3% | InterPro motif | **RNP-1 motif** | **3.16** | **3.6E-8** |
| | | | | Cellular function | **RNA binding** | **2.07** | **3.8E-6** |
| | | | | Cellular component | **hnRNP complex** | **9.12** | **4.1E-4** |
| Prostate (Schaner *et al., 2003, supra*) | 54675 | Prostate carcinoma *vs.* normal prostate | 1.3% | InterPro motif | **RNP-1 motif** | **2.06** | **2.2E-6** |
| | | | | | e.g. hnRNP C | | 1.5E-4 |
| | | | | Cellular function | **RNA binding** | **1.66** | **2.7E-6** |
| | | | | Cellular component | **Nucleolus** | 4.03 | **5.9E-5** |
| | | | | | e.g. nucleophosmin | | 2.4E-4 |
| | | | | | **hnRNP** complex | 9.61 | 6.3E-5 |
| Prostate (Vanaja *et al.,* 2006) | 44928 | Metastatic *vs.* primary vs. benign prostate | 2.4% | InterPro motif | **RNP-1 motif** | **2.22** | **2.2E-5** |
| | | | | | e.g. hnRNP C | | 5.6E-4 |
| | | | | Cellular function | **RNA binding** | **3.4** | **5.8E-15** |
| | | | | Cellular component | **Nucleus** | **1.39** | **2.6E-9** |
| | | | | | **hnRNP complex** | **11.51** | 2.3E-5 |
| | | | | | **Nucleolus** | **7.84** | 7.2E-8 |
| | | | | | e.g. nucleophosmin | | 1.2E-5 |
| Prostate (Singh *et al.,* 2002) | 9892 | Prostate tumour *vs.* non-tumour prostate | 4.9% | InterPro motif | **RNP-1 motif** | **3.08** | **1.9E-8** |
| | | | | | e.g. hnRNP A0 | | 3.2E-4 |
| | | | | | La/SS-B | | 9.5E-4 |
| | | | | Cellular function | **RNA binding** | **3.43** | **1.5E-19** |
| | | | | Cellular component | **hnRNP complex** | **10.52** | **1.2E-5** |
| Prostate (Vanaja *et al., 2006, supra*) | 44928 | Metastatic *vs*. primary *vs*. benign prostate | 2.4% | InterPro motif | RNP-1 motif | 2.22 | 2.2E-5 |
| | | | | | e.g. hnRNP C | | 5.6E-4 |
| | | | | Cellular function | RNA binding | 3.4 | 5.8E-15 |
| | | | | Cellular component | Nucleus | 1.39 | 2.6E-9 |
| | | | | | hnRNP complex | 11.51 | 2.3E-5 |
| | | | | | Nucleolus | 7.84 | 7.2E-8 |
| | | | | | e.g. nucleophosmin | | 1.2E-5 |
| Salivary gland (Frierson *et al.,* 2002) | 10142 | Adenoid Cystic Carcinoma of Salivary Gland *vs.* Normal Salivary Gland | 13.5% | InterPro motif | **RNP-1** motif | **4.96** | 9.7E-17 |
| | | | | | e.g. hnRNP H3 | | 6.6E-9 |
| | | | | | hnRNP A2/B1 | | 2.0E-4 |
| | | | | | ......La/SS-B | | 6.4E-4 |
| | | | | Cellular function | **RNA binding** | **3.01** | 3.7E-15 |
| | | | | Cellular component | **Nucleus** | **2.05** | 3.4E-24 |
| | | | | | **hnRNP complex** | **8.76** | **5.1E-4** |

**TABLE 6**

| **Tissue type (reference)** | **No. genes in study** | **Comparison** | **% of overexpressed genes** | **P-value for La mRNA over-expression** |
|---|---|---|---|---|
| Lymphoma (23) | 22215 | IgG-Myc fusion *vs.* fusion-negative lymphoma | 27.9% | **1.1E-12** |
| Brain (11) | 44792 | grade IV *vs*. grade III astrocytoma | 9.6% | **2.9E-10** |
| Lymphoma (23) | 22215 | Definite Burkitts Lymphoma *vs*. not definite | 27.9% | **3.4E-9** |
| Seminoma (29) | 44760 | Adult male germ cell tumor *vs*. normal testis | 40.1% | **4.7E-7** |
| Mammary epithelial cells - oncogene transfected (18) | 54675 | Activated H-ras vs GFP transfected primary cells | 16.1% | **2E-6** |
| Brain (11) | 44792 | dead *vs*. alive in astrocytoma 5 year survival | 1.0% | **6.5E-5** |
| Lung (22) | 11158 | Lung adenocarcinoma vs. normal lung | 31.1% | **2.6E-5** |
| Mammary epithelial cells - oncogene transfected (18) | 54675 | c-Myc vs GFP transfected primary cells | 3.7% | **2E-5** |
| Multiple cancers (30) | 15708 | Progression of primary cancer *vs*. normal tissue | 11.1% | **5E-4** |
| Lung (31) | 22646 | Lung adenocarcinoma *vs*. normal Lung | 8.6% | **1.6E-4** |
| Colon (19) | 6745 | Colon adenocarcinoma *vs*. normal colon | 7.2% | **1.3E-4** |
| Tongue (32) | 12558 | Tongue squamous cell carcinoma *vs*. normal tongue | 23.5% | **1.3E-4** |
| Multiple cancers (30) | 14252 | Progression of metastatic cancer *vs*. primary cancer | 4.8% | **1.2E-4** |
| Oral (33) | 14119 | Oral squamous cell carcinoma *vs*. oral squamous epithelium | 4.2% | **1.2E-4** |
| Brain (34) | 44692 | Grade V *vs*. Grade III glioma | 13.4% | **0.001** |
| Head-Neck (35) | 22215 | Head and neck squamous cell carcinoma *vs*. normal oral Mucosa | 21.4% | **0.001** |
| Melanoma | 22283 | Melanoma *vs*. normal skin and nevus | 30.5% | **0.001** |
| Ovary (36) | 4995 | Ovarian adenocarcinoma *vs*. normal ovary | 11.3% | **0.001** |
| Lung (31) | 22646 | squamous cell carcinoma *vs*. normal lung | 8.6% | **0.002** |
| Prostate (27) | 9892 | Prostate tumor *vs.* non-tumor prostate | 4.9% | **0.002** |
| Lung (22) | 12241 | Metastatic *vs*. primary adenocarcinoma | 48.8% | **0.003** |
| Ovarian (37) | 63174 | Serous ovarian carcinoma *vs*. ovarian surface epithelium | 2.3% | **0.003** |
| Brain (11) | 44792 | Grade IV necrosis positive vs. negative astrocytoma | 0.6% | **0.004** |
| Brain (13) | 12625 | Glioblastoma multiforme *vs*. normal white matter | 17% | **0.006** |
| Lung (22) | 10030 | squamous cell lung carcinoma *vs*. normal lung | 7.1% | **0.006** |
| Liver (21) | 22033 | Metastasis to liver *vs*. hepatocellular carcinoma | 6.2% | **0.007** |
| Lung (22) | 10881 | Small cell lung cancer *vs*. normal lung | 26.6% | **0.014** |
| Breast (16) | 22283 | Relapse vs. no disease in 5-year disease free survival study in breast carcinoma | 1.1% | **0.016** |
| Cell line (38) | 22215 | Camptothecin-treated HeLa cells *vs*. non-treated | 0.9% | **0.024** |
| Brain (34) | 44692 | Dead *vs*. alive in 3-year survival study of glioma | 5.9% | **0.025** |
| Breast (39) | 44611 | Alive *vs*. Dead in 5-year survival study of breast carcinoma | 0.6% | **0.036** |
| Breast (40) | 7937 | Breast carcinoma *vs*. benign breast | 4.6% | **0.037** |
| Prostate (41) | 19111 | Lymph node metastasis *vs*. prostate cancer | 3.9% | **0.049** |

### EXAMPLE 4

### IN VITRO RATIONALE FOR TARGETTING OF THE RIBONUCLEOPROTEIN La IN A MOUSE TUMOUR MODEL

### Materials and Methods

### Materials

The suppliers of the materials are identified in brackets after each material. Cell culture media, RPMI-1640, DMEM and Ham's F12, and fetal calf serum (FCS) (JRH Biosciences Inc., Lenexa, KS); Trypsin-EDTA solution, trypan blue, propidium iodide (PI), bovine serum albumin (BSA), BCIP/NBT premixed substrate solution for alkaline phophatase (AP), hydrocortisone, monodansylcadaverine (MDC) and staurosporine (STS) and mouse anti-human β-tubulin mAb (TUB 2.1) (Sigma-Aldrich Co., St. Louis, MO). Hybond-P membrane (PVDF) and protein G purification columns (Amersham Biosciences, Piscataway, NJ). BCA Protein Reagent Assay (Pierce Biotechnology Inc., Rockford, IL). Anti-poly(ADP-ribose) polymerase (PARP) monoclonal antibody (mAb) clone C-2-10 and anti-proliferating cell nuclear antigen (PCNA) mAb clone PC10 (Oncogene Research Products, Cambridge, MA). Trichostatin A (TSA), anti-phospho-histone H2AX (Ser139) clone JBW301 biotin-conjugated mAb and anti-human H2A polyclonal antibody (Millipore Inc., Billerica, MA). Anti-β fodrin mAb (Chemicon International, Temecula, CA). Anti-β actin affinity-purified rabbit polyclonal antibody, Fluorescein isothiocyanate (FITC)-conjugated goat anti-rabbit IgG and AP-conjugated goat anti-rabbit IgG antibodies (Rockland, Gilbertsville, PA). The anti-La/SS-B 3B9 mAb hybridoma is a murine IgG₂ₐ autoantibody, which is crossreactive with human La and which was prepared by Dr M. Bachmann (Oklahoma Medical Research Foundation, OK, USA), was a kind gift from Dr T.P. Gordon (Department of Immunology, Allergy and Arthritis, Flinders Medical Centre, SA, Australia). The isotype control Sal5 (1D4.5) mAb hybridoma, prepared by Dr L.K. Ashman (Medical Science Building, University of Newcastle, NSW, Australia), was kindly supplied by Dr S. McColl (School of Molecular Biosciences, University of Adelaide, SA, Australia). The mAb were affinity-purified using protein G columns and FITC-conjugates were prepared according to the manufacturer's instructions (Sigma-Aldrich Co., St. Louis, MO). Anti-human nucleolin mAb, anti-human nucleophosmin (NPM) mAb, anti-mouse IgG antibody conjugated to Alexa₄₈₈, streptavidin-Alexa₄₈₈, streptavidin-PE, 7-amino-actinomycin D (7-AAD) and biotinylated cadaverine (Invitrogen, Carlsbad, CA). Lymphoprep (Axis-Shield PoC AS, Norton, MA). Etoposide (Pfizer Inc., NY, USA), cyclophosphamide and cisplatin (Bristol-Myers Squibb Company, Princeton, NJ) and gemcitabine (Eli Lilly, Indianapolis, IN) were obtained from the Royal Adelaide Hospital Cytotoxics Pharmacy (Adelaide, SA, Australia).

### Cell culture

Suspension cultures of Jurkat and U-937 leukemia cell lines were maintained in RPMI-1640 containing 5% FCS and passaged by splitting at 1:10 every 72h. Cultures of adherent MDA-MB-231, MCF-7, PC-3, LNCaP and A549 cancer cell lines were maintained in RPMI-1640 containing 5% FCS and passaged every 48-72h at a 1:4 dilution after detachment with trypsin-EDTA solution. Adherent sub-confluent cultures of the pancreatic adenocarcinoma cell line, PANC-1, were routinely cultured in RPMI-1640 containing 10% FCS and passaged as above. The squamous cell carcinoma cell line, SCC-25, was cultured in a 1:1 mixture of DMEM and Ham's F12 medium supplemented with 400ng/mL hydrocortisone and 5% FCS and passaged after detachment using trypsin-EDTA solution. Peripheral blood mononuclear cells (PBMC) were isolated from fresh heparinized blood obtained from normal healthy volunteer donors using Lymphoprep separation and cultured overnight in RPMI-1640 containing 5% FCS to separate adherent cells from those remaining in suspension. Cytofluographic analysis indicated that >70% of both suspension and adherent cells were CD3⁺ and CD14⁺, respectively. Hence, CD3- and CD14-enriched PBMC preparations will be described as peripheral blood lymphocytes and monocytes, respectively. Clonetics conditioned cultures of primary human cells were maintained according to the manufacturer's instructions (Cambrex Corporation, East Rutherford, NJ) and included cultures of Human Mammary Epithelial Cells (HMEC), Prostate Epithelial Cells (PrEC) and Normal Human Bronchial Epithelium (NHBE). Finally, buccal cells were isolated from the gum lining of normal healthy volunteer donors as described.

### Induction of apoptosis or cell death and cell permeabilization

Apoptosis or cell death was induced by adding cytotoxic drugs to culture media at the specified concentrations. In some experiments, cells were starved by serum deprivation. In other experiments, TSA used at the specified concentrations was prepared from 1 mg/mL stock solution in absolute ethanol. Control (untreated) cells had ≥90% viability determined by PI or trypan blue staining (data not shown). Viable cells were fixed and permeabilized by incubating cells at 5x10⁶ cells/mL in 2% w/v paraformaldehyde solution in PBS (150mM sodium phosphate and 150 sodium chloride, pH 7.2) for 10min followed by 1:10 dilution in absolute methanol (at -20°C) for 1-3 min. before a final wash with PBS.

### Flow cytometry

Indirect immunofluorescence staining was performed using purified mouse antibodies at 5µg/mL for 30 min. at room temperature (RT) in PBS followed by Alexa₄₈₈-conjugated anti-mouse IgG at 2µg/mL for 30 min. at RT in PBS. Fluorescence was detected using the FL-1 channel (530nm filter). Cell viability was assessed by the exclusion of PI (0.5µg/mL) and detected using the FL-2 channel (585nm filter) or by the exclusion of 7-AAD (2µg/mL for 15 min. at RT) and detected using the FL-3 channel (>650nm filter). Staining for γH2AX was performed using 0.2µg/mL of anti-γH2AX-biotin for 30 min. at RT followed by 2µg/mL of streptavidin-PE or streptavidin-Alexa₄₈₈. Staining of polyclonal antibodies (anti-actin or anti-H2A) was performed using 5µg/mL of antibody solution for 30 min. at RT. Control incubations were performed using protein G purified rabbit IgG from normal rabbit serum (IMVS, SA, Australia). Cells were washed then incubated (30min. at RT) with 2µg/mL of FITC-conjugated anti-rabbit IgG antibody, which was detected using the FL-1 channel. Samples were acquired immediately by a Becton-Dickinson FACScan™ flow cytometry system (BD Biosciences, San Jose, CA). Acquisition was standardized to 10,000 events or in some cases standardized to a set time for acquisition (in seconds) to allow comparison of cell counts in different incubations. Flow cytometry data were analyzed using WinMDI v2.8 (Scripps Research Institute, La Jolla, CA). Unless otherwise specified, no gating was performed in any of the reported analyses. Specific binding of antibodies was calculated as the difference in mean fluorescence intensities (MFI) between the test antibody and the control isotype antibody and expressed as the Net MFI ± standard error of the mean (SEM), which was calculated from replicate incubations (n>2).

### SDS polyacrylamide gel electrophoresis (SDS-PAGE) and immunoblotting

Cell lysates were prepared in SDS lysis solution (2% w/v SDS, 10% v/v glycerol and 62.5 mM Tris-HCl) and protein concentration was determined using BCA protein reagent assay kit according to the manufacturer's instructions. Bromophenol blue (0.05% w/v) and β-mercaptoethanol (5% v/v) were added to lysates after BCA assay measurement. SDS-PAGE was performed according to the manufacturer's instructions with the Hoefer Mighty Small II SE 250 electrophoresis system (Amersham Biosciences, Piscataway, NJ) under reducing conditions using 12% resolving polyacrylamide gel as per Laemmli's method. The transfer of the polyacrylamide gel to Hybond-P membrane was carried out according to the manufacturer's instructions using the TE 22 Mini Tank Transfer Unit (Amersham Biosciences, Piscataway, NJ). Immunoblotting was done using standard methods in which staining with 3B9 was followed by staining with AP-conjugated anti-mouse IgG mAb (Jackson ImmunoResearch Laboratories Inc., West Grove, PA) or anti-actin (N-20) affinity purified goat polyclonal antibody (Santa Cruz Biotechnology Inc., Santa Cruz, CA) followed by AP-conjugated anti-goat IgG mAb (Jackson ImmunoResearch). Some blots were developed using the BCIP/NBT premixed solution as specified by the manufacturer and analyzed using GelPro Analyzer v3.1 (Media Cybernetics Inc., Silver Spring, MD).

Other blots were developed using the ECF^{™} substrate (Amersham Biosciences, Piscataway, NJ) and scanned using the Fluorlmager^{™} 595 (Molecular Dynamics, Amersham Biosciences, Piscataway, NJ) with a 488nm excitation laser and the emissions were collected using a 570nm filter.

### Assays of transglutaminase-mediated crosslinking

Transglutaminase 2 (TG2)-mediated protein-protein crosslinking in apoptotic cells was investigated using a modification of a previously described method. Briefly, cells were resuspended at 1x10⁶ cells/mL in PBS containing 1% Triton X-100, 0.2µg/mL sulforhodamine 101 and 0.1µM SytoxGreen. Samples were vortexed and incubated for 5min. before analysis by flow cytometry where fluorescence from Sytox Green and sulforhodamine were detected using FL-1 and FL-3, respectively. The degree of crosslinking in apoptotic cells was calculated as the ratio of the MFI of sulforhodamine staining in apoptotic cells to the MFI in control cells (protein crosslinking ratio). In other experiments, cells were incubated with PBS or 1% Triton X-100 in PBS for 10min. at RT with intermittent vortexing.

To test inhibition of protein crosslinking, cultured cells were incubated with increasing concentrations of the competitive TG2inhibitor, monodansylcadaverine (MDC). MDC was dissolved in DMSO at 25mM and then diluted in culture media before it was added to cell cultures at the specified concentrations for the specified duration. Incorporation of the TG2 substrate, biotinylated cadaverine, in cellular proteins was used as an index of TG2 activity. Cells were incubated with increasing concentrations of cisplatin with or without 100µM cadaverine-biotin (from 25mM stock solution in DMSO). Cells were collected after 48h, washed extensively with PBS and incubated with streptavidin-Alexa₄₈₈ for cytofluographic analysis. In similar assays, cadaverine-biotin labelled cells were lysed for SDS-PAGE with or without prior immunoprecipitation using 3B9 mAb. PAGE gels were transferred to PVDF membranes and probed using streptavidin-AP to visualise both the total pool of TG2 substrates and 3B9-reactive TG2 substrates.

### Confocal laser scanning microscopy

Cells were stained using immunofluorescence methods described above and then spotted onto glass slides using the cytospin method. The prepared slides were mounted with coverslips using non-fluorescence mounting medium (Dako, Carpinteria, CA). Slides were analyzed using a BioRad Olympus Confocal microscope with appropriate filters and under constant conditions of laser voltage, iris aperture and photomultiplier tube amplification.

### Chromatin-binding assay

Jurkat cells were incubated in the presence or absence of 20µg/mL cisplatin and pelleted 3h after treatment to prepare soluble nuclear and chromatin fractions as decribed. Samples of these fractions were fractionated using 12% SDS-PAGE for immunoblotting as described above. Membranes were probed with 1 µg/mL 3B9, 2µg/mL anti-H2A antibodies or 0.2µg/mL anti-γH2AX-biotin followed by the appropriate AP-conjugated secondary antibodies or strepatividin-AP. The presence of H2A in chromatin and not soluble fractions was used to assess the quality of preparation of the chromatin fractions.

### Fluorescent microscopy of γH2AX and 3B9 co-localisation

Chamber slides were seeded with PANC-1 cells and incubated overnight in RPMI-1640 containing 10% FCS before replacement with medium containing 20µg/mL cisplatin alone or in combination with TSA. After 3h, cells were fixed and permeabilized using paraformaldehyde and methanol as described above. Cells were washed and blocked with 5% BSA solution in PBS then stained with 10µg/mL 3B9 followed by 2µg/mL Alexa₄₈₈-conjugated anti-mouse IgG antibody. Cells were washed and incubated with 0.2µg/mL anti-γH2AX-biotin followed by 2µg/mL streptavidin-PE. Finally, cells were washed and coverslips were mounted using non-fluorescent mounting media (Dako, Glostrup, Denmark). Alexa₄₈₈ and PE were excited using a 488nm laser and fluorescence was detected using filters 1 and 2, respectively, of an Olympus fluorescence microscope. Samples stained separately with 3B9 or γH2AX showed that there was no fluorescence bleeding between the two different filters using the specified fluorophores (data not shown).

### Bioinformatics analysis

Oncomine is a database of microarray data, which holds data from 962 studies of which 209 were analyzed. The database contains 14,177 microarrays from 35 cancer types (information publicly available at the website www.oncomine.org). Several cancer signatures have been deduced from large scale analysis of data in the database (24-28). We analyzed the 209 studies using the Advanced Analysis module limiting results to overexpressed genes only and gene enrichment was selected using the following options: (1) InterPro for analysis of motifs in the overexpresed genes, (2) Gene Ontogeny (GO) molecular function for analysis of functions of overexpressed genes, and (3) GO cellular component for cellular compartmentation of the overexpressed genes. Two paramaters were used to describe the gene sets deduced from the above analysis (a) Odds Ratio and (b) P-value, which were provided by the database.

### Statistical analysis

Statistical comparisons were performed using GraphPad Prism v4 (GraphPad Software, San Diego, CA). Generally, two-way analysis of variance (ANOVA) was used to deduce significant differences among the results. The Bonferroni post-test comparison was used to report P values. P values are denoted as: *, *P*<0.05; **, *P*<0.01; ***, *P*<0.001.

### Results

### La is overexpressed in human malignant cells and is induced by DNA-damaging drugs

As Figure 10 illustrates, the 3B9 target antigen, La, is overexpressed in malignant cells with respect to the corresponding primary cell type. In addition, data mining of Oncomine, which is a cancer gene expression database, indicates that nucleolar proteins and proteins containing the RNA Recognition Motif (RRM) such as La are overexpressed at mRNA level in many human cancers and in normal cells transfected with oncogenes such *c-Myc.* Analysis of La/SS-B mRNA expression indicated that La mRNA was overexpressed in malignancy (Tables 2 and 3). Together, these results support the notion that La protein and mRNA overexpression is a feature of malignancy in common with other components of the transcriptional and translational apparatus. It was also inferred that La expression was cell cycle-dependent. Jurkat cells were synchronized by double-thymidine block and 3B9 binding to Jurkat cells, which were fixed and permeabilized at the different phases of the cell cycle, was maximal during S phase (data not shown).

Synchronisation of Jurkat cells by double-thymidine block demonstrates that maximal binding of Apomab, and by inference maximal expression of La, occurs during S phase of the cell cycle (Fig. 11). In further support of the idea that La expression is cell cycle dependent (Fig. 11), the primary cells obtained from Clonetics^{®}, which are cultured in medium supplied by the manufacturer and which contained the activating and mitogenic compound PMA were examined. It was observed that anti-La antibody binding to these permeabilised primary cells is greater than that found in other primary cells such as buccal mucosal cells, which we had freshly isolated. Consequently, we reasoned that PMA as a mitogenic effector may affect the level of La expression in primary and malignant cells. We tested this hypothesis using three cancer cell lines (Fig. 12), and PMA increases La expression.

Cytofluographic analysis of etoposide-treated Jurkat cells showed a time-dependent loss of cell membrane integrity, which was measured by binding of propidium iodide (PI) to intracellular nucleic acids. Despite the loss of cell membrane integrity, dead and/or dying cells did not accumulate Sal5 mAb, which is an isotype control antibody of irrelevant specificity (upper panel, Fig. 13A). In contrast, there was a time-dependent increase in accumulation of 3B9, which indicated its specific binding to an intracellular antigen (lower panel, Fig. 13A). The same pattern of binding to dead tumour cells was observed for the human La-specific SW3 mAb and affinity-purified La-specific polyclonal human autoimmune sera (data not shown). Similar accumulation was also observed using other mAb specific for intracellular antigens such as PARP1, PCNA, actin, tubulin and fodrin (data not shown) or after using other DNA damaging agents such as cyclophosamide, cisplatin and staurosporine (data not shown). These results indicate that dead cells contain a reservoir of intracellular antigens, which may potentially be targeted by mAb for diagnostic and therapeutic purposes.

Nonetheless, significant differences in binding levels of the mAb specific for the different intracellular antigens were observed after induction of Jurkat cell apoptosis with the DNA-damaging drug, cisplatin. Antigen-specific mean fluorescence intensity (MFI) of cisplatin-treated Jurkat cells was compared with the MFI of control untreated Jurkat cells, which were fixed and permeabilized (Fig. 13B). While the comparison with control cells showed that antigens such as NPM, La and H2A were at least retained in Jurkat cells after cisplatin-induced cell death, La antigen and H2A were 'created' or 'induced' after cisplatin-induced cell death (Fig. 13B). The 3B9 mAb bound La in the 7-AAD-stained nucleoplasm of control permeabilized cells whereas 3B9 bound La diffusely in the cytoplasm of cisplatin-treated Jurkat cells in which 7-AAD⁺ nuclear fragmentation was apparent (Fig. 13C), which indicated that cisplatin treatment of Jurkat cells induced an apoptotic mode of cell death. This finding was confirmed by demonstrating loss of mitochondrial membrane potential after cisplatin treatment of Jurkat cells (data not shown).

Further evidence for induction of 3B9 binding by DNA-damaging agents was obtained by comparing serum-deprivation with cisplatin-treatment of various malignant cell types. In MCF-7, MDA-MB-231, A549 and PC-3 cancer cell lines, significantly greater 3B9-specific binding to the dead malignant cells was observed after use of the DNA-damaging agent (Fig. 13D). Finally, the overexpression of La observed in permeabilized malignant cells with respect to permeabilized counterpart primary cells was still observed after both malignant and normal cells were treated with cisplatin (Fig. 13E). As shown in Figure 13E, the specific binding of 3B9 to the various malignant cell types was at least two-fold higher than in the corresponding normal cell types.

### 3B9 binding is indicative of DNA damage during drug-induced apoptosis

As Fig. 14A illustrates, the extent of 3B9-specific binding per dead Jurkat cell after induction of apoptosis using the DNA-damaging agent, cisplatin, was proportional to cisplatin dose. Increasing the dose of cisplatin increased the amount of DNA damage, which was measured by cytofluographic detection of the DNA damage marker, γH2AX (inset, Fig. 14A). The correlation of 3B9-specific binding with DNA damage may result from increased expression and/or redistribution and/or accessibility of the La target antigen. Evidence was obtained for redistribution of the La antigen to double stranded breaks (DSB) after observing an increased association of both La and γH2AX with the chromatin fraction derived from cisplatin-treated Jurkat cells (Fig. 14B). Also examined were cisplatin-treated cells of the PANC-1 pancreatic cancer cell line for evidence of DNA damage. As shown in Figure 14C, γH2AX staining co-localized with 3B9 staining of La antigen in nuclei having apoptotic morphology. Together, these data suggest a role for La in DNA-damage responses.

Further evidence for the involvement of La in the DNA-damage response was obtained using chemotherapy-resistant PANC-1 cells. Neither gemcitabine (Fig. 15A) nor the histone deacetylase (HDAC) inhibitor, trichostatin A (TSA), (data not shown) as single agents induced significant levels of cell death among PANC-1 cells. In contrast, the combination of gemcitabine and TSA produced a significantly greater proportion of 7-AAD⁺ dead PANC-1 cells (Fig. 15A). Moreover, significantly greater 3B9-specific binding to 7-AAD⁺ PANC-1 cells was induced when gemcitabine and TSA were used in combination (Fig. 15B). In comparison with control PANC-1 cells, treatment of PANC-1 cells with TSA alone did not alter 3B9 binding (data not shown). In correlation, γH2AX staining increased significantly when PANC-1 cells were treated with the combination of gemcitabine and TSA compared with either gemcitabine treatment alone or no treatment (Fig. 15C). In comparison with untreated PANC-1 cells (upper panels, Fig. 15D), fluorescence microscopy confirmed increased nuclear γH2AX staining in PANC-1 cells, which were treated with gemcitabine and TSA and which showed apoptotic morphology (lower panels, Fig. 15D)

### Cisplastin-treatment of Jurkat cells causes apoptosis and intranuclear accumulation of La

As illustrated in Fig. 16A, staining with the mitochondrial-permeant dye, rhodamine-123, and the DNA binding dye, 7-AAD, confirms that cisplatin-treated Jurkat cells undergo an apoptotic mode of cell death. After 24h treatment with cisplatin, Jurkat cells are smaller and more internally complex, which is consistent with apoptosis induction (Fig. 16A, left-hand panels). Unlike control cells, cisplatin-treated cells bind 7-AAD and show loss of mitochondrial retention of rhodamine-123 (Fig. 16A, right-hand panels). The mitochondrial loss of rhodamine-123 is characteristic of apoptosis (Fig. 16B).

The same dose of cisplatin is used to treat Jurkat cells that are subsequently permeabilised and fixed and stained with mAb specific for a number of nuclear antigens (Fig. 17). The brightness of staining per cell, which is expressed as the antigen-specific mean fluorescence intensity (MFI) of the cisplatin-treated Jurkat cells is compared with the MFI of control untreated Jurkat cells, which are also permeabilised, fixed and stained. The expression of nucleophosmin (NPM) is as high in permeabilised and fixed cisplatin-treated Jurkat cells as in control cells. On the other hand, detection of highly expressed PCNA is markedly reduced after cisplatin treatment. Similarly, other nuclear proteins such as PARP, hTERT, nucleolin and lamin B together with the cytoplasmic cytoskeletal protein actin are all less detectable in permeabilised and fixed apoptotic Jurkat cells compared with permeabilised and fixed control cells (Fig. 17A , B and C). In contrast, detection of the La antigen is significantly increased in apoptotic compared with control Jurkat cells, particularly 48h after apoptosis induction. Similarly, histone H2A is increased in apoptotic Jurkat cells 72h post-apoptosis induction. These data indicate that while antigens such as NPM, La and H2A are retained in Jurkat cells during cisplatin-induced apoptosis, some antigens such as La in particular, and perhaps H2A, are 'created' or 'induced' during apoptosis induced by DNA-damaging agents (Fig. 17D).

### La becomes "fixed " in dead and dying malignant cells

Resistance of apoptotic cells to the non-ionic detergent, Triton X-100, has been reported and correlates with the activity of tissue transglutaminase or transglutaminase 2 (TG2), which mediates protein-protein crosslinking. It was found that during apoptosis, the proportion of 7-AAD⁺ cells and the level of 3B9 binding to these cells were comparable irrespective of Triton X-100 treatment (Fig 18A), which suggested that crosslinking was an integral component of apoptosis in this in *vitro* system. Immunoblot analysis of Jurkat cell lysates demonstrated that 3B9 recognized a 48kDa band, which is consistent with the known molecular weight of La and which remained unchanged during a 72h culture period both in amount and integrity (Fig. 18B). In contrast, in lysates of cisplatin-treated Jurkat cells, 3B9 identified higher molecular weight and SDS-insoluble La-containing complexes, which accumulated as cisplatin-induced apoptosis progressed, together with apoptosis-related La cleavage products (Fig. 18B). The low molecular weight La-specific bands represent caspase-generated cleavage products, which have been reported following apoptosis. Consistent with the cytofluographic findings (Fig. 18A), the SDS-stable high molecular weight bands may represent La antigen covalently bound in higher order complexes with either itself and/or other proteins.

Next, it was hypothesized that inhibition of TG2 activity reduces 3B9 binding to apoptotic cells because of reduced protein-protein crosslinking. First, the effect of increasing doses of cisplatin on the activity of TG2 in Jurkat cells was investigated using the TG2 substrate cadaverine-biotin. Incorporation of cadaverine-biotin into Jurkat cells showed a dose-dependent relationship to cisplatin concentration (Fig. 18C). Based on these results, cisplatin at 20µg/mL was chosen to induce maximal activity of TG2. Then the level of protein crosslinking in apoptotic cells was investigated. An assay was usedin which apoptotic cells were incubated in Triton X-100 solution containing the fluorescent protein-binding dye, sulforhodamine. Sulforhodamine staining in apoptotic cells was higher than in viable cells thus creating a crosslinking ratio to compare protein crosslinking in cisplatin-treated Jurkat cells with or without use of the TG2 inhibitor, monodansylcadaverine (MDC) (Fig. 18D). As shown in Figure 18D, MDC had a dose-dependent inhibitory effect on protein crosslinking. Similarly, 3B9-specific binding to apoptotic Jurkat cells was inhibited in a dose-dependent manner by MDC (Fig. 18E). The concentration of MDC (± SEM) required to inhibit half of the binding of 3B9 was 260±1, 110±1 and 150±1 µM at 24, 48 and 72h, respectively. Together, these results indicated that the La antigen was retained in apoptotic cells by a generalized protein crosslinking process, which was most likely mediated by TG2.

### Protein crosslinking covalently attaches 3B9 to the interior of leaky dead malignant cells during apoptosis

Using etoposide or cisplatin, Jurkat cells were induced to undergo apoptosis in the presence of 3B9 or its Sal5 isotype control mAb. 3B9 binding to apoptotic Jurkat cells was detectable even after stripping of the Jurkat cells with Triton X-100, which suggests that the protein cross-linking process in apoptotic cells includes the cross-linking of bound antigen-specific mAb (Fig. 19A). Furthermore, and importantly, the detergent resistance of 3B9 binding to apoptotic Jurkat cells (Fig. 19A) was much more evident than it was to apoptotic CD3⁺-enriched lymphocytes (Fig. 19B). Confocal microscopic analysis of apoptotic Jurkat cells and apoptotic CD3⁺-enriched lymphocytes supported the contention that 3B9 preferentially binds malignant cells. Reflecting the per cell measure of fluorescence intensity given by the MFI, apoptotic Jurkat cells (Fig. 19C) stain much more intensely with sulforhodamine (red) and 3B9 (green) than their counterpart primary CD3⁺-enriched lymphocytes (Fig. 19D). Moreover, this intense staining is well preserved after Triton X-100 treatment, which suggests that 3B9 is itself cross-linked to other sulforhodamine-staining proteins in the apoptotic Jurkat cells. The lack of 3B9 staining of control Jurkat cells indicates that 3B9 binding depends on induction of apoptosis and consequent loss of cell membrane integrity (Fig. 19A and C). Similar results were obtained for the matched pair of U937 monocytic leukemia cells and normal CD14-enriched peripheral blood monocytes (data not shown). Altogether, these data suggest that along with other proteins in dying malignant cells, both 3B9 and the target antigen La are covalently crosslinked as the result of a TG2-dependent process.

### EXAMPLE 5

### IN VIVO TARGETTING OF THE RIBONUCLEOPROTEIN La IN A MOUSE TUMOUR MODEL

### Materials and Methods

### Cell culture and mAb production

The EL4 murine T-lymphoblastic lymphoma cell line was obtained from American Type Cell Culture (TIB-39) and murine thymocytes were freshly prepared from 6-8 week old C57BL/6 mice. Cells were routinely cultured in RPMI-1640 containing 5% FCS (JRH Biosciences Inc., Lenexa, KS) and passaged every 48-72h at 1:4 dilution. 3B9 and the relevant control (Sal5) were prepared as described previously (first paper). Fluorescein isothiocyanate (FITC) conjugated of these agents was prepared as described by manufacturer's instructions (Sigma-Aldrich Co., St. Louis, MO).

### Induction of Apoptosis

Apoptosis in cultures was induced by adding 20µg/mL etoposide (Pfizer Inc., NY, USA) and 20µg/mL cyclophosphamide (Bristol-Myers Squibb Company, Princeton, NJ) to the culture media. Data presented here from control (untreated cells) originated from samples with higher than 90% viability as determined by PI or trypan blue (Sigma-Aldrich Co., St. Louis, MO) staining (data not shown). Permeabilization of viable cells was performed by 10 min. incubation in 2% w/v paraformaldehyde solution in PBS (150 mM sodium phosphate and 150 mM sodium chloride, pH 7.2) at 5 x 10⁶ cells/mL, which was diluted 1:10 with -20°C-cold absolute methanol (1-3 min.) before washing with PBS.

### Flow cytometry

Direct immunofluorescence staining was performed using 1-2 x 10⁵ cells at 10⁶ cells/mL for 30 min. at room temperature (RT) in PBS containing 0.1% bovine serum albumin (BSA, Sigma-Aldrich Co., St. Louis, MO) and 5µg/mL of FITC-conjugated 3B9 or FITC-conjugated Sal5 isotype control mAb. Indirect immunofluorescence staining was performed using purified mouse antibodies (5µg/mL for 30 min. at RT in PBS) followed by anti-mouse IgG conjugated to Alexa₄₈₈ (Invitrogen, Carlsbad, CA) (2µg/mL for 30 min. at RT in PBS) and detected using the FL-1 channel (530-nm filter). Cell viability was assessed by the exclusion of PI (0.5µg/mL) and detected using the FL-2 channel (585-nm filter) or by the exclusion of 7-AAD (Invitrogen, Carlsbad, CA) (2µg/mL for 15 min. at RT) and detected using the FL-3 channel (>650 nm filter). Samples were acquired immediately by Becton-Dickinson FACScan™ flow cytometry system (BD Biosciences, San Jose, CA). Acquisition was standardized to 10,000 events. Flow cytometry data was analyzed using WinMDI v 2.8 (Scripps Research Institute, La Jolla, CA). Unless otherwise specified, no gating was performed in any of the reported analyses. Specific binding of antibodies was calculated as the difference in the mean fluorescent intensity (MFI) from the test antibodies and that from control isotype antibody and was expressed as the Net MFI ± standard error of the mean (SEM) calculated from replicate incubations (n > 2).

### SDS polyacrylamide gel electrophoresis (SDS PAGE) and Western blotting

Cell lysates were prepared in SDS lysis solution (2% w/v SDS, 10% v/v glycerol and 62.5 mM Tris-HCl) and protein concentration was determined using BCA protein reagent assay kit according to the manufacturer's instructions (Pierce Biotechnology Inc., Rockford, IL). Bromophenol blue (0.05% w/v) and β-mercaptoethanol (5% v/v) (Sigma-Aldrich Co., St. Louis, MO) were added to lysates after BCA assay measurement. An amount of 12µg of lysate was added to each lane for SDS-PAGE. SDS-PAGE was performed according to the manufacturer's instructions with the Hoefer^{®} Mighty Small II SE 250 electrophoresis system (Amersham Biosciences, Piscataway, NJ) under reducing condition using 12% resolving polyacrylamide gel as per Laemmli's method. The transfer of the polyacrylamide gel to Hybond-P membrane was carried out according to the manufacturer's instructions using the TE 22 Mini Tank Transfer Unit (Amersham Biosciences, Piscataway, NJ). Immunoblotting was done using standard methods in which staining with 3B9 was followed by staining with alkaline phosphatase (AP)-conjugated anti-mouse IgG mAb (Jackson ImmunoResearch Laboratories, Inc., West Grove, PA). Blots were developed using the BCIP/NBT premixed solution as specified by manufacturer (Sigma-Aldrich Co., St. Louis, MO) and analyzed using GelPro Analyzer v3.1 (Media Cybernetics Inc., Silver Spring, MD).

### Radioligand binding studies

3B9 and matched control were labelled with ¹⁴C by incubating the hybridoma cells (35 million cells) in 35mL RPMI-1640 containing 5% FCS in the production module of miniPERM bioreactor (Vivascience GmbH, Hannover, Germany) and 400mL of RPMI-1640 containing 5% FCS and 250µCi of D-[U-¹⁴C]glucose and 250µCi of L-[U-¹⁴C]leucine (Amersham Biosciences, Piscataway, NJ) in the nutrient module. The bioreactor was incubated in 5% CO₂ humidified air at 37°C on a bottle-rotating device for 5 days. The medium in the production chamber was collected for purification using Protein G purification columns. Radioactivity of purified antibodies (10µL sample) was counted in UltimaGold^{™} scintillation liquid (1mL) for 20 min using Packard Tri-Carb 3100 β-counter (PerkinElmer Inc., Wellesley, MA) regularly calibrated using supplied ¹⁴C standards. Protein concentration was determined using BCA protein reagent assay. The specific radioactivity of ¹⁴C-control and ¹⁴C-3B9 was 120.3 and 130.8dpm/µg, respectively.

Saturation binding assays were performed by incubating apoptotic EL4 cells (5 x 10⁵ cells) after 48h treatment with etoposide and cyclophosphamide with increasing concentrations of ¹⁴C-3B9 in the presence (to measure non-specific binding) or absence (to measure total binding) of a 50-fold molar excess of unlabelled 3B9. After 30 min., cells were washed thoroughly using PBS and radioactivity was measured using the □-counter as described above. Specific binding was calculated as the difference between total and non-specific binding and plotted as a function of the concentration of ¹⁴C-3B9. Competition binding assays were carried out by incubating apoptotic EL4 cells with 100 nM ¹⁴C-3B9 in the presence of increasing concentrations of unlabelled 3B9. Radioactivity was measured as described above and plotted as a function of unlabelled 3B9 concentration. Association assays were performed by incubation of apoptotic EL4 cells with 100 nM ¹⁴C-3B9 in the presence or absence of a 50-fold molar excess of unlabelled 3B9 for the specified times. Samples were washed, radioactivity was measured and specific binding was plotted as function of time. Dissociation assays were performed after incubation of apoptotic EL4 cells with 100 nM ¹⁴C-3B9 in the absence or presence of 50-fold molar excess of unlabelled 3B9 for 30 min. at RT. Cells were washed, incubated at 37°C in PBS and samples were removed at the specified times. These samples were washed with PBS, radioactivity was measured and specific binding was plotted as function of time.

### EL4 tumour model in C57BL/6 mice

The EL4 thymic lymphoblastic lymphoma is a robust model of apoptosis, which is induced by the cytotoxic drugs, cyclophosphamide and etoposide (14). Subcutaneous EL4 tumour implants were established in 6-8 week old syngeneic C57BL/6 mice where 10⁶ EL4 cells were injected subcutaneously in the right flank of each mouse. Mice were housed and treated as per protocols approved by the Animal Ethics Committee at The University of Adelaide and the Animal Ethics Committee at the Institute of Medical and Veterinary Sciences (IMVS). Once the tumour reached 1cm diameter, mice were randomly divided into control or treatment groups.

Treatment comprised intraperitoneal injections of cytotoxic chemotherapy given in one of the following five regimens of reducing dose intensity. The respective cyclophosphamide and etoposide doses are indicated and are mg/kg. (i) full dose as published: 100, 76 at 0h and 24h; (ii) half dose 2d: 50, 38 at 0h and 24h; (iii) half dose 1d: 50, 38 at 0h; (iv) quarter dose 2d: 25, 19 at 0h and 24h; (v) quarter dose 1d: 25, 19 at 0h.

### Flow cytometry analysis of control and chemotherapy treated EL4 tumours

Control and chemotherapy-treated mice bearing EL4 tumours were euthanized 48h after treatment and tumours were excised. Excised tumour tissue was disrupted by incubation in RPMI-1640 containing 2mg/mL collagenase (Sigma-Aldrich Co., St. Louis, MO) for 30 min. at 37°C with constant shaking. Single cell suspensions were washed with PBS and used for immunofluorescent staining with 3B9-FITC or Sal-FITC and PI and flow cytometry analysis as described earlier.

### ¹⁴C-3B9 biodistribution in EL4 tumour bearing mice

Control and chemotherapy treated mice received an intravenous injection of ¹⁴C-3B9 or ¹⁴C-Sal5 (control) at time 0. After 48h, mice were euthanized, whole blood was collected by cardiac puncture, and EL4 tumours and other tissues were collected for radioactivity measurement. Serum and body tissues were solubilized using 1mL Solvable^{™} (PerkinElmer Inc., Wellesley, MA) for 2h at 50°C, and then decolorized using 100µL H₂O₂ (30%) (Sigma-Aldrich Co., St. Louis, MO). UltimaGold^{™} scintillation liquid (1mL) was added and samples were counted for 10min. using the β-counter.

### ¹¹¹In-DOTA-3B9 biodistribution and pharmacokinetics in EL4 tumour bearing mice

Protein G purified 3B9 or Sal5 mAb were mixed at 2.5mg/mL in 0.1M sodium bicarbonate and 0.1M sodium phosphate buffer (pH8.5) with 50-fold molar excess of DOTA-NHS-Ester (Macrocyclics, Dallas, TX) dissolved in DMSO (Sigma-Aldrich Co., St. Louis, MO). DMSO represented <10% of the final reaction, which was incubated for 2h at 23°C with constant vigorous shaking. DOTA-NHS-Ester was added at a 50-fold molar excess to the antibody as this ratio was found to minimize inactivation of 3B9 and showed favorable *in vivo* biodistribution compared to Sal5 conjugated at the same ratio and to 3B9 conjugated at higher ratios (data not shown). Addition of 1.5M Tris-HCl (pH8.8) at a final dilution of 1:10 was used to stop the reaction, which was loaded onto a 100kDa-cutoff microconcentrator (Millipore, Billerica, MA). Reactions were buffer-exchanged using 5x500µL washes of PBS. The concentration of IgG was measured using a BCA protein assay kit and the concentration of conjugated DOTA was measured using a modification of a published Cu:Arsenazo(III) assay. The ligand/protein (L/P) ratio of 7.5 was calculated as the ratio of the concentrations (µM) of DOTA-immunoconjugates to IgG. The reaction was stored at 4°C before radiolabeling.

Purified DOTA-immunoconjugate solutions were concentrated and buffer-exchanged into metal free 0.2M ammonium acetate buffer (pH5.5) (Sigma-Aldrich Co., St. Louis, MO) containing 6mg/mL ascorbic acid (Sigma-Aldrich Co., St. Louis, MO). Indium-111 chloride (PerkinElmer Inc., Wellesley, MA) was diluted in the same buffer (5mCi/mL; 185MBq/mL) and mixed with DOTA immunoconjugates (10mg/mL). Radiolabeling reactions were incubated at 37°C for 2h then mixed with an equal volume of 0.2M ammonium acetate buffer (pH8) containing 5mM EDTA to quench free and loosely bond radionuclide. Quenched reactions were loaded onto 100kDa-cutoff microconcentrators and purified by three washes of 500µL endotoxin free PBS. The incorporation of ¹¹¹In in the purified reactions was determined using instant thin layer chromatography (ITLC) which was performed using ITLC-SG strips (Pall Corporation, East Hills, NY) in 0.2M ammonium acetate solution (pH 8) containing 5mM EDTA as mobile phase. Briefly, aliquots (2µL) of purified reactions and free ¹¹¹In in 0.2M ammonium acetate and 5mM EDTA were used for ITLC in the mobile phase and the origin and solvent front halves of strips were counted using a Cobra 5010 gamma counter (PerkinElmer Inc., Wellesley, MA) normalized for ¹¹¹In counting using a 100-350keV counting window. While 99% of radioactivity on the ITLC-SG strip was at the solvent front when ITLC was performed using ¹¹¹InCl₃ solution, 99% of radioactivity in the DOTA-conjugates remained at the origin indicating the complete incorporation of ¹¹¹In in the DOTA-3B9 conjugate (data not shown). ¹At 0h, ¹¹¹In-DOTA-3B9 (72µg in 100µL of PBS) was given by intravenous injection to EL4 tumour-bearing mice, which were left untreated or treated with cytotoxic chemotherapy as described above. Mice were euthanized at selected time points, blood was collected by cardiac puncture, and tumours and organs were collected. Blood and organs were weighed and placed in gamma-counter tubes and radioactivity was measured using normalized Cobra5010 gamma counter. Radioactivity in organs was normalized to the weight of the organs and accumulation was calculated as the percentage of radioactivity per gram in the organs to the radioactivity of the injected dose of ¹¹¹In-DOTA-3B9 at time 0 (%ID/g). Time activity curves for blood and tumours were constructed using GraphPad Prism v.4 (GraphPad Software, San Diego, CA) for each treatment group where accumulation %ID/g (±SEM, n=5) plotted as a function of time. Curves were fitted to one-phase exponential decay for blood and one-phase exponential association for tumours and the half-life (t_{1/2}) was provided from the fitted models. In the case of tumour accumulation, the fitted association model also provided a value for maximal accumulation at saturation together with the corresponding standard error of this measurement, which we report as %ID/g ± SEM at saturation. Data points did not deviate from the fitted models as judged by runs test performed using the fitting software and the regression value of the fitted model was provided.

### Statistical analysis

Statistical comparisons were preformed using GraphPad Prism. Two-way analysis of variances (2-way ANOVA) was used to deduce significant differences in the results. The Bonferroni post-test in the 2-way ANOVA function in GraphPad prism was used to report P values. P values less than 0.05 were considered significant where one, two and three asterisks denote P values less than 0.05, 0.01 and 0.001, respectively.

### Results

### La is overexpressed in malignant EL4 cells and La-specific mAb binding to dead EL4 cells is induced by cytotoxic drug treatment

Cytofluographic analysis of cultured EL4 lymphoma cells, which were stained with the DNA binding dye 7-AAD as a test of membrane integrity, indicated that although <10% of the cultured cells demonstrated evidence of spontaneous cell death, these dead cells did not bind La-specific mAb 3B9 (Fig. 20A). Nevertheless, after fixation and permeabilization of the EL4 cells, 3B9 binding to 7-AAD⁺ cells was evident, which indicated that La autoantigen could be accessed using a specific mAb (Fig. 20B). Furthermore, after EL4 cells were treated with the cytotoxic and DNA-damaging drugs, cyclophosphamide and etoposide, the intensity of 3B9 binding to the resulting 7-AAD⁺ EL4 cells was greater than to fixed and permeabilized 7-AAD⁺ cells (Fig. 20C). As illustrated in Fig. 20D, per cell binding of La-specific mAb to dead EL4 cells killed by cytotoxic drug treatment was significantly higher than the binding to dead cells killed by fixation and permeabilization, which suggested that cytotoxic drug treatment either induced higher levels of target expression and/or increased the accessibility of the epitope. In addition, the 3B9 binding to the dead EL4 cells killed with cytotoxic drugs was preserved after the dead cells were treated with a non-ionic detergent, which indicated that the La target antigen had been cross-linked in the dead cells. Fig. 20D also illustrates that, after fixation and permeabilization, the per cell binding of 3B9 mAb was significantly higher for the malignant EL4 cell than for its normal murine counterpart cell type of the thymocytes. This cytofluographic evidence of La overexpression in EL4 cells was corroborated by immunoblot analysis of cell lysates prepared from thymocytes and EL4 cells (inset, Fig. 20D). The binding of 3B9 to dead thymocytes was the same irrespective of whether they were killed by cytotoxic drugs or fixation and permeabilization, which contrasts with the significantly increased binding of 3B9 to dead EL4 cells after cytotoxic drug treatment (Fig. 20D).

### ¹⁴C-labelled 3B9 binds specifically to EL4 cells

Next, biosynthetically labelled 3B9 mAb was used to further characterize the binding of La-specific mAb to dead EL4 cells, which were killed with cytotoxic drugs. It was found that ¹⁴C-3B9 bound dead EL4 cells in a specific and saturable manner. The concentration of ¹⁴C-3B9 required to reach half-maximal saturation was 18nM and maximal binding was ∼7500 femtomole/million dead cells (Fig. 21A). Half-maximal saturation of ¹⁴C-3B9 binding occurred within 5 minutes at room temperature (Fig. 21B). The concentration of unlabelled 3B9 required to inhibit half-maximal binding of ¹⁴C-3B9 (IC₅₀) was estimated to be 118nM (Fig. 21C). Dissociation of bound 3B9 was minimal when cells were incubated in PBS for 30 minutes at 37°C (Fig. 21D). Altogether, these data describe specific, rapid and high affinity binding of ¹⁴C-3B9 to dead tumour cells. Binding was not detected when EL4 cells were tested in the absence of cytotoxic drugs (data not shown), which indicated that binding depended on the induction of cell death.

### Cytotoxic chemotherapy increases the target for 3B9 in the tumour mass

*The in vitro* findings depicted in Fig. 20 were confirmed *in vivo* using the EL4 lymphoma model. After the use of cytotoxic chemotherapy, the fraction of PI⁺ cells in the tumour explants increased significantly from a mean (±SEM) of 50±2% to 70±1% (*P*<0.001). Similarly, the 3B9⁺ subset of PI⁺ cells increased significantly from 15±1% to 38±2% (P<0.01) with use of cytotoxic chemotherapy whereas isotype control staining did not alter with use of chemotherapy (Fig. 22). Only the PI⁺ subpopulation of tumour cells displayed binding with the 3B9-FITC conjugate, which indicated that the La antigen was recognized specifically in dead tumour cells. In order to describe the effect of chemotherapy on 3B9 targeting to La, we analyzed the frequency distributions of PI⁺ cells, which bound FITC conjugates and which were defined in the upper right quadrant of each density plot (Fig 22). As illustrated in the representative histograms in Fig. 22, the specific binding of 3B9-FITC to PI⁺ cells was significantly augmented in tumours exposed *in vivo* to cytotoxic chemotherapy (net MFI ± SEM of 18±3 with chemotherapy and 1±3 without chemotherapy, *P*<0.05).. These results provide *in vivo* evidence of increased per cell binding of 3B9, which may reflect increased expression and/or increased availability of the 3B9 epitope in dead cells obtained from tumours that were exposed to cytotoxic chemotherapy.

### 3B9 targets EL4 tumours in vivo especially after cytotoxic chemotherapy

The biodistribution of La-specific mAb was studied in the EL4 lymphoma model using intrinsically or extrinsically labelled 3B9 mAb. First, biosynthetically labelled ¹⁴C-3B9 mAb was used to demonstrate that tumour accumulation of 3B9 mAb was an inherent property of its antigen-binding activity. EL4 tumour-bearing mice were treated with the full dose schedule of cyclophosphamide and etoposide and simultaneously administered 100µg each of either ¹⁴C-3B9 or ¹⁴C-Sal5 isotype control mAb before analysis of the mice after 48 hours. The ¹⁴C-Sal5 mAb did not accumulate significantly in any organ or tissue including the tumour. In contrast and compared with all other organs, we found that ¹⁴C-3B9 accumulated significantly in serum and in the tumour (*P*<0.001). Moreover, after the mice were treated with cytotoxic chemotherapy, only the tumours accumulated significantly more ¹⁴C-3B9 (*P*<0.001) (data not shown).

In the second series of experiments, 3B9 conjugated to a metal chelator, DOTA, was radiolabelled with ¹¹¹In and used to investigate the biodistribution and pharmacokinetics in EL4 tumour-bearing mice before and after chemotherapy treatment (Fig. 23&24). The clearance of ¹¹¹In-DOTA-3B9 from blood of control mice and mice treated with quarter dose 2d or with half dose 1d was not significantly different (*P>0.05*). On the other hand, the clearance of ¹¹¹In-DOTA-3B9 from blood of mice treated with quarter dose 1 d or with half dose 2d was significantly faster than that in control mice (*P<0.001*) (Fig. 23). The accumulation of 3B9 in the tumours of mice treated quarter dose 2d (40±3%ID/g, n=5) and half dose 1d (37±3%ID/g, n=5) was significantly different from that in control mice (20±1%ID/g, n=5) *(P<0.05).* The more rapid clearance of 3B9 from blood of mice treated with quarter dose 1d or half dose 2d was associated with more significant accumulation of the agent in the tumours, (48±8%ID/g, n=5) and (47±6%ID/g, n=5), respectively *(P<0.01)* (Fig. 23). Despite the exponential growth of control tumours (measured as tumour weight, Fig. 23), weight-normalized tumour accumulation (%ID/g) of ¹¹¹In-DOTA-3B9, which has a physical half-life of 2.8 days, was unchanged, suggesting that rates of tumour cell death balanced tumour cell proliferation to allow continued binding of 3B9 to these growing tumours. Finally, the reduction of tumour weights in mice treated with cytotoxic chemotherapy and the accumulation of ¹¹¹In-DOTA-3B9 in these tumours was associated with increased levels of cell death measured by cytofluographic analysis (% of 7-AAD⁺ cells) (Fig 23 - inset).

The specificity of ¹¹¹In-DOTA-3B9 towards the dying tumours was expressed as the ratio of tumour accumulation to that in normal organs (tumour/organ ratio, Fig. 24). At 24h after chemotherapy and ¹¹¹In-DOTA-3B9 administration, only the tumour/muscle ratio was significantly higher compared to control mice (*P<0.001,* data not shown). Compared to control mice, the tumour/organ ratio at 48h (Fig. 24A) and 72h (Fig. 24B) after treatment was significantly higher for all organs except for blood. Further evidence for tumour specificity was obtained from biodistribution studies of ¹⁴C-3B9 in EL4 tumour-bearing mice which were extended to include lower doses of the radiolabelled agent. As illustrated in Fig. 25, there was no significant accumulation of ¹⁴C-3B9 in any tissue except the tumour and only after administration of cytotoxic chemotherapy. Tumour accumulation of ¹⁴C-3B9 was dose-dependent and the sigmoidal dose-response relationship suggested that the binding of the target was specific and saturable. In comparison to untreated mice, tumour uptake of ¹⁴C-3B9 with chemotherapy was significantly higher and demonstrated fold increases of 1.9, 1.9 and 1.8 at ¹⁴C-3B9 doses of 25 µg, 50 µg and 100 %g, respectively. Irrespective of the use of chemotherapy, no significant difference in the tumour uptake of ¹⁴C-3B9 was observed at a 5µg dose. These data further support the concept that 3B9 selectively targets malignant tissues rather than critical normal tissues. Altogether, 3B9, using two different radiotracer formats, showed specific and preferential accumulation to EL4 tumours which was augmented by chemotherapy treatment of this chemo-responsive tumour model.

### EXAMPLE 6

### The La antigen La is a TG2 substrate

A biotinylated form of cadaverine, which is a polyamine TG2 substrate and which consequently becomes covalently bound to other TG2 substrates, is used to identify protein targets of cross-linking during apoptosis. As shown in Fig. 26A, the incorporation of cadaverine-biotin in apoptotic Jurkat cells was confirmed by fluorocytometric analysis using streptavidin-Alexa₄₈₈. Immunoblot analysis of Jurkat cells induced to undergo apoptosis in the presence of cadaverine-biotin results in the detection of additional protein bands labelled with cadaverine-biotin (lane 2 in inset, Fig. 26). These data indicate that cadaverine-biotin is incorporated in an SDS-stable manner into cellular proteins during apoptosis. Immunoprecipitation with Apomab of Jurkat cell lysates after treatment of the cells with cadaverine-biotin during apoptosis induction demonstrates incorporation of cadaverine-biotin into a putative La antigen band as detected by streptavidin-AP on the immunoblot (arrow, lane 2, Fig. 26B).

### EXAMPLE 7

### DNA-DAMAGING CHEMOTHERAPY INDUCES ANTI-LA ANTIBODY BINDING IN PRIMARY HUMAN MALIGNANT CELLS

Two studies were performed on patient material to indicate that Apomab binding was augmented by DNA-damaging cytotoxic chemotherapy. In the first study, primary ALL blasts from a chemonaïve patient were treated *in vitro* with cytotoxic drugs and then analysed (Fig. 27&28). In the second study, circulating tumour cells of a patient receiving this first cycle of chemotherapy for extensive stage small cell lung cancer (SCLC) were analysed *in vitro* (Fig. 29).

### Treatment and analysis of acute lymphoblastic leukemic blasts in vitro

To purify primary ALL blasts for further analyses, Ficoll gradient separation was performed using a 10 mL peripheral blood sample from a newly diagnosed and untreated ALL patient. The buffy coat was collected and washed with culture medium before incubation for 10 min in red cell lysis buffer. Cells were washed, aliquots were removed for immediate analysis while other aliquots were incubated or not in different concentrations of cytotoxic drugs singly or in combination. At 24h, 48h, and 72h after treatment was initiated *in vitro,* ALL blasts were stained with 10 µg/mL Sal5 control or Apomab then with 2 µg/mL Alexa₄₈₈-conjugated anti-mouse IgG. Cells were washed and incubated with 2 µg/mL 7-AAD then analysed by flow cytometry immediately.

The level of Apomab binding to permeabilised primary ALL blasts (inset, Fig. 28) was similar to the level found in blasts dying spontaneously in culture (control, Fig. 28). Apomab binding was highest after ALL blasts were treated with double strand DNA break (DSB)-inducing agents, etoposide and/or cisplatin, and peaked 48h after treatment (Fig. 28).

### Analysis in vitro of circulating small lung cancer cells of a patient treated with cytotoxic drugs

Anti-La antibody binding to circulating tumour cells in peripheral blood of a 73-year-old male patient, GP, who had extensive-stage SCLC was studied. The patient, GP, who had not previously been treated for his cancer, was administered cytotoxic chemotherapy using carboplatin at AUC 5 on day 1 together with etoposide 120 mg/m² on days 1, 2 and 3. Heparinised peripheral blood samples were drawn from the patient before chemotherapy (0h) and 24, 48 and 72 hours after initiation of chemotherapy. To demonstrate that GP's blood contained circulating tumour cells (CTC), his blood was enriched before and after cytotoxic drug treatment for BerEP4-expressing cells, which were present minimally if at all in the peripheral blood of a normal healthy volunteer (control) (Fig. 29, upper row of panels). Forty-eight hours after cytotoxic chemotherapy, there was a large increase in the number of dead (7-AAD⁺) CTC, which clearly and specifically bound Apomab (Fig. 29, third row of panels). At 72h post-treatment, Apomab-specific staining was also detected on peripheral blood 7-AAD⁺ material of GP that has scatter characteristics or apoptotic bodies (Fig. 29, fourth row of panels). The greatest increase of 7-AAD⁺ cells in GP's blood was observed 48 hours after initiation of cytotoxic chemotherapy and the 7-AAD⁺ cells had the highest binding of Apomab (Fig. 29B).

Although the laboratory findings were not correlated with a formal radiological evaluation of overall tumour response to chemotherapy, GP did have a palpable and painful sternal mass before cytotoxic chemotherapy, which became markedly less painful and shrunk significantly in size within two weeks of cytotoxic chemotherapy administration.

In conclusion, these results using patient tumour material indicate that anti-La antibody identifies malignant cells that have died as the result of cytotoxic drug administration and, hence, this antibody may be useful for predicting early chemotherapy responses in cancer patients.

### DNA-damaging antineoplastic induces the intrinsic apoptosis pathway and induces sustained Apomab to apoptotic cells unlike extrinsic pathway activation, culture stress, serum withdrawal or primary necrosis

Apoptotic changes after intrinsic pathway activation took longer than after extrinsic pathway activation. During activation of the intrinsic pathway by cisplatin or γ-radiation, evidence of DNA damage was observed at the early 5h time point in permeabilised cells by γ -phosphorylation of H2AX and, simultaneously, by significantly increased antibody binding, which reflects increased expression of the La target antigen (Fig. 30A&B). DSB and early induction of La expression were most prominent and sustained after γ-radiation (Fig. 30B). These changes preceded apoptosome formation that is marked by mitochondrial membrane permeabilisation (reduced retention of rhodamine 123) and caspase-3 activation. During activation of the extrinsic pathway by Fas ligation, apoptosis of Jurkat cells was induced more rapidly than by the intrinsic pathway inducers of cisplatin and γ-radiation because caspase-3 activation was evident only 5h after treatment. At this time point also were detected marked elevations of γ H2AX and La antigen, which subsided 24h later as Jurkat cells were losing cell membrane integrity manifest as 7-AAD staining (Fig. 30C). After Fas ligation, DSB may occur as the cell undergoes apoptosis and is dismantled into apobodies.

In contrast, cells that were overgrown *in vitro* (Fig. 30D) or starved by serum deprivation (Fig. 30E), did not induce La expression manifest as increased anti-La antibody binding, and in fact antibody binding to the permeabilised cells declined significantly with time after the stressful stimulus. Under these stressful conditions, loss of cell membrane integrity manifest as 7-AAD staining did not occur until 72h after the stress. These conditions often induce ER stress and cells may survive by undergoing autophagy before finally succumbing to a death that is not apparently marked by apoptotic features. These *in vitro* conditions may mirror the endogenous tumour cell death resulting from adverse micronenvironmental conditions *in vivo* such as hypoxia, acidosis, and ischemia. Importantly, these *in vitro* conditions did not result in induction of the La target antigen, and thus did not augment anti-La antibody binding. Finally, a primary necrotic insult such as heat did not induce either apoptosis or La expression and antibody binding despite earlier loss of cell membrane integrity (Fig. 30F).

Further analysis indicated differences in the course of apoptosis after γ-radiation vis à vis cisplatin treatment (Fig. 31). For irradiated cells, activation of caspase-3 occurred in parallel to the conversion of apoptotic cells to apoptotic bodies (apobodies), which were smaller (lower FSC), more internally complex or granular (higher SSC) with intermediate 7-AAD-staining. In response to this apoptotic stimulus, γH2AX and La induction was apparent at 5h before caspase-3 activation and persisted (Fig. 31B). Interestingly, La induction persisted in all cells whereas γH2AX foci were lost in apobodies reflecting loss of H2AX γ-phosphorylation. Another interpretation is that La partitioned to both RNA- and DNA-containing apobodies, which may be mutually exclusive, whereas γH2AX foci associated only with DNA (Fig. 31B).

After Fas ligation, similar considerations applied to activation of caspase-3. As cells turned into apobodies, caspase-3 was activated simultaneously. Caspase activation indicated that cells were being dismantled into apobodies and the caspase-mediated processes likely included DNA degradation. Hence, the creation of simultaneous DSB seemed to be responsible for the striking induction of γH2AX foci and the concomitant induction of La. Activated caspase-3 activation persisted in cells as they converted to apobodies. On the other hand, the γH2AX and La induction occurred initially and transiently in all cells, and so appeared to be a process that was executed in response to the death stimulus applied to all cells and then was complete (Fig. 31C).

In confirmation that cisplatin treatment and γ-radiation of Jurkat cells induces expression of La antigen, immunoblots of cell lysates prepared shortly after apoptosis induction but before loss of cell membrane integrity were probed with anti-La antibodies or anti-actin antibodies as a loading control (Fig. 32A). In contrast to starvation of Jurkat cells by serum withdrawal, densitometric scanning of the immunoblots (Fig. 32B) indicates that relatively more La was expressed in Jurkat cells after both cisplatin treatment and γ-radiation (Fig. 32C).

To show that anti-La antibody bound a variety of other dead human malignant cell lines after cytotoxic treatment, Apomab or its Sal5 isotype control mAb was used to stain malignant cells after treatment with the pan-tyrosine kinase inhibitor (STS), the topoisomerase II inhibitor, etoposide, or the tubule depolymerising agent, vincristine (Fig. 33 and Table 6).

### EXAMPLE 8

### DNA-DAMAGING CHEMOTHERAPY INDUCES Apomab BINDING IN VIVO

### Aims

*In vivo* studies were performed to quantify dead cells in EL4 tumours before and after CE chemotherapy, and to measure anti-La antibody uptake by these tumours. To provide evidence *in vivo* of induction of the La antigen by DNA-damaging chemotherapy, the relationship between tumour anti-La antibody uptake and the percentage of dead cells within tumours were examined. Anti-La antibody uptake by control tumours was compared with tumour anti-La antibody uptake after CE chemotherapy to determine if it exceeded the level expected if it were only proportionate to the tumour content of dead cells.

### Treatment and analysis of cell death in EL4 tumours

C57BL/6 mice bearing EL4 tumours were left untreated (n=3) or treated by intraperitoneal injection (IPI) of 19.5mg/kg etoposide and 25mg/kg of cyclophosphamide (*n*=3)*.* Mice were killed at specified time points, tumours were excised, minced into pieces <10mm³ using scissors, and 0.1 g of minced tumour weighed and placed in 10 mL 2 mg/mL solution of collagenase Type 1 in HBSS containing 2.5 mM Ca²⁺. Solutions were incubated at 37°C with constant rotation for 1h. Digested tumour cell suspensions were passed sequentially through syringes with 19 G, 23 G then 25 G needles. Suspensions were centrifuged at 2000 rpm for 5 min and pellets were washed with 10 mL HBSS. Washed pellets were resuspended in 1 mL HBSS and 100 µL aliquots were stained for 10 min at room temperature (RT) in duplicate with 2 µg/mL 7-AAD. Samples were analysed using flow cytometry for the percentage of cells that bound 7-AAD to measure the percentage of dead cells (7-AAD⁺).

### Treatment and analysis of anti-La antibody (Apomab) binding to EL4 tumours in vivo

C57BL/6 mice bearing EL4 tumours were left untreated (*n*=5) or treated with chemotherapy (n=5) as described above. All mice were also injected intravenously with ¹¹¹In-Apomab. Mice were killed at specified time points, tumours were excised, weighed, and placed in gamma counting tubes to measure radioactivity. Measured radioactivity was normalised to tumour weight (cpm/g) and accumulation was calculated as the percentage of weight-normalised radioactivity counts to total radioactivity counts of injected dose at time 0h (% cpm/g in tumour / cpm of injected ¹¹¹In-Apomab at time 0h).

### Results

In comparison with untreated control mice (Fig. 34A), treatment of mice with CE chemotherapy increased the EL4 tumour cell death rate (Fig.34B). Similarly, treating EL4 tumour-bearing mice with CE chemotherapy augmented tumour accumulation of ¹¹¹In-Apomab (data not shown). To determine the rate of tumour accumulation of Apomab itself, time-activity data were corrected for ¹¹¹Indium decay because ¹¹¹In-Apomab accumulation represented 97.0, 78.3, 61.3, 48.0, and 37.5% of Apomab accumulation at 24, 48, 72, and 96h post-injection, respectively. It is readily apparent that this correction shows that the rate of Apomab accumulation in control tumours matched the tumour cell death rate (Fig.34A) whereas the rate of Apomab accumulation in treated tumours exceeded the tumour cell death rate (Fig.34B).

To demonstrate this finding more explicitly, Apomab binding to dead tumour cells was related directly to the tumour content of dead cells at each time point (Fig. 35). In control tumours, the ratio of Apomab accumulation (%ID/g) to tumour content of dead cells (%7-AAD⁺ cells) never exceeded 100% and indeed declined as tumours grew whereas, after chemotherapy, the ratio increased beyond 100% with time. It is hypothesised that DNA-damaging antineoplastic treatment induces expression of the La antigen and so augments per cell binding of Apomab, which has been demonstrated previously *in vitro* and as *ex vivo* staining of tumour cell suspensions obtained from mice treated or not with DNA-damaging chemotherapy.

In conjunction with the data showing therapeutic efficacy of La-directed monoRIT, at least two significant inferences may be drawn from these observations:
(i) The first step of DNA-damaging antineoplastic therapy creates additional targets for binding of armed versions of Apomab or other potential dead tumour cell-interacting radioligands, and thus creates a self-amplifying or feed-forward loop for the second step of circulating tumour-binding activity.
(ii) Delivery of armed Apomab to tumour cells dying as the result of DNA-damaging antineoplastic therapy initiates cell death among nearby viable tumour cells and this bystander killing amplifies the effect of the original targeting by creating targets that were not at first manifest within the tumour. According to this method, we hypothesise that bystander killing will be most efficient if the immunoconjugate induces DSB. Such conjugates include ionising radiation and calicheamicin.

Furthermore, cytotoxic radiosensitising drugs such as cisplatin, the antimetabolites gemcitabine and 5-fluorouracil, and the taxanes, will augment the potency of La-directed radioimmunotherapy (RIT) by lowering the threshold for tumour cell death. Therefore, it would be expected that appropriately scheduled administration of a cytotoxic radiosensitising drug and La-directed RIT would lower the effective dose of RIT required for tumour cell kill.

Other than ionising radiation, the radiomimetic drug, cisplatin, topoisomerase inhibitors, DNA-intercalating agents, which are all antineoplastic treatments well known to induce double-stranded DNA breaks (DSB) and some which have been shown to augment Apomab binding to apoptotic tumour cells *in vitro,* newer agents induce DSB *via* novel mechanisms of action. For example, PARP inhibitors induce DSB and sensitise to apoptosis those tumour cells that lack DNA repair mechanisms based homologous recombination (HR) such as BRCA nullizygous tumour cells, or tumour cells with features of 'BRCAness' (N Turner et al. Nat Rev Cancer 4, 1-6, 2004) because HR is required to repair endogenous DNA damage (HE Bryant et al. Nature 434, 913-917, 2005; H Farmer et al. Nature 434, 917-921, 2005). The antimetabolite and masked DNA chain terminator, gemcitabine, induces few DSB unless the S-phase checkpoint is abrogated by a class of drug known as CHK1/2 inhibitors when DSB become greatly increased and apoptosis ensues (G McArthur et al. J Clin Oncol 24, 3045a, 2006; MA Morgan et al. Cancer Res 65, 6835-6842, 2005). Similar effects were exerted by CHK1/2 inhibitors after ionising radiation induced G₂ and S checkpoints (J Falck et al. Nature 410, 842-847, 2001; H Zhao et al. PNAS 99, 14795-14800, 2002).

### EXAMPLE 9

### COMBINATION OF CHEMOTHERAPY AND RADIOIMMUNOTHERAPY WITH Apomab

### LnCap tumour model

LnCap cells (5x10⁶ cells) prepared in 50:50 PBS:matrigel were injected in the right flanks of 6-8 weeks old Balb/c female nude mice. When tumours reached 120cm³ in volume mice were randomly divided into 6 groups of 5-10 mice each (tumour volumes were not significantly different before treatment) (Figure 36) (Table 7)

### Control: no treatment

RIT: 2.5MBq of ⁹⁰Y-Apomab (25µg) on day 1
Chemo: 50mg/kg gem and 1mg/kg cis on day 1, 50mg/kg gem on day 4, 50mg/kg gem and 1mg/kg cis on day 7 and 50mg/kg gem on day 10
Chemo->RIT: 50mg/kg gem and 1mg/kg cis on day 1, 50mg/kg gem on day 4, 50mg/kg gem and 1mg/kg cis on day 7, RIT on day 8 and 50mg/kg gem on day 10
Chemo->RIT: 50mg/kg gem and 1mg/kg cis on day 1, RIT on day 2, 50mg/kg gem on day 4 and 50mg/kg gem and 1mg/kg cis on day 8
Chemo+RIT: 25mg/kg gem and 0.5mg/kg cis and RIT on day 1, 25mg/kg gem and RIT on day 4 and 25mg/kg gem and 0.5mg/kg cis on day 7

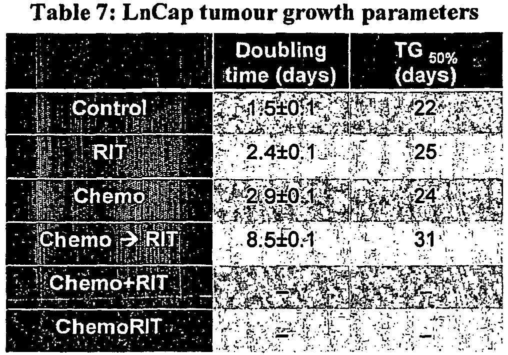

Growth curves were fitted to exponential growth curve (R2> 0.90) using GraphPad Prism. Doubling time and tumour growth to 50% were provided by the fitted curve.

### PANIC-1 tumour model

PANC-1 cells (5x10⁶ cells) prepared in 50:50 PBS:matrigel were injected in the right flanks of 6-8 weeks old Balb/c female nude mice. When tumours reached 50cm³ in volume mice were randomly divided into 4 groups of 5 mice each (tumour volumes were not significantly different before treatment) (Figure 37) (Table 8)
Group 1: Control, no treatment
Group 2: RIT, 2.5MBq of ⁹⁰Y-Apomab (25µg) on day 7
Group 3: Chemo, 50mg/kg gem and 1mg/kg cis on day 6, 50mg/kg gem on day 9 and 50mg/kg gem and 1mg/kg cis on day 12
Group 4: Chemo and RIT combined

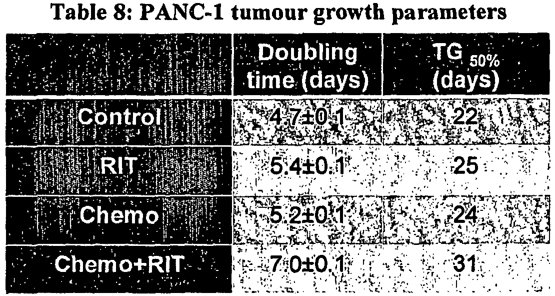

Growth curves (previous slides) were fitted to exponential growth curve (R2> 0.92) using GraphPad Prism. Doubling time and tumour growth to 50% were provided by the fitted curve.

### EL4 tumour model

### 90Y-Apomab therapy in EL4 tumour model

- C57 mice bearing EL4 tumours left untreated or treated with full dose chemotherapy were injected (24h post chemo) with escalating doses of 90Y-Apomab
- Mice did not show any signs or irreversible toxicity (weight loss, hunched posture, diarrhoea, reluctance to move or eat, Figure 38)
- High doses of 90Y-Apomab provided cure when used alone
- Combination of RIT and chemotherapy showed cure at even low doses of RIT where 80% and 100% cure was obtained by combination of chemo and 0.46 and 0.93MBq if 90Y-Apomab, respectively (Figure 39).

### RIT with 90Y-Apomab is specific (Figure 40)

- C57 mice bearing EL4 tumours were left untreated or treated with full dose chemotherapy
- Mice were injected (24h post chemo) with 0.46 or 0.93MBq of 90Y-Apomab or 90Y-Sal5 (control)
- Mono RIT with 90Y-Apomab slightly increased survival of EL4-bearing mice compared to 90Y-Sal5
- Combination of 90Y-Sal5 with full dose chemo was toxic at both 0.46MBq and 0.93MBq doses
- Combination of 90Y-Apomab with full dose chemo showed 80% and 100% cure at 0.46 and 0.93MBq, respectively

### Lewis Lung Carcinoma model

LL2 tumours were grown in C57 mice (9 days) then treated with gemcitabine and cisplatin (**A**) or gemcitabine, cisplatin and TSA (**B**). Tumour volume was measured and the percentage change in tumour volume was calculated. Combination of RIT and chemo±TSA showed synergy in comparison to single agent treatments (Figure 41).

Tumour doubling time (tDt) and time for growth to 50% (TG50) were measured from growth curves of LL2 tumour model treated with 1 cycle therapy (Table 9) or 2 cycle therapy (Table 10).

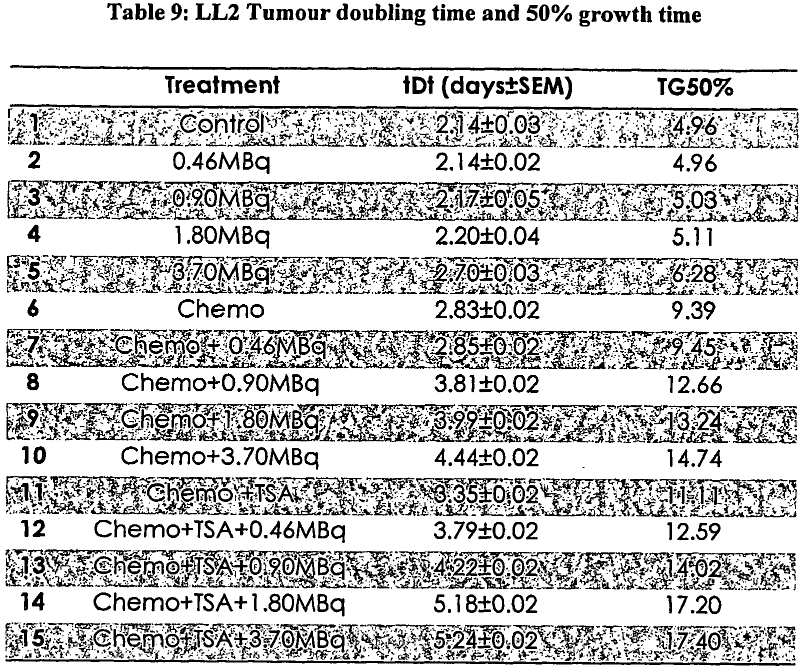

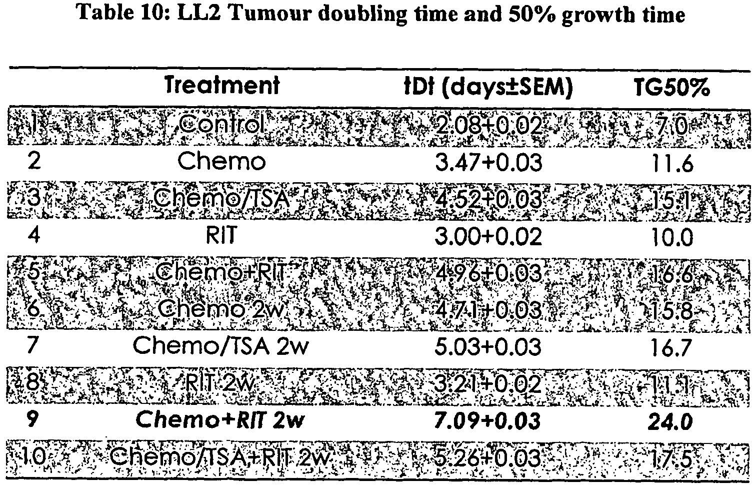

### EXAMPLE 10

### RADIOSENSITISING CHEMOTHERAPY LOWERS THE THRESHOLD FOR EFFICACY OF LA-DIRECTED RIT USING THE LEWIS LUNG CARCINOMA MODEL

### Chemotherapy

50mg/kg gemcitabine and 2.5mg/kg cisplatin on day 0 and 50mg/kg gemcitabine on day 1.

### Radioimmunotherapy (RIT)

⁹⁰Y-DOTA-Apomab was administered at doses of 0.46 Gy, 0.90 Gy, 1.80 Gy, or 3.70 Gy per mouse alone or in combination with chemotherapy.

### Tumour volume measurements

Volume of Lewis Lung Carcinoma-2 (LL2) tumours was calculated from bi-dimensional measurements made using callipers. Change in tumour volume was calculated as the percentage of tumour volume change compared to that on day 0 (dayₜ - day₀) divided by the volume on day 0 (% [dayₜ - day₀]/day₀).

### Cell death measurements

Tumours were excised from control and treated mice after completion of chemotherapy treatment on day 1 (0h), day 2 (24h), day 3 (48h), day 4 (72h), and day 5 (96h). Single cell suspensions were prepared from the excised tumours (n=4 per time point) and analysed for the content of dead cells using fluorocytometric analysis of 7-AAD staining.

### Statistical analyses

Comparisons were made using two-way analysis of variance and a Bonferroni post-test comparison for cell death measurements, and two-way analysis of variance and a Tukey post-test comparison (Prism GraphPad v.4,0) for tumour doubling time estimations.

### Interpretation of results

The doubling times of tumours from untreated control mice and mice given chemotherapy or RIT singly or in combination are tabulated (see Table 11 and Figures 42-45). Chemotherapy alone increased the tumour content of dead cells by a maximum of 10% and, in comparison with control tumours, significantly retarded tumour growth (*P*<0.001). RIT alone had no impact on LL2 growth until at the highest dose of 3.70 Gy per mouse, tumour growth was significantly delayed compared with chemotherapy alone (*P*<0.01). Carcinomas such as LL2 are known to be relatively radioresistant and hence no bystander killing effect of RIT alone was demonstrated. However, the addition of La-directed RIT to chemotherapy significantly delayed tumour growth in a dose-dependent manner at doses of 0.90 Gy and above and thus, in addition, suggested a threshold effect.

In contrast, EL4 tumours were cured after La-directed RIT was administered at doses of 1.80 Gy or 3.70 Gy per mouse, and thus indicated that the bystander killing exerted by radiation crossfire was effective in a lymphoma model because lymphoma is highly radiosensitive. Again, in contrast, the dead cell content of EL4 lymphomas approached 70% 7-AAD⁺ cells after mice were given chemotherapy, and some of the synergistic therapeutic effect achieved by the combining low-dose chemotherapy and low-dose RIT in the treatment of EL4 lymphoma-bearing mice could be attributed to the augmented dead cell content of tumours in mice given chemotherapy.

Therefore, given that chemotherapy produced only a 10% increase in the content of dead cells in LL2 tumours and that RIT alone was ineffective, the addition of RIT to chemotherapy greatly inhibited LL2 tumour growth because the radiosensitising cisplatin and gemcitabine chemotherapy lowered the threshold for induction of cell death by RIT.

**Table 11: LL2 tumour doubling times**

| | **Treatment** | **tDt (days±SEM)** |
|---|---|---|
| **1** | Control | 2.14±0.03 |
| **2** | 0.46MBq | 2.14±0.02 |
| **3** | 0.90MBq | 2.17±0.05 |
| **4** | 1.80MBq | 2.20±0.04 |
| **5** | 3.70MBq | 2.70±0.03 |
| **6** | Chemo | 2.83±0.02 |
| **7** | Chemo + 0.46MBq | 2.85±0.02 |
| **8** | Chemo+0.90MBq | 3.81±0.02 |
| **9** | Chemo+1.80MBq | 3.99±0.02 |
| **10** | Chemo+3.70MBq | 4.44±0.02 |

### EXAMPLE 11

### COMBINATION TREATMENT OF CYTOTOXIC DRUGS AND THE HISTONE DEACETYLASE INHIBITOR (HDACi), TRICHOSTATIN A (TSA) INDUCES CELL DEATH AMONG MALIGNANT CELLS AND PROVIDES GREATER OPPORTUNITY FOR ANTI-LA ANTIBODY (Apomab) BINDING

Further data are presented in support of this hypothesis using combination treatment with cytotoxic drugs and the histone deacetylase inhibitor (HDACi), trichostatin A (TSA), to show that the combination is very effective in inducing cell death among malignant cells and, therefore, provides the opportunity to maximise target creation.

Adding TSA to cisplatin treatment of Jurkat cells *in vitro* produces a TSA dose-dependent increase in Apomab-specific binding to dead Jurkat cells (Fig. 36). As depicted in Figures 36 and 37, it is hypothesised that the observed effect results from TSA augmenting the DNA damage response initiated by cisplatin, which together 'induce' La in dead malignant cells. As illustrated in Figure 38, exposure of Jurkat cells to cisplatin is required at the time that TSA is added to the Jurkat cell cultures for an increase in Apomab-specific binding to be manifest. If increased Apomab binding does occur as the result of enhanced DNA damage, then these data would be consistent with findings in which HDACi increase DNA damage initiated by conventional DNA-damaging agents. DNA damage, which is measured by the phosphorylation of H2A to create γH2AX, was induced by epirubicin in cell lines and potentiated by the HDAC inhibitors, suberoylanilide hydroxamic acid (SAHA) (Marchion DC et al. J Cell Biochem 92,223-237, 2004) and valproic acid (Marchion DC et al. Mol Cancer Ther 4, 1993-2000, 2005). Nonetheless, La is 'induced' in malignant cells after cisplatin treatment with or without the addition of TSA, As Figure 39 shows, the induced La becomes 'fixed' in the dead malignant cells and its detection is clearly shown to be detergent resistant.

Similar synergistic interactions were found between gemcitabine and TSA in cells of the pancreatic cancer cell line, PANC-1, *in vitro.* In these experiments, the TSA doses of 200, 100, 50, 25 and 12.5ng/mL are equivalent to TSA concentrations of 667, 333, 167, 83 and 42nM, respectively. Together with the chosen concentrations of gemcitabine, TSA significantly inhibited proliferation of PANC-1 cells *in vitro* (Piacentini P et al.Virchows Arch 448, 797-804, 2006). Moreover, a moderate dose-dependent effect of TSA was observed on the induction of markers of apoptosis among cultures of PANC-1 cells *in vitro* (Donadelli M et al. Mol Carcinogenesis 38, 59-69, 2003).

Using the MTS proliferation assay, it was observed that TSA alone inhibited proliferation of PANC-1 cells *in vitro.* Addition of gemcitabine completely inhibited PANC-1 proliferation at a TSA concentration of 50nM whereas at a TSA concentration of 200nM, gemcitabine significantly reduced the numbers of PANC-1 cells in the cultures (Fig. 40). As illustrated in the cell death assay (Fig. 41), which was performed in parallel using the same experimental design, it is clear that the reduction in PANC-1 cell numbers at the TSA concentration of 200nM resulted from augmented cell death in the gemcitabine-containing cultures. Moreover, this assay shows that enhancement of Apomab-specific binding to the dead 7-AAD⁺ PANC-1 cells only occurs 48 hours after the induction of cell death and this effect is not observed at the 72 hour time point (Fig. 41). A further such assay was performed at the 48 hour time point and again showed the synergistic effect of combination treatment with TSA and gemcitabine on both cell death and Apomab-specific binding to the dead PANC-1 cells at all concentrations of gemcitabine when the TSA concentrations were either 100ng/mL or 200ng/mL (Fig. 42).

While gemcitabine may not be viewed as a conventional DNA-damaging agent, recent evidence indicates that it produces γH2AX DNA damage foci in a PC-3 tumour xenograft model (McArthur GA et al. J Clin Oncol 24(18S) 3045a, 2006). The synergistic effect of TSA on the induction of cell death among gemcitabine-treated PANC-1 cells together with Apomab-specific binding to the dead cells was evident at the lowest concentration of gemcitabine.

Altogether, it is believed that the data illustrated in Figures 36-42 support the contention that La, as the target antigen for Apomab binding in dead malignant cells, is a prime example of a target that is created by the use of anti-neoplastic treatment. La is 'induced' by the action of anti-neoplastic treatment, particularly DNA-damaging drugs, via an unknown mechanism, and its retention in dead malignant cells as a target antigen is enhanced by 'fixation', which probably results from the activity of apoptosis-induced transglutaminase-2.

### EXAMPLE 12

### METHODS FOR OPTIMIZING RADIOIMMUNOCONJUGATES (RIC) AND THEIR EFFECTS ON ACTIVITY

### Materials and Methods

### Cell culture and antibody production

Suspension cultures of Jurkat leukemia cell line and EL4 murine T-lymphoblastic lymphoma cell line were maintained in RPMI-1640 containing 5% FCS (JRH Biosciences Inc., Lenexa, KS) and passaged by splitting at 1:10 every 72h. The anti-La/SS-B 3B9 mAb hybridoma (Tran et al. 2002, ArthritisRheum. 46(1):202-8) is a murine IgG₂ₐ autoantibody, which is crossreactive with human La and which was prepared by Dr M. Bachmann (Oklahoma Medical Research Foundation, OK), was a kind gift from Dr T.P. Gordon (Department of Immunology, Allergy and Arthritis, Flinders Medical Centre, SA, Australia). The isotype control Sal5 (1D4.5) mAb hybridoma, prepared by Dr L.K. Ashman (Medical Science Building, University of Newcastle, NSW, Australia), was kindly supplied by Dr S. McColl (School of Molecular Biosciences, University of Adelaide, SA, Australia). Hybridoma were cultured in RPMI-1640 containing 5% FCS and the produced antibodies were affinity-purified from culture media using protein G columns. FITC-conjugates were prepared according to the manufacturer instructions (Sigma-Aldrich Co., St. Louis, MO).

### Conjugation of mAb with DOTA-NHS-ESTER

DOTA-NHS-ESTER (Macrocyclics, Dallas, TX) was dissolved in DMSO (Sigma-Aldrich Co., St. Louis, MO) at 25mg/mL. Purified 3B9 and Sal5 in 0.1 M sodium phosphate/0.1 M sodium bicarbonate buffer (pH 8.6) were mixed at 2mg/mL with 50-, 100-, 150- and 200-fold molar excess of DOTA-NHS-ESTER. Control reactions were prepared using equivalent volume of DMSO to replace the NHS-ESTER compound. Reactions were incubated at 23°C for 2 h with constant rotation and were stopped using 1.5M Tris-HCl (pH 8.3) at 10% v/v final concentration. Removal of unconjugated DOTA and buffer exchange of immunoconjugates was achieved by 5x500µL PBS washes in 100kDa cut off microconcentrators as described by manufacturer instructions (Millipore, Billerica, MA). Conjugates were stored at 4°C for further use.

### Determination of DOTA/antibody ratio

Protein concentration was determined using the BCA protein assay kit as described by manufacturer instructions (Pierce Biotechnology Inc., Rockford, IL). Protein concentration was converted from units of mg/mL to µM based on the Mr of IgG (150'000 g/mol). The concentration of DOTA was determined using a modified form of the previously described Arsenazo(III) assay (Pippin et al. 1992, Bioconjug Chem 3(4):342-5; Dadachova et al. 1999, Nucl Med Biol, 26(8):977-82; Brady et al. 2004, Nucl Med Biol, 31(6):795-802). Briefly, assays were performed in 96-wells titre plates to reduce the volume used. Stock solutions of Cu:Arsenazo(III) were prepared using 100µL of 1mg/mL standard Cu atomic absorption solution (Sigma-Aldrich Co., St. Louis, MO), 0.875mg Arsenazo(III) (Sigma-Aldrich Co., St. Louis, MO) and 3mL of metal free 5M ammonium acetate (Sigma-Aldrich Co., St. Louis, MO) in a final 10 mL volume of milliQ water and stored at room temperature (RT) in the dark. Standard concentrations of DOTA were prepared using DOTA-NHS-ESTER dissolved in milliQ water. Aliquots (10)µL) of conjugation reactions and standard DOTA solutions were mixed with 190µL of working dilution (see figure legends) of Cu:Arsenazo(III) solution in milliQ water, incubated at 37°C for 30 min and absorbance was measured at 630nm. The concentration of conjugated DOTA was interpolated from the standard curve constructed for the relationship between DOTA standard solutions and the absorbance of Cu:Arsenazo(III) reagent. DOTA/antibody ratio in prepared conjugates was calculated as the ratio of DOTA concentration (µM) to IgG concentration (µM).

### SDS polyacrylamide gel electrophoresis (SDS-PAGE) and Western blotting

SDS-PAGE was performed as per manufacturer instructions using the Hoefer^{®} Mighty Small II SE 250 electrophoresis system (Amersham Biosciences, Piscataway, NJ) under reducing condition using 12% resolving polyacrylamide gel as per Laemmli (Laemmli U.K., 2005, J. Biol Regul Homeost Agents 19(3-4):105-112). Transfer of polyacrylamide gel onto Hybond-P membrane was carried out as per manufacturer instruction using the TE 22 Mini Tank Transfer Unit (Amersham Biosciences, Piscataway, NJ). After blocking with 5% skim milk, probing was performed using 5µg/mL ¹¹¹In-labelled 3B9 (prepared as described below). Membranes were washed then exposed to x-ray films (3h at RT) which were developed and documented.

### Determination of Rf value in native PAGE

Native polyacrylamide gel electrophoresis (native PAGE) was used to determine the relative fractionation (Rf) value for unmodified and conjugated antibody. Briefly, 7% native gel was prepared using 100µL APS, 20µL TEMED (Sigma-Aldrich Co., St. Louis, MO), 1.75mL of 40% acrylamide (Biorad®) and 1.25mL of 1.5MTris-HCl (pH 8.6) in a final 10mL volume of milliQ water. Native PAGE running buffer was prepared by dissolving 3.4g of glycine and 1.2g of Tris in 500mL of milliQ water (pH adjusted to 8.5). Unmodified and conjugated antibody were mixed with equal volume of native PAGE loading dye (20% v/v glycerol, 1.5M Tris-HCl and 0.05% w/v bromophenol blue in milliQ water) and samples were loaded onto gel. Electrophoresis was performed at 200V for 2.5h and gel was stained using brilliant blue R250 (BBR250) solution (1g of BBR250 [Sigma-Aldrich Co., St. Louis, MO] in 200mL methanol and 100mL H₂O). The Rf values were determined using the gel analysis program, GelPro Analyzer^{™} v3.1 (Media Cybernetics Inc., Silver Spring, MD).

### Flow cytometry

Jurkat cell were fixed and permeabilized by incubation at 1x107 cells/mL in 2% paraformaldehyde for 10min, diluted 1:10 in -20°C methanol (5 min) then washed extensively using PBS. Direct immunofluorescent staining was performed using 5µg/mL of 3B9-FITC or 5µg/mL Sal5-FITC. Indirect immunofluorescent assays were performed using permeabilized cells incubated with 5µg/mL of unmodified 3B9,3B9-DOTA or matching irrelevant isotype (Sal5 or Sal5-DOTA) for 30 min at RT. Cells were washed then incubated in 2µg/mL of goat anti-mouse IgG Alexa₄₈₈-conjugated antibody (Invitrogen, Carlsbad, CA) for 30 min at RT. Cells were washed and samples were acquired using Becton-Dickinson FACScan™ flow cytometry system (BD Biosciences, San Jose, CA). Acquisition was standardized to 10'000 events Flow cytometry data was analyzed using WinMDI v 2.8 (Scripps Research Institute, La Jolla, CA).

Activity of 3B9-DOTA conjugates was tested by their ability to inhibit the binding of 3B9-FITC. Briefly, fixed/permeabilized Jurkat cells were incubated for 30 min at RT with 5µg/mL of 3B9-FITC in the absence or presence of 5, 25, 50 and 100µg/mL of 3B9-DOTA conjugates. Cells were washed and analyzed by flow cytometry as described above.

### Recognition of Fc and Fab regions of DOTA-conjugated IgG

Samples (0.1µg) of unmodified or DOTA-conjugated IgG in native form (PBS) were applied onto nitrocellulose membranes (Amersham Biosciences, Piscataway, NJ). Membranes were dried and blocked with 5% skim milk for 30 min at RT. Membranes were incubated (30 min at RT) with 1µg/mL of goat antibodies conjugated with biotin and directed against the mouse IgG or against the Fc or (Fab)₂ regions of mouse IgG (Rockland, Gilbertsville, PA). Membranes were washed and incubated with 0.1µg/mL streptavidin-alkaline phosphatase (AP) (Rockland, Gilbertsville, PA) for 15 min at RT. Membranes were washed and developed using the BCIP/NBT premixed solution as specified by manufacturer (Sigma-Aldrich Co., St. Louis, MO) and analyzed using GelPro Analyzer™.

### Kinetics of labelling of mAb-DOTA with Terbium

Arsenazo(III) reagent was utilized to study the kinetics of labelling of 3B9-DOTA with Terbium (Tb). TbCl₃ (Sigma-Aldrich Co., St. Louis, MO) was diluted in milliQ water at 20mg/ml. Stock of Arsenazo(III):metal complexes was prepared by mixing 18.75µL of TbCl₃ solution with 1.75mg Arsenazo(III) in 3mL of 5M ammonium acetate in a final 10mL of milliQ water. Stock was diluted 1:10 and 90 µL was added to 17.5µg 3B9-DOTA solutions (10 µL). Absorbance at 630nm was measured kinetically at 37°C every 1 min for 1h.

### Radiolabelling of mAb-DOTA with ¹¹¹In

3B9-DOTA or Sal5-DOTA conjugates were buffer exchanged into 0.2M ammonium acetate (pH 5.5) containing 6mg/mL ascorbic acid by four washes in 100kDa cut off microconcentrators. Indium-111 (¹¹¹InCl3, PerkinElmer Inc., Wellesley, MA) and DOTA-conjugates were incubated for 2 h at 37°C at concentrations of 2mCi/mL (74 MBq/mL) of ¹¹¹In and 2mg/mL of conjugates in (i.e. 1:1 mCi:mg or 37MBq:mg ratio) 0.2M ammonium acetate buffer containing 6mg/mL ascorbic acid. In some cases, ascorbic acid which enhances the incorporation of ¹¹¹In into the conjugates was eliminated from the radiolabelling reaction as interferes with the BCA protein assay kit. Incorporation of ¹¹¹In was measured as described in next section.

For purification of ¹¹¹In-labelled conjugates, radiolabelling reactions were washed twice with 400µL volumes of 0.2M ammonium acetate containing 5mM EDTA (pH 8.0) then 3 times with 400µL volumes of PBS in 100kDa cut off microconcentrators as described earlier. Protein concentration of purified ¹¹¹In-labelled conjugates was measured using the BCA assay kit and the radioactivity concentration was measured using a specified volume placed in gamma counting tubes and counted using Cobra5010 gamma counter (PerkinElmer Inc., Wellesley, MA) which was normalized for ¹¹¹In counting at 100-350 keV window. Specific radioactivity was measured as cpm from gamma counter per µg of protein present in the counted volume.

### Instant thin Layer chromatography

Incorporation efficiency was measured using instant thin layer chromatography (ITLC). Briefly, 2µL aliquots of labelling reactions were mixed with 2µL of 0.2M ammonium acetate containing 5mM EDTA (pH 8.0) and applied at 1 cm from the bottom of 1x9 cm ITLC-SG strips (Pall Corporation, East Hills, NY). Chromatography was performed in 0.2M ammonium acetate containing 5mM EDTA (pH 8.0) as mobile phase and was stopped when solvent front reached 1 cm from the top of the strips. Strips were cut in two halves where the bottom half represented the origin (ori) and the top half represented the solvent front (SF). The activity of the two halves was measured using gamma counter. Incorporation efficiency was calculated as the percentage of activity remaining at the origin to the total activity of the strip [% ori/(ori + SF)].

### In vivo biodistribution of DOTA-conjugates

We have previously described an *in vivo* model of apoptosis which we have shown specific and preferential accumulation of 3B9 in tumors of chemotherapy treated mice (Al-Ejeh *et al.,* 2007). Briefly, EL4 thymic lymphoma cells (1x10⁶ cells) were injected subcutaneously in the right flanks of 6-8 weeks old C57BL/6 female mice. Mice were housed and treated as per protocols approved by the Animal Ethics Committee at The University of Adelaide and the Animal Ethics Committee at the Institute of Medical and Veterinary Sciences (IMVS). Once the tumor reached 1cm diameter, mice were treated by intraperitoneal injections of cytotoxic chemotherapy (38mg/kg etoposide and 50mg/kg cyclophosphamide) given at time 0 and 24h. DOTA-conjugates labelled with ¹¹¹In (100µg) was injected intravenously at time 0. Mice were euthanized at 48h, blood was collected by cardiac puncture, and tumors and organs were collected. Blood and organs were weighed and placed in gamma-counter tubes and radioactivity was measured using gamma counter. Radioactivity in organs was normalized to the weight of the organs and accumulation was calculated as the percentage of radioactivity per gram in the organs to the radioactivity of the injected dose at time 0 (%ID/g).

### Statistical analysis

Statistical comparisons were preformed using GraphPad Prism v.4 (GraphPad Software, San Diego, CA). Two-way analysis of variances (2-way ANOVA) was used to deduce significant differences in the results. The Bonferroni post-test in the 2-way ANOVA function in GraphPad prism was used to report P values. P values less than 0.05 were considered significant where one, two and three asterisks denote P values less than 0.05, 0.01 and 0.001, respectively.

### Results

### Modification of Cu:Arsenazo(III) assay

Previously described assays for the measurement of metal chelators attached to antibodies uses a standard curve for the absorbance (deep blue color) of Cu:Arsenazo(III) solutions of different concentrations. The absorbance of this reagent when mixed with immunoconjugates is then used to interpolate (from standard Cu:Arsenazo(III) curve) the concentration of Cu remaining after chelation which allows calculation of chelated Cu thus.representing the concentration of metal chelator (Pippin *et al.* 1992 *supra*; Dadachova *et al.* 1999 *supra*; Brady *et al.* 2004 *supra*)*.* We found that while Cu:Arsenazo(III) standard curve represented dilutions of the deep blue color of the reagent (Figure 53A), mixtures of the reagent with increasing concentrations of free DOTA (Figure 53B), DTPA or EDTA (data not shown) does not follow the dilution of the reagent itself. This was clearly evident when using high concentration of the metal chelator where chelation of Cu resulted in the disappearance of the blue color and the appearance of the color related to Arsenazo(III) solution (Figure 53C). Therefore, the assay was modified where Cu:Arsenazo(III) reagent mixed with standard solutions of metal chelators at different concentrations was used to construct standard curves for the relationship between absorbance and the concentration of metal chelator (Figure 53C). The absorbance of immunoconjugates mixed with the reagent was directly used to interpolate the concentration of metal chelator from the standard curve.

### DOTA/antibody ratio is manipulated by conjugation condition

Conjugation of 3B9 with DOTA-NHS-ESTER was performed in three separate occasions. The average DOTA/antibody ratios were 2.8±0.3, 8.2±0.3,11.6±1.1 and 15.0±0.7 for the conjugation conditions of 50-, 100-, 150- and 200-fold molar excess of DOTA-NHS-ESTER to 3B9 (Figure 54A). In correlation with increased DOTA/antibody ratio with increasing concentration of DOTA-NHS-ETSER, the Rf value of 3B9 increased in native PAGE with the addition of more DOTA molecules. As shown in Figure 54B, the average Rf value for unmodified 3B9 was 0.23±0.01 whereas the average Rf values were 0.65±0.01, 0.82±0.01, 0.92±0.01 and 0.97±0.01 when it was conjugated at 50-, 100-, 150- and 200-fold molar excess of DOTA-NHS-ESTER. This change in the Rf value with increasing the DOTA/antibody ratio is expected due to the replacement of the positive charge on the primary amine residues on the antibody by three negative charges on the carboxyl-groups of attached DOTA.

### Increased conjugation affects antibody avidity

The avidity of DOTA-conjugated 3B9 was investigated using competition binding assays. As shown in Figure 55, 3B9-DOTA conjugates competed the binding of 33.3nM 3B9-FITC. The IC₅₀ for competition was 64±4,128±8,155±9 and 223±13nM for conjugates prepared at 50-, 100-, 150-and 200-fold molar excess of DOTA-NHS-ESTER. The IC₅₀ was significantly higher for the 100-fold conjugates (*P<0.01*), 150-fold conjugates (*P<0.001*) and 200-fold conjugates (*P<0.001*) compared to that for the 50-fold conjugates. This indicated a significant loss of avidity with increasing the concentration of DOTA-NHS-ESTER in the conjugation reaction. In fact, a linear correlation described the relationship between the IC₅₀ of 3B9-DOTA conjugates and their DOTA/antibody ratios (Figure 55 - inset).

### Modification of Fc and Fab region during conjugation

Detection of 3B9-DOTA conjugates using antibodies against whole IgG molecule or antibodies against the Fc and (Fab)₂ regions decreased significantly compared to that of unmodified 3B9. Samples of 3B9 and 3B9-DOTA conjugates fractioned in non-reducing SDS-PAGE showed comparable amounts of protein in these samples as judged by the intensity of BBR250 staining (Figure 56A). Probing of identical samples of 3B9 and 3B9-DOTA applied onto nitrocellulose using secondary antibodies decreased with increasing concentration of DOTA-NHS-ESTER added to the conjugation reaction (Figure 56B). The optical density of detection using secondary antibodies was normalized to the optical density of BBR250 staining and plotted as a function of the DOTA/antibody ratio in these preparations (Figure 56C). These data indicate modification of the Fab region and to a larger extent the Fc region which were significant enough to affect recognition by secondary antibodies.

Further evidence for the modification of 3B9 during conjugation both in structure and avidity was obtained using indirect immunofluorescent staining of permeabilized Jurkat cells. As shown in Figure 56D, increased DOTA/antibody ratio was associated with a decline in the mean fluorescent intensity (MFI) for target staining in permeabilized Jurkat cells.

### Chelation rate and metal loading capacity is affected by the DOTAlantibody ratio

The absorbance of Terbium:Arsenazo(III) reagent mixed with 3B9-DOTA conjugates decreased during 1h incubation at 37°C (Figure 57A), indicating chelation of terbium as seen in previous Cu:Arsenazo(III) assays. The rate of chelation (min⁻¹) was measured for the one-phase exponential decay model fitted using GraphPad (*r*²=0.90) and showed a linear correlation with the DOTA/antibody ratio (Figure 57A-inset). This indicated that both the rate of chelation and the amount of chelated metal is directly proportional to the amount of DOTA attached to the antibody. Additional evidence for the increased metal loading capacity with increased DOTA/antibody ratio was obtained from the specific radioactivity of ¹¹¹In-labelled 3B9-DOTA (Figure 57B). It is noteworthy that both the heavy and light chains were labelled with ¹¹¹In as judged from the autoradiograph of reducing SDS-PAGE fractionation of ¹¹¹In-DOTA-3B9 samples (Figure 57B-inset), confirming the modification of both chains during conjugation.

### Modulation of antibody properties during conjugation is reflected in vivo

There has been reported the specific and saturable accumulation of 3B9 in EL4 tumors after cytotoxic chemotherapy treatment compared to the irrelevant control of matched isotype, Sal5 (Al-Ejeh *et al.*, 2007). This model was used to investigate the significance of the above described modification of antibody properties after conjugation *in vivo.* Biodistribution of 3B9-DOTA in chemotherapy treated mice was affected when prepared at 200-fold molar excess of DOTA-NHS-ESTER compared to that prepared at 50-fold excess (Figure 58). Accumulation was significantly lower in the tumor (*P<0.001*) and blood (P*<0.05*) and significantly higher (*P<0.001*) in the liver and spleen of mice injected with 3B9-DOTA prepared at 200-fold molar excess of DOTA-NHS-ESTER compared to that prepared using 50-fold excess (Figure 58).

### BIBLIOGRAPHY

Alon, U., Barkai, N., Notterman, D. A., Gish, K., Ybarra, S., Mack, D. and Levine, A. J. (1999). Broad patterns of gene expression revealed by clustering analysis of tumour and normal colon tissues probed by oligonucleotide arrays. Proc Natl Acad Sci USA 96(12): 6745-50.
Arias, E., Anderson, R.N., Kung, H.C., Murphy, S.L. and Kochanek, K.D. (2003). Deaths: final data for 2001. National Vital Statistics Reports 52: 111-115.
Bender, H., Takahashi, H., Adachi, K., Belser, P., Liang, S.H., Prewett, M., Schrappe, M., Sutter, A., Rodeck, U. and Herlyn, D. (1992). Immunotherapy of human glioma xenografts with unlabeled, 131I-, or 125I-labeled monoclonal antibody 425 to epidermal growth factor receptor. Cancer Research 52: 121-126.
Bertram, J.S. (2000). The molecular biology of cancer. Molecular Aspects of Medicine 21: 167-223.
Bhattacharjee, A., Richards, W. G., Staunton, J., Li, C., Monti, S., Vasa, P., Ladd, C., Beheshti, J., Bueno, R., Gillette, M., Loda, M., Weber, G., Mark, E. J., Lander, E. S., Wong, W., Johnson, B. E., Golub, T. R., Sugarbaker, D. J. and Meyerson, M. (2001). Classification of human lung carcinomas by mRNA expression profiling reveals distinct adenocarcinoma subclasses. Proc Natl Acad Sci US A 98(24): 13790-5.
Biggiogera et al., Biologie Cellulaire 96(8): 603-15. 2004
Biggiogera, M., Bottone, M. G., Scovassi, A. I., Soldani, C., Vecchio, L. and Pellicciari, C. (2004). Rearrangement of nuclear ribonucleoprotein (RNP)-containing structures during apoptosis and transcriptional arrest. Biologie Cellulaire 96(8): 603-15.
Bild, A. H., Yao, G., Chang, J. T., Wang, Q., Potti, A., Chasse, D., Joshi, M. B., Harpole, D., Lancaster, J. M., Berchuck, A., Olson, J. A., Jr., Marks, J. R., Dressman, H. K., West, M. and Nevins, J. R. (2006). Oncogenic pathway signatures in human cancers as a guide to targeted therapies. Nature 439(7074): 353-7.
Britz-Cunningham, S.H. and Adelstein, S.J. (2003). Molecular targeting with radionuclides: state of the science. Journal of Nuclear Medicine 44: 1945-1961.
Brown JM, Attardi LD. Nature Rev Cancer 5, 231-237, 2005
Cancer in Australia 2000 (2003). Australian Institute of Health and Welfare (AIHW) and Australasian Association of Cancer Registries (AACR),. AIHW cat. no. CAN 18 Canberra, AIHW, Cancer Series no. 23.
Carpenter, B., MacKay, C., Alnabulsi, A., MacKay, M., Telfer, C., Melvin, W. T. and Murray, G. I. (2006). The roles of heterogeneous nuclear ribonucleoproteins in tumour development and progression. Biochimica et Biophysica Acta 1765(2): 85-100.
Chen, X., Cheung, S. T., So, S., Fan, S. T., Barry, C., Higgins, J., Lai, K. M., Ji, J., Dudoit, S., Ng, I. O., Van De Rijn, M., Botstein, D. and Brown, P. O. (2002). Gene expression patterns in human liver cancers. Mol Biol Cell 13(6): 1929-39.
Christiansen, J. and Rajasekaran, A.K. (2004). Biological impediments to monoclonal antibody-based cancer immunotherapy. Molecular Cancer Therapy 3: 1493-1501.
Dadachova, E., Nosanchuk, J.D., Shi, L., Schweitzer, A.D., Frenkel, A., Nosanchuk, J.S. and Casadevall, A. (2004). Dead cells in melanoma tumours provide abundant antigen for targeted delivery of ionizing radiation by a mAb to melanin. Proceedings of the National Academy of Sciences of the United States of America 101: 14865-14870.
Dairkee, S. H., Ji, Y., Ben, Y., Moore, D. H., Meng, Z. and Jeffrey, S. S. (2004). A molecular 'signature' of primary breast cancer cultures; patterns resembling tumour tissue. BMC Genomics 5(1): 47.
Frierson, H. F., Jr., El-Naggar, A. K., Welsh, J. B., Sapinoso, L. M., Su, A. I., Cheng, J., Saku, T., Moskaluk, C. A. and Hampton, G. M. (2002). Large scale molecular analysis identifies genes with altered expression in salivary adenoid cystic carcinoma. Am J Pathol 161(4): 1315-23.
Giordano, T. J., Thomas, D. G., Kuick, R., Lizyness, M., Misek, D. E., Smith, A. L., Sanders, D., Aljundi, R. T., Gauger, P. G., Thompson, N. W., Taylor, J. M. and Hanash, S. M. (2003). Distinct transcriptional profiles of adrenocortical tumours uncovered by DNA microarray analysis. Am J Pathol 162(2): 521-31.
Gordon, G. J., Rockwell, G. N., Jensen, R. V., Rheinwald, J. G., Glickman, J. N., Aronson, J. P., Pottorf, B. J., Nitz, M. D., Richards, W. G., Sugarbaker, D. J. and Bueno, R. (2005). Identification of novel candidate oncogenes and tumour suppressors in malignant pleural mesothelioma using large-scale transcriptional profiling. Am J Pathol 166(6): 1827-40.
Gorer, P.A. (1950). Studies in antibody response of mice to tumour inoculation. British Journal of Cancer 4: 372-379.
Griffiths, G.L., Govindan, S.V., Sgouros, G., Ong, G.L., Goldenberg, D.M. and Mattes, M.J. (1999). Cytotoxicity with Auger electron-emitting radionuclides delivered by antibodies. International Journal of Cancer 81: 985-992.
Grisendi, S., Mecucci, C., Falini, B. and Pandolfi, P. P. (2006). Nucleophosmin and cancer. Nature Reviews. Cancer 6(7): 493-505.
Hampton, T. (2004). Tool helps cancer scientists mine genes. Jama 292(17): 2073.
Hummel, M., Bentink, S., Berger, H., Klapper, W., Wessendorf, S., Barth, T. F., Bernd, H. W., Cogliatti, S. B., Dierlamm, J., Feller, A. C., Hansmann, M. L., Haralambieva, E., Harder, L., Hasenclever, D., Kuhn, M., Lenze, D., Lichter, P., Martin-Subero, J. I., Moller, P., Muller-Hermelink, H. K., Ott, G., Parwaresch, R. M., Pott, C., Rosenwald, A., Rosolowski, M., Schwaenen, C., Sturzenhofecker, B., Szczepanowski, M., Trautmann, H., Wacker, H. H., Spang, R., Loeffler, M., Trumper, L., Stein, H. and Siebert, R. (2006). A biologic definition of Burkitt's lymphoma from transcriptional and genomic profiling. N Engl JMed 354(23): 2419-30.
Khatua, S., Peterson, K. M., Brown, K. M., Lawlor, C., Santi, M. R., LaFleur, B., Dressman, D., Stephan, D. A. and MacDonald, T. J. (2003). Overexpression of the EGFFt/FKBP12/HHIF-2alpha pathway identified in childhood astrocytomas by angiogenesis gene profiling. Cancer Res 63(8): 1865-70.
Laemmli, U.K. (1970). Cleavage of structural proteins during the assembly of the head of bacteriophage T4. Nature 227: 680-685.
Liang, Y., Diehn, M., Watson, N., Bollen, A. W., Aldape, K. D., Nicholas, M. K., Lamborn, K. R., Berger, M. S., Botstein, D., Brown, P. O. and Israel, M. A. (2005). Gene expression profiling reveals molecularly and clinically distinct subtypes of glioblastoma multiforme. Proc Natl Acad Sci USA 102(16): 5814-9.
Liu, S. and Edwards, D.S. (2001). Bifunctional chelators for therapeutic lanthanide radiopharmaceuticals. Bioconjugate Chemistry 12: 7-34.
Maggi, L. B., Jr. and Weber, J. D. (2005). Nucleolar adaptation in human cancer. Cancer Investigation 23(7): 599-608.
Milas L et al. Cancer Res 59, 107-114, 1999
Notterman, D. A., Alon, U., Sierk, A. J. and Levine, A. J. (2001). Transcriptional gene expression profiles of colorectal adenoma, adenocarcinoma, and normal tissue examined by oligonucleotide arrays. Cancer Res 61(7): 3124-30.
O'Donoghue, J.A., Bardies, M. and Wheldon, T.E. (1995). Relationships between tumour size and curability for uniformly targeted therapy with beta-emitting radionuclides. Journal of Nuclear Medicine 36: 1902-1909.
Phillips, H. S., Kharbanda, S., Chen, R., Forrest, W. F., Soriano, R. H., Wu, T. D., Misra, A., Nigro, J. M., Colman, H., Soroceanu, L., Williams, P. M., Modrusan, Z., Feuerstein, B. G. and Aldape, K. (2006). Molecular subclasses of high-grade glioma predict prognosis, delineate a pattern of disease progression, and resemble stages in neurogenesis. Cancer Cell 9(3): 157-73.
Rhodes, D. R. and Chinnaiyan, A. M. (2005). Integrative analysis of the cancer transcriptome. Nat Genet 37 Suppl: S31-7.
Rhodes, D. R., Kalyana-Sundaram, S., Mahavisno, V., Barrette, T. R., Ghosh, D. and Chinnaiyan, A. M. (2005). Mining for regulatory programs in the cancer transcriptome. Nat Genet 37(6): 579-83.
Rhodes, D. R., Yu, J., Shanker, K., Deshpande, N., Varambally, R., Ghosh, D., Barrette, T., Pandey, A. and Chinnaiyan, A. M. (2004a). Large-scale meta-analysis of cancer microarray data identifies common transcriptional profiles of neoplastic transformation and progression. Proc Natl Acad Sci USA 101(25): 9309-14.
Rhodes, D. R., Yu, J., Shanker, K., Deshpande, N., Varambally, R., Ghosh, D., Barrette, T., Pandey, A. and Chinnaiyan, A. M. (2004b). ONCOMINE: a cancer microarray database and integrated data-mining platform. Neoplasia 6(1): 1-6.
Rickman, D. S., Bobek, M. P., Misek, D. E., Kuick, R., Blaivas, M., Kurnit, D. M., Taylor, J. and Hanash, S. M. (2001). Distinctive molecular profiles of high-grade and low-grade gliomas based on oligonucleotide microarray analysis. Cancer Res 61(18): 6885-91.
Schaner, M. E., Ross, D. T., Ciaravino, G., Sorlie, T., Troyanskaya, O., Diehn, M., Wang, Y. C., Duran, G. E., Sikic, T. L., Caldeira, S., Skomedal, H., Tu, I. P., Hernandez-Boussard, T., Johnson, S. W., O'Dwyer, P. J., Fero, M. J., Kristensen, G. B., Borresen-Dale, A. L., Hastie, T., Tibshirani, R., van de Rijn, M., Teng, N. N., Longacre, T. A., Botstein, D., Brown, P. O. and Sikic, B. I. (2003). Gene expression patterns in ovarian carcinomas. Mol Biol Cell 14(11): 4376-86.
Sellers, W.R. and Fisher, D.E. (1999). Apoptosis and cancer drug targeting. Journal of Clinical Investigation 104: 1655-1661.
Shai, R., Shi, T., Kremen, T. J., Horvath, S., Liau, L. M., Cloughesy, T. F., Mischel, P. S. and Nelson, S. F. (2003). Gene expression profiling identifies molecular subtypes of gliomas. Oncogene 22(31): 4918-23.
Singh, D., Febbo, P. G., Ross, K., Jackson, D. G., Manola, J., Ladd, C., Tamayo, P., Renshaw, A. A., D'Amico, A. V., Richie, J. P., Lander, E. S., Loda, M., Kantoff, P. W., Golub, T. R. and Sellers, W. R. (2002). Gene expression correlates of clinical prostate cancer behavior. Cancer Cell 1(2): 203-9.
Symon Z et al. Int J Radiat Oncol Biol Physics 53, 140-145, 2002
Vanaja, D. K., Ballman, K. V., Morlan, B. W., Cheville, J. C., Neumann, R. M., Lieber, M. M., Tindall, D. J. and Young, C. Y. (2006). PDLIM4 repression by hypermethylation as a potential biomarker for prostate cancer. Clin Cancer Res 12(4): 1128-36.
Waldmann, T.A. (1991). Monoclonal antibodies in diagnosis and therapy. Science 252: 1657-1662.
Wang, Y., Klijn, J. G., Zhang, Y., Sieuwerts, A. M., Look, M. P., Yang, F., Talantov, D., Timmermans, M., Meijer-van Gelder, M. E., Yu, J., Jatkoe, T., Berns, E. M., Atkins, D. and Foekens, J. A. (2005). Gene-expression profiles to predict distant metastasis of lymph-node-negative primary breast cancer. Lancet 365(9460): 671-9.
Xue, L.Y., Butler, N.J., Makrigiorgos, G.M., Adelstein, S.J. and Kassis, A.I. (2002). Bystander effect produced by radiolabeled tumour cells in vivo. Proceedings of the National Academy of Sciences of the United States of America 99: 13765-13770.

## Claims

1. One or more DNA damaging agents and an immunointeractive molecule directed to La, which immunointeractive molecule is covalently or non-covalently linked to an anti-neoplastic agent which down-regulates the growth of neoplastic cells, for use in the treatment of a neoplasm or a neoplastic condition in a subject, wherein:
(i) said DNA damaging agent is for initial administration to said subject to induce the damage of at least a subpopulation of neoplastic cells, wherein said cell damage is **characterised by** the exposure of La to the extracellular environment and an increase in the level of La in said cell; and
(ii) said anti-neoplastic agent is for subsequent administration to said subject to induce the downregulation of the growth of viable bystander neoplastic cells,
wherein said DNA damaging agent is selected from the group consisting of a chemotherapy agent, a cytotoxic agent, a radioisotope, an inhibitor of poly-(ADP ribosyl) transferase, an antineoplastic agent, a pro-apoptotic agent, a CHK1/2 inhibitor, a histone deactylase inhibitor, a tumour necrosis factor-related apoptosis inducing ligand, a BH3 mimetic and a DNA dependent protein kinase inhibitor and wherein said immunointeractive molecule is an antibody or a fragment thereof or a T-cell associated antigen-binding molecule preferably wherein said subject is a human.

2. Use of:
(i) one or more DNA damaging agents; and
(ii) an immunointeractive molecule directed to La, which immunointeractive molecule is covalently or non-covalently linked to an anti-neoplastic agent which down-regulates neoplastic cell growth,
in the manufacture of a medicament for the treatment of a neoplasm or a neoplastic condition in a subject wherein:
(i) said DNA damaging agent is for initial administration to said subject to induce the damage of at least a subpopulation of neoplastic cells, wherein said cell damage is **characterised by** the exposure of La to the extracellular environment and an increase in the level of La in said cell; and
(ii) said anti-neoplastic agent linked to said immunointeractive molecule is for subsequent administration to said subject to induce the downregulation of growth of viable bystander neoplastic cells,
wherein said DNA damaging agent is selected from the group consisting of a chemotherapy agent, a cytotoxic agent, a radioisotope, an inhibitor of poly-(ADP ribosyl) transferase, an antineoplastic agent, a pro-apoptotic agent, a CHK1/2 inhibitor, a histone deactylase inhibitor, a tumour necrosis factor-related apoptosis inducing ligand, a BH3 mimetic and a DNA dependent protein kinase inhibitor and wherein said immunointeractive molecule is an antibody or a fragment thereof or a T-cell associated antigen-binding molecule preferably wherein said subject is a human.

3. The agents and immunointeractive molecule for use according to claim 1 or the use according to claim 2 wherein said neoplastic cell or neoplasm is malignant, preferably wherein said neoplastic cell or neoplasm is metastatic.

4. The agents and immunointeractive molecule for use according to claim 1 or 3 or the use according to claim 2 wherein said neoplasm is a central nervous system tumour, retinoblastoma, neuroblastoma, a paediatric tumour, a head and neck cancer such as squamous cell cancer, breast or prostate cancer, lung cancer, kidney cancer such as renal cell adenocarcinoma, oesophagogastric cancer, hepatocellular carcinoma, pancreaticobiliary neoplasia such as adenocarcinoma and an islet cell tumour, colorectal cancer, cervical cancer, anal cancer, uterine or other reproductive tract cancer, urinary tract cancer such as of the ureter or bladder, a germ cell tumour such as a testicular germ cell tumour or an ovarian germ cell tumour, ovarian cancer such as ovarian epithelial cancer, carcinoma of unknown primary human immunodeficiency associated malignancy such as Kaposi's sarcoma, lymphoma, leukemia, malignant melanoma, sarcoma, an endocrine tumour such as of the thyroid gland, a mesothelioma or other pleural or peritoneal tumour, a neuroendocrine tumour or a carcinoid tumour, preferably wherein said neoplasm is a solid tumour.

5. The agents and immunointeractive molecule for use according to any one of claims 1 or 3 to 4 or the use according to claim 2 wherein said cell damage is cellular and nuclear membrane damage, preferably wherein said cell damage is the induction of cell death.

6. The agents and immunointeractive molecule for use according to claim 5 or the use according to claim 5 wherein said cell damage is induced by a DNA damaging agent selected from Actinomycin D, Arsenic Trioxide, Bleomycin, Busulfan, Carboplatin, Carmustine, Chlorambucil, Cisplatin, Cyclophosphamide, Daunorubicin, Doxorubicin, Epirubicin, Etoposide, Hydroxyurea, Idarubicin, Ifosfamide, Irinotecan, Lomustine, Melphalan, Mitomycin, Mitoxantrone, Oxaliplatin, Procarbizine, Streptozocin, Thiotepa, Topotecan, Treosulfan.

7. The agents and immunointeractive molecule for use according to claim 5 or the use according to claim 5 wherein said DNA damaging agent is selected from:
(i) a radioisotope
(ii) gemcitabine together with a CHK1/2 inhibitor
(iii) irinotecan together with a CHK1/2 inhibit
(iv) a histone deacetylase inhibitor
(v) tumour necrosis factor related apoptosis-inducing ligand (TRAIL)
(vi) a cytotoxic agent
(vii) a BH3 mimetic
preferably wherein said CHK1/2 inhibitor is CBP-501 or AZD7762, or wherein said histone deacetylase inhibitor is vorinostat or wherein said BH3 mimetic is ABT737, or wherein said TRAIL is administered together with a cytotoxic agent.

8. The agents and immunointeractive molecule for use according to any one of claims 1 or 3 to 7 or the use according to any one of claims 2 to 7, wherein said antibody is a monoclonal antibody or a fragment thereof.

9. The agents and immunointeractive molecule for use according to any one of claims 1 or 3 to 7 or the use according to any one of claims 2 to 7, wherein said antibody is a polyclonal antibody or a fragment thereof.

10. The agents and immunointeractive molecule for use according to any one of claims 1 or 3 to 9 or the use according to any one of claims 2 to 9, wherein said antibody or fragment thereof is selected from a single chain antibody, a deimmunised antibody, an Fv, Fab, Fab', or F(ab')₂ fragment, a single chain Fv fragment, a disulphide-stabilised Fv fragment, or a single variable region domain (dAb), or wherein said T-cell associated antigen-binding molecule is a TABM.

11. The agents and immunointeractive molecule for use according to claim 10 or the use according to claim 10, wherein the deimmunised antibody is a humanized antibody.

12. The agents and immunointeractive molecule for use according to any one of claims 1 or 3 to 7 or the use according to any one of claims 2 to 7, wherein said anti-neoplastic agent kills tumour cells.

13. The agents and immunointeractive molecule for use according to any one of claims 1 or 3 to 7 or use according to any one of claims 2 to 7, wherein said anti-neoplastic agent is a toxic molecule.

14. The agents and immunointeractive molecule for use according to claim 13 or the use according to claim 13 wherein said immunointeractive molecule is linked to an anti-neoplastic agent selected from:
- a cytokine
- a chemokine
- an immune response modulator such as N-formyl-methionyl-lencylphenylalanine or JBT 2002
- a toxin, preferably wherein said toxin is a radioisotope or ricin, colicheamicin, a prodrug such as ADEPT, a catalytic antibody or maytansinoid.

15. The agents and immunointeractive molecule for use according to claim 14 or the use according to claim 14 wherein said radioisotope is a 3 particle emitter, preferably wherein said β particle emitter is ¹¹¹Indium, ¹³¹Iodine, ⁹⁰Yttrium, ¹⁸⁸Rhenium, ⁶⁷ Copper, ¹⁷⁷Lutelium or a particle emitter preferably wherein said α particle emitter is ²¹³Bi or ¹⁴⁹Tb.

16. The agents and immunointeractive molecule for use according to any one of claims 1 or 3 to 15 or use according to any one of claims 2 to 15 wherein said La is localised to the cytoplasm, preferably wherein said immunointeractive molecule becomes fixed.

17. A kit comprising a DNA damaging agent and an immunointeractive molecule directed to La, which immunointeractive molecule is covalently or non-covalently linked to an anti-neoplastic agent which down-regulates neoplastic cell growth wherein
(i) said DNA damaging agent is for initial administration to a subject to induce the damage of at least a subpopulation of neoplastic cells and said cell damage is **characterised by** the exposure of La to the extracellular environment; and
(ii) said anti-neoplastic agent is for subsequent administration to said subject to downregulate the growth of neoplastic cells
wherein said DNA damaging agent is selected from the group consisting of a chemotherapy agent, a cytotoxic agent, a radioisotope, an inhibitor of poly-(ADP ribosyl) transferase, an antineoplastic agent, a pro-apoptotic agent, a CHK1/2 inhibitor, a histone deactylase inhibitor, a tumour necrosis factor-related apoptosis inducing ligand, a BH3 mimetic and a DNA dependent protein kinase inhibitor and wherein said immunointeractive molecule is an antibody or a fragment thereof or a T-cell associated antigen-binding molecule.

## Patentansprüche

1. Ein oder mehrere DNS-schädigende Wirkstoffe und ein immuno-interaktives Molekül gerichtet auf La, dieses immuno-interaktive Molekül ist kovalent oder nicht-kovalent verbunden mit einem anti-neoplastischen Wirkstoff, der das Wachstum neoplastischer Zellen herunterregelt, zur Verwendung zur Behandlung eines Neoplasmas oder einer neoplastischen Kondition bei einer Versuchsperson, wobei:
(i) der genannte DNS-schädigende Wirkstoff vorgesehen ist zur Erstverabreichung an die genannte Versuchsperson, um zumindest eine Subpopulation neoplasticher Zellen zu schädigen, wobei diese Zellschädigung **dadurch gekennzeichnet** ist, das La einer extrazellulären Umgebung und einer Steigerung des La-Niveaus in der genannten Zelle ausgesetzt ist; und
(ii) der genannte anti-neoplastische Wirkstoff vorgesehen ist zur darauf folgenden Verabreichung an diese Versuchsperson, zur Anregung der Herunterreglung des Wachstums von lebensfähigen neoplastischen Zellen in der Umgebung,
wobei der genannte DNS-schädigende Wirkstoff aus einer Gruppe ausgewählt wird, die besteht aus einem chemotherapeutischen Wirkstoff, einem zytotoxischen Wirkstoff, einem Radioisotop, einem Poly-(ADP ribosyl)-Transferase-Inhibitor, einem anti-neoplastischen Wirkstoff, einem pro-apoptotischen Wirkstoff, einem CHK1/2-Inhibitor, einem Histon-Deacetylase-Inhibitor, einem TRAIL, einem BH3-Mimetikum und einem DNS-abhängigen Proteinkinase-Inhibitor und wobei das genannte immuno-interaktive Molekül ein Antikörper oder ein Antikörperfragment oder ein T-Zellen assoziiertes, Antigen-bindendes Molekül ist, wobei die genannte Versuchsperson vorzugsweise ein Mensch ist

2. Verwendung von:
(i) einem oder mehreren DNS-schädigenden Wirkstoffen; und
(ii) einem immuno-interaktives Molekül, auf La gerichtet, dieses immuno-interaktive Molekül ist kovalent oder nicht-kovalent verbunden mit einem anti-neoplastischen Wirkstoff, der das neoplastische Zellwachstum herunterregelt,
bei der Herstellung eines Medikamentes zur Behandlung eines Neoplasmas oder einer neoplastischen Kondition bei einer Versuchsperson, wobei:
(i) der genannte DNS-schädigende Wirkstoff vorgesehen ist zur Erstverabreichung an die genannte Versuchsperson, um zumindest eine Subpopulation neoplastischer Zellen zu schädigen, wobei diese Zellschädigung **dadurch gekennzeichnet** ist, das La einer extrazellulären Umgebung und einer Steigerung des La-Niveaus in der genannten Zelle ausgesetzt ist; und
(ii) der genannte anti-neoplastische Wirkstoff, verbunden mit diesem immuno-interaktiven Molekül, vorgesehen ist zur darauf folgenden Verabreichung an diese Versuchsperson, zur Anregung der Herunterreglung des Wachstums von lebensfähigen neoplastischen Zellen in der Umgebung,
wobei der genannte DNS-schädigende Wirkstoff aus einer Gruppe ausgewählt wird, die besteht aus einem chemotherapeutischen Wirkstoff, einem zytotoxischen Wirkstoff, einem Radioisotop, einem Poly-(ADP ribosyl) transferase-Inhibitor, einem anti-neoplastischen Wirkstoff, einem pro-apoptotischen Wirkstoff, einem CHK1/2-Inhibitor, einem Histon-Deacetylase-Inhibitor, einem TRAIL, einem BH3-Mimetikum und einem DNS-abhängigen Proteinkinase-Inhibitor und wobei das genannte immuno-interaktive Molekül ein Antikörper oder ein Antikörperfragment oder ein T-Zellen assoziiertes, Antigen-bindendes Molekül ist, wobei die genannte Versuchsperson vorzugsweise ein Mensch ist.

3. Die Wirkstoffe und immuno-interaktiven Moleküle zur Verwendung gemäß Anspruch 1 oder die Verwendung gemäß Anspruch 2, wobei die genannte neoplastische Zelle bösartig ist, vorzugsweise wenn die genannte neoplastische Zelle metastatisch ist.

4. Die Wirkstoffe und immuno-interaktiven Moleküle zu Verwendung gemäß Anspruch 1 oder 3 oder die Verwendung gemäß Anspruch 2, wobei dieses Neoplasma ein Tumor des zentralen Nervensystems, ein Retinoblastom, ein Neuroblastom, ein pädiatrischer Tumor, ein Kopf- und Halskarzinome, ein Plattenepithelkarzinom , Brust- oder Prostatakreps, Lungenkrebs, Nierenkrebs, wie Adenokarzinom der Niere, ösophagogastrischer Krebs, ein hepatozelluläres Karzinom, eine pankreato-biliäre Neoplasie wie Adenokarzinom und ein Inselzelltumor, kolorektale Karzinome, Gebärmutterhalskrebs, ein Analkarzinom, Gebärmutterkrebs oder andere Karzinome der Fortpflanzungsorgane, Krebserkrankung der Harnwege, wie Harnröhrenkrebs oder Blasenkrebs, ein Keimzelltumor wie Hodentumor oder Eierstocktumor, Eierstockkrebs, wie Epithelkrebs, ein Karzinom aufgrund unbekannter humaner primärer Immundefizienz-assoziierter Bösartigkeit, wie Kaposi-Sarkom, Lymphom, Leukämie, malignes Melanom, Sareom, eine endokriner Tumor, zum Beispiel der Schilddrüse, ein Mesotheliom oder ein anderer pleuraler oder peritonealer Tumor, ein Neuroendokrin-Tumor oder ein Karzinoid, vorzugsweise wenn das genannte Neoplasma ein solider Tumor ist.

5. Die Wirkstoffe und das immuno - interaktive Molekül zur Verwendung gemäß irgendeinem der Ansprüche 1 oder 3 bis 4 oder die Verwendung gemäß Anspruch 2, wobei es sich bei der genannten Zellschädigung um Zell- und Kernmembranschädigung handelt, wobei es sich bei dieser Zellschädigung vorzugsweise um eine Induktion des Zelltodes handelt.

6. Die Wirkstoffe und das immuno-interaktive Molekül zur Verwendung gemäß Anspruch 5 oder die Verwendung gemäß Anspruch 5, wobei die genannte Zellschädigung induziert wird durch einen DNS-schädigenden Wirkstoff, ausgewählt aus Actinomycin D, Arsentrioxid, Bleomycin, Busulfan, Carboplatin, Carmustin, Chlorambucil, Cisplatin, Cyclophosphamid, Daunorubicin, Doxorubicin, Epirubicin, Etoposid, Hydroxyurea, Idarubicin, Ifosfamid, Irinotecan, Lomustin, Melphalan, Mitomycin, Mitoxantron, Oxaliplatin, Procarbizin, Streptozocin, Thiotepa, Topotecan, Treosulfan.

7. Die Wirkstoffe und das immuno-interaktive Molekül zur Verwendung gemäß Anspruch 5 oder die Verwendung gemäß Anspruch 5, wobei dieser DNS-schädigende Wirkstoff ausgewählt wird unter:
(i) einem Radioisotop
(ii) Gemcitabin zusammen mit einem CHK 1/2-Inhibitor
(iii) Irinotecan zusammen mit einem CHK 1/2-Inhibitor
(iv) einem Histon-Deacetylase-Inhibitor
(v) einem Tumornekrosefaktor-verwandten Apoptose-induzierenden Liganden (TRAIL)
(vi) einem zytotoxischen Wirkstoff
(vii) einem BH3-Mimetikum
wobei es sich bei diesem CHK 1/2-Inhibitor vorzugsweise um CBP-501 oder AZD7762 handelt, oder wobei es sich bei dem genannten Histon-Deacetylase-Inhibitor um Vorinostat handelt, oder wobei das genannte BH3-Mimetikum ABT137 ist, oder wobei dieser TRAIL zusammen mit einem zytotoxischen Wirkstoff verabreicht wird.

8. Die Wirkstoffe und das immuno-interaktive Molekül zur Verwendung gemäß irgendeinem der Ansprüche 1 oder 3 bis 7 oder die Verwendung gemäß irgendeinem der Ansprüche 2 bis 7, wobei es sich bei dem genannten Antikörper um einen monoklonalen Antikörper oder ein Antikörperfragment handelt.

9. Die Wirkstoffe und das immuno-interaktive Molekül zur Verwendung gemäß irgendeinem der Ansprüche 1 oder 3 bis 7 oder die Verwendung gemäß irgendeinem der Ansprüche 2 bis 7, wobei es sich bei dem genannten Antikörper um einen polyklonalen Antikörper oder ein Antikörperfragment handelt.

10. Die Wirkstoffe und das immuno-interaktive Molekül zur Verwendung gemäß irgendeinem der Ansprüche 1 oder 3 bis 9 oder die Verwendung gemäß irgendeinem der Ansprüche 2 bis 9, wobei der genannte Antikörper oder das Antikörperfragment ausgewählt aus einem einkettigen Antikörper, einem deimmunisierten Antikörper, einem Fv, Fab, Fab', or F(ab')₂ Fragment, einem einkettigen Fv-Fragment, einem disulfidstabilisierten Fv-Fragment, oder einem Einzeldomänen-Antikörper (dAb), oder wobei es sich bei dem T-Zellen assoziierten, Antigen-bindenden Molekül um ein TABM handelt.

11. Die Wirkstoffe und das immuno-interaktive Molekül zur Verwendung gemäß Anspruch 10 oder die Verwendung gemäß Anspruch 10, wobei dieser DNS-schädigende Wirkstoff ein humanisierter Antikörper ist.

12. Die Wirkstoffe und das immuno-interaktive Molekül zur Verwendung gemäß irgendeinem der Ansprüche 1 oder 3 bis 7 oder die Verwendung gemäß irgendeinem der Ansprüche 2 bis 7, wobei der genannte anti- neoplastische Wirkstoff Tumorzellen abtötet.

13. Die Wirkstoffe und das immuno-interaktive Molekül zur Verwendung gemäß irgendeinem der Ansprüche 1 oder 3 bis 7 oder die Verwendung gemäß irgendeinem der Ansprüche 2 bis 7, wobei der genannte anti- neoplastische Wirkstoff ein toxisches Molekül ist.

14. Die Wirkstoffe und das immuno-interaktive Molekül zur Verwendung gemäß Anspruch 13 oder die Verwendung gemäß Anspruch 13, wobei dieser DNS-schädigende Wirkstoff verknüpft ist mit einem anti- neoplastischen Wirkstoff, ausgewählter unter:
- ein Zytokin
- ein Chemokin
- ein Immunabwehrmodulator wie N-formyl-methionyllencylphenylalanin oder JBT 2002
- ein Toxin, wobei vorzugsweise dieses Toxin ein Radioisotop oder Ricin, Colicheamicin, ein Prodrug wie ADEPT, ein katalytischer Antikörper oder Maytansinoid.

15. Die Wirkstoffe und das immuno-interaktive Molekül zur Verwendung gemäß Anspruch 14 oder die Verwendung gemäß Anspruch 14, wobei das genannte Radioisotop ein 3 Partikelemitter ist, wobei vorzugsweise dieser β-Partikelemitter ¹¹¹Indium, ¹³¹Iodine, ⁹⁰Yttrium, ¹⁸⁸Rhenium, ⁶⁷Copper, ¹⁷⁷Lutelium oder ein Partikelemitter ist, wobei vorzugsweise der genannte α Partikelemitter ²¹³Bi oder ¹⁴⁹Tb ist.

16. Die Wirkstoffe und das immuno-interaktive Molekül zur Verwendung gemäß irgendeinem der Ansprüche 1 oder 3 bis 15 oder die Verwendung gemäß irgendeinem der Ansprüche 2 bis 15, wobei das genannte La zum Zytoplasma lokalisiert ist, wobei vorzugsweise das genannte immuno-interaktive Molekül fixiert wird.

17. Ein Set bestehend aus einem DNS-schädigenden Wirkstoff und einem immuno-interaktiven Molekül gerichtet auf La, dieses immuno-interaktive Molekül ist kovalent oder nicht-kovalent verbunden mit einem anti-neoplastischen Wirkstoff, der das Wachstum neoplastischer Zellen herunterregelt, wobei
(i) der genannte DNS-schädigende Wirkstoff vorgesehen ist zur Erstverabreichung an eine Versuchsperson, um zumindest eine Subpopulation neoplasticher Zellen zu schädigen, und diese Zellschädigung **dadurch gekennzeichnet** ist, das La einer extrazellulären Umgebung ausgesetzt ist; und
(ii) der genannte anti-neoplastische Wirkstoff vorgesehen ist zur darauf folgenden Verabreichung an diese Versuchsperson, zur Herunterreglung des Wachstums von neoplastischen Zellen, wobei der genannte DNS-schädigende Wirkstoff aus einer Gruppe ausgewählt wird, die besteht aus einem chemotherapeutischen Wirkstoff, einem zytotoxischen Wirkstoff, einem Radioisotop, einem Poly-(ADP ribosyl) transferase-Inhibitor, einem anti-neoplastischen Wirkstoff, einem pro-apoptotischen Wirkstoff, einem CHK1/2-Inhibitor, einem Histon-Deacetylase-Inhibitor, einem TRAIL, einem BH3-Mimetikum und einem DNS-abhängigen Proteinkinase-Inhibitor und wobei das genannte immuno-interaktive Molekül ein Antikörper oder ein Antikörperfragment oder ein T-Zellen assoziiertes, Antigen-bindendes Molekül ist.

## Revendications

1. Un ou plusieurs agents endommageant l'ADN et molécule immuno-interactive dirigée contre La, laquelle molécule immuno-interactive est liée de façon covalente ou non covalente à un agent antinéoplasique qui régule négativement la croissance de cellules néoplasiques, destinés à être utilisés dans le traitement d'un néoplasme ou d'un état néoplasique chez un sujet, où :
(i) ledit agent endommageant l'ADN est destiné à une administration initiale audit sujet afin d'induire un dommage à au moins une sous-population de cellules néoplasiques, où ledit dommage cellulaire est **caractérisé par** l'exposition de La à l'environnement extracellulaire et une augmentation du taux de La dans ladite cellule ; et
(ii) ledit agent antinéoplasique est destiné à une administration ultérieure audit sujet afin d'induire la régulation négative de la croissance de cellules néoplasiques de voisinage viables,
où ledit agent endommageant l'ADN est sélectionné dans le groupe consistant en un agent de chimiothérapie, un agent cytotoxique, un radio-isotope, un inhibiteur de la poly-(ADP ribosyl)transférase, un agent antinéoplasique, un agent pro-apoptotique, un inhibiteur de la CHK1/2, un inhibiteur d'histone désacétylase, un ligand induisant une apoptose apparenté au facteur de nécrose tumorale, un mimétique de BH3 et un inhibiteur de protéines kinases ADN-dépendant, et où ladite molécule immuno-interactive est un anticorps ou un fragment de celui-ci ou une molécule de liaison à un antigène associée aux cellules T, de préférence où ledit sujet est un humain.

2. Utilisation de :
(i) un ou plusieurs agents endommageant l'ADN ; et
(ii) une molécule immuno-interactive dirigée contre La, laquelle molécule immuno-interactive est liée de façon covalente ou non covalente à un agent antinéoplasique qui régule négativement la croissance de cellules néoplasiques,
dans la fabrication d'un médicament destiné au traitement d'un néoplasme ou d'un état néoplasique chez un sujet, dans laquelle :
(i) ledit agent endommageant l'ADN est destiné à une administration initiale audit sujet afin d'induire un dommage à au moins une sous-population de cellules néoplasiques, où ledit dommage cellulaire est **caractérisé par** l'exposition de La à l'environnement extracellulaire et une augmentation du taux de La dans ladite cellule ; et
(ii) ledit agent antinéoplasique lié à ladite molécule immuno-interactive est destiné à une administration ultérieure audit sujet afin d'induire la régulation négative de la croissance de cellules néoplasiques de voisinage viables,
où ledit agent endommageant l'ADN est sélectionné dans le groupe consistant en un agent de chimiothérapie, un agent cytotoxique, un radio-isotope, un inhibiteur de la poly-(ADP ribosyl)transférase, un agent antinéoplasique, un agent pro-apoptotique, un inhibiteur de la CHK1/2, un inhibiteur d'histone désacétylase, un ligand induisant une apoptose apparenté au facteur de nécrose tumorale, un mimétique de BH3 et un inhibiteur de protéines kinases ADN-dépendant, et où ladite molécule immuno-interactive est un anticorps ou un fragment de celui-ci ou une molécule de liaison à un antigène associée aux cellules T, de préférence où ledit sujet est un humain.

3. Agents et molécule immuno-interactive destinés à être utilisés selon la revendication 1 ou utilisation selon la revendication 2, où ladite cellule néoplasique ou ledit néoplasme est maligne/malin, de préférence où ladite cellule néoplasique ou ledit néoplasme est métastatique.

4. Agents et molécule immuno-interactive destinés à être utilisés selon la revendication 1 ou 3 ou utilisation selon la revendication 2, où ledit néoplasme est une tumeur du système nerveux central, un rétinoblastome, un neuroblastome, une tumeur pédiatrique, un cancer de la tête et du cou comme un cancer à cellules squameuses, le cancer du sein ou de la prostate, le cancer du poumon, le cancer du rein comme un adénocarcinome à cellules rénales, un cancer oesophagogastrique, un carcinome hépatocellulaire, une néoplasie pancréatico-biliaire comme un adénocarcinome et une tumeur des îlots de Langerhans, le cancer colorectal, le cancer du col de l'utérus, le cancer de l'anus, le cancer de l'utérus ou un autre cancer de l'appareil reproducteur, un cancer des voies urinaires tel que de l'uretère ou de la vessie, une tumeur des cellules germinales comme une tumeur des cellules germinales testiculaires ou une tumeur des cellules germinales ovariennes, le cancer de l'ovaire comme un cancer épithélial de l'ovaire, un carcinome de malignité primaire inconnue associée à l'immunodéficience humaine comme le sarcome de Kaposi, un lymphome, une leucémie, un mélanome malin, un sarcome, une tumeur endocrine telle que de la thyroïde, un mésothéliome ou une autre tumeur pleurale ou péritonéale, une tumeur neuroendocrine ou une tumeur carcinoïde, de préférence où ledit néoplasme est une tumeur solide.

5. Agents et molécule immuno-interactive destinés à être utilisés selon l'une quelconque des revendications 1 ou 3 à 4 ou utilisation selon la revendication 2, où ledit dommage cellulaire est un dommage de la membrane cellulaire et nucléaire, de préférence où ledit dommage cellulaire est l'induction de la mort cellulaire.

6. Agents et molécule immuno-interactive destinés à être utilisés selon la revendication 5 ou utilisation selon la revendication 5, où ledit dommage cellulaire est induit par un agent endommageant l'ADN sélectionné parmi l'actinomycine D, le trioxyde d'arsenic, la bléomycine, le busulfan, le carboplatine, la carmustine, le chlorambucil, le cisplatine, le cyclophosphamide, la daunorubicine, la doxorubicine, l'épirubicine, l'étoposide, l'hydroxyurée, l'idarubicine, l'ifosfamide, l'irinotécan, la lomustine, le melphalan, la mitomycine, la mitoxantrone, l'oxaliplatine, la procarbazine, la streptozocine, le thiotépa, le topotécan, le tréosulfan.

7. Agents et molécule immuno-interactive destinés à être utilisés selon la revendication 5 ou utilisation selon la revendication 5, où ledit agent endommageant l'ADN est sélectionné parmi :
(i) un radio-isotope
(ii) la gemcitabine avec un inhibiteur de la CHK1/2
(iii) l'irinotécan avec un inhibiteur de la CHK1/2
(iv) un inhibiteur d'histone désacétylase
(v) un ligand induisant une apoptose apparenté au facteur de nécrose tumorale (TRAIL)
(vi) un agent cytotoxique
(vii) un mimétique de BH3
de préférence dans lesquels ledit inhibiteur de la CHK1/2 est le CBP-501 ou l'AZD7762, ou dans lesquels ledit inhibiteur d'histone désacétylase est le vorinostat ou dans lesquels ledit mimétique de BH3 est l'ABT737, ou dans lesquels ledit TRAIL est administré avec un agent cytotoxique.

8. Agents et molécule immuno-interactive destinés à être utilisés selon l'une quelconque des revendications 1 ou 3 à 7 ou utilisation selon l'une quelconque des revendications 2 à 7, où ledit anticorps est un anticorps monoclonal ou un fragment de celui-ci.

9. Agents et molécule immuno-interactive destinés à être utilisés selon l'une quelconque des revendications 1 ou 3 à 7 ou utilisation selon l'une quelconque des revendications 2 à 7, où ledit anticorps est un anticorps polyclonal ou un fragment de celui-ci.

10. Agents et molécule immuno-interactive destinés à être utilisés selon l'une quelconque des revendications 1 ou 3 à 9 ou utilisation selon l'une quelconque des revendications 2 à 9, dans lesquels ledit anticorps ou fragment de celui-ci est sélectionné parmi un anticorps simple chaîne, un anticorps désimmunisé, un fragment Fv, Fab, Fab', ou F(ab')₂, un fragment Fv simple chaîne, un fragment Fv stabilisé par une liaison disulfure, ou un anticorps à un seul domaine de région variable (dAb), ou dans lesquels ladite molécule de liaison à un antigène associée aux cellules T est une TABM.

11. Agents et molécule immuno-interactive destinés à être utilisés selon la revendication 10 ou utilisation selon la revendication 10, où l'anticorps désimmunisé est un anticorps humanisé.

12. Agents et molécule immuno-interactive destinés à être utilisés selon l'une quelconque des revendications 1 ou 3 à 7 ou utilisation selon l'une quelconque des revendications 2 à 7, où ledit agent antinéoplasique tue des cellules tumorales.

13. Agents et molécule immuno-interactive destinés à être utilisés selon l'une quelconque des revendications 1 ou 3 à 7 ou utilisation selon l'une quelconque des revendications 2 à 7, où ledit agent antinéoplasique est une molécule toxique.

14. Agents et molécule immuno-interactive destinés à être utilisés selon la revendication 13 ou utilisation selon la revendication 13, où ladite molécule immuno-interactive est liée à un agent antinéoplasique sélectionné parmi :
- une cytokine
- une chimiokine
- un modulateur de la réponse immunitaire comme la N-formyl-méthionyl-leucylphénylalanine ou le JBT 2002
- une toxine, de préférence où ladite toxine est un radio-isotope ou la ricine, la calichéamicine, un promédicament comme une ADEPT, un anticorps catalytique ou un maytansinoïde.

15. Agents et molécule immuno-interactive destinés à être utilisés selon la revendication 14 ou utilisation selon la revendication 14, où ledit radio-isotope est un émetteur de particules β, de préférence où ledit émetteur de particules β est l' indium¹¹¹, l' iode¹³¹, l'yttrium⁹⁰, le rhénium¹⁸⁸, le cuivre⁶⁷, le lutétium¹⁷⁷, ou un émetteur de particules α, de préférence où ledit émetteur de particules α est le Bi²¹³ ou le Tb¹⁴⁹.

16. Agents et molécule immuno-interactive destinés à être utilisés selon l'une quelconque des revendications 1 ou 3 à 15 ou utilisation selon l'une quelconque des revendications 2 à 15, où ladite La est localisée dans le cytoplasme, de préférence où ladite molécule immuno-interactive devient fixée.

17. Kit comprenant un agent endommageant l'ADN et une molécule immuno-interactive dirigée contre La, laquelle molécule immuno-interactive est liée de façon covalente ou non covalente à un agent antinéoplasique qui régule négativement la croissance de cellules néoplasiques, où
(i) ledit agent endommageant l'ADN est destiné à une administration initiale à un sujet afin d'induire un dommage à au moins une sous-population de cellules néoplasiques et ledit dommage cellulaire est **caractérisé par** l'exposition de La à l'environnement extracellulaire ; et
(ii) ledit agent antinéoplasique est destiné à une administration ultérieure audit sujet afin de négativement réguler la croissance de cellules néoplasiques
où ledit agent endommageant l'ADN est sélectionné dans le groupe consistant en un agent de chimiothérapie, un agent cytotoxique, un radio-isotope, un inhibiteur de la poly-(ADP ribosyl)transférase, un agent antinéoplasique, un agent pro-apoptotique, un inhibiteur de la CHK1/2, un inhibiteur d'histone désacétylase, un ligand induisant une apoptose apparenté au facteur de nécrose tumorale, un mimétique de BH3 et un inhibiteur de protéines kinases ADN-dépendant, et où ladite molécule immuno-interactive est un anticorps ou un fragment de celui-ci ou une molécule de liaison à un antigène associée aux cellules T.
